# EUROPEAN PATENT SPECIFICATION

(11) **EP 3 464 598 B1**
(45) Date of publication and mention of the grant of the patent: **06.09.2023**
(21) Application number: 17807660.0
(22) Date of filing: 05.06.2017
(51) Int. Cl.: C12N 15/115, C12Q 1/68, G01N 33/553, A61K 31/7088, A61K 38/28, A61K 48/00, A61P 7/02

(54) **APTAMER-BASED ANALYTE ASSAYS**
APTAMER-BASIERTE ANALYTENTESTS
DOSAGES D'ANALYTES BASÉS SUR LES APTAMÈRES

(30) Priority: 03.06.2016 US 201662345697 P; 03.06.2016 US 201662345641 P; 06.06.2016 US 201662346374 P
(43) Date of publication of application: 10.04.2019
(73) Proprietor: The Trustees of Columbia University in the City of New York, New York, NY 10027 (US)
(72) Inventor: STOJANOVIC, Milan, Ridgewood, NJ 07450 (US); YANG, Kyungae, New York, NY 10032 (US); TAYLOR, Steven, Jersey City, NJ 07307 (US); MILOSAVIC, Nenad, Hackensack, NJ 07601 (US)
(74) Representative: Kilburn & Strode LLP
(86) International application number: PCT/US2017/035958
(87) International publication number: WO 2017/210683

(56) References cited:
- EP-A1- 1 992 705
- WO-A2-2005/051174
- US-A1- 2015 064 696
- YANG KYUNG-AE ET AL: "In vitroselection and amplification protocols for isolation of aptameric sensors for small molecules", METHODS, ACADEMIC PRESS, NL, vol. 106, 4 May 2016 (2016-05-04), pages 58-65, XP029677551, ISSN: 1046-2023, DOI: 10.1016/J.YMETH.2016.04.032
- MANJULA RAJENDRAN ET AL: "Selection of fluorescent aptamer beacons that light up in the presence of zinc", ANALYTICAL AND BIOANALYTICAL CHEMISTRY, SPRINGER, BERLIN, DE, vol. 390, no. 4, 30 November 2007 (2007-11-30), pages 1067-1075, XP019584825, ISSN: 1618-2650
- NUTIU R ET AL: "In vitro Selection of Structure-Switching Signaling Aptamers", ANGEWANDTE CHEMIE, INTERNATIONAL EDITION, WILEY-VCH, DE, vol. 144, no. 7, 4 February 2005 (2005-02-04), pages 1061-1065, XP003019996, ISSN: 1433-7851, DOI: 10.1002/ANIE.200461848
- DENG, B ET AL.: 'Aptamer binding assays for proteins: The thrombin example - A review' ANALYTICA CHIMICA ACTA vol. 837, 21 July 2014, pages 1 - 15, XP028862975

## Description

### PRIORITY CLAIM

This patent claims priority to United States Provisional Applications Nos. 62/345,641 and 62/345,697, both filed June 3, 2016, and United States Provisional Application No. 62/346,374 filed June 6, 2016.

### GRANT INFORMATION

This invention was made with Government Support under Grant No. RGM104960 awarded by the National Institutes of Health and Grant No. CCF1518715 awarded by the National Science Foundation. The Government has certain rights in the invention.

### 1. INTRODUCTION

The present disclosure relates to aptamer-based assays to capture and/or detect analytes, including small molecules that offer limited mutually non-competitive epitopes to antibodies and are therefore difficult to detect or measure using traditional antibody sandwich-type assays.

### 2. BACKGROUND OF THE INVENTION

Non-competitive sandwich assays employ two different binding elements to capture and then detect an analyte ¹⁻⁵. For example, a typical example of a sandwich assay format is an enzyme linked immunosorbent assay (ELISA) on plates, wherein one antibody (capture antibody), directed toward a target analyte (e.g., a protein), is bound to a solid support (e.g., a plate), the analyte is bound, and then a second antibody (the "detection antibody") bearing a detectable moiety is introduced that binds to a different site on the captured analyte. Because alternative binding sites are typically present on the analyte, in the presence of excess reagent, these assays can be more sensitive than competitive assays, detect ligand with increasing signal, and have lower noise. The sandwich principle also allows more stringent washing protocols that minimizes nonspecific interactions. The principle of two or more binding elements interacting with a target analyte (i.e., "sandwiching") is also used in other assays beyond ELISA, such as lateral flow assays or latex-bead agglutination assays.

The sandwich approach becomes problematic with small analytes that can be too small to bind to two antibodies at once (e.g., steroids or catecholamines or even some small peptides such as vasopressin) or that are non-immunogenic (e.g., molecules such as phenylalanine or glucose). When antibodies against small molecules were available, several idiosyncratic approaches ⁶⁻⁹ have produced ELISA-like assays for small molecules.

Aptamers are oligonucleotide-based receptors that can bind to small molecules ⁹⁻¹⁵. Aptamers have been used before in traditional sandwich assays, however, traditional sandwich assays depend upon availability of more than one binding site in the analyte. Therefore, there is a need for means for detecting small molecules that overcome the problems due to the lack of multiple epitopes. Yang et al. (2016) Methods Vol. 106, pages 58-65 describes in vitro selection and amplification protocols for isolation of aptameric sensors for small molecules. Manjula et al. (20078) Analytical and Bioanalytical Chemistry Vol. 390, pages 1067-107 describes selection of fluorescent aptamer beacons that light up in the presence of zinc. Nutiu et al. (2005) Vol. 144, pages 1061-1065 describes in vitro selection of Structure-Switching signaling aptamers. EP1992705 A1 relates to detecting interactions using aptamers.

### 3. SUMMARY OF THE INVENTION

Aspects of the invention for which protection is sought are as defined in the appended set of claims. The description may contain additional technical information, which although not part of the claimed invention, is provided to place the invention in a broader technical context and to illustrate possible related technical developments.

In a first aspect of the invention there is provided an assay for testing a sample for the presence and/or amount of an analyte of interest comprising contacting at least a portion of the sample with effective amounts of (1) a primary aptamer comprising a core sequence that binds to the analyte and (2) an anti-aptamer which is complementary to at least a portion of the primary aptamer, wherein the primary aptamer and/or anti-aptamer comprise a detectable moiety(ies) which detect whether the primary aptamer and anti-aptamer are bound to each other or unbound; and wherein a primary aptamer bound to the analyte does not bind to its anti-aptamer, and wherein the primary aptamer comprises a fluorescent label and the anti-aptamer comprises a quencher moiety; wherein the analyte, primary aptamer and anti-aptamer are selected from the group consisting of: (a) the analyte is deoxycorticosterone 21-glucoside (DOG) and either (i) the primary aptamer comprises SEQ ID NO: 62 and the anti-aptamer comprises SEQ ID NO:69, or (ii) the primary aptamer comprises SEQ ID NO: 76 and the anti-aptamer comprises SEQ ID NO:77; (b) the analyte is aldosterone (ALDS) and either (i) the primary aptamer comprises SEQ ID NO: 63 and the anti-aptamer comprises the complement of SEQ ID NO: 63, or (ii) the primary aptamer comprises SEQ ID NO: 79 and the anti-aptamer comprises SEQ ID NO:80; (c) the analyte is cortisol (CS) and the primary aptamer comprises SEQ ID NO: 64 and the anti-aptamer comprises the complement of SEQ ID NO: 64; (d) the analyte is testosterone (TES) and the primary aptamer comprises SEQ ID NO: 65 and the anti-aptamer comprises the complement of SEQ ID NO: 65; (e) the analyte is Phe-CpRh and the primary aptamer comprises SEQ ID NO: 66 and the anti-aptamer comprises the complement of SEQ ID NO: 66; and (f) the analyte is phenylalanine (Phe) and the primary aptamer comprises SEQ ID NO: 67 and the anti-aptamer comprises the complement of SEQ ID NO: 67.

In a second aspect of the invention there is provided a method of detecting or measuring the presence or amount of an analyte of interest in a sample, comprising (i) contacting at least a portion of a sample with effective amounts of a primary aptamer and an anti-aptamer that is complementary to at least a portion of the primary aptamer, said primary aptamer and/or anti-aptamer comprising a moiety which allows the amount of primary aptamer bound to analyte to be detected and/or measured, under conditions that would permit duplex formation between the primary aptamer and anti-aptamer if target analyte were not present; and (ii) detecting the amount of primary aptamer that is not bound to anti-aptamer, and wherein the primary aptamer comprises a fluorescent label and the anti-aptamer comprises a quencher moiety ; wherein the analyte, primary aptamer and anti-aptamer are selected from the group consisting of: (a) the analyte is deoxycorticosterone 21-glucoside (DOG) and either i. the primary aptamer comprises SEQ ID NO: 62 and the anti-aptamer comprises SEQ ID NO:69, or ii. the primary aptamer comprises SEQ ID NO: 76 and the anti-aptamer comprises SEQ ID NO: 77; (b) the analyte is aldosterone (ALDS) and either iii. the primary aptamer comprises SEQ ID NO: 63 and the anti-aptamer comprises the complement of SEQ ID NO: 63, or iv. the primary aptamer comprises SEQ ID NO: 79 and the anti-aptamer comprises SEQ ID NO:80; (c) the analyte is cortisol (CS) and the primary aptamer comprises SEQ ID NO: 64 and the anti-aptamer comprises the complement of SEQ ID NO: 64; (d) the analyte is testosterone (TES) and the primary aptamer comprises SEQ ID NO: 65 and the anti-aptamer comprises the complement of SEQ ID NO: 65; (e) the analyte is Phe-CpRh and the primary aptamer comprises SEQ ID NO: 66 and the anti-aptamer comprises the complement of SEQ ID NO: 66; and (f) the analyte is phenylalanine (Phe) and the primary aptamer comprises SEQ ID NO: 67 and the anti-aptamer comprises the complement of SEQ ID NO: 67.

The method may further comprise (i) contacting at least a portion of the sample with effective amounts of (a) a primary aptamer comprising a fluorescent label and (b) an anti-aptamer, comprising a moiety that quenches fluorescence of said fluorescent label if primary aptamer and anti-aptamer are bound together in a duplex, under conditions that would permit duplex formation between primary aptamer and anti-aptamer to occur if analyte were not present; and (ii) detecting the amount of fluorescence. Wherein the primary aptamer comprises a core sequence that binds to the analyte and a portion complementary to a sensor oligonucleotide, the method may further comprise contacting at least a portion of the sample with an effective amounts of a sensor oligonucleotide one or more of which is bound to a detectable moity(ies) which can detect whether the primary aptamer and sensor oligonucleotide are bound to each other or whether primary aptamer is bound to analyte. Wherein the primary aptamer comprises a core sequence that binds to the analyte and a portion that, when primary aptamer is bound to analyte, binds to a secondary "sandwich" aptamer, and the method may further comprise contacting at least a portion of the sample with an effective amounts of a secondary "sandwich" aptamer; one or more of which is bound to a detectable moity(ies) which can detect whether the primary aptamer and secondary "sandwich" aptamer are bound to each other or unbound.The present disclosure relates to aptamer-based assays to capture and detect analytes that may not lend themselves to antibody-based assays. Three different exemplary assays are provided, as well as a variety of aptamers comprising a core sequence and an operative sequence, where the operative sequence can be varied depending upon the assay to be used.

A first set of instances provide for an "anti-aptamer assay" in which a sample to be tested for the presence and/or amount of an analyte of interest is contacted with effective amounts of (1) a primary aptamer comprising a core sequence that binds to the analyte and (2) an "anti-aptamer" which is complementary to at least a portion of the primary aptamer, wherein the primary aptamer and/or anti-aptamer comprise a detectable moiety(ies) which detect whether the primary aptamer and anti-aptamer are bound to each other or unbound; and wherein a primary aptamer bound to the analyte does not bind to an anti-aptamer . The analyte competes with anti-aptamer for binding to primary aptamer, so that the amount of bound (or unbound) anti-aptamer correlates with the amount of analyte present.

A second set of instances provide for a "pseudo-sandwich assay" in which a sample to be tested for the presence and/or amount of an analyte of interest is contacted with effective amounts of (1) a primary aptamer comprising a core sequence that binds to the analyte as well as at least a portion that is complementary to a structure -switching "sensor oligonucleotide"; (2) a sensor oligonucleotide, optionally bound to a solid support, which optionally further comprises a portion complementary to a "comp" (for "complementary") oligonucleotide; wherein the primary aptamer and/or sensor oligonucleotide and/or comp oligonucleotide comprise a detectable moiety(ies) which can detect whether the primary aptamer and sensor oligonucleotide are bound to each other or unbound; and wherein the primary aptamer and sensor oligonucleotide form a partially double stranded helix that detectably dissociates, or does not form, when the primary aptamer is bound to analyte. For example, a test sample may be added to an effective amount of complexes comprised of primary aptamer and sensor oligonucleotide and comp oligonucleotide; the amount of primary aptamer released from the complexes correlates with the amount of analyte in the test sample (and the comp and sensor oligonucleotides form an at least partially double-stranded structure). As a further example, and not by way of limitation, the complexes may be linked to a solid suppport, the test sample applied, and then the primary aptamer released may be detected. As a more specific non-limiting example, the sensor oligonucleotide may be linked to a solid support and complexed to primary aptamer, the resulting complexes may then be contacted with test sample, and the amount of released primary aptamer detected, for example, washed off the support.

A third set of instances provides for a "sandwich assay" in which a sample to be tested for the presence and/or amount of an analyte of interest is contacted with effective amounts of (1) a primary aptamer comprising a core sequence that binds to the analyte as well as at least a portion that binds to a secondary aptamer when it is bound to analyte; and (ii) a sandwich aptamer (also referred to herein as a "secondary aptamer") which binds to primary aptamer provided that the primary aptamer is bound to analyte to form a ternary complex (the "sandwich"); wherein the primary aptamer and/or sandwich aptamer comprise a detectable moiety(ies) which can detect whether the primary aptamer and sandwich oligonucleotide are bound to each other or unbound. For example, a test sample may be added to effective amounts of primary aptamer and sandwich aptamer, and then the amounts of primary aptamer/sandwich aptamer complexes, or the amount of unbound primary aptamer or sandwich aptamer, may be detected.

Also provided herein are consensus core sequences, core sequences, and primary aptamers that can bind to primary aptamers of interest, including glucose, hydrocortisone, phenylalanine, dehydroisoandrosterone, deoxycortisone, testosterone, aldosterone, dopamine, sphingosine-1-phosphate, serotonin, melatonin, tyrosine, tobramycin, amikacin, methylene blue, ammonium ion, boronic acid, epinephrine, creatinine and vasopressin.

Further provided are associated anti-aptamer, sensor, comp and sandwich aptamers/oligonucleoti des.

Further instanceprovide for kits comprising the aforementioned primary aptamers and/or oligonucleotides.

### 4. BRIEF DESCRIPTION OF THE FIGURES

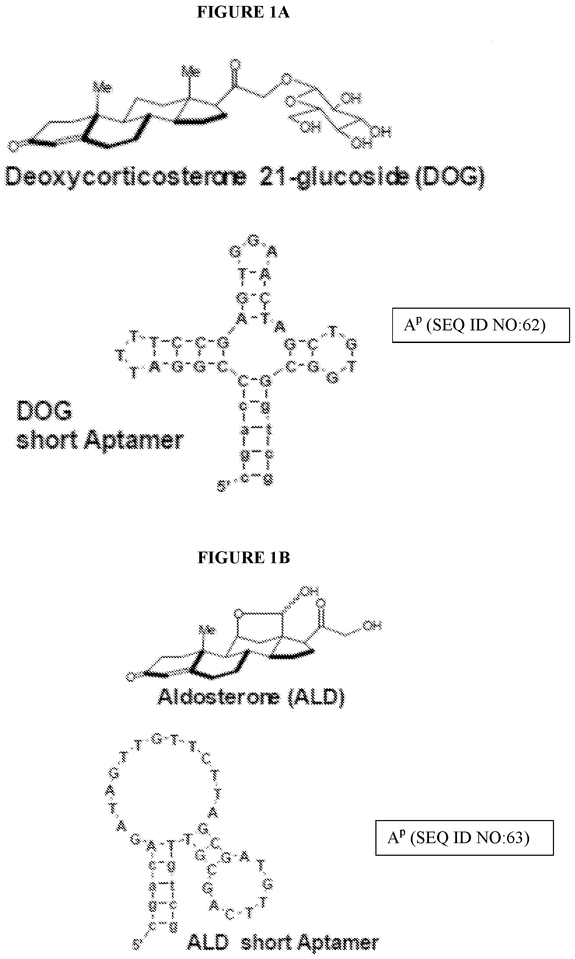

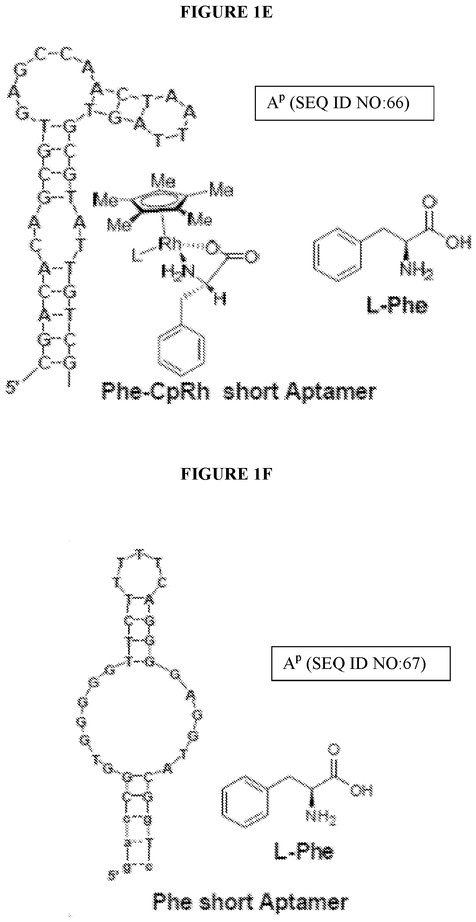

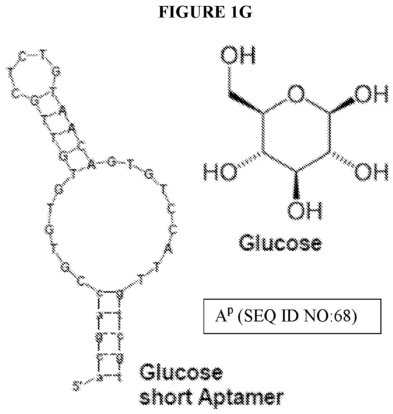

FIGURE 1A-G. Structures of primary aptamers (short forms) and corresponding target analytes. (A) Primary aptamer (SEQ ID NO:62, a subsequence of SEQ ID NO: 12) and its target analyte, deoxycorticosterone 21-glucoside ("DOG"); (B) Primary aptamer (SEQ ID NO:63) and its target analyte, aldosterone ("ALDS"); (C) Primary aptamer (SEQ ID NO:64, a subsequence of SEQ ID NO:5) and its target analyte, cortison ("CS"); (D) Primary aptamer (SEQ ID NO:65, a subsequence of SEQ ID NO: 13) and its target analyte, testosterone ("TES"); (E) Primary aptamer (SEQ ID NO:66) and its target analyte, Phe-CpRh; (F) Primary aptamer (SEQ ID NO:67) and its target analyte, L-Phenylalanine; (G) Primary aptamer (SEQ ID NO:68, a subsequence of SEQ ID NO: 1) and its target analyte, glucose.

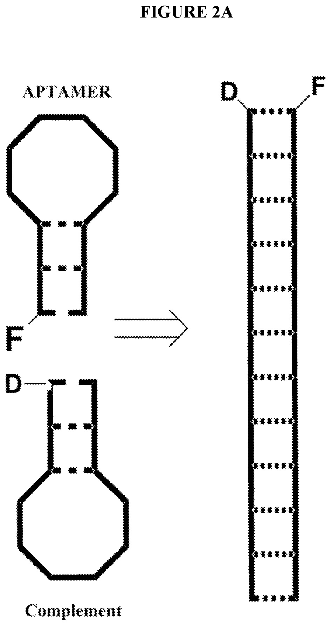

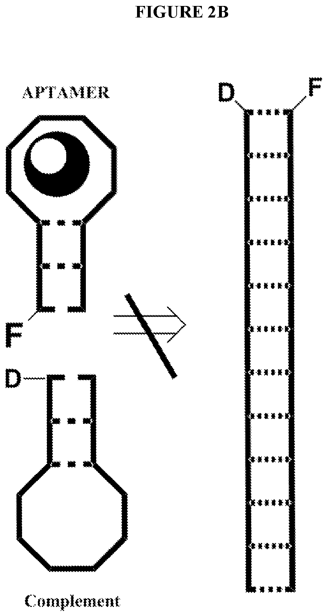

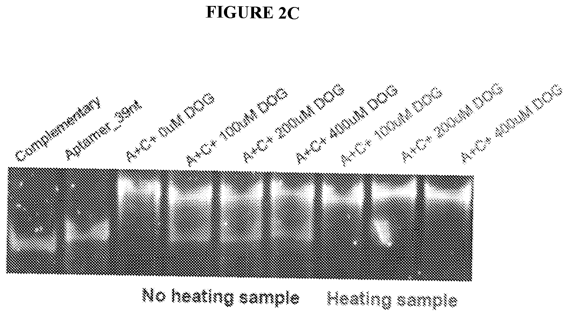

FIGURE 2A-C. Design principle of "anti-aptamer assay." (A) Effective amounts of primary aptamer ("aptamer") labeled with fluorescent label (e.g. F = fluorescein) and its "complement" oligonucleotide (a.k.a. "anti-aptamer") labeled with quencher (e.g., D = dabcyl quencher) can hybridize to form a duplex. Fluorescence of the label on the aptamer is quenched upon duplex formation. (B) In the presence of target analyte, primary aptamer binds to analyte instead of its complement anti-aptamer, and the fluoresence of its label is not quenched. (C) Shows increasing fluorescence with increasing concentrations of target analyte DOG in unheated samples but not heated samples (because heating interferes with analyte/aptamer binding).

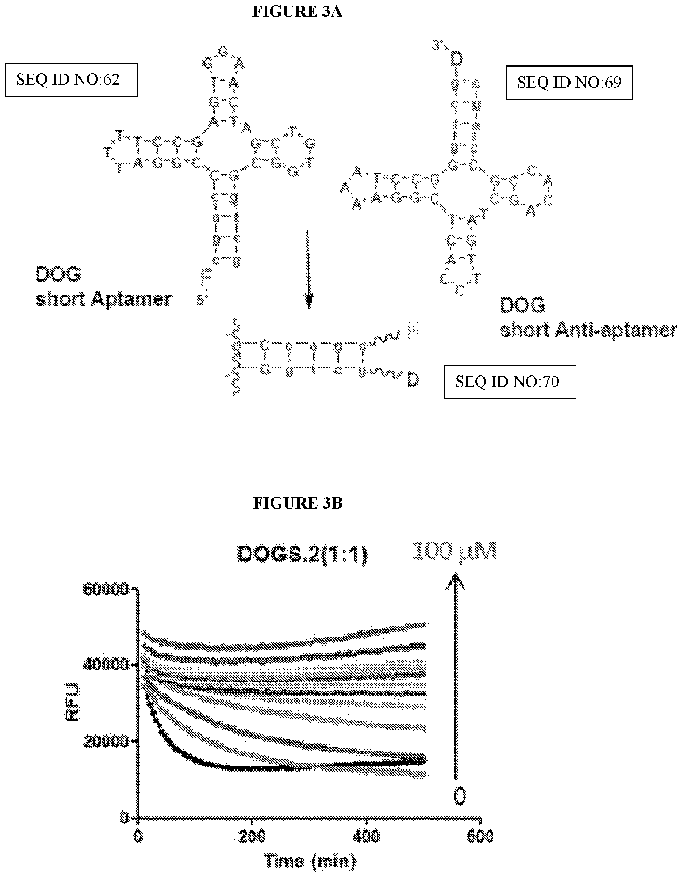

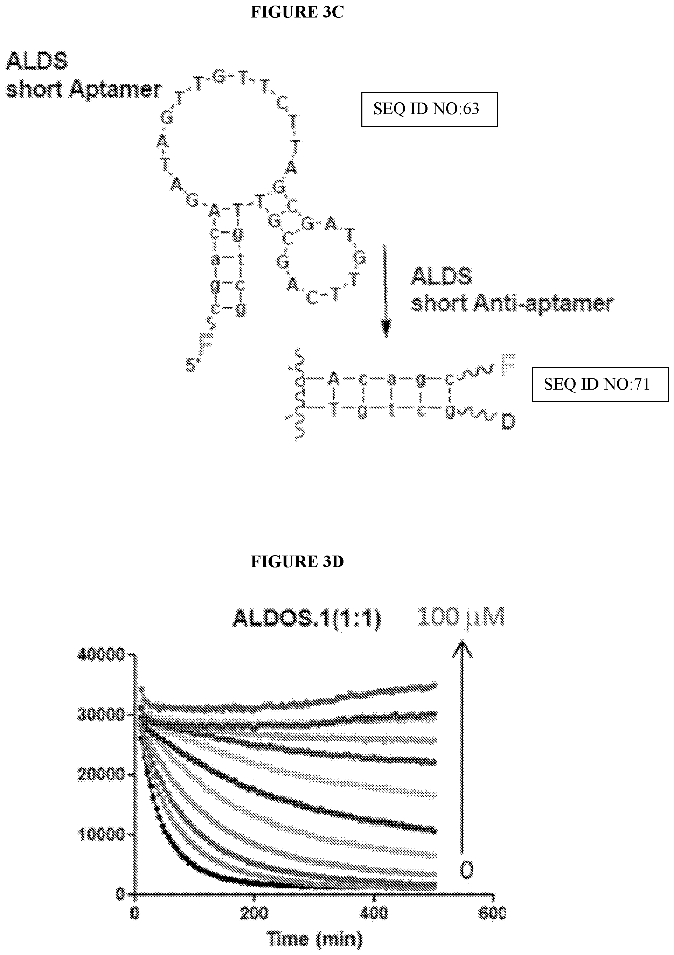

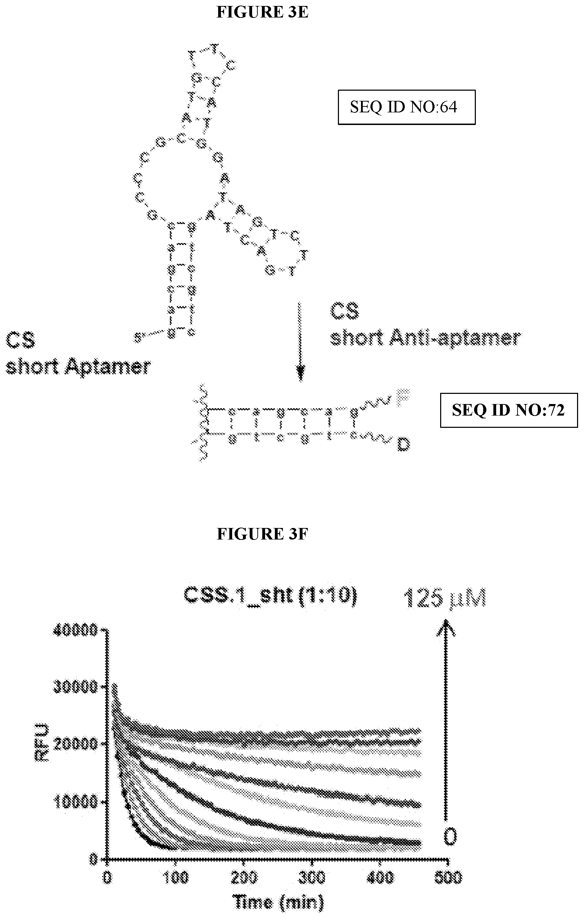

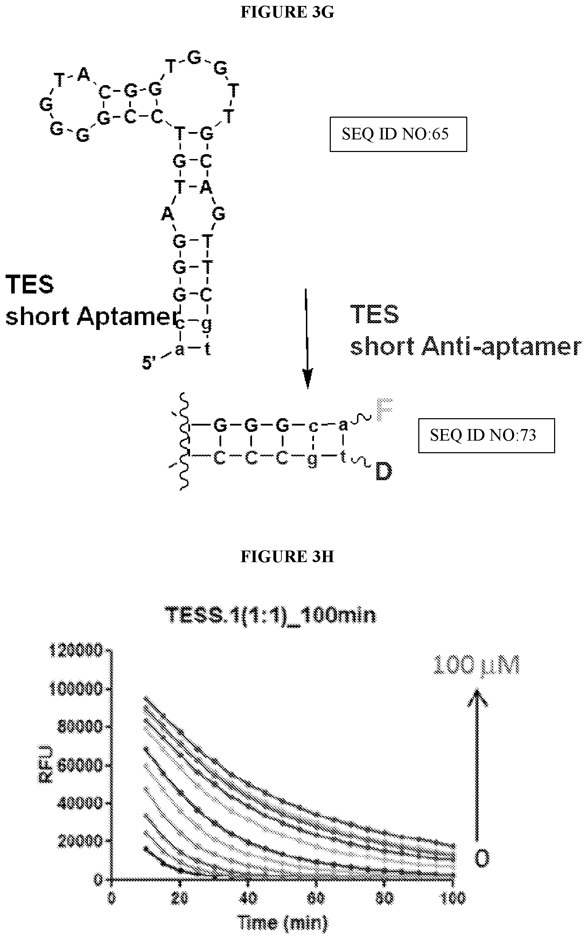

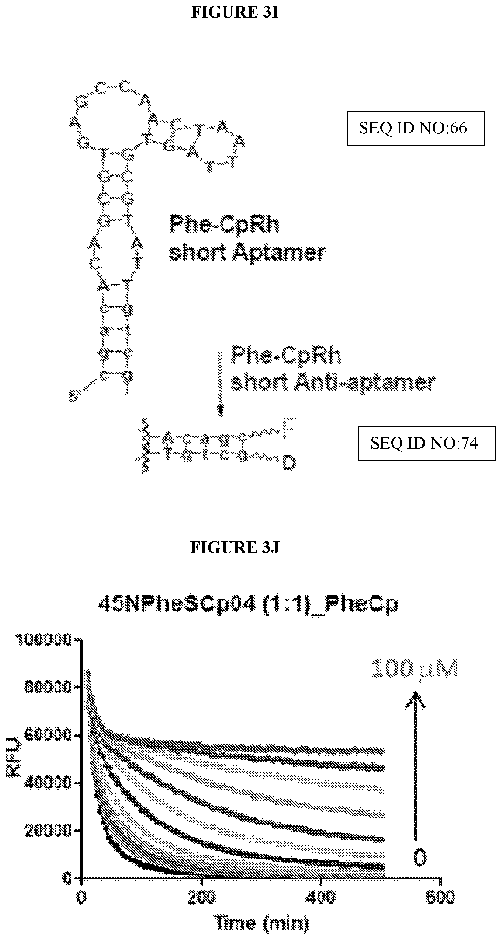

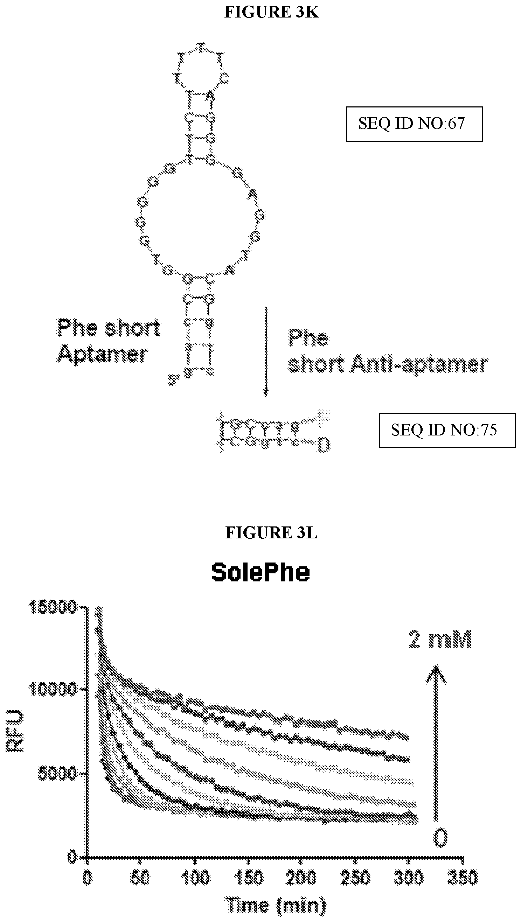

FIGURE 3A-L. Examples of results of anti-aptamer assays for various target analytes using "short-form" primary aptamers. (A) Primary aptamer for deoxycorticosterone 21-glucoside ("DOG") (SEQ ID NO:62), linked to fluorescent label (e.g. F = fluorescein) and its complement anti-aptamer (SEQ ID NO:69) labeled with quencher (e.g., D = dabcyl quencher) can hybridize to form a duplex (schematically indicated by SEQ ID NO:70); less duplex forms in the presence of DOG and fluorescent signal increases. (B) 1:1 ratio of primary aptamer and anti-aptamer of (A) are combined with increasing concentrations of DOG, from 0 to 100 µM. The graph shows fluoresence over time. (C) Primary aptamer (SEQ ID NO:63) for aldosterone ("ALDS"), linked to fluorescent label (e.g. F = fluorescein) and its complement anti-aptamer labeled with quencher (e.g., D = dabcyl quencher) can hybridize to form a duplex (schematically indicated by SEQ ID NO:71); less duplex forms in the presence of ALDS and fluorescent signal increases. (D) 1:1 ratio of primary aptamer and anti-aptamer of (C) are combined with increasing concentrations of ALDS, from 0 to 100 µM. The graph shows fluoresence over time. (E) Primary aptamer for cortisol ("CS") (SEQ ID NO:64), linked to fluorescent label (e.g. F = fluorescein) and its complement anti-aptamer labeled with quencher (e.g., D = dabcyl quencher) can hybridize to form a duplex (schematically indicated by SEQ ID NO:72); less duplex forms in the presence of CS and fluorescent signal increases. (F) 1:10 ratio of primary aptamer and anti-aptamer of (E) are combined with increasing concentrations ofCS, from 0 to 125 µM. The graph shows fluoresence over time. (G) Primary aptamer for testosterone ("TES") (SEQ ID NO:65), linked to fluorescent label (e.g. F = fluorescein) and its complement anti-aptamer labeled with quencher (e.g., D = dabcyl quencher) can hybridize to form a duplex (schematically indicated by SEQ ID NO:73); less duplex forms in the presence of TES and fluorescent signal increases. (H) 1:1 ratio of primary aptamer and anti-aptamer of (G) are combined with increasing concentrations ofTES, from 0 to 100 µM. The graph shows fluoresence over time. (I) Primary aptamer for Phe-CpRh (SEQ ID NO:66), linked to fluorescent label (e.g. F = fluorescein) and its complement anti-aptamer labeled with quencher (e.g., D = dabcyl quencher) can hybridize to form a duplex (schematically indicated by SEQ ID NO:74); less duplex forms in the presence of DOG and fluorescent signal increases. (J) 1:1 ratio of primary aptamer and anti-aptamer of (I) are combined with increasing concentrations of Phe-CpRh, from 0 to 100 µM. The graph shows fluoresence over time. (K) Primary aptamer for phenylalanine ("Phe") (SEQ ID NO:67), linked to fluorescent label (e.g. F = fluorescein) and its complement anti-aptamer labeled with quencher (e.g., D = dabcyl quencher) can hybridize to form a duplex (schematically indicated by SEQ ID NO:75); less duplex forms in the presence of Phe and fluorescent signal increases. (L) 1: 1 ratio of primary aptamer and anti-aptamer of (K) are combined with increasing concentrations of Phe, from 0 to 2 mM. The graph shows fluoresence over time. These results demonstrate the target analyte -concentration dependent effect on hybridization of the aptamer and anti-aptamer strands.

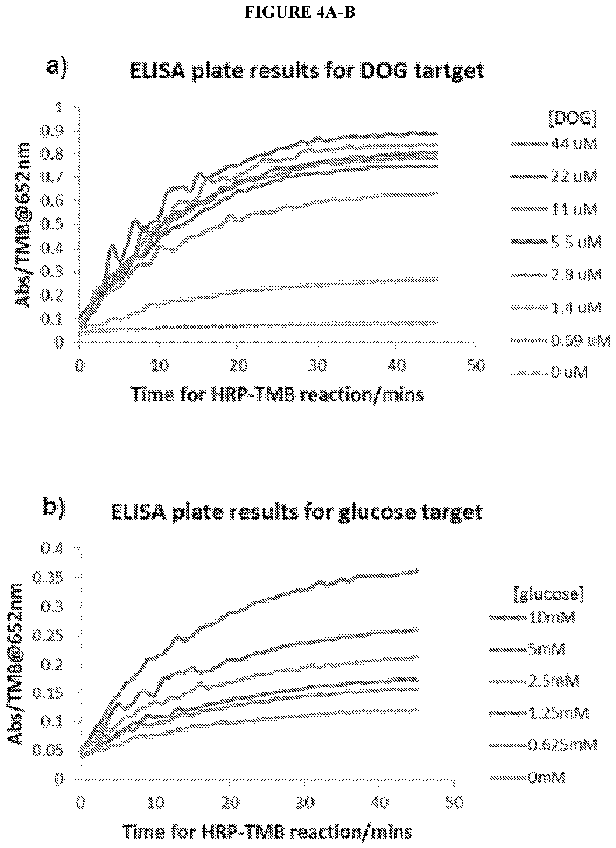

FIGURE 4A-C. Aptamer/anti-aptamer (short-form) results in plate-based assay format. Signal is from the reaction between TMB and HRP-tagged anti-aptamer complement. (A) DOG: anti-aptamer coated plate exposed to a solution of 1µM DOGaptamer and DOG target for 30mins. (B) Glucose: a solution of 2 µM glucose-aptamer and glucose target exposed to 0.2 µM anti-aptamer for 10mins, then "pulled-down" on to a streptavidin-coated plate. (C) Phenylalanine: a solution of 1 µM Phe-aptamer and phenylalanine target exposed to 0.2 µM anti-aptamer for 5mins, then "pulled-down" on to a streptavidin-coated plate.

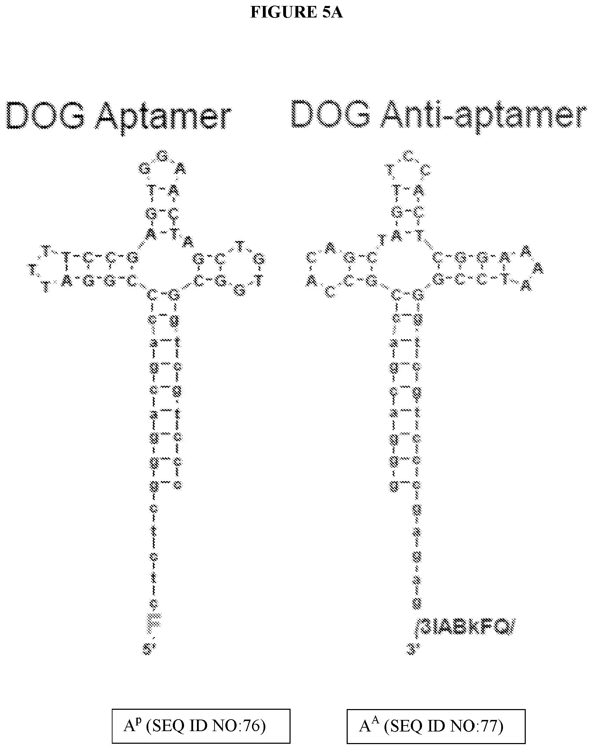

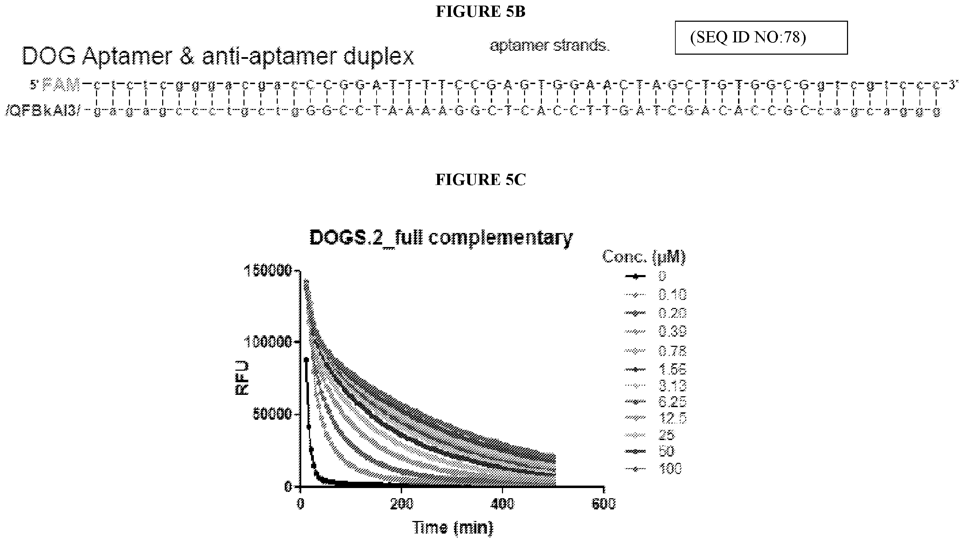

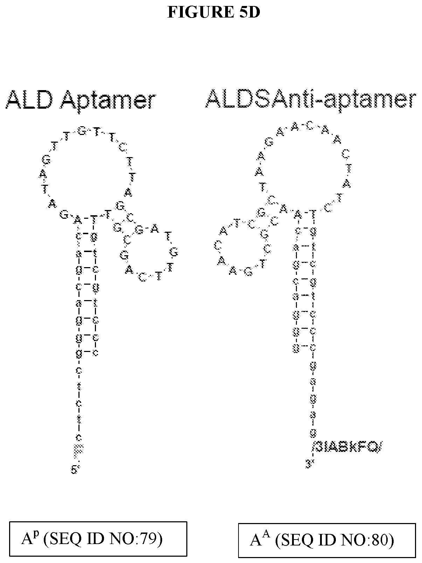

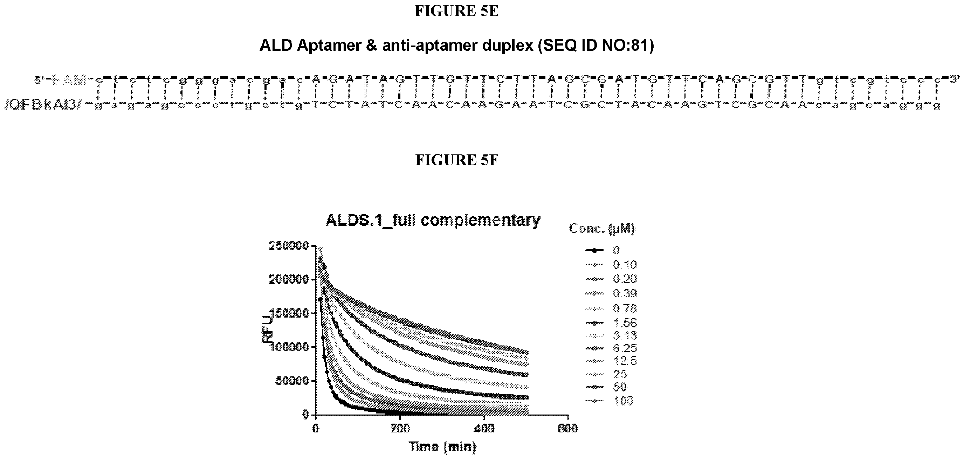

FIGURE 5A-F. Examples of results of anti-aptamer assays for various target analytes using "long-form" primary aptamers. (A) A long-form of a primary aptamer for deoxycorticosterone 21-glucoside ("DOG") (SEQ ID NO:76, which comprises SEQ ID NOS:62 and 12), linked to fluorescent label (e.g. FAM = fluorescein) and its complement anti-aptamer (SEQ ID NO:77) labeled with quencher (e.g., QFBkA13 quencher) can hybridize to form a duplex (B; SEQ ID NO:78). (C) Fluorescent signal over time after mixing aptamer and anti-aptamer in the presence of various concentrations of target. These results demonstrate the target-concentration dependent effect on hybridization of the aptamer and anti-aptamer strands. (D) A long-form of a primary aptamer for aldosterone ("ALDS") (SEQ ID NO:79, which comprises SEQ ID NO:63), linked to fluorescent label (e.g. FAM =fluorescein) and its complement anti-aptamer (SEQ ID NO:80) labeled with quencher (e.g., QFBkA13 ("Iowa Black"; Integrated DNA Technologies) quencher) can hybridize to form a duplex (E; SEQ ID NO:81). (F) Fluorescent signal over time after mixing aptamer and anti-aptamer in the presence of various concentrations of target. These results demonstrate the target-concentration dependent effect on hybidization of the aptamer and anti-aptamer strands.

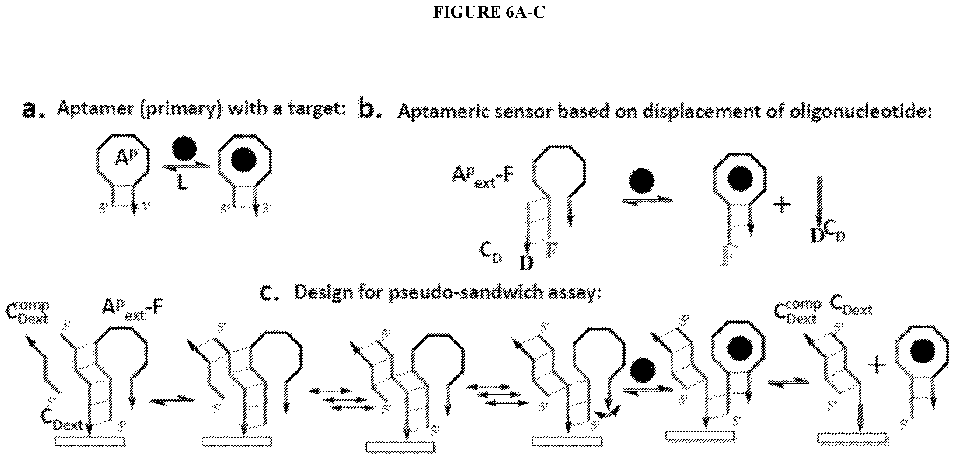

FIGURE 6A-C. Design principles of pseudo-sandwich assays. (A) Primary aptamer (A^{P}) in complex with its ligand (L). (B) A^{P} is extended (A^{P}ₑₓₜ) and complementary oligonucleotide (C_{D}) is added to obtain a structure-switching sensor (shown here in a set-up to achieve fluorescent signaling; F = fluorescein and D = dabcyl quencher). This is an equilibrium process, and a system, if attached to a surface to achieve ELISA-like format, cannot be washed without removing components. (C) Pseudo-sandwich assay overcomes these problems and translate equilibrium reactions into stable double helical structure. To achieve this, C_{D} is extended into C_{Dext} and then C_{Dext}^{comp} is used to establish a different equilibrium. If C_{Dext} is attached to surface (one possible implementation, in the other one C_{Dext}^{comp} is attached to surface), upon binding of ligand, C_{Dext} and C_{Dext}^{comp} form a stable double helical complex that can be, if formed on surface, extensively washed.

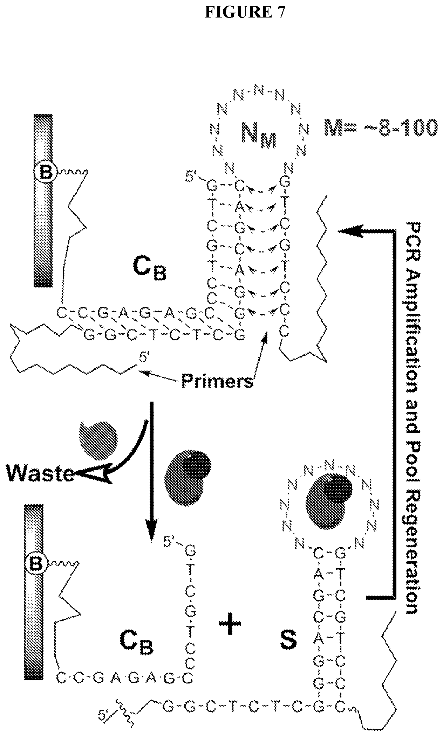

FIGURE 7. Selection of primary aptamers. In the depicted schematic representation of a solution-phase selection an oligonucleotide library (N_{M}, with randomized region M having 8 to 100 nucleotides, flanked by constant regions, i.e., primers for the PCR amplification, where N can be any one of A, T, G, or C) is attached to an agarose-streptavidin column via a biotinylated complementary oligonucleotide (C_{B}). Exposure to a target (blue shape; red shape is a counter-target that ensures specificity through elimination of cross-reactivity, causes elution of receptors in which a stem (S) is stabilized, and these sequences are then amplified. Figure discloses SEQ ID NOS 308, 309, 308, and 309, respectively, in order of appearance.
FIGURE 8A-C. Design principles for sandwich assay for small molecules and concept of secondary "sandwich" aptamers. (A) A^{P} in complex with its L. (B) Secondary "sandwich" aptamer (A^{S}) binds to A^{P} only when it is in a complex with L to form a ternary complex. (C) If A^{P} is attached to surface, A^{S} will attach itself to a complex of A^{P} with its L, thus forming a sandwich.

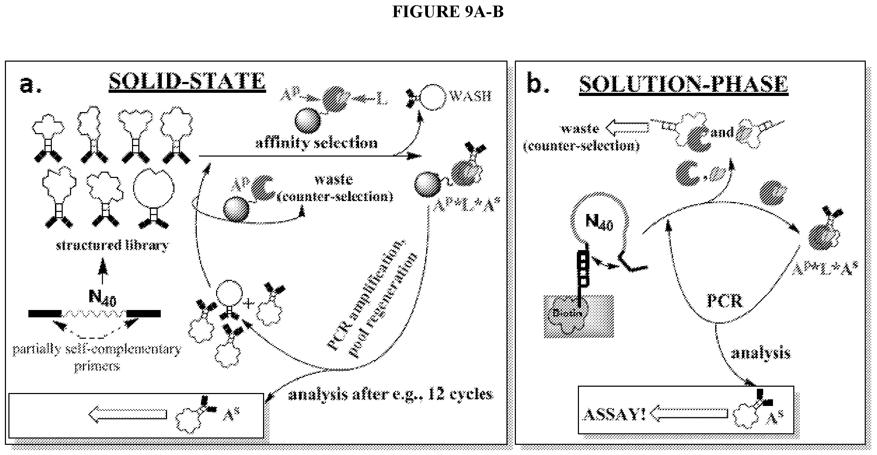

FIGURE 9A-B. Selection of secondary "sandwich" aptamers. (A) A solid-state selection: A target aptamer (A^{p}) is attached to a matrix (e.g., beads), incubated with a library (e.g., structured N₄₀, with partially self-complementary primers forming a stem; alternative is unstructured N_{M}) in the presence of L to isolate aptamer candidates with affinity for A^{P∗}L complex. These oligonucleotides are PCR-amplified, single-stranded species regenerated, and then used in the next selection cycle. The process is repeated until convergence is reached, pool cloned and sequenced, leading to A^{S} candidates. The counter-selection is performed by elimination of binders to A^{P} in the absence of L. (B) The process in solution-phase uses pre-structured library attached to matrix via complementary oligonucleotides, and A^{S} candidates are selected because their loop is closed through binding to A^{P} and they get released from the solid surface. Counter-selection in this case is against A^{P} without ligand, and ligand itself.
FIGURE 10A-C. Primary aptamer for glucose as used in pseudosandwich assay. (A) Primary aptamer (SEQ ID NO:1) bound to sensor oligonucleotide (SEQ ID NO: 82). (B) Core/pocket (SEQ ID NO:83); left-most strand of hairpin is 5' end. (C) Dose/fluorescent response curve.
FIGURE 11A-C. Primary aptamer for phenylalanine as used in pseudosandwich assay. (A) Primary aptamer (SEQ ID NO:2) bound to sensor oligonucleotide (SEQ ID NO: 84). (B) Core/pocket (SEQ ID NO:85); left-most strand of hairpin is 5' end. (C) Dose/fluorescent response curve.
FIGURE 12A-C. Primary aptamer for phenylalanine as used in pseudosandwich assay. (A) Primary aptamer (SEQ ID NO:3) bound to sensor oligonucleotide (SEQ ID NO: 86). (B) Core/pocket (SEQ ID NO:87); left-most strand of hairpin is 5' end. (C) Dose/fluorescent response curve.
FIGURE 13A-C. Primary aptamer for phenylalanine as used in pseudosandwich assay. (A) Primary aptamer (SEQ ID NO:4) bound to sensor oligonucleotide (SEQ ID NO:88). (B) Core/pocket (SEQ ID NO:89); left-most strand of hairpin is 5' end. (C) Dose/fluorescent response curve.
FIGURE 14A-C. Primary aptamer for hydrocortisone as used in pseudosandwich assay. (A) Primary aptamer (SEQ ID NO:5) bound to sensor oligonucleotide (SEQ ID NO:90). (B) Core/pocket (SEQ ID NO:91); left-most strand of hairpin is 5' end. (C) Dose/fluorescent response curve.
FIGURE 15A-C. Primary aptamer for hydrocortisone as used in pseudosandwich assay. (A) Primary aptamer (SEQ ID NO:6) bound to sensor oligonucleotide (SEQ ID NO:92). (B) Core/pocket (SEQ ID NO:93); left-most strand of hairpin is 5' end. (C) Dose/fluorescent response curve.
FIGURE 16A-C. Primary aptamer for hydrocortisone as used in pseudosandwich assay. (A) Primary aptamer (SEQ ID NO:7) bound to sensor oligonucleotide (SEQ ID NO:94). (B) Core/pocket (SEQ ID NO:95); left-most strand of hairpin is 5' end. (C) Dose/fluorescent response curve.
FIGURE 17A-C. Primary aptamer for dehyrdroisoandrosterone and deoxycorticosterone as used in pseudosandwich assay. (A) Primary aptamer (SEQ ID NO:8) bound to sensor oligonucleotide (SEQ ID NO:96); left-most strand of hairpin is 5' end. (B) Core/pocket (SEQ ID NO:97); left-most strand of hairpin is 5' end. (C) Dose/fluorescent response curve.
FIGURE 18A-C. Primary aptamer for dehydroisoandrosterone as used in pseudosandwich assay. (A) Primary aptamer (SEQ ID NO:9) bound to sensor oligonucleotide (SEQ ID NO:98). (B) Core/pocket (SEQ ID NO:99); left-most strand of hairpin is 5' end. (C) Dose/fluorescent response curve.
FIGURE 19A-C. Primary aptamer for dehydroisoandrosterone as used in pseudosandwich assay. (A) Primary aptamer (SEQ ID NO:10) bound to sensor oligonucleotide (SEQ ID NO:100). (B) Core/pocket (SEQ ID NO:101); left-most strand of hairpin is 5' end. (C) Dose/fluorescent response curve.
FIGURE 20A-C. Primary aptamer for dehydroisoandrosterone as used in pseudosandwich assay. (A) Primary aptamer (SEQ ID NO:11) bound to sensor oligonucleotide (SEQ ID NO:102). (B) Core/pocket (SEQ ID NO:103); left-most strand of hairpin is 5' end. (C) Dose/fluorescent response curve.
FIGURE 21A-C. Primary aptamer for deoxycorticosterone as used in pseudosandwich assay. (A) Primary aptamer (SEQ ID NO:12) bound to sensor oligonucleotide (SEQ ID NO:104). (B) Core/pocket (SEQ ID NO:105); left-most strand of hairpin is 5' end. (C) Dose/fluorescent response curve.
FIGURE 22A-C. Primary aptamer for testosterone as used in pseudosandwich assay. (A) Primary aptamer (SEQ ID NO:13) bound to sensor oligonucleotide (SEQ ID NO:106). (B) Core/pocket (SEQ ID NO:107); left-most strand of hairpin is 5' end. (C) Dose/fluorescent response curve.
FIGURE 23A-C. Primary aptamer for testosterone as used in pseudosandwich assay. (A) Primary aptamer (SEQ ID NO:14) bound to sensor oligonucleotide (SEQ ID NO:108). (B) Core/pocket (SEQ ID NO:109); left-most strand of hairpin is 5' end. (C) Dose/fluorescent response curve.
FIGURE 24A-C. Primary aptamer for testosterone as used in pseudosandwich assay. (A) Primary aptamer (SEQ ID NO:15) bound to sensor oligonucleotide (SEQ ID NO:110). (B) Core/pocket (SEQ ID NO:111); left-most strand of hairpin is 5' end. (C) Dose/fluorescent response curve.
FIGURE 25A-C. Primary aptamer for testosterone as used in pseudosandwich assay. (A) Primary aptamer (SEQ ID NO:16) bound to sensor oligonucleotide (SEQ ID NO: 112). (B) Core/pocket (SEQ ID NO:113); left-most strand of hairpin is 5' end. (C) Dose/fluorescent response curve.
FIGURE 26A-C. Primary aptamer for testosterone as used in pseudosandwich assay. (A) Primary aptamer (SEQ ID NO:17) bound to sensor oligonucleotide (SEQ ID NO: 114). (B) Core/pocket (SEQ ID NO:115); left-most strand of hairpin is 5' end. (C) Dose/fluorescent response curve.
FIGURE 27A-C. Primary aptamer for sphingosine-1-phosphate (d18:1) as used in pseudosandwich assay. (A) Primary aptamer (SEQ ID NO:18) bound to sensor oligonucleotide (SEQ ID NO: 116). (B) Core/pocket (SEQ ID NO:117); left-most strand of hairpin is 5' end. (C) Dose/fluorescent response curve.
FIGURE 28A-C. Primary aptamer for dopamine as used in pseudosandwich assay. (A) Primary aptamer (SEQ ID NO:19) bound to sensor oligonucleotide (SEQ ID NO: 118). (B) Core/pocket (SEQ ID NO:119); left-most strand of hairpin is 5' end. (C) Dose/fluorescent response curve.
FIGURE 29A-C. Primary aptamer for dopamine as used in pseudosandwich assay. (A) Primary aptamer (SEQ ID NO:20) bound to sensor oligonucleotide (SEQ ID NO: 120). (B) Core/pocket (SEQ ID NO:121); left-most strand of hairpin is 5' end. (C) Dose/fluorescent response curve.
FIGURE 30A-C. Primary aptamer for dopamine as used in pseudosandwich assay. (A) Primary aptamer (SEQ ID NO:21) bound to sensor oligonucleotide (SEQ ID NO: 122). (B) Core/pocket (SEQ ID NO:123); left-most strand of hairpin is 5' end. (C) Dose/fluorescent response curve.

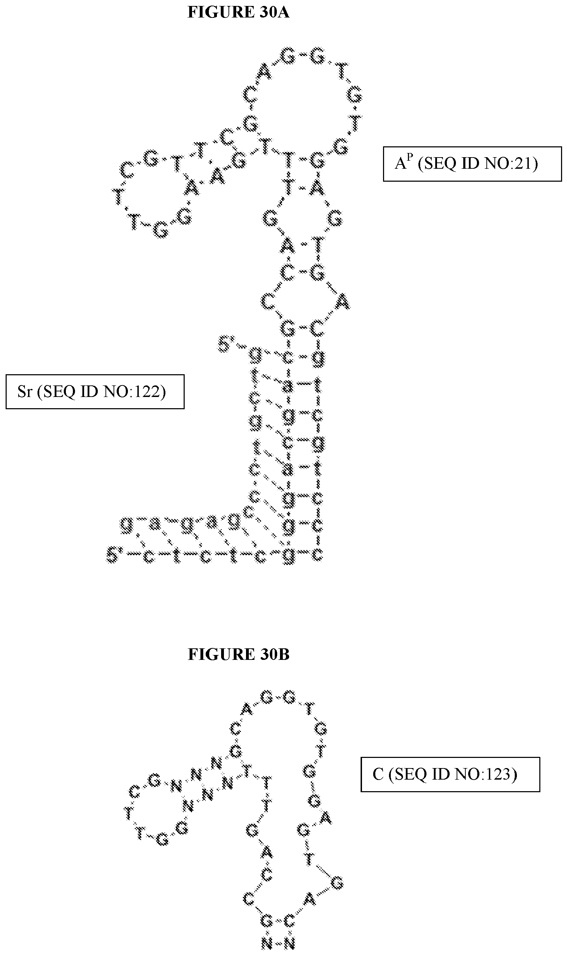

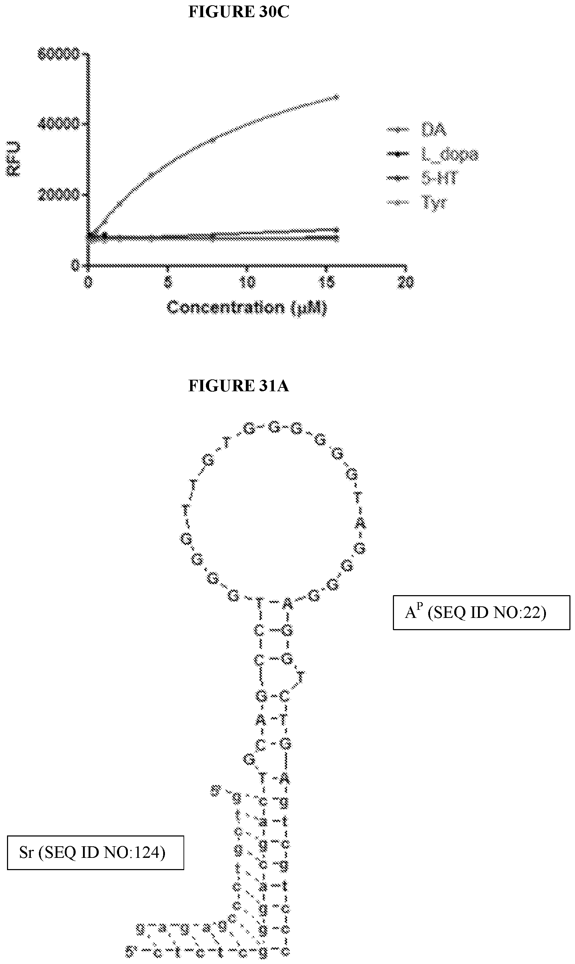

FIGURE 30A-C. Primary aptamer for dopamine as used in pseudosandwich assay. (A) Primary aptamer (SEQ ID NO:21) bound to sensor oligonucleotide (SEQ ID NO: 122). (B) Core/pocket (SEQ ID NO:123); left-most strand of hairpin is 5' end. (C) Dose/fluorescent response curve.

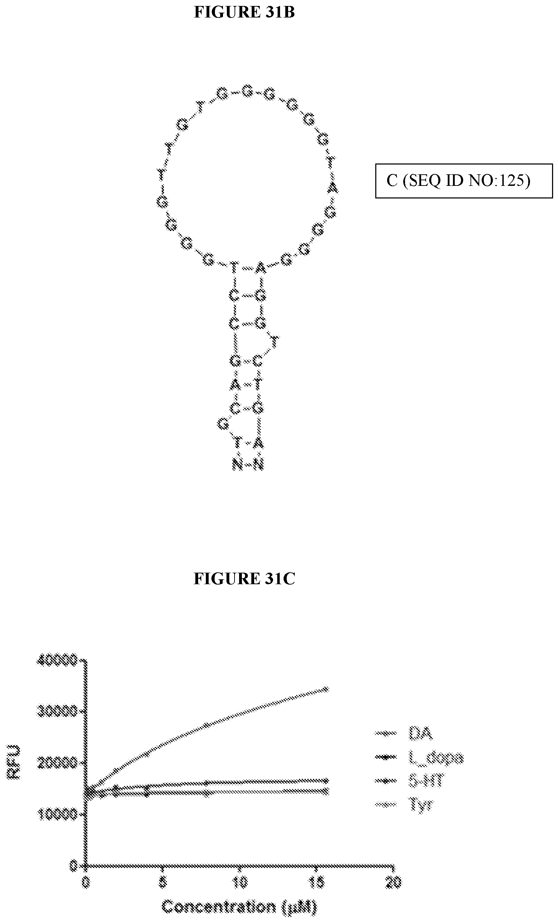

FIGURE 31A-C. Primary aptamer for dopamine as used in pseudosandwich assay. (A) Primary aptamer (SEQ ID NO:22) bound to sensor oligonucleotide (SEQ ID NO: 124). (B) Core/pocket (SEQ ID NO:125); left-most strand of hairpin is 5' end. (C) Dose/fluorescent response curve.

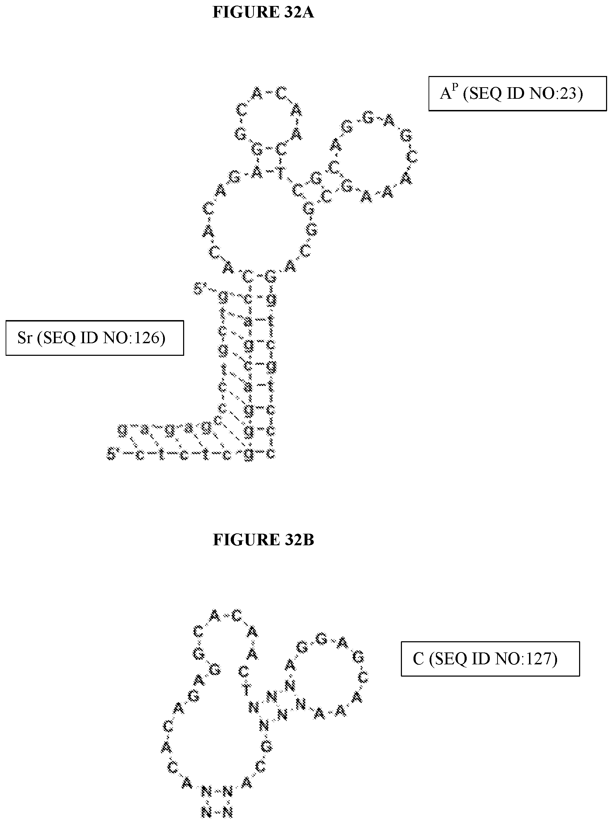

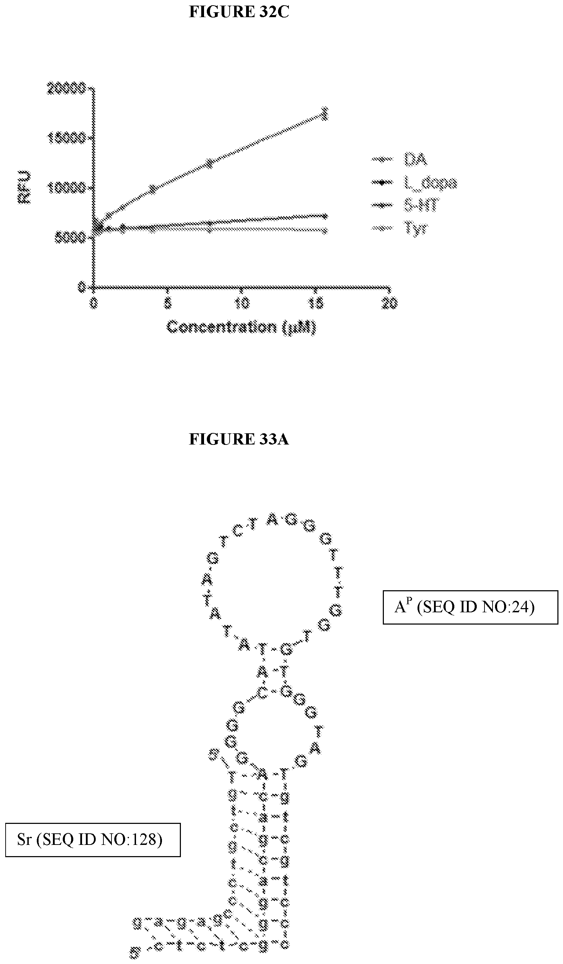

FIGURE 32A-C. Primary aptamer for dopamine as used in pseudosandwich assay. (A) Primary aptamer (SEQ ID NO:23) bound to sensor oligonucleotide (SEQ ID NO: 126). (B) Core/pocket (SEQ ID NO: 127); left-most strand of hairpin is 5' end. (C) Dose/fluorescent response curve.

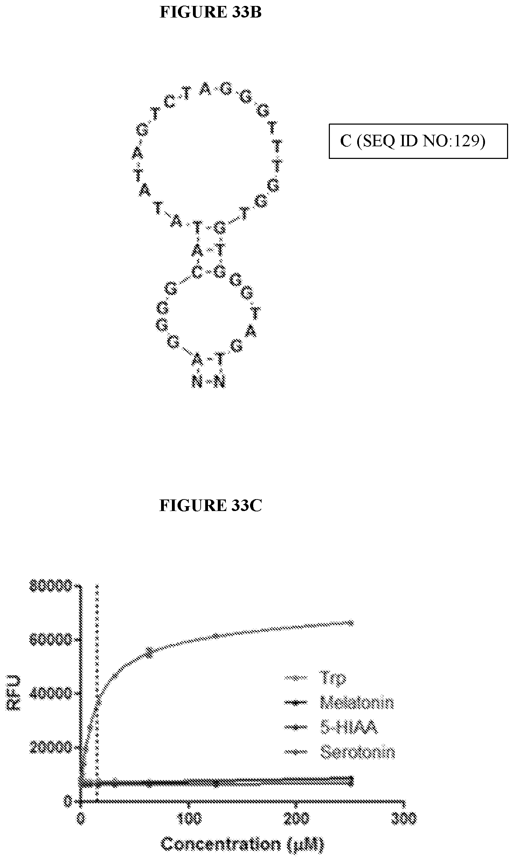

FIGURE 33A-C. Primary aptamer for dopamine as used in pseudosandwich assay. (A) Primary aptamer (SEQ ID NO:24) bound to sensor oligonucleotide (SEQ ID NO: 128). (B) Core/pocket (SEQ ID NO: 129); left-most strand of hairpin is 5' end. (C) Dose/fluorescent response curve.

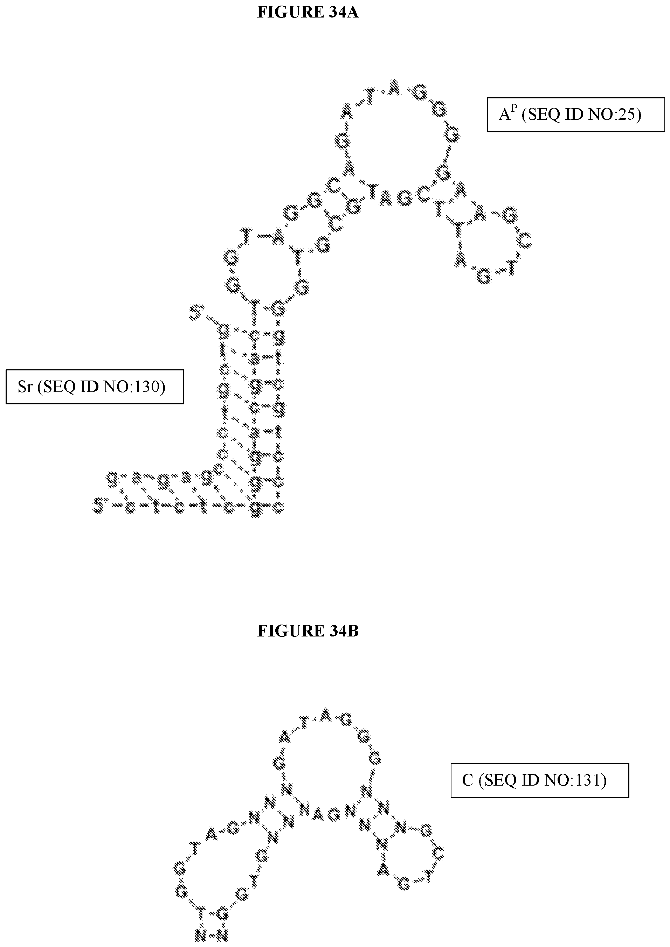

FIGURE 34A-C. Primary aptamer for dopamine as used in pseudosandwich assay. (A) Primary aptamer (SEQ ID NO:25) bound to sensor oligonucleotide (SEQ ID NO: 130). (B) Core/pocket (SEQ ID NO: 131); left-most strand of hairpin is 5' end. (C) Dose/fluorescent response curve.

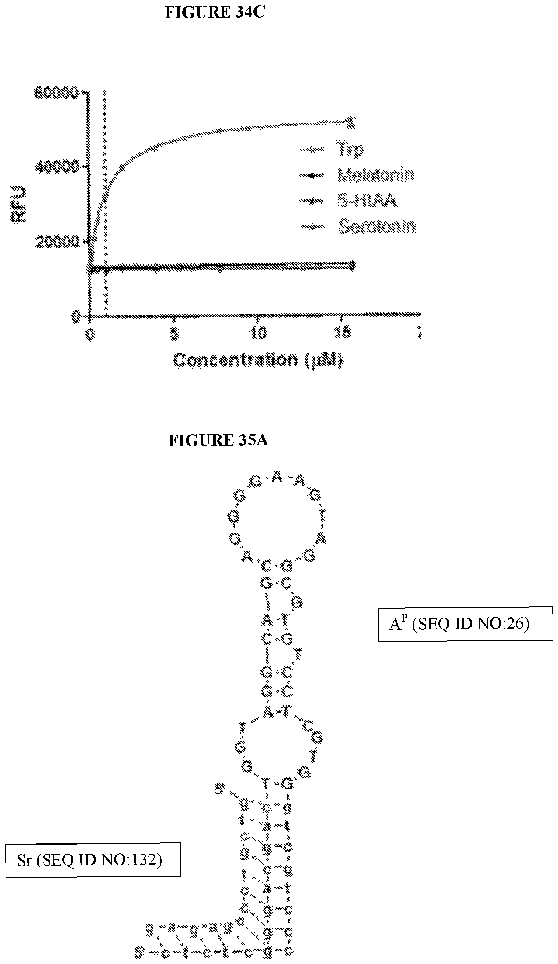

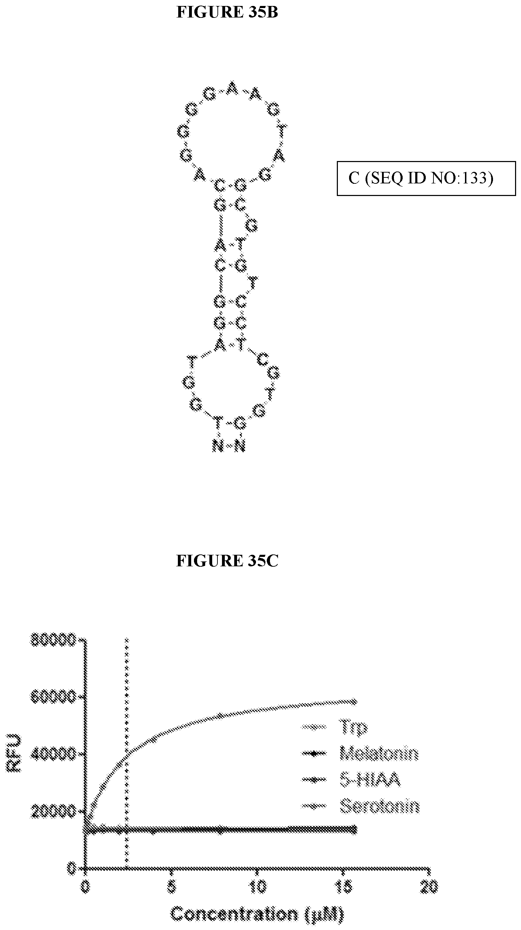

FIGURE 35A-C. Primary aptamer for dopamine as used in pseudosandwich assay. (A) Primary aptamer (SEQ ID NO:26) bound to sensor oligonucleotide (SEQ ID NO: 132). (B) Core/pocket (SEQ ID NO: 133); left-most strand of hairpin is 5' end. (C) Dose/fluorescent response curve.

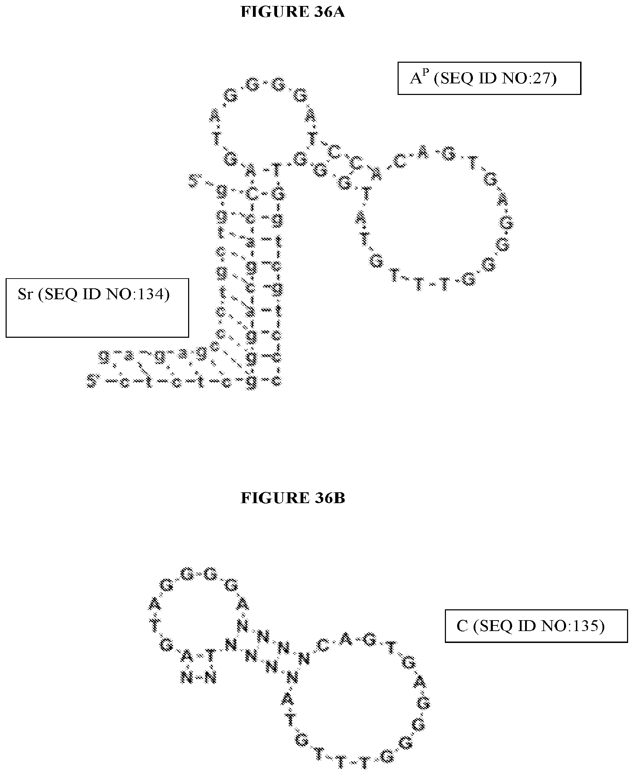

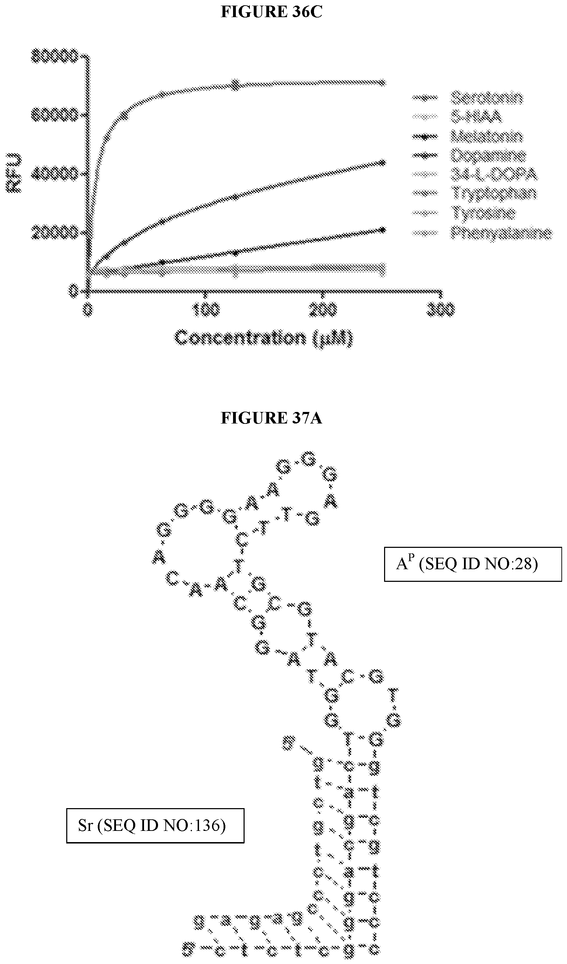

FIGURE 36A-C. Primary aptamer for dopamine as used in pseudosandwich assay. (A) Primary aptamer (SEQ ID NO:27) bound to sensor oligonucleotide (SEQ ID NO: 134). (B) Core/pocket (SEQ ID NO: 135); left-most strand of hairpin is 5' end. (C) Dose/fluorescent response curve.

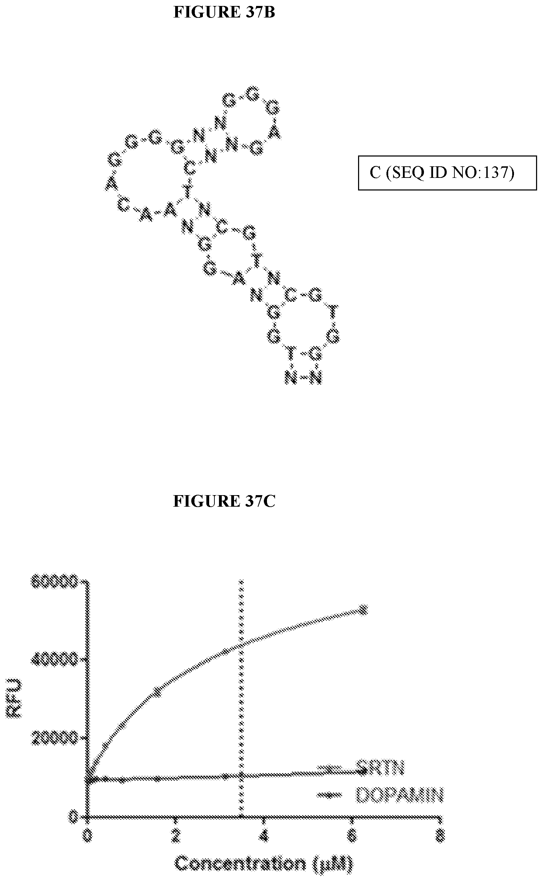

FIGURE 37A-C. Primary aptamer for dopamine as used in pseudosandwich assay. (A) Primary aptamer (SEQ ID NO:28) bound to sensor oligonucleotide (SEQ ID NO: 136). (B) Core/pocket (SEQ ID NO: 137); left-most strand of hairpin is 5' end. (C) Dose/fluorescent response curve.

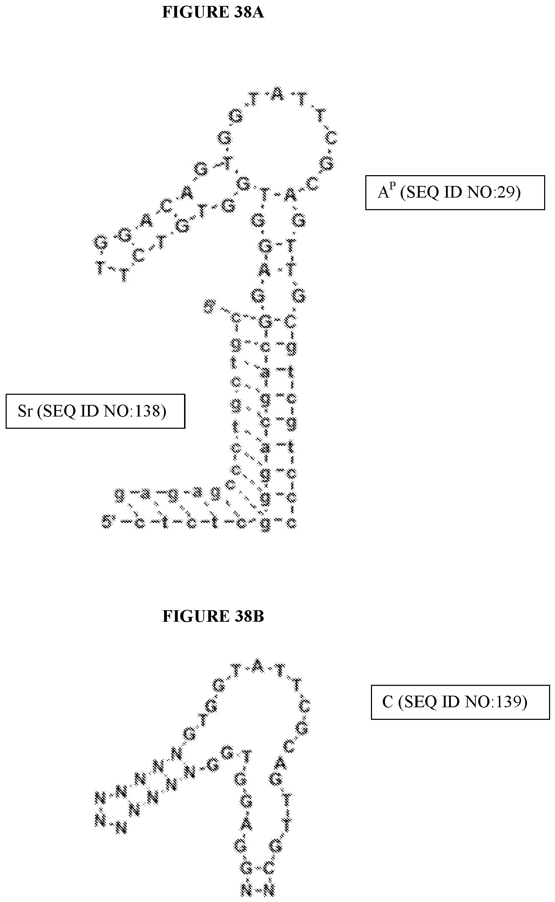

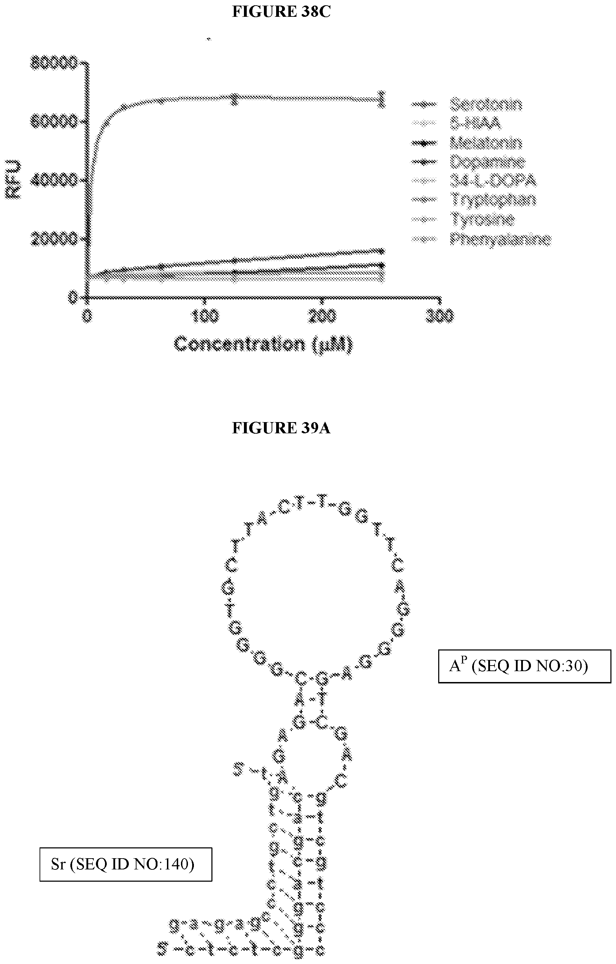

FIGURE 38A-C. Primary aptamer for dopamine as used in pseudosandwich assay. (A) Primary aptamer (SEQ ID NO:29) bound to sensor oligonucleotide (SEQ ID NO: 138). (B) Core/pocket (SEQ ID NO: 139); left-most strand of hairpin is 5' end. (C) Dose/fluorescent response curve.

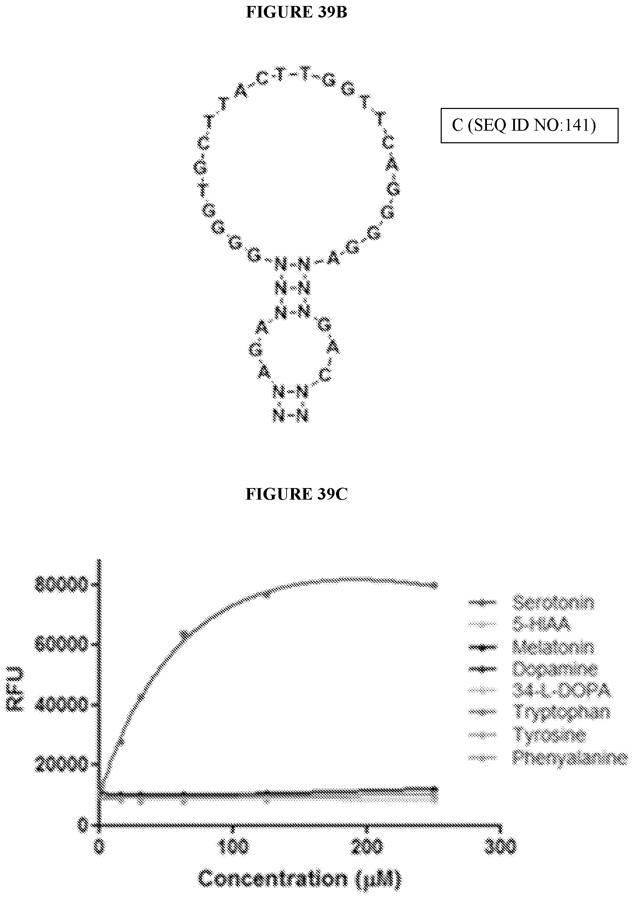

FIGURE 39A-C. Primary aptamer for dopamine as used in pseudosandwich assay. (A) Primary aptamer (SEQ ID NO:30) bound to sensor oligonucleotide (SEQ ID NO: 140). (B) Core/pocket (SEQ ID NO: 141); left-most strand of hairpin is 5' end. (C) Dose/fluorescent response curve.

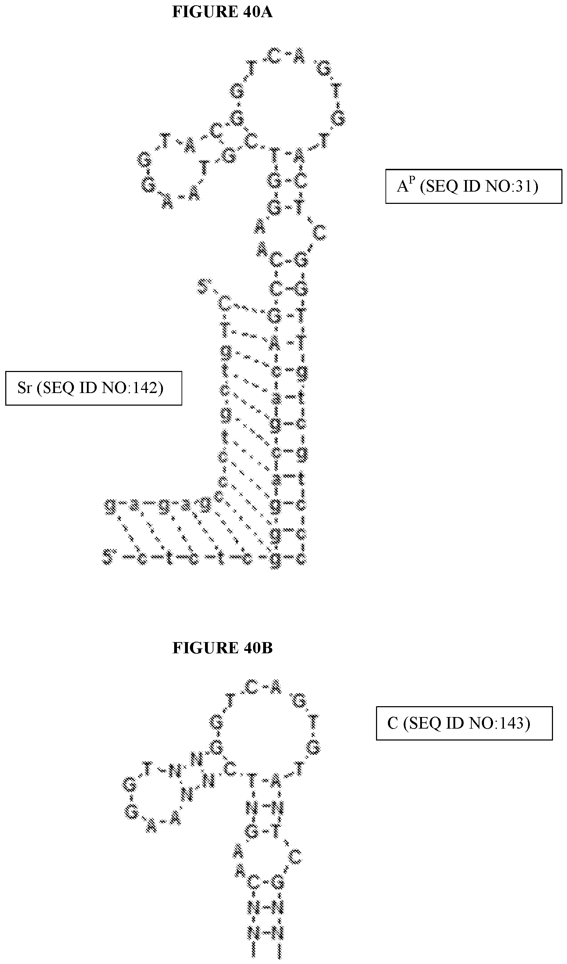

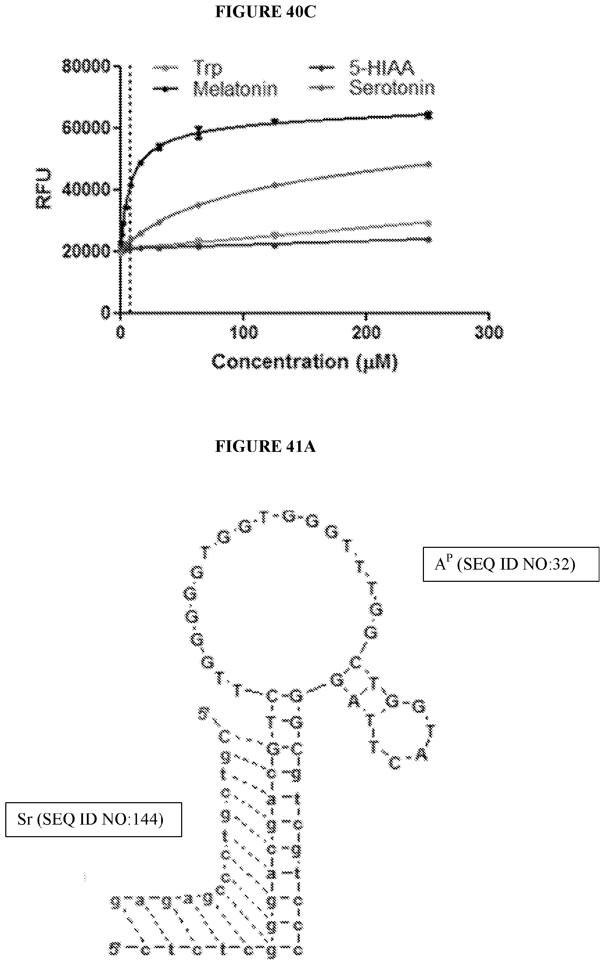

FIGURE 40A-C. Primary aptamer for dopamine as used in pseudosandwich assay. (A) Primary aptamer (SEQ ID NO:31) bound to sensor oligonucleotide (SEQ ID NO: 142). (B) Core/pocket (SEQ ID NO: 143); left-most strand of hairpin is 5' end. (C) Dose/fluorescent response curve.

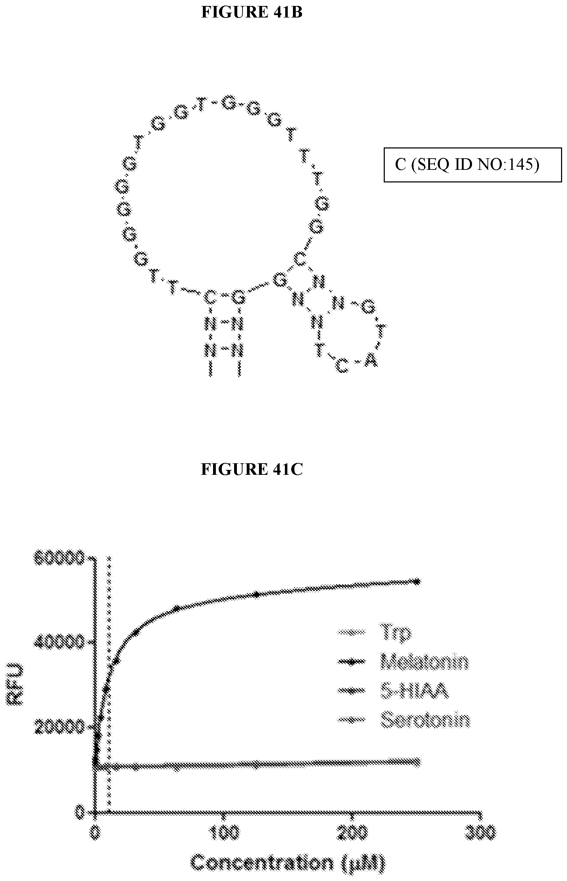

FIGURE 41A-C. Primary aptamer for dopamine as used in pseudosandwich assay. (A) Primary aptamer (SEQ ID NO:32) bound to sensor oligonucleotide (SEQ ID NO: 144). (B) Core/pocket (SEQ ID NO: 145); left-most strand of hairpin is 5' end. (C) Dose/fluorescent response curve.

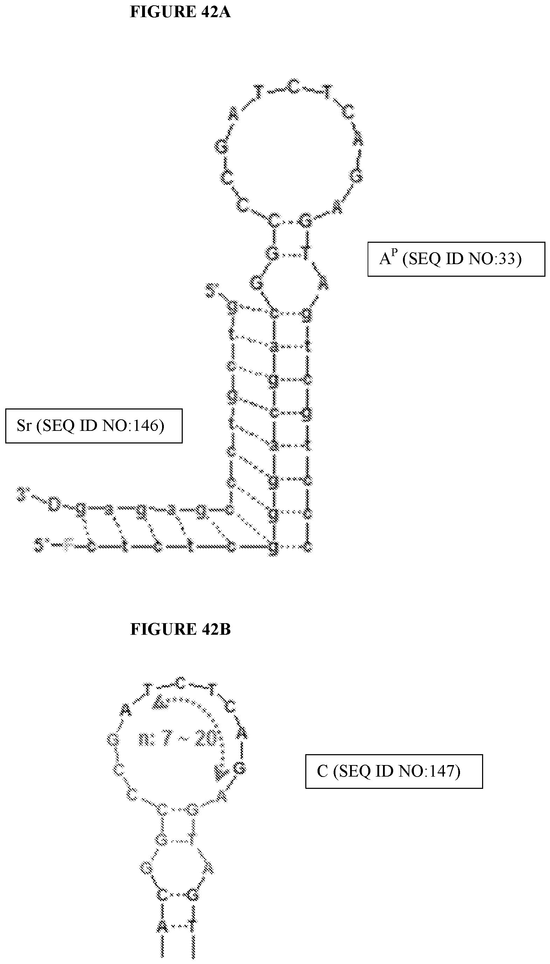

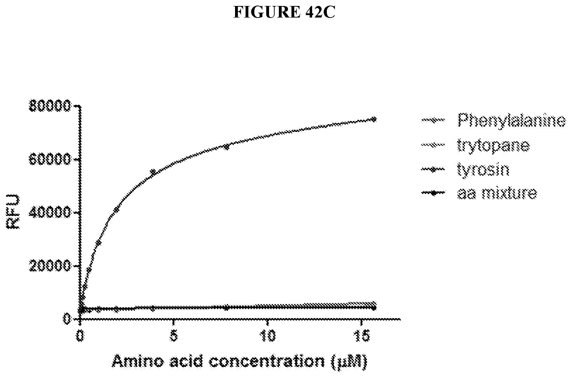

FIGURE 42A-C. Primary aptamer for dopamine as used in pseudosandwich assay. (A) Primary aptamer (SEQ ID NO:33) bound to sensor oligonucleotide (SEQ ID NO: 146). (B) Core/pocket (SEQ ID NO: 147); left-most strand of hairpin is 5' end. (C) Dose/fluorescent response curve.

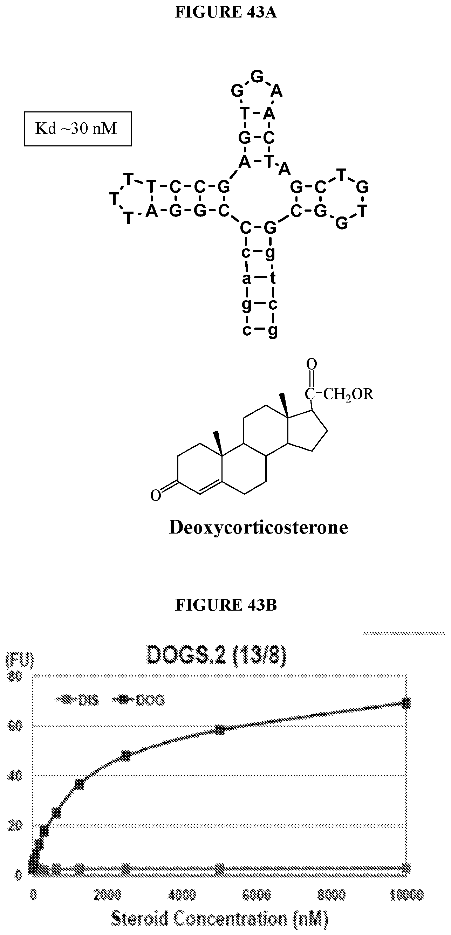

FIGURE 43A-B. Primary aptamer for deoxycorticosterone (SEQ ID NO: 62) as used in pseudosandwich assay. In certain non-limiting embodiments, the deoxycorticosterone-binding primary aptamer has a binding affinity (dissociation constant, Kd) for deoxycorticosterone that is about 30 nM (in an aqueous solution at room temperature or 25°C).

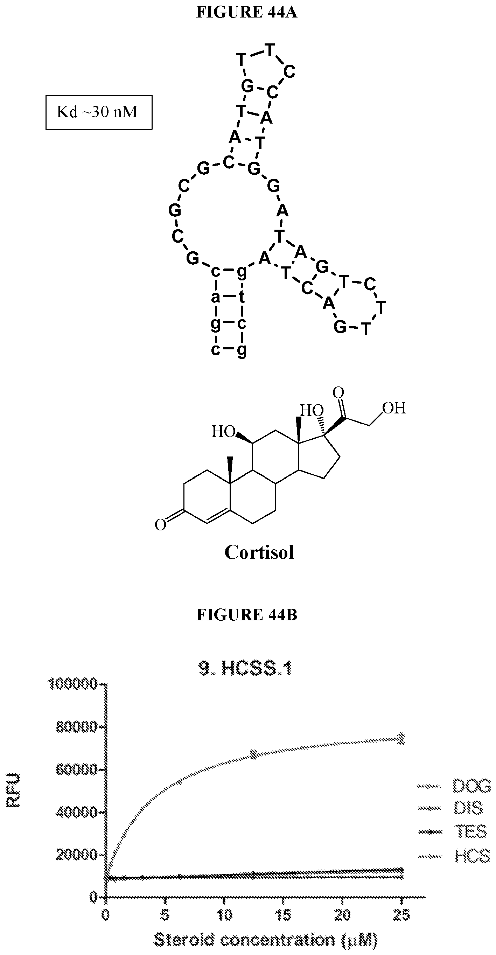

FIGURE 44A-B. Primary aptamer for cortisol (SEQ ID NO: 148) as used in pseudosandwich assay. In certain non-limiting instances, the cortisol-binding primary aptamer has a binding affinity (dissociation constant, Kd) for cortisol that is about 30 nM (in an aqueous solution at room temperature or 25°C).

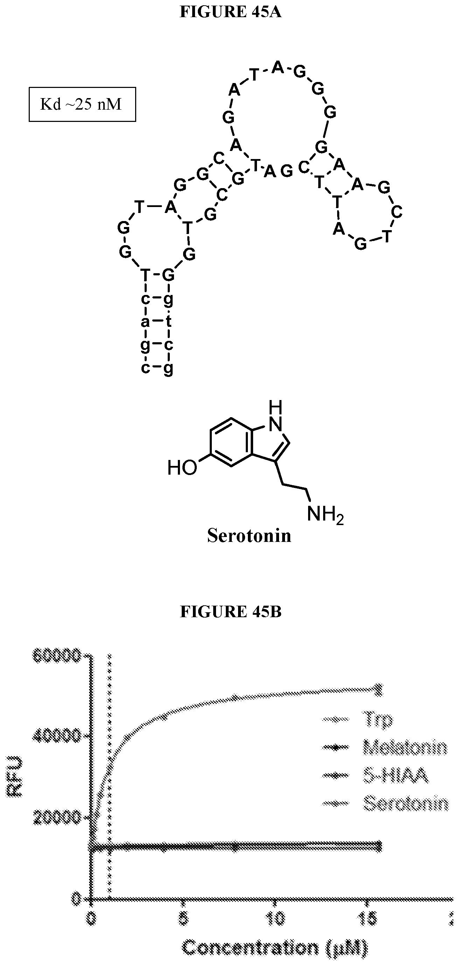

FIGURE 45A-B. Primary aptamer for serotonin (SEQ ID NO: 58) as used in pseudosandwich assay. In certain non-limiting instances, the serotonin-binding primary aptamer has a binding affinity (dissociation constant, Kd) for serotonin that is about 25 nM (in an aqueous solution at room temperature or 25°C).

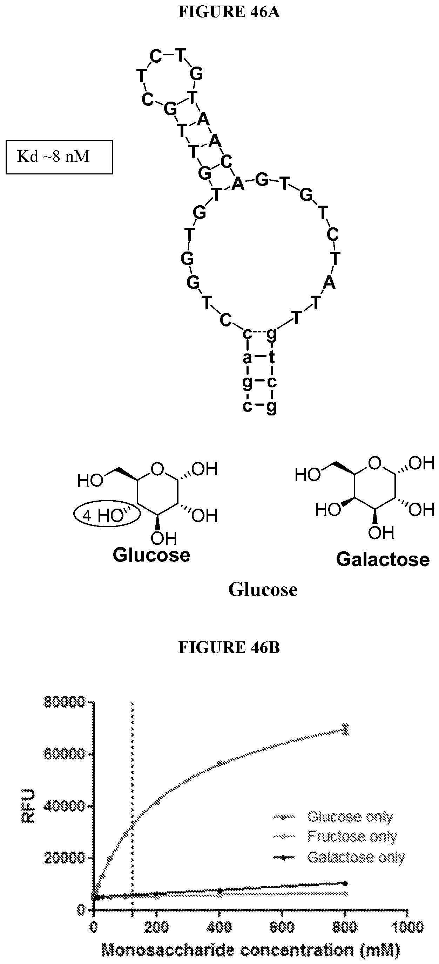

FIGURE 46A-B. Primary aptamer for glucose (SEQ ID NO: 149) as used in pseudosandwich assay. In certain non-limiting instances, the glucose-binding primary aptamer has a binding affinity (dissociation constant, Kd) for glucose that is about 8 nM (in an aqueous solution at room temperature or 25°C) and binds selectively with glucose versus galactose.

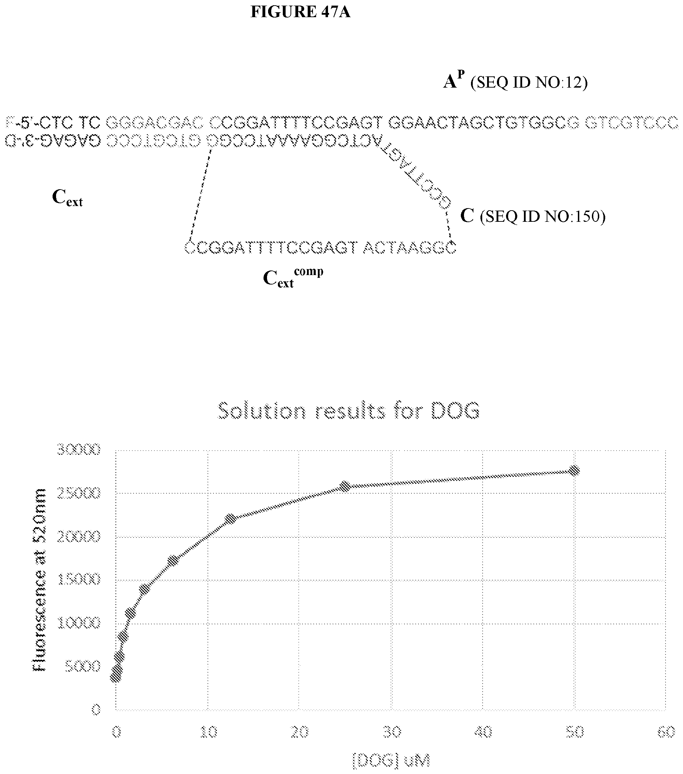

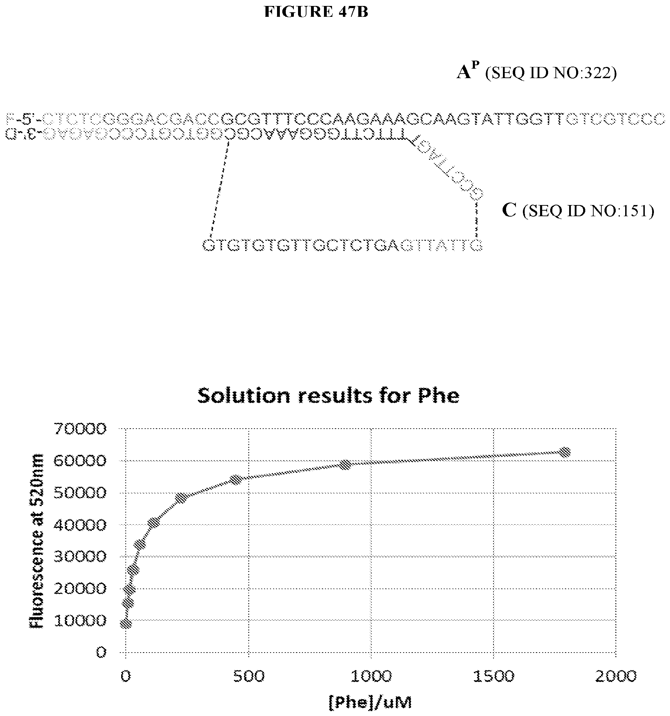

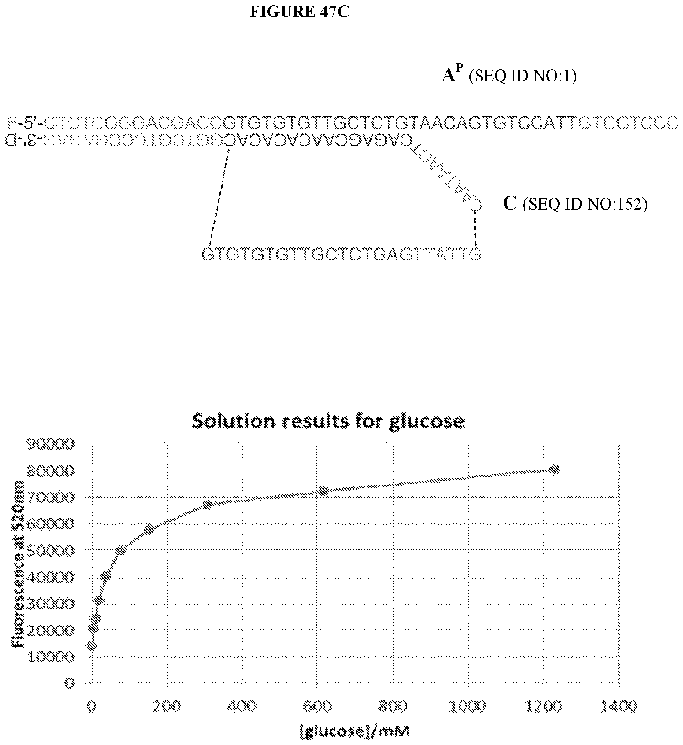

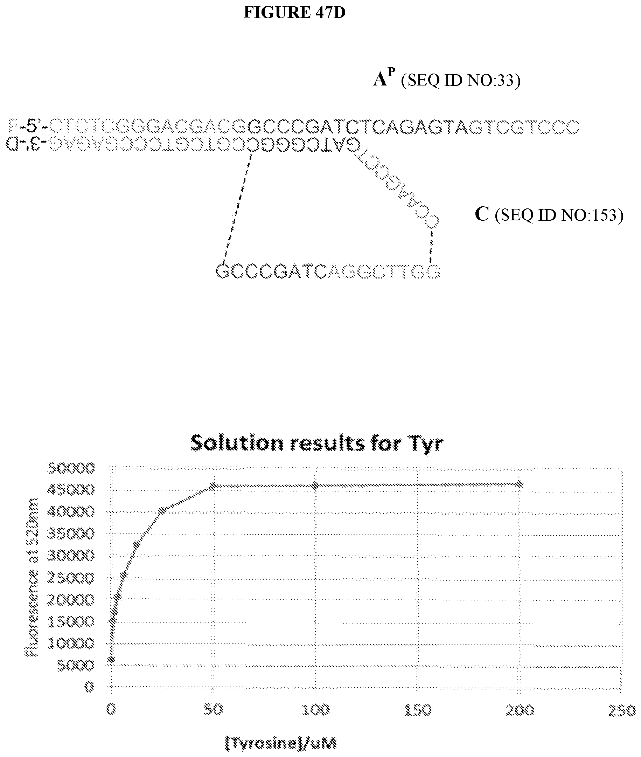

FIGURE 47A-F. Examples of pseudosandwich assays, as practiced in solution and on a plate. (A) Example of pseudosandwich assay in solution . Sequences that are participating in a pseudosandwich assays, A^{P} is primary aptamer for deoxycorticosterone 21-glucoside (DOG) (shown labeled with Fluorescein) (SEQ ID NO: 12; (SEQ ID NO: 150). Cₑₓₜ is competitor that is extended (labeled with quencher, Q), while Cₑₓₜ^{comp} is complementary oligonucleotide which forms stable double helix .Dose- fluorescence response curve showing formation of double helix between Cₑₓₜ and Cₑₓₜ^{comp}, and release of fluorescently labeled aptamer (FIGURE 6B; in this case Cₑₓₜ was labeled with a quencher, Q, and A^{P} with fluorescein, F). (B) Example of pseudosandwich assay in solution. Sequences that are participating in a pseudosandwich assays, Ap is primary aptamer for L-phenylalanine (shown labeled with Fluorescein) (SEQ ID NO: 322; (SEQ ID NO: 151). Cₑₓₜ is competitor that is extended (labeled with quencher, Q), while Cₑₓₜ^{comp} is complementary oligonucleotide which forms stable double helix. Dose- fluorescence response curve showing formation of double helix between Cₑₓₜ and Cₑₓₜ^{comp}, and release of fluorescently labeled aptamer (FIGURE 6B; in this case Cₑₓₜ was labeled with a quencher, Q, and A^{P} with fluorescein, F). (C) Example of pseudosandwich assay in solution. Sequences that are participating in a pseudosandwich assays, Ap is primary aptamer for D-glucose (shown labeled with Fluorescein) (SEQ ID NO: 1; (SEQ ID NO: 152). Cₑₓₜ is competitor that is extended (labeled with quencher, Q), while Cₑₓₜ^{comp} is complementary oligonucleotide which forms stable double helix. Dose- fluorescence response curve showing formation of double helix between Cₑₓₜ and Cₑₓₜ^{comp}, and release of fluorescently labeled aptamer (FIGURE 6B; in this case Cₑₓₜ was labeled with a quencher, Q, and A^{P} with fluorescein, F). (D) Example of pseudosandwich assay in solution. Sequences that are participating in a pseudosandwich assays, Ap is primary aptamer for L-tyrosine (shown labeled with Fluorescein) (SEQ ID NO:33; (SEQ ID NO: 153). Cₑₓₜ is competitor that is extended (labeled with quencher, Q), while Cₑₓₜ^{comp} is complementary oligonucleotide which forms stable double helix. Dose- fluorescence response curve showing formation of double helix between Cₑₓₜ and Cₑₓₜ^{comp}, and release of fluorescently labeled aptamer (FIGURE 6B; in this case Cₑₓₜ was labeled with a quencher, Q, and A^{P} with fluorescein, F). (E) Example of pseudosandwich assay plate. Sequences as used in assay on plates (SEQ ID NO: 12; (SEQ ID NO: 154); sequences are the same as in FIGURE 47A but sandwich is adapted to be formed on a plate (horseradish peroxidase-streptavidin conjugate (HRP-STV) used to amplify sandwich). Dose-color intensity response indicating ligand-dose-dependent sandwich formation on the surface of plate. (F) Example of pseudosandwich assay plate. Sequences as used in assay on plates (SEQ ID NO: 322; (SEQ ID NO: 155); sequences are the same as in FIGURE 47B but sandwich is adapted to be formed on a plate (horseradish peroxidase-streptavidin conjugate (HRP-STV) used to amplify sandwich). Dose-color intensity response indicating ligand-dose-dependent sandwich formation on the surface of plate.

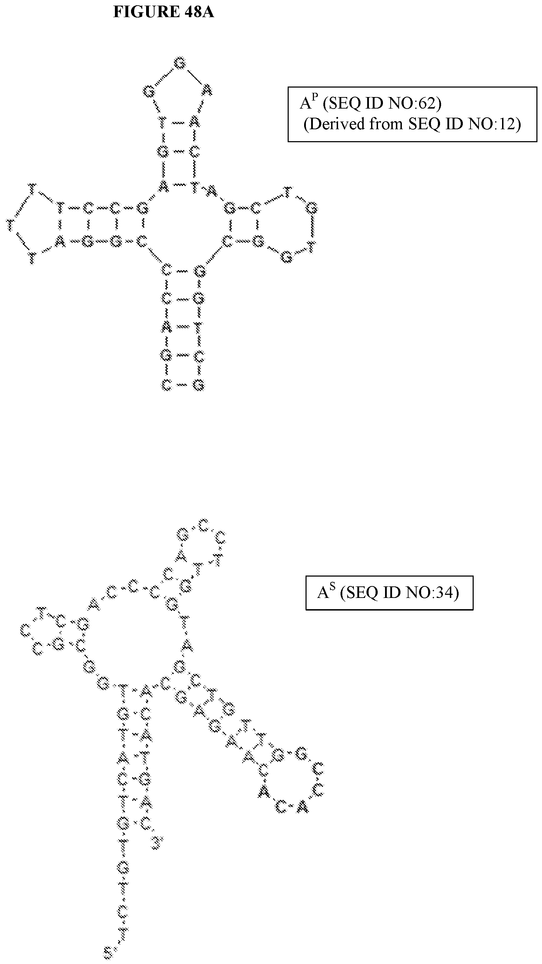

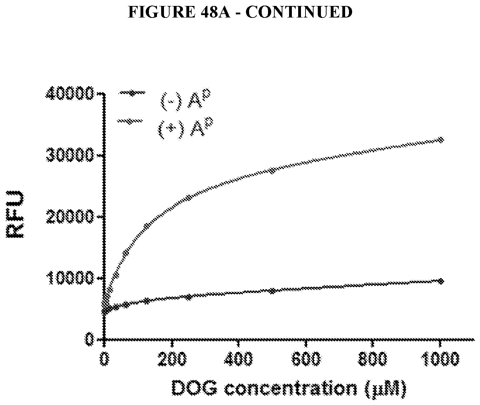

FIGURE 48A-E. Examples of secondary aptamers and examples of sandwich assays in solution and on a plate. (A) Example of secondary aptamers and assays in solution. Deoxycorticosterone as the targeted molecule that was used to form a complex with a primary aptamer. Presumed secondary structure of a primary aptamer (A^{P}) as used in selection of secondary aptamers (SEQ ID NO:62; derived from SEQ ID NO: 12). Presumed secondary structure of secondary aptamer (A^{S}) that binds to the structure (SEQ ID NO:34). Dose- fluorescence response curve to increasing quantities of target is shown as red curve, while blue is control in which primary aptamer is eliminated. For the purpose of this assay secondary aptamer was labeled with fluorescein, and Cₑₓₜ with quencher was added to it. (B) Example of secondary aptamers and assays in solution. Deoxycorticosterone as the targeted molecule that was used to form a complex with a primary aptamer. Presumed secondary structure of a primary aptamer (A^{P}) as used in selection of secondary aptamers (SEQ ID NO: 62; derived from SEQ ID NO: 12). Presumed secondary structure of secondary aptamer (A^{S}) that binds to the structure (SEQ ID NO:35). Dose- fluorescence response curve to increasing quantities of target is shown as red curve, while blue is control in which primary aptamer is eliminated. For the purpose of this assay secondary aptamer was labeled with fluorescein, and Cₑₓₜ with quencher was added to it. (C) Example of secondary aptamers and assays in solution. Serotonin as the targeted molecule that was used to form a complex with a primary aptamer. Presumed secondary structure of a primary aptamer (A^{P}) as used in selection of secondary aptamers (SEQ ID NO:58; derived from SEQ ID NO:25). Presumed secondary structure of secondary aptamer (A^{S}) that binds to the structure (SEQ ID NO:59). Dosefluorescence response curve to increasing quantities of target is shown as red curve, while blue is control in which primary aptamer is eliminated. For the purpose of this assay secondary aptamer was labeled with fluorescein, and Cₑₓₜ with quencher was added to it. (D) Example of sandwich assay on plate. Serotonin as the targeted molecule that was used to form a complex with a primary aptamer. Presumed secondary structure of a primary aptamer (A^{P}) as used in selection of secondary aptamers (SEQ ID NO:58; derived from SEQ ID NO:25). Presumed secondary structure of secondary aptamer (A^{S}) that binds to the structure (SEQ ID NO:59; Derived from SEQ ID NO: 36). Dose-color intensity response curve to ligand; color is developed by HRP-STV reaction.

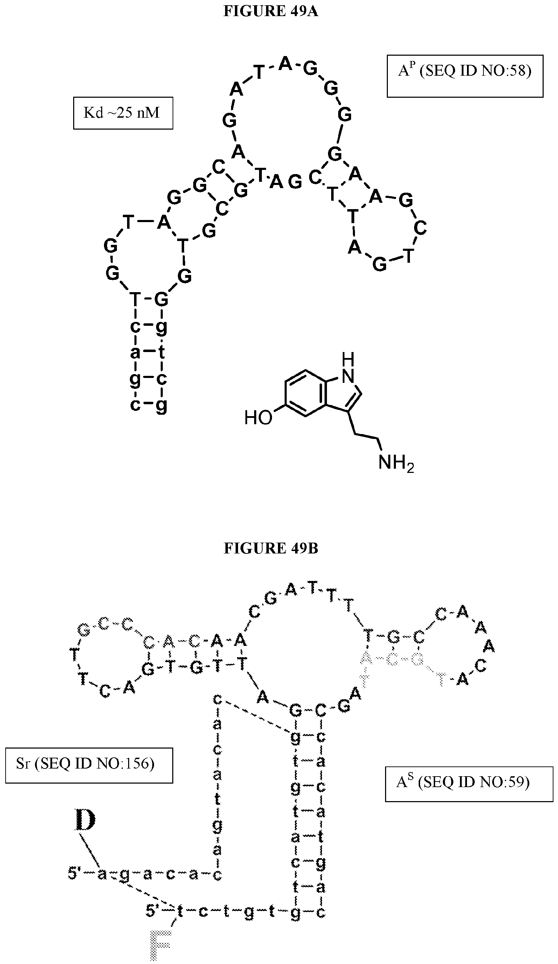

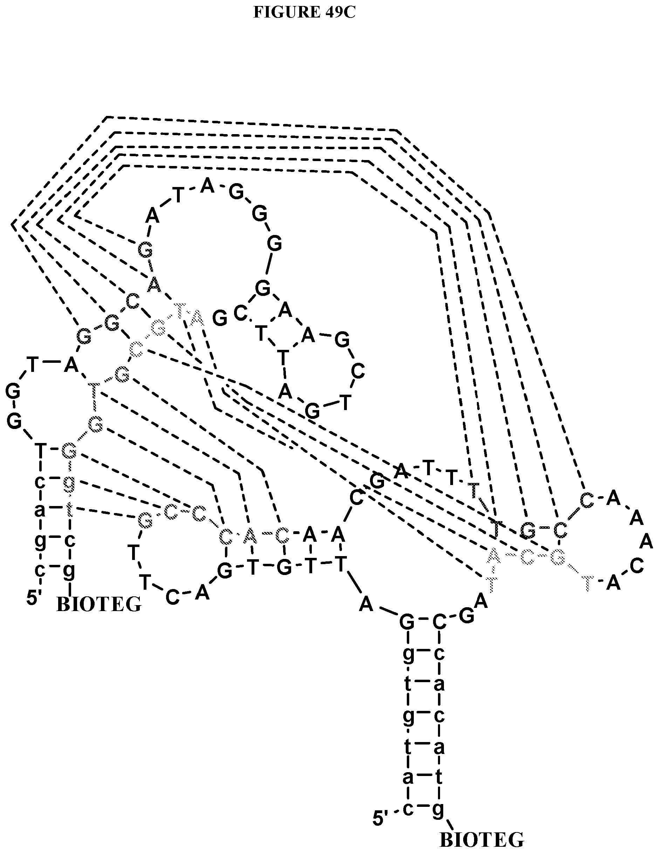

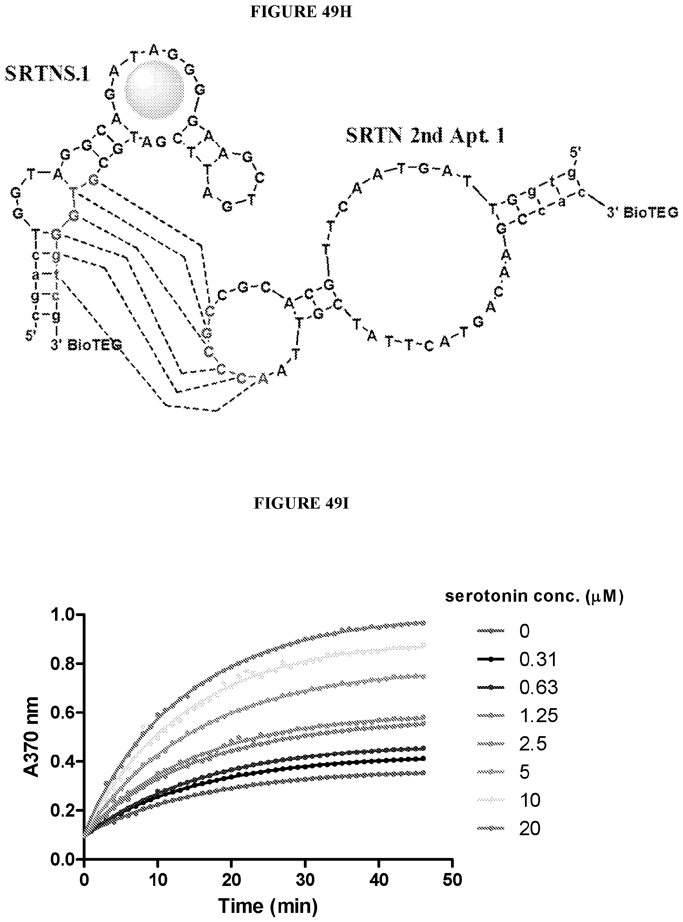

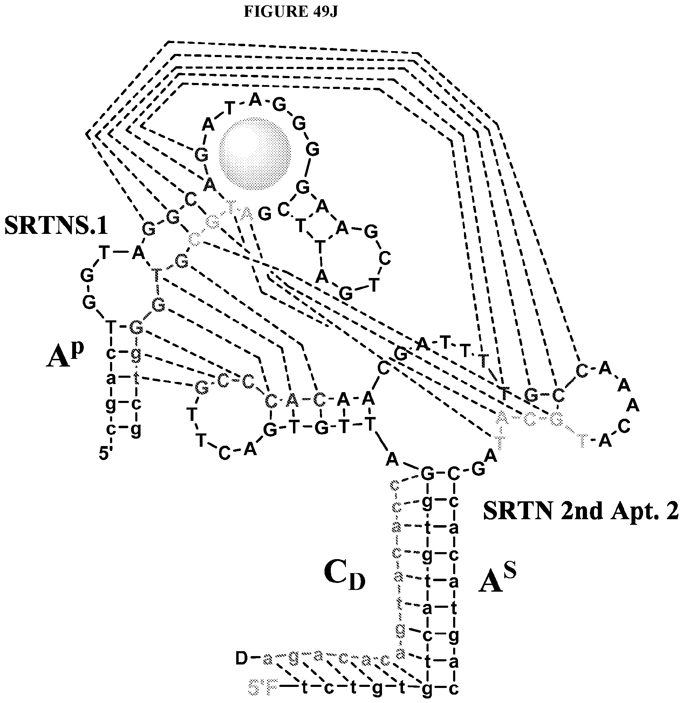

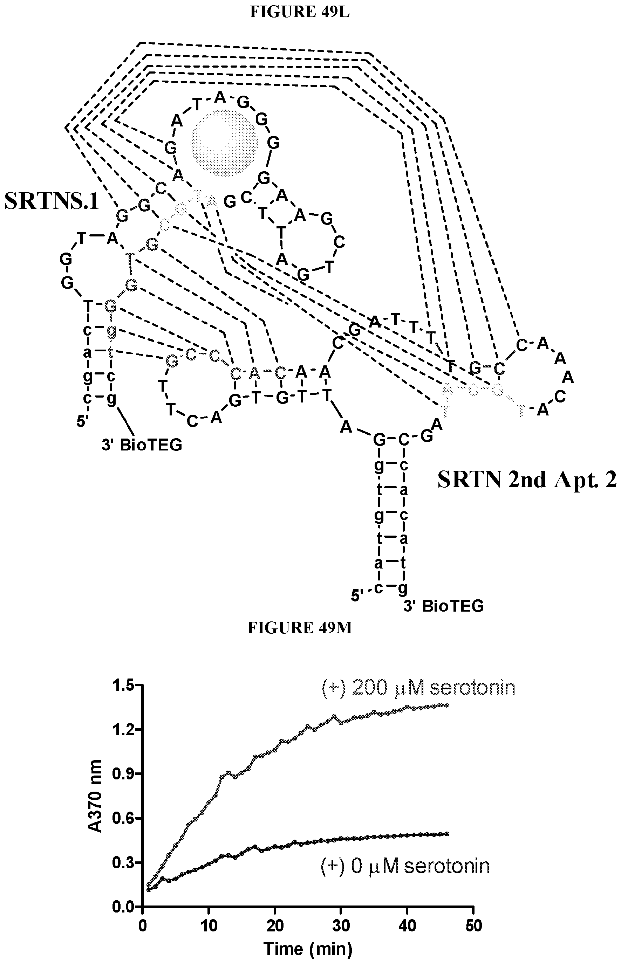

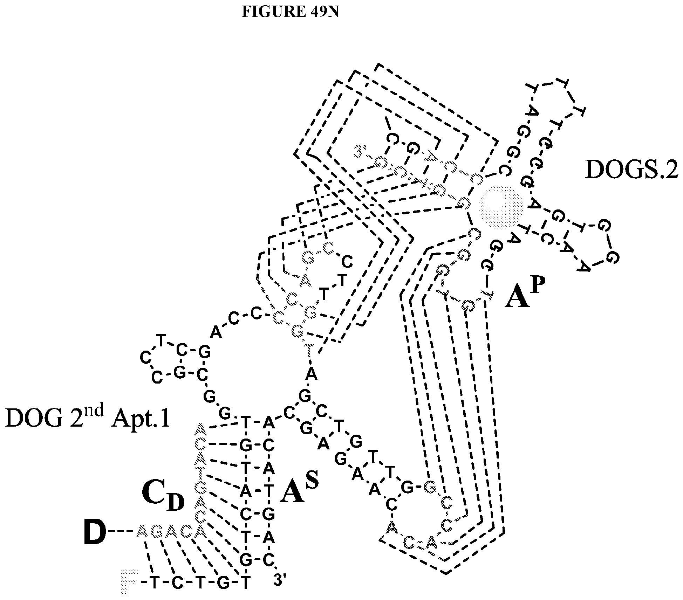

FIGURE 49A-Q. Exemplary Sandwich assays. Figure 49C discloses SEQ ID NOS 310-311, respectively, in order of appearance. Figure 49H discloses SEQ ID NOS 312-313, respectively, in order of appearance. Figure 49J discloses SEQ ID NOS 314-316, respectively, in order of appearance. Figure 49L discloses SEQ ID NOS 317-318, respectively, in order of appearance. Figure 49N discloses SEQ ID NOS 319-321, respectively, in order of appearance.

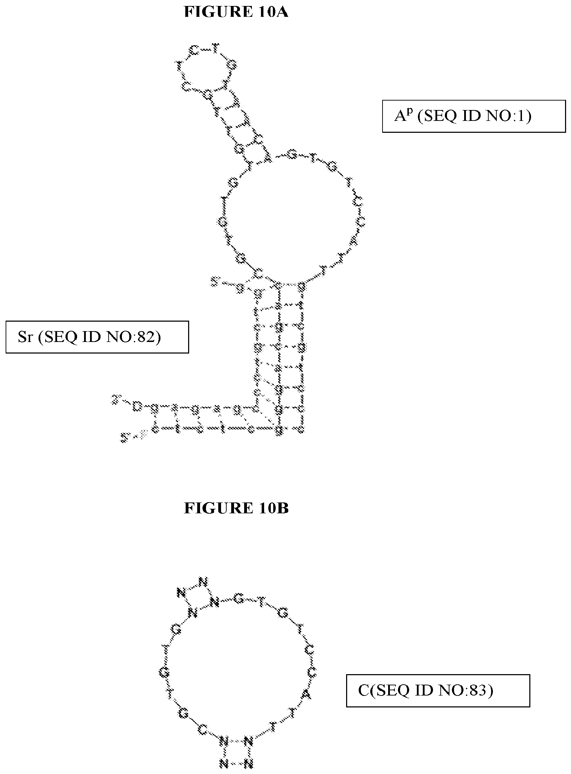

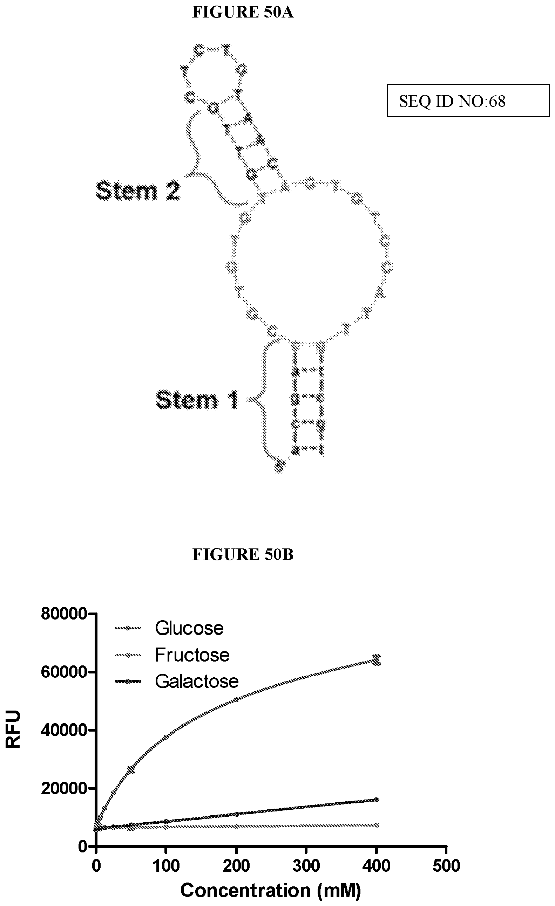

FIGURE 50A-B. (A) Glucose-binding primary aptamer (SEQ ID NO:68) showing stem and loop structures. (B) Dose/fluorescent response curve resulting from a primary aptamer comprising SEQ ID NO:68 and additional operative sequence complementary to a sensor oligonucleotide analogous to FIGURE 10A-C, showing selective binding to glucose (top curve) versus galactose (middle curve) or fructose (bottom curve).

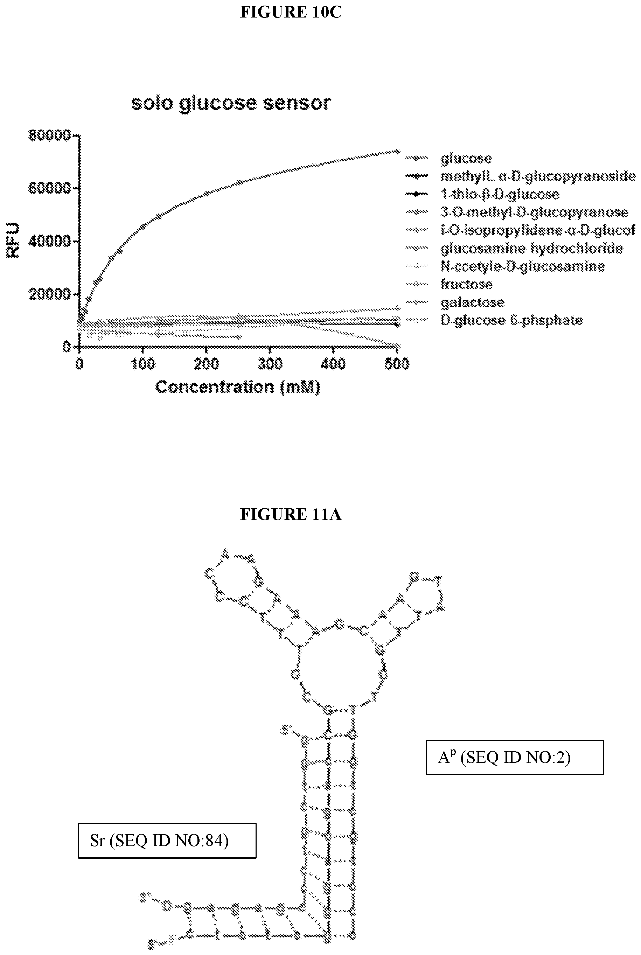

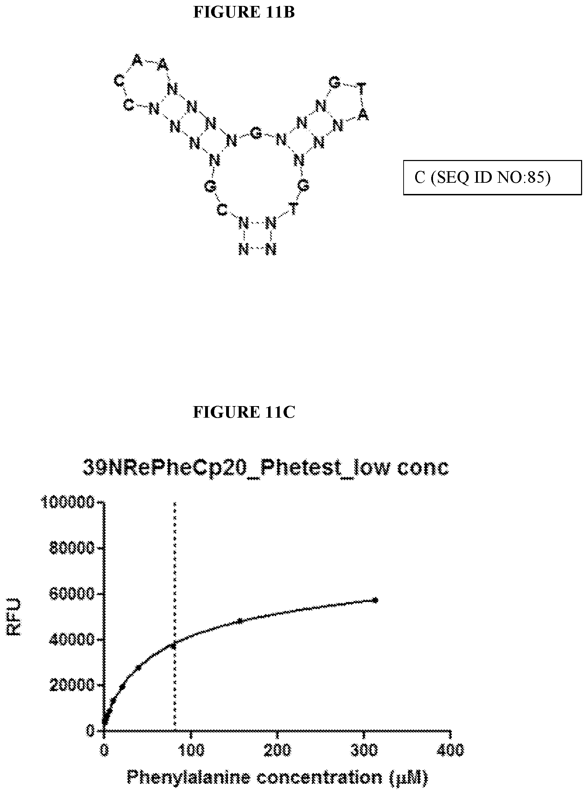

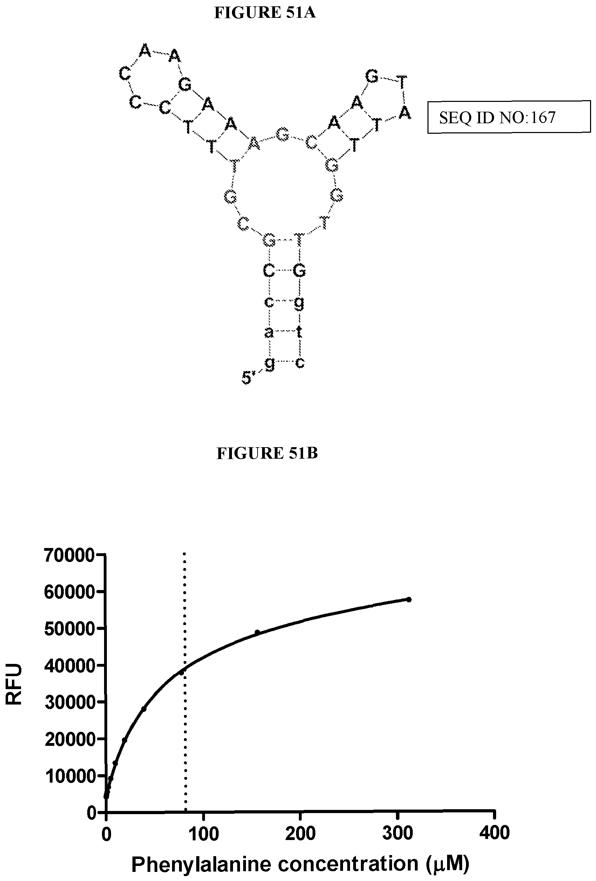

FIGURE 51A-B. (A) Phenylalanine-binding primary aptamer (SEQ ID NO:167) showing stem and loop structures. (B) Dose/fluorescent response curve resulting from a primary aptamer comprising SEQ ID NO:167 and additional operative sequence complementary to a sensor oligonucleotide analogous to FIGURE 11A-C, showing binding to phenylalanine.

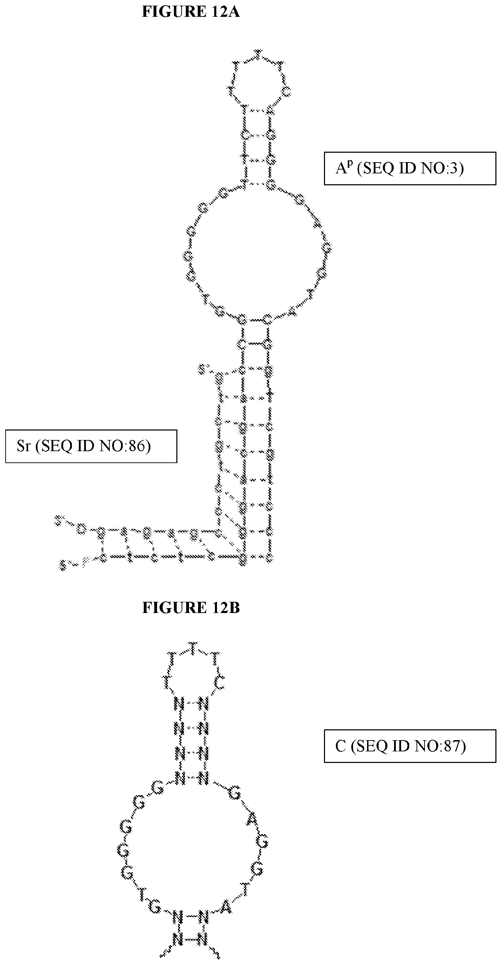

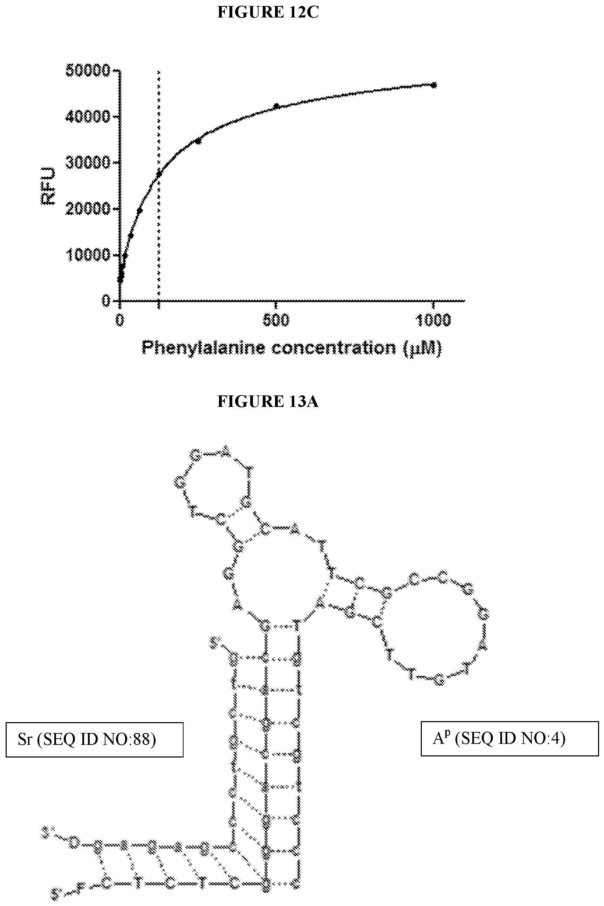

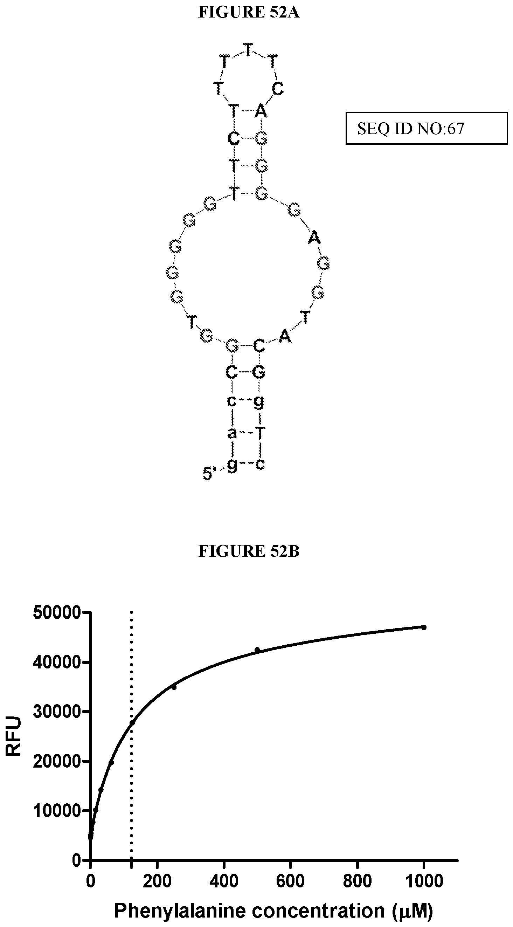

FIGURE 52A-B. (A) Phenylalanine-binding primary aptamer (SEQ ID NO:67) showing stem and loop structures. (B) Dose/fluorescent response curve resulting from a primary aptamer comprising SEQ ID NO:67 and additional operative sequence complementary to a sensor oligonucleotide analogous to FIGURE 12A-C, showing binding to phenylalanine.

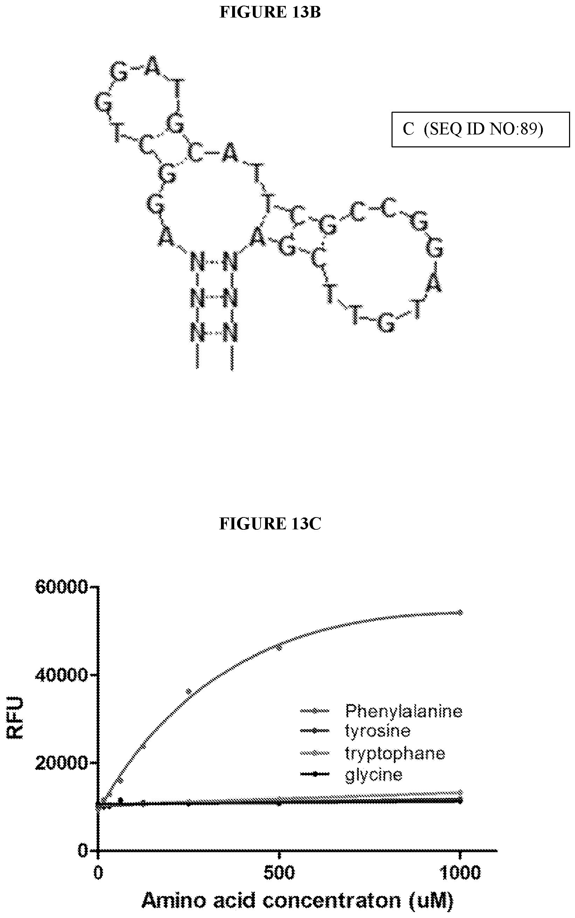

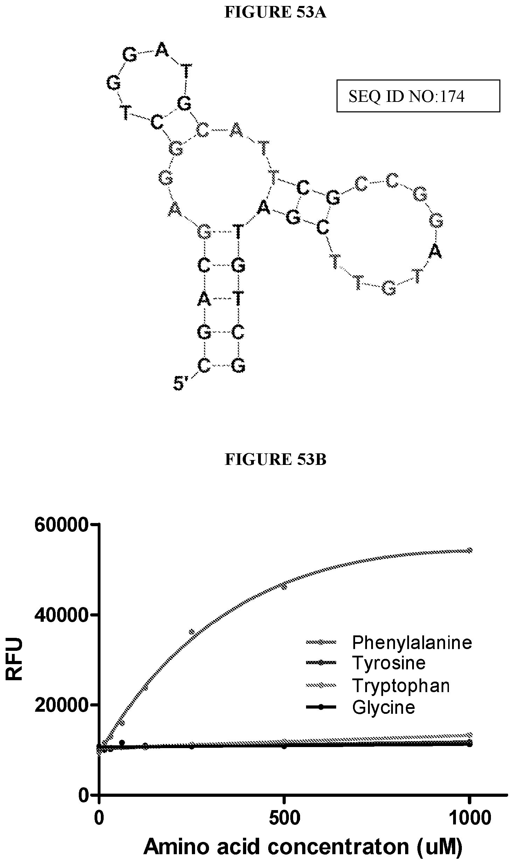

FIGURE 53A-B. (A) Phenylalanine-binding primary aptamer (SEQ ID NO:174) showing stem and loop structures. (B) Dose/fluorescent response curve resulting from a primary aptamer comprising SEQ ID NO:174 and additional operative sequence complementary to a sensor oligonucleotide analogous to FIGURE 13A-C, showing binding to phenylalanine (top curve) relative to tyrosine, glycine and tryptophan.

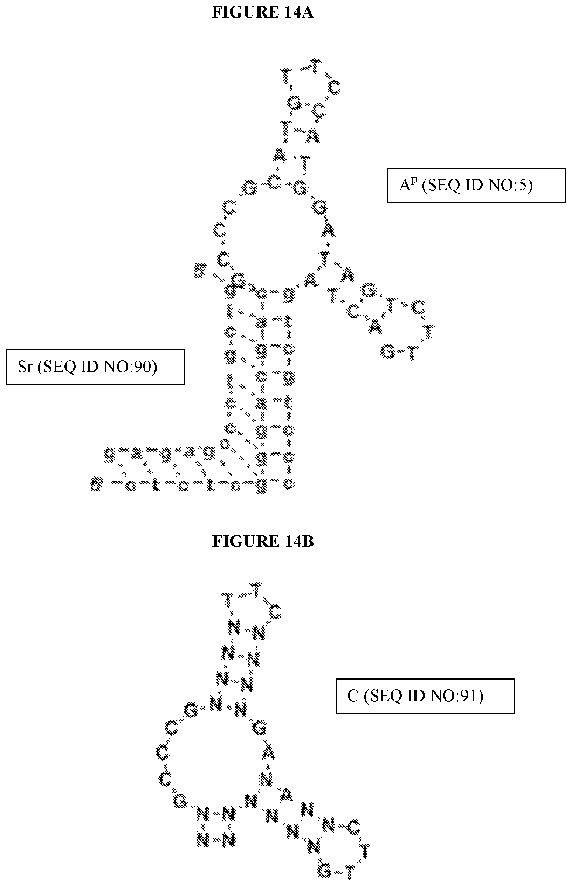

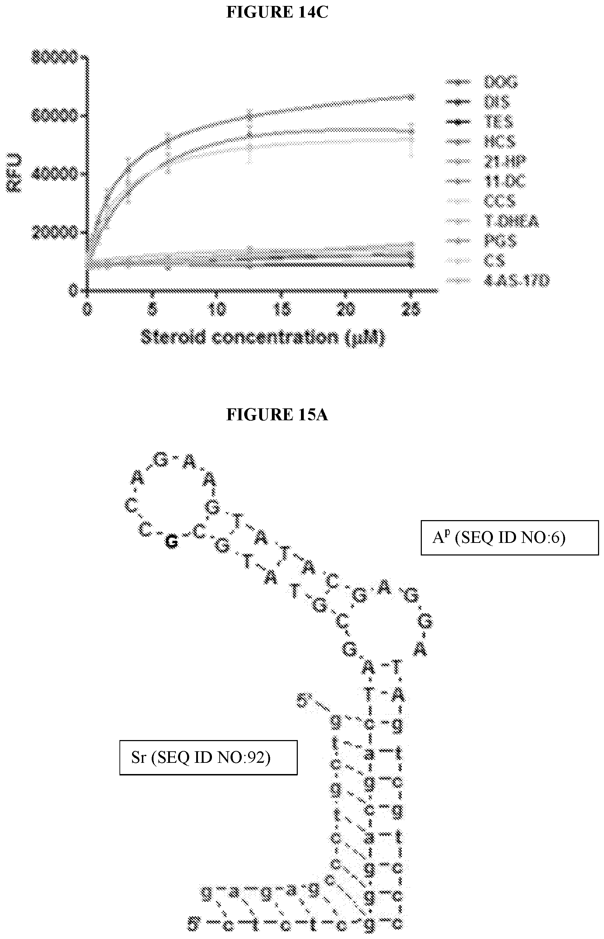

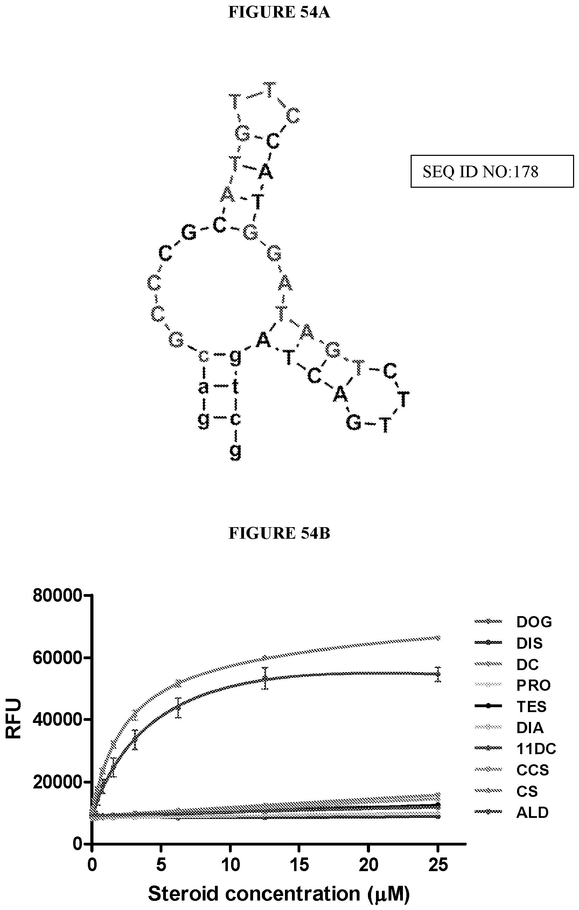

FIGURE 54A-B. (A) Hydrocortisone-binding primary aptamer (SEQ ID NO:178) showing stem and loop structures. (B) Dose/fluorescent response curve resulting from a primary aptamer comprising SEQ ID NO:178 and additional operative sequence complementary to a sensor oligonucleotide analogous to FIGURE 14A-C, showing binding to hydrocortisone (top curve) relative to other steroids.

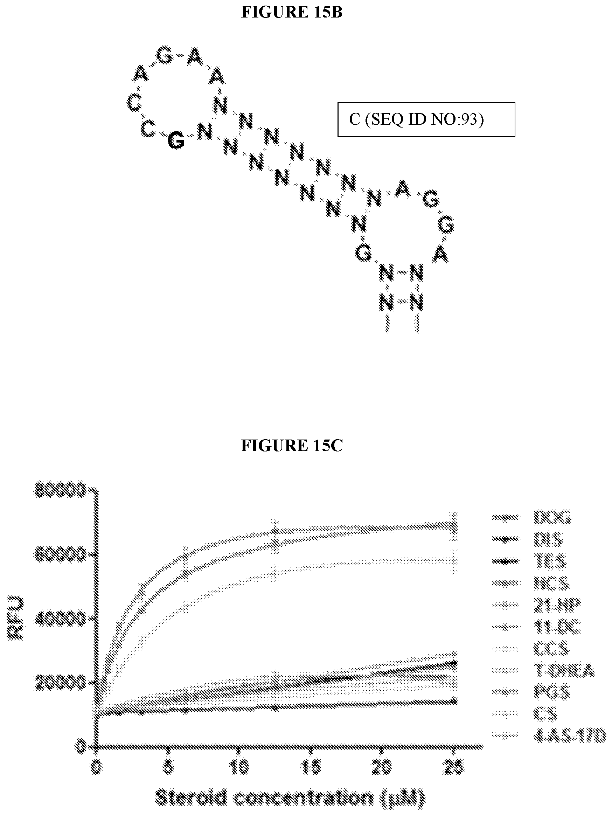

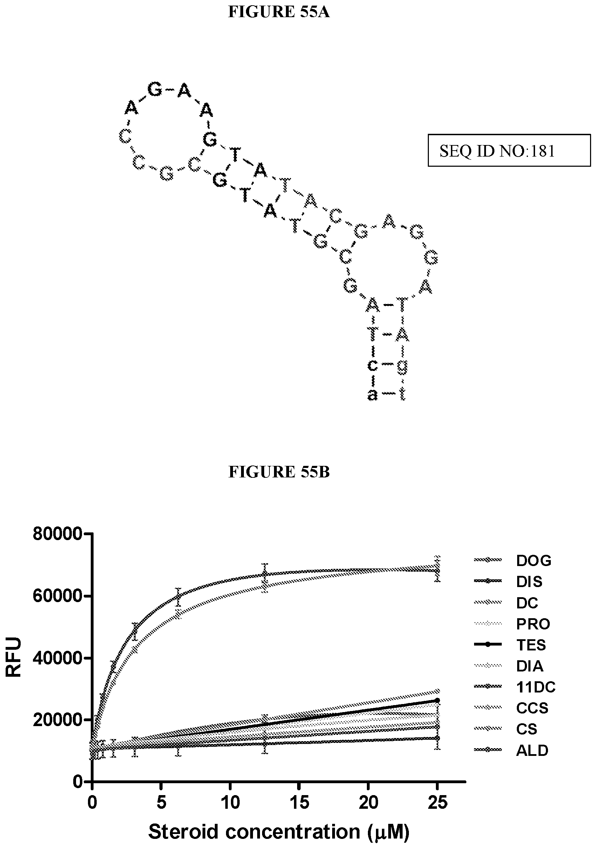

FIGURE 55A-B. (A) Hydrocortisone-binding primary aptamer (SEQ ID NO:181) showing stem and loop structures. (B) Dose/fluorescent response curve resulting from a primary aptamer comprising SEQ ID NO:181 and additional operative sequence complementary to a sensor oligonucleotide analogous to FIGURE 15A-C, showing binding to hydrocortisone (top curve) relative to other steroids.

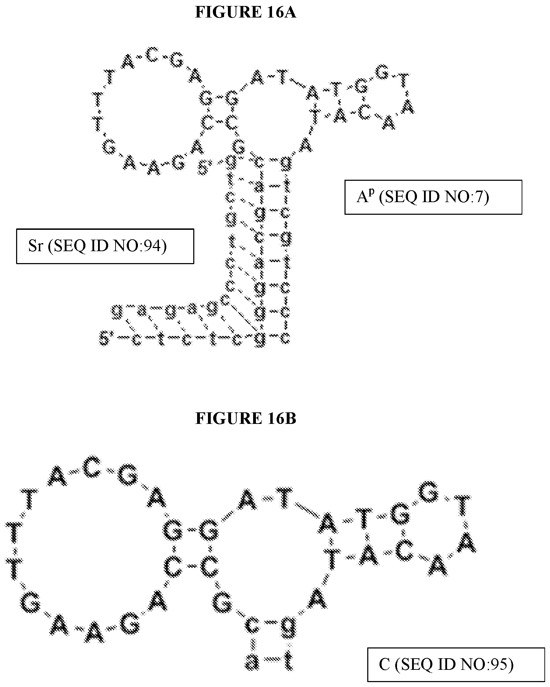

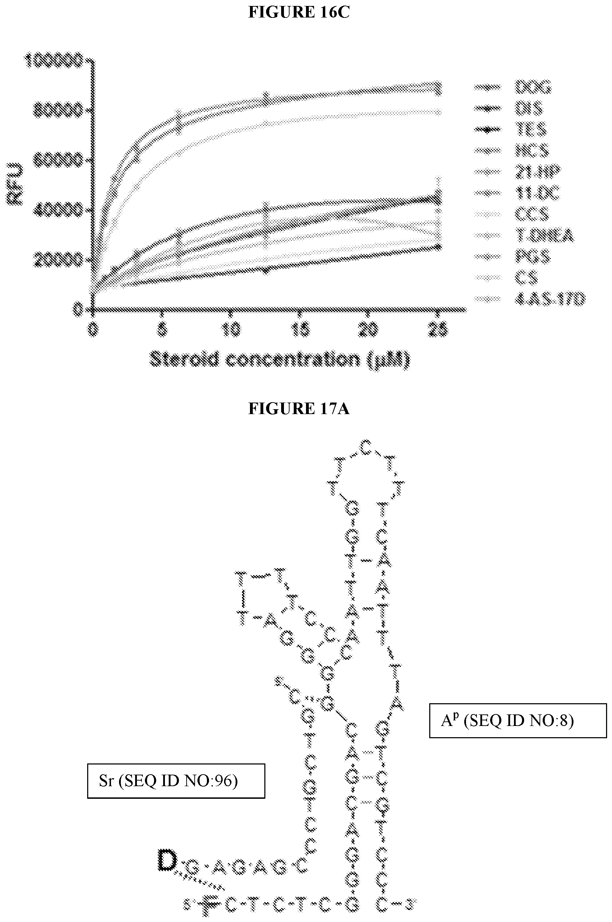

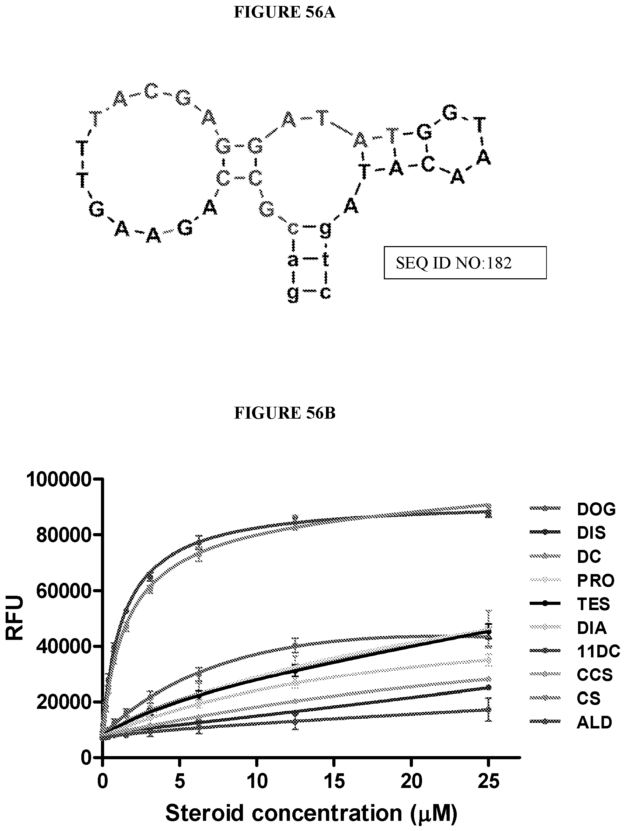

FIGURE 56A-B. (A) Hydrocortisone-binding primary aptamer (SEQ ID NO:182) showing stem and loop structures. (B) Dose/fluorescent response curve resulting from a primary aptamer comprising SEQ ID NO:182 and additional operative sequence complementary to a sensor oligonucleotide analogous to FIGURE 16A-C, showing binding to hydrocortisone (top curve) relative to other steroids.

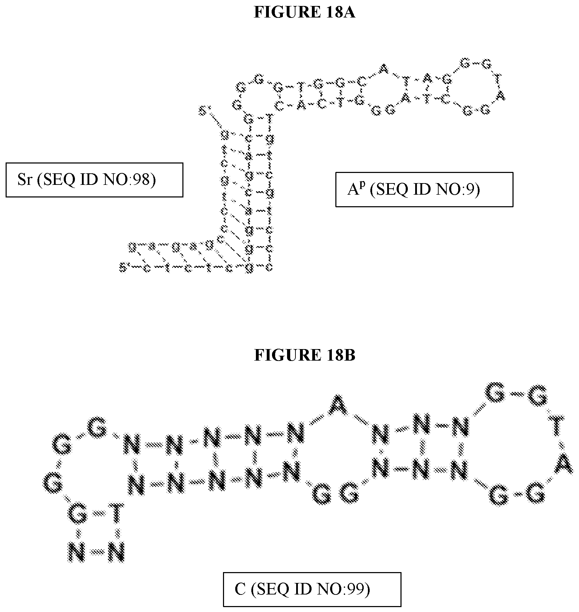

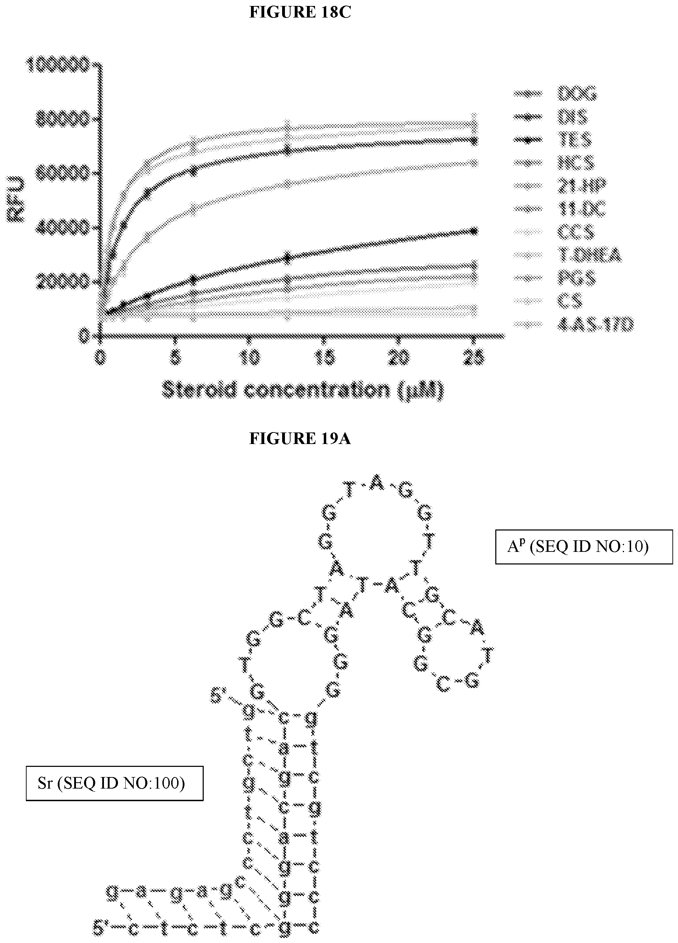

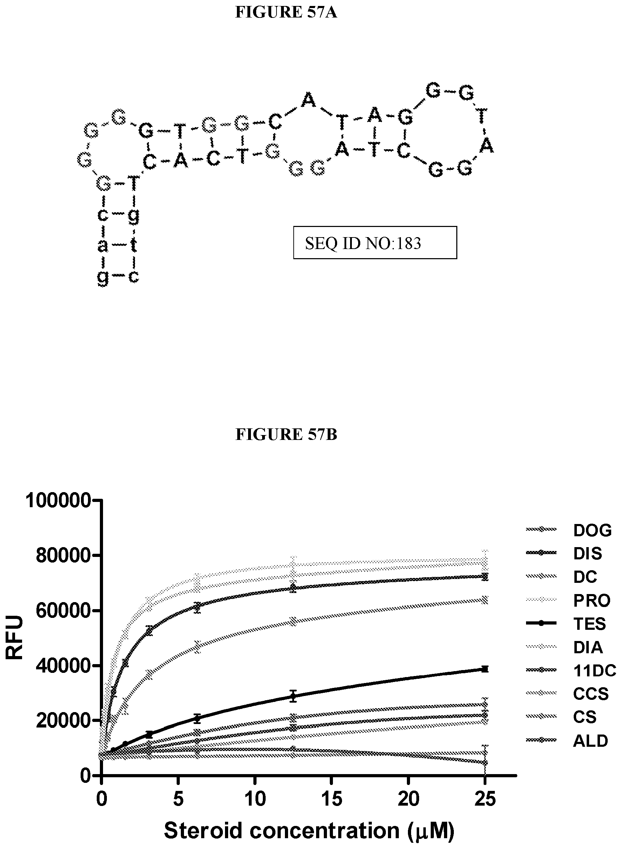

FIGURE 57A-B. (A) Dehydroisoandrosterone-binding primary aptamer (SEQ ID NO: 183) showing stem and loop structures. (B) Dose/fluorescent response curve resulting from a primary aptamer comprising SEQ ID NO:183 and additional operative sequence complementary to a sensor oligonucleotide analogous to FIGURE 18A-C, showing binding to dehydroisoandrosterone(top curve) relative to other steroids.

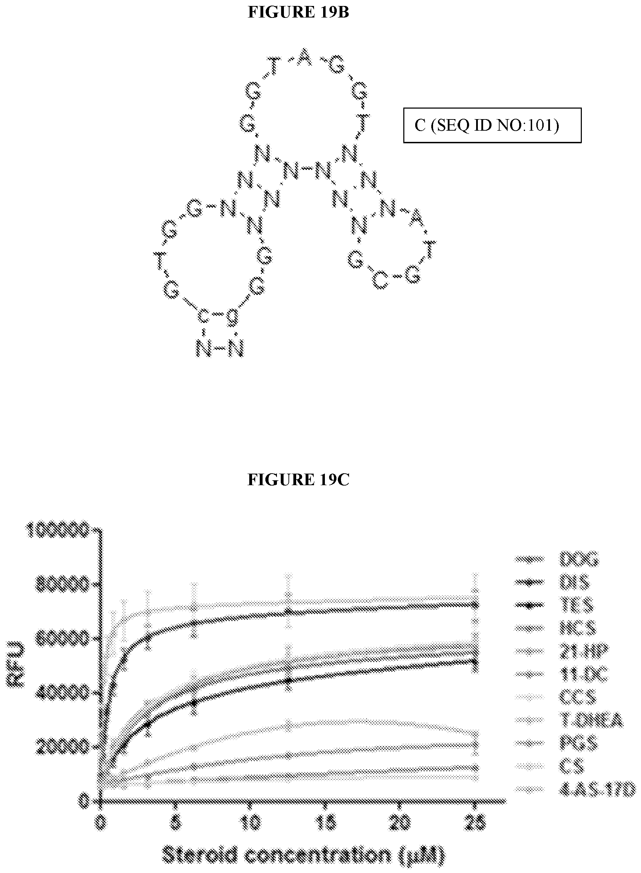

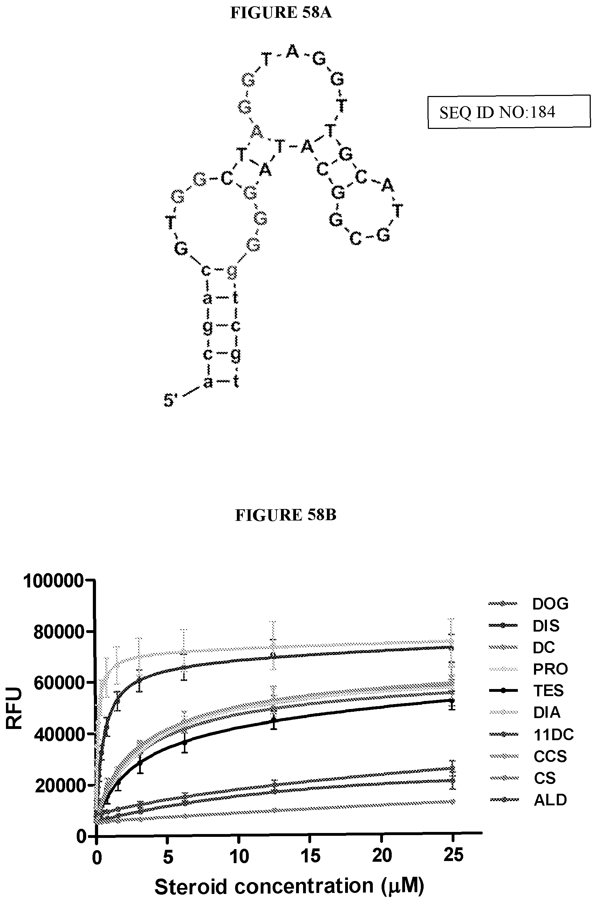

FIGURE 58A-B. (A) Dehydroisoandrosterone-binding primary aptamer (SEQ ID NO: 184) showing stem and loop structures. (B) Dose/fluorescent response curve resulting from a primary aptamer comprising SEQ ID NO:184 and additional operative sequence complementary to a sensor oligonucleotide analogous to FIGURE 19A-C, showing binding to dehydroisoandrosterone (top curve) relative to other steroids.

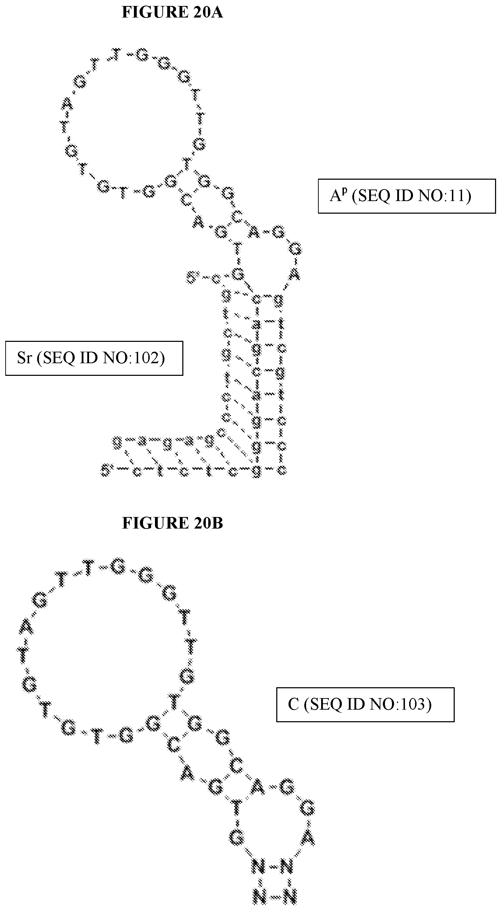

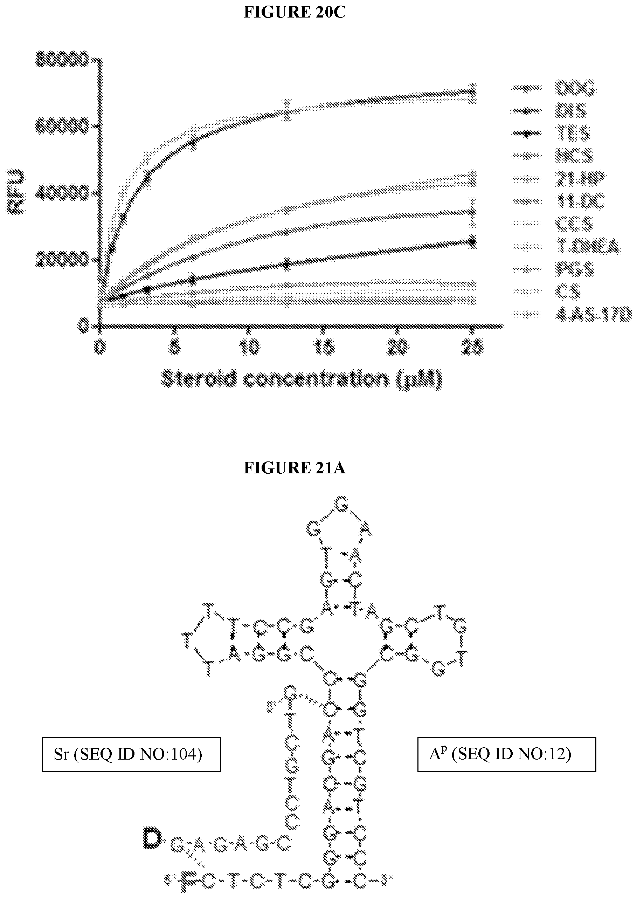

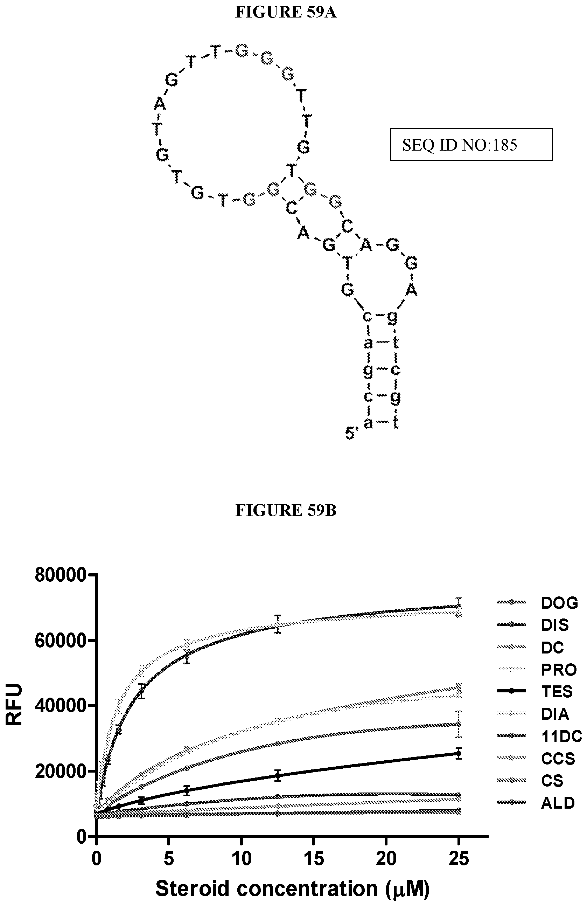

FIGURE 59A-B. (A) Dehydroisoandrosterone-binding primary aptamer (SEQ ID NO: 185) showing stem and loop structures. (B) Dose/fluorescent response curve resulting from a primary aptamer comprising SEQ ID NO:185 and additional operative sequence complementary to a sensor oligonucleotide analogous to FIGURE 20A-C, showing binding to dehydroisoandrosterone (top curve) relative to other steroids.

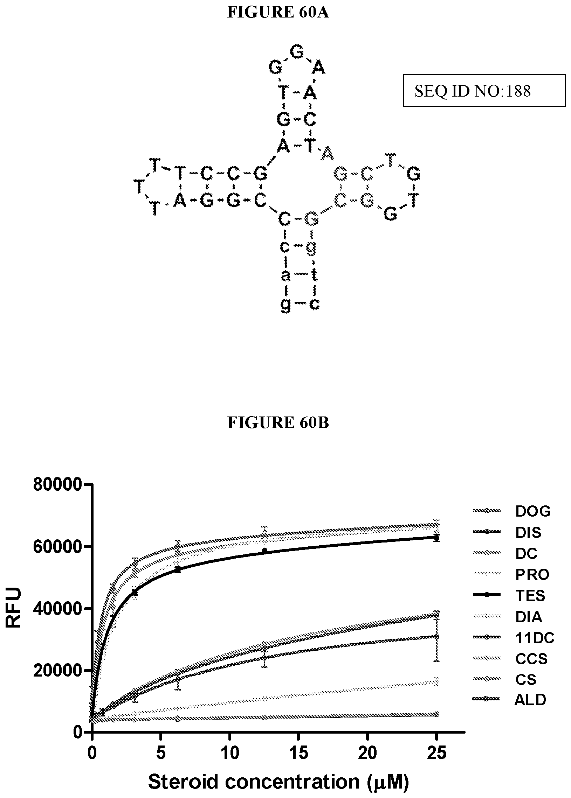

FIGURE 60A-B. (A) Deoxycorticosterone-binding primary aptamer (SEQ ID NO: 188) showing stem and loop structures. (B) Dose/fluorescent response curve resulting from a primary aptamer comprising SEQ ID NO:188 and additional operative sequence complementary to a sensor oligonucleotide analogous to FIGURE 21A-C, showing binding to deoxycorticosterone (top curve) relative to other steroids.

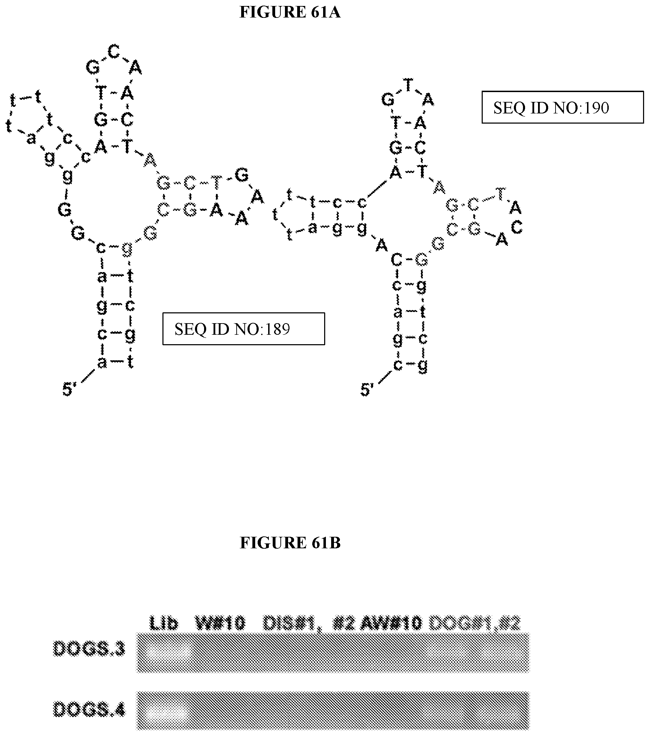

FIGURE 61A-B. (A) Deoxycorticosterone-binding primary aptamers (SEQ ID NOS:189 and 190) showing stem and loop structures. (B) Gel analysis showing selective binding.

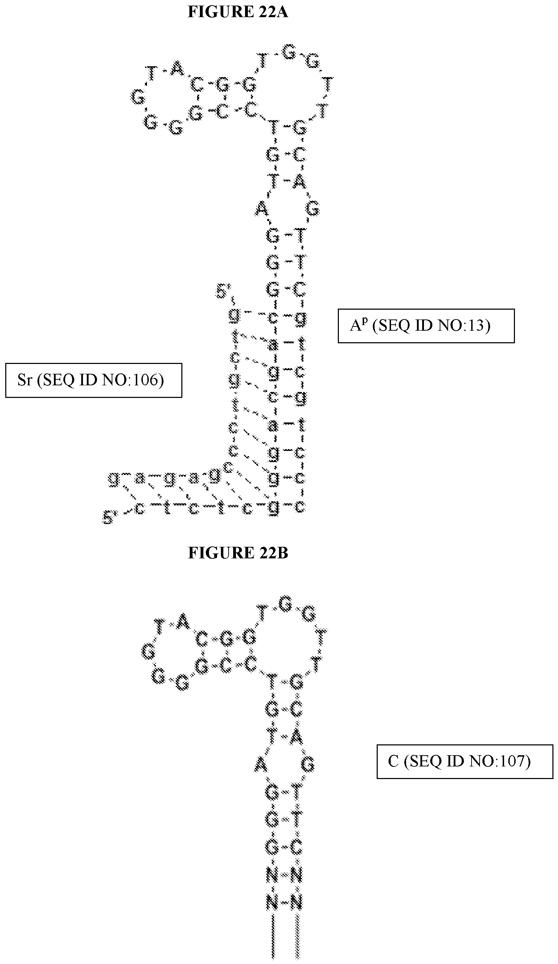

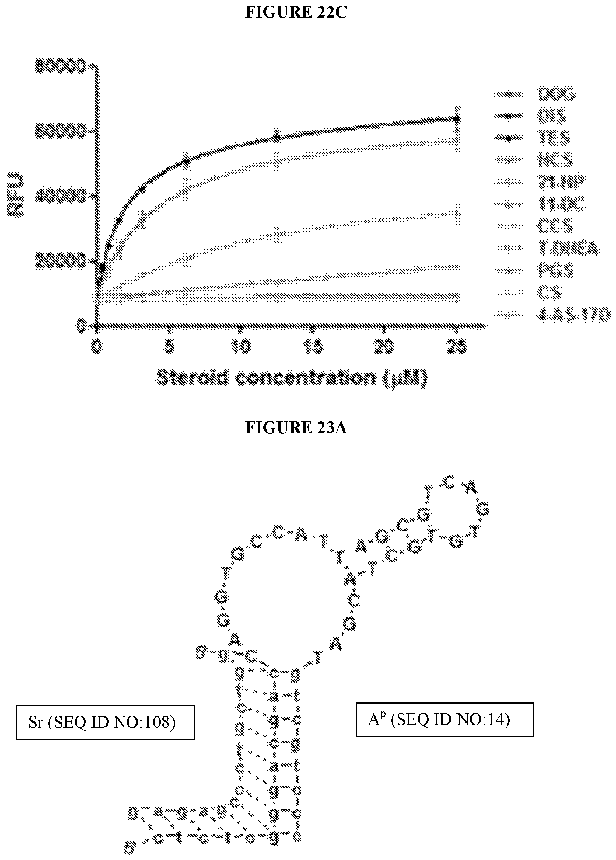

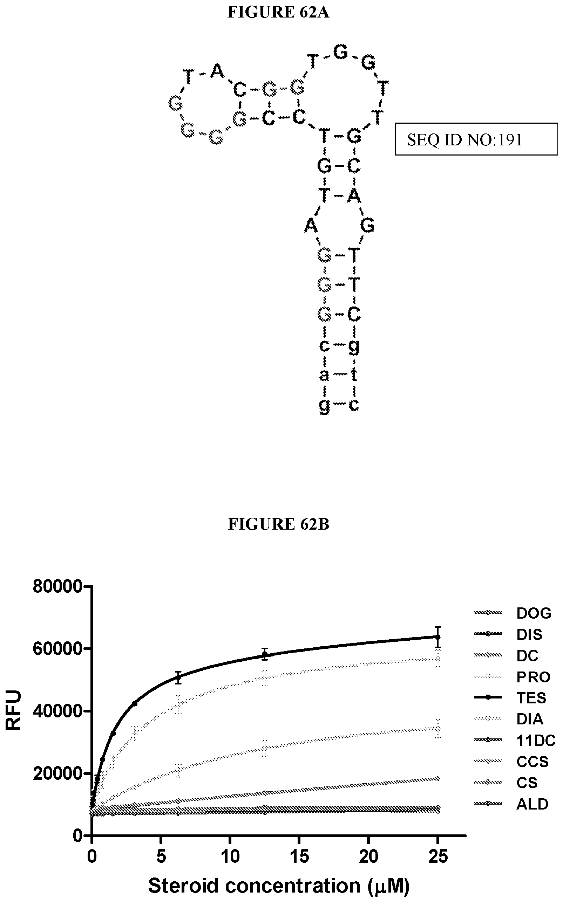

FIGURE 62A-B. (A) Testosterone-binding primary aptamer (SEQ ID NO:191) showing stem and loop structures. (B) Dose/fluorescent response curve resulting from a primary aptamer comprising SEQ ID NO:191 and additional operative sequence complementary to a sensor oligonucleotide analogous to FIGURE 22A-C, showing binding to testosterone (top curve) relative to other steroids.

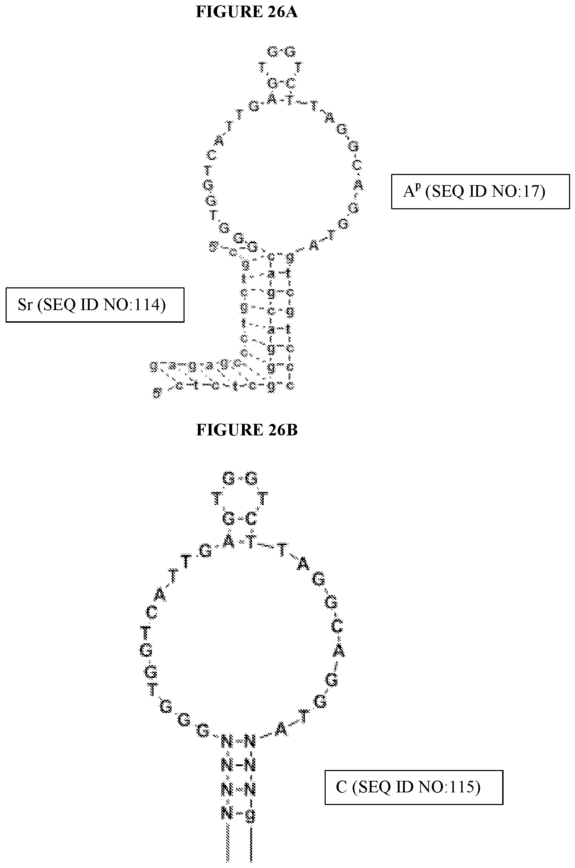

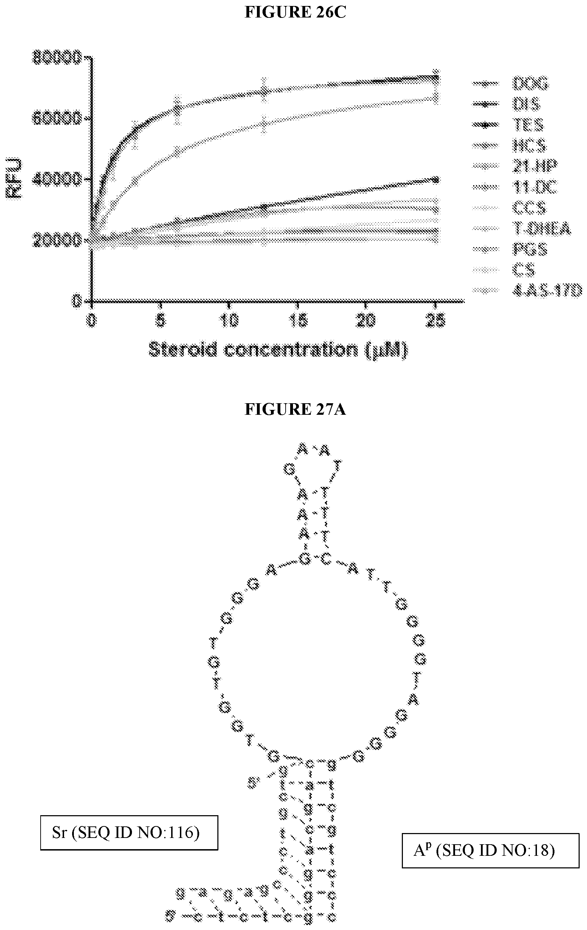

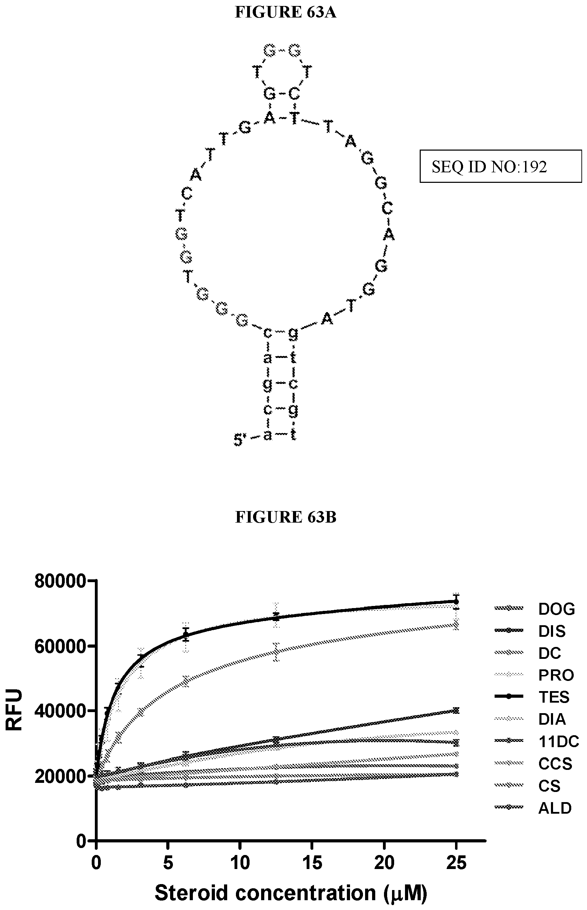

FIGURE 63A-B. (A) Testosterone-binding primary aptamer (SEQ ID NO:192) showing stem and loop structures. (B) Dose/fluorescent response curve resulting from a primary aptamer comprising SEQ ID NO:192 and additional operative sequence complementary to a sensor oligonucleotide analogous to FIGURE 26A-C, showing binding to testosterone (top curve) relative to other steroids.

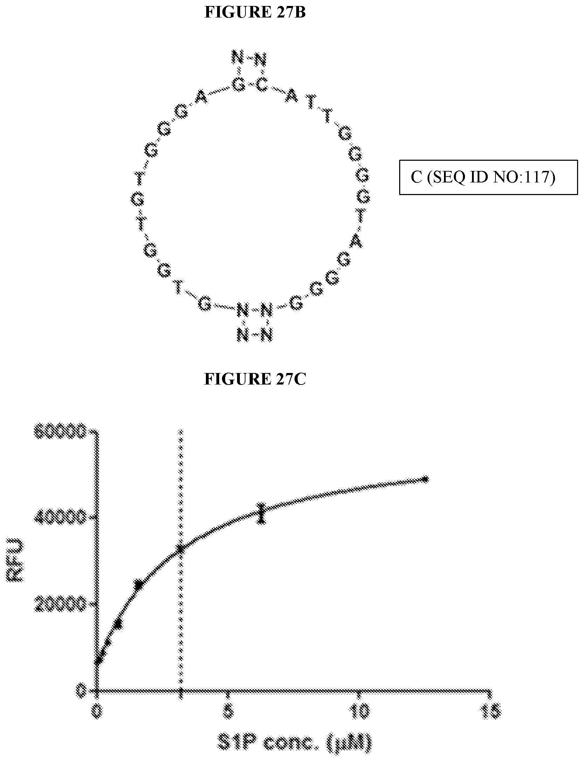

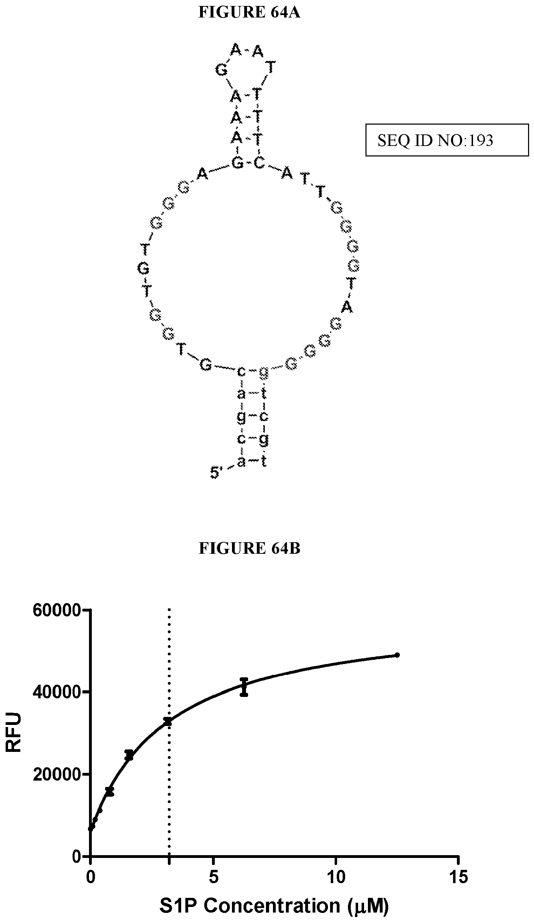

FIGURE 64A-B. (A) Sphingosine-1-phosphate-binding primary aptamer (SEQ ID NO: 193) showing stem and loop structures. (B) Dose/fluorescent response curve resulting from a primary aptamer comprising SEQ ID NO: 13 and additional operative sequence complementary to a sensor oligonucleotide analogous to FIGURE 27A-C, showing binding to sphingosine-1-phosphate.

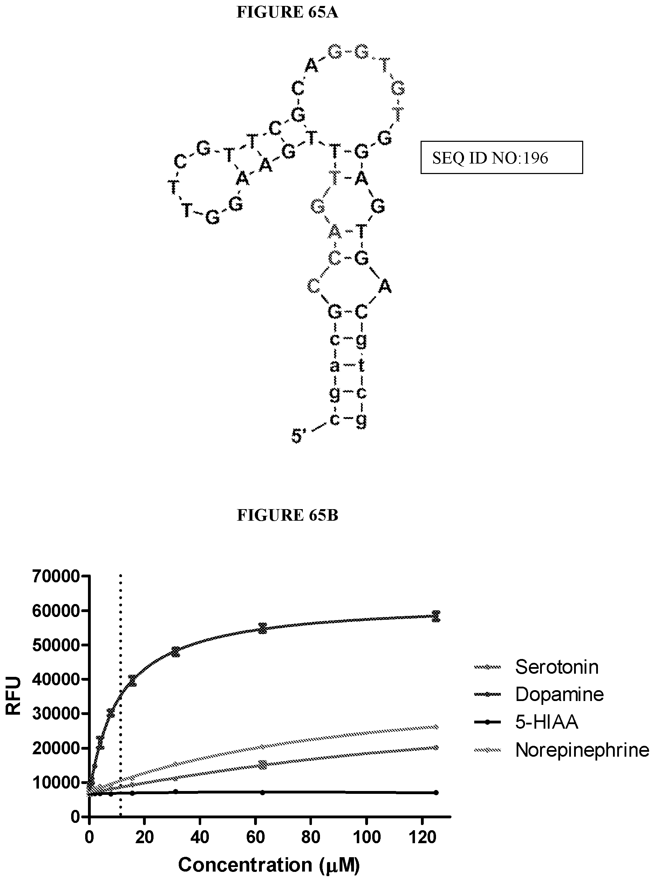

FIGURE 65A-B. (A) Dopamine-binding primary aptamer (SEQ ID NO:196) showing stem and loop structures. (B) Dose/fluorescent response curve resulting from a primary aptamer comprising SEQ ID NO:196 and additional operative sequence complementary to a sensor oligonucleotide analogous to FIGURE 30A-C, showing binding to dopamine (top curve) relative to (curves in descending order) norephinephrine, serotonin and 5-HIAA.

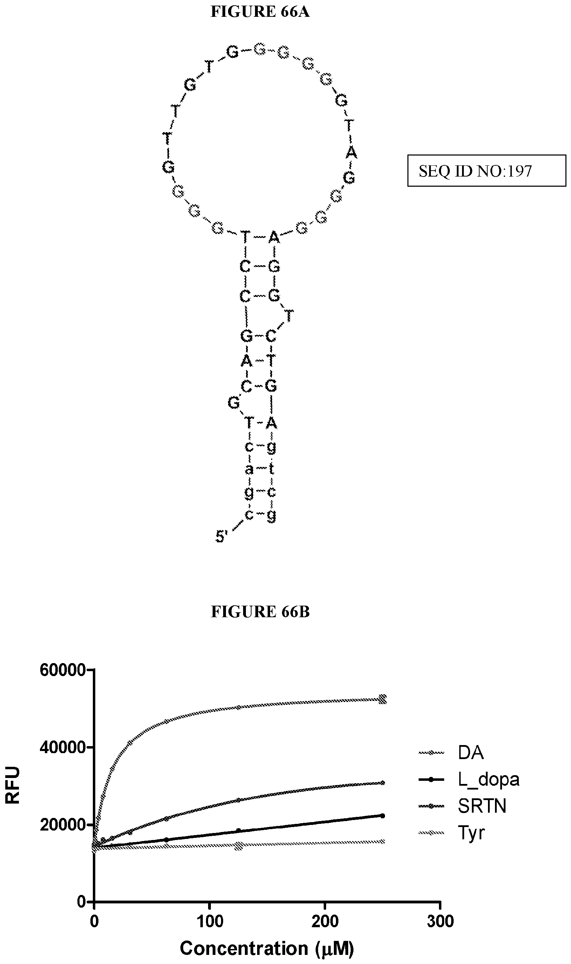

FIGURE 66A-B. (A) Dopamine-binding primary aptamer (SEQ ID NO:197) showing stem and loop structures. (B) Dose/fluorescent response curve resulting from a primary aptamer comprising SEQ ID NO:197 and additional operative sequence complementary to a sensor oligonucleotide analogous to FIGURE 31A-C, showing binding to dopamine (top curve) relative to (curves in descending order) L-dopa, serotonin and tyrosine.

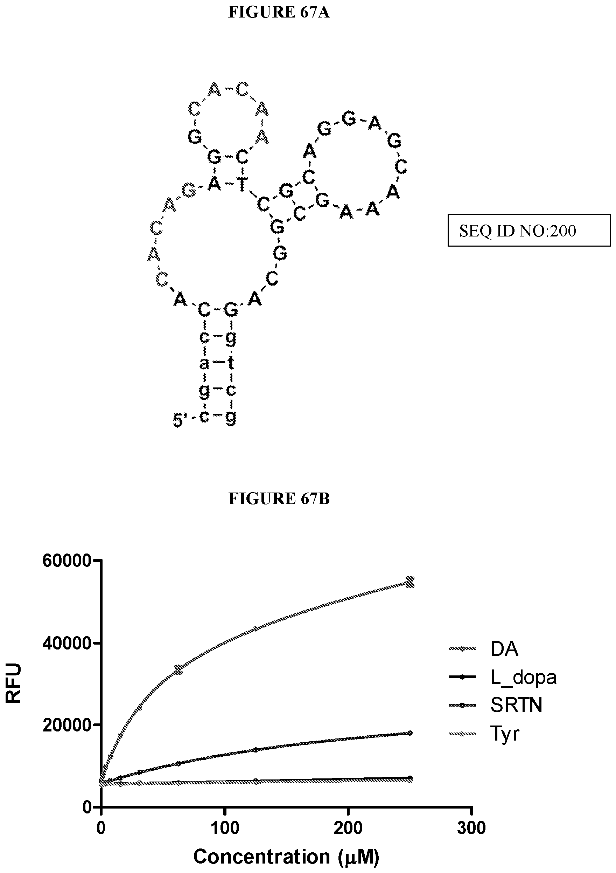

FIGURE 67A-B. (A) Dopamine-binding primary aptamer (SEQ ID NO:200) showing stem and loop structures. (B) Dose/fluorescent response curve resulting from a primary aptamer comprising SEQ ID NO:200 and additional operative sequence complementary to a sensor oligonucleotide analogous to FIGURE 32A-C, showing binding to dopamine (top curve) relative to (curves in descending order) serotonin and tyrosine.

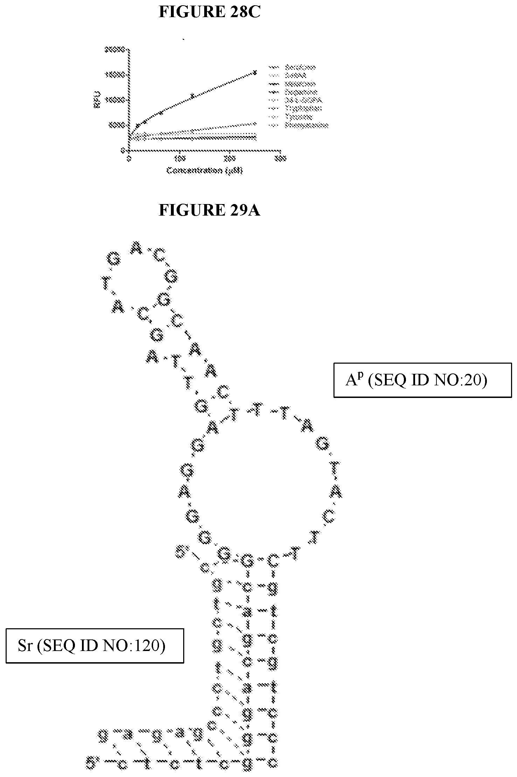

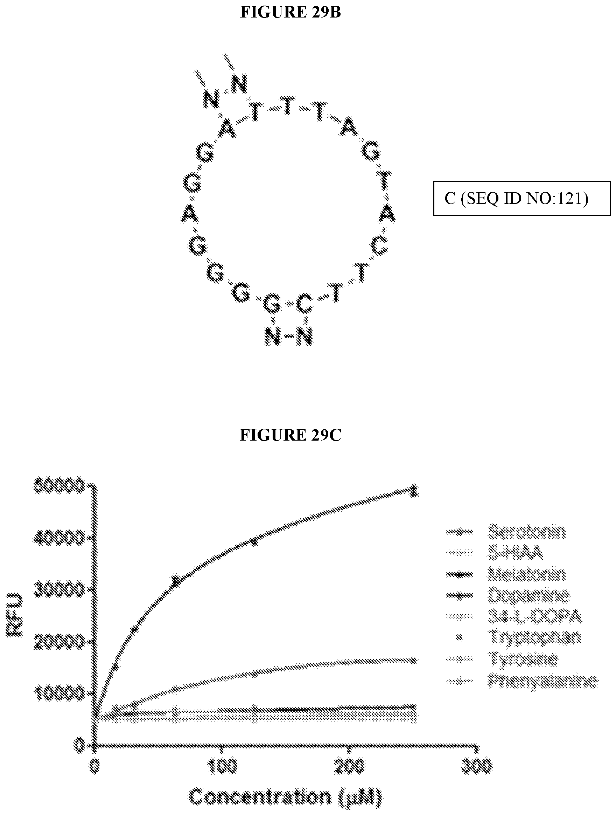

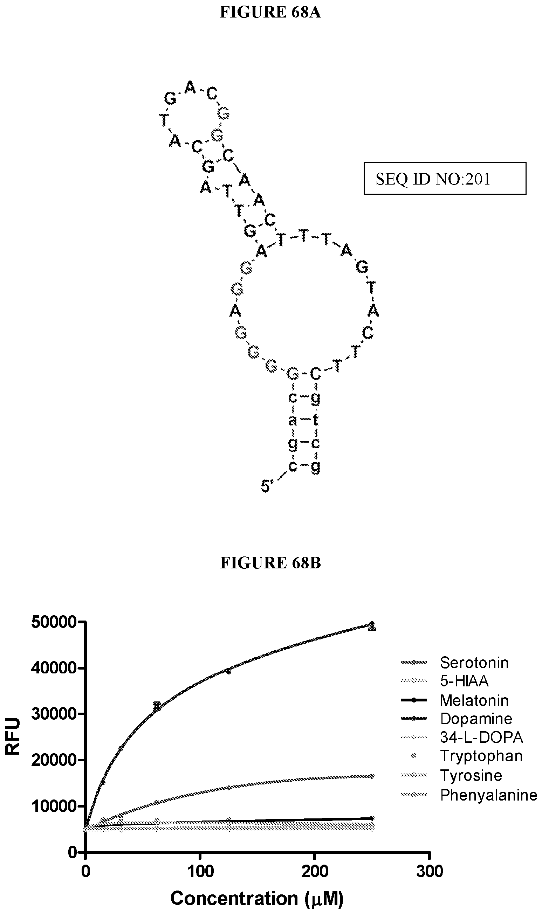

FIGURE 68A-B. (A) Dopamine-binding primary aptamer (SEQ ID NO:201) showing stem and loop structures. (B) Dose/fluorescent response curve resulting from a primary aptamer comprising SEQ ID NO:201 and additional operative sequence complementary to a sensor oligonucleotide analogous to FIGURE 29A-C, showing binding to dopamine (top curve) relative to (curves in descending order) L-dopa, serotonin and then various others, including tyrosine and melatonin.

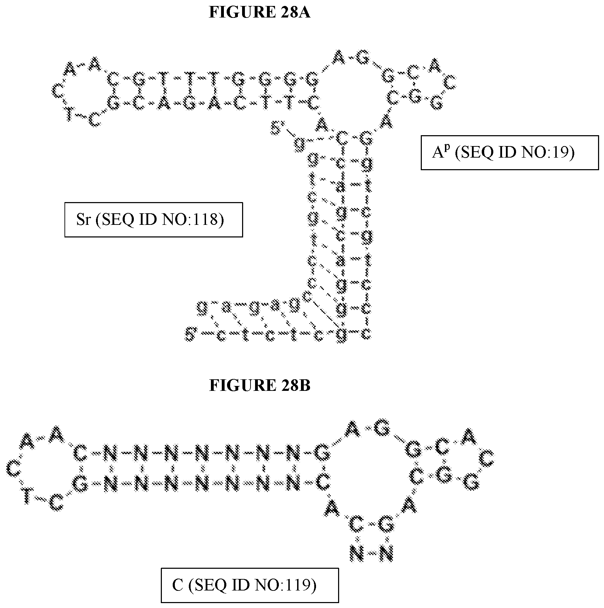

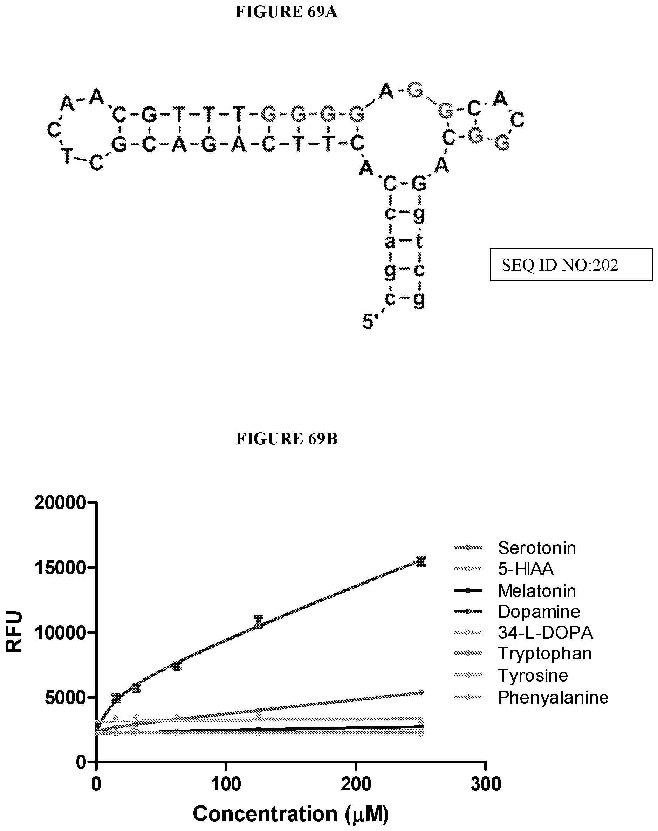

FIGURE 69A-B. (A) Dopamine-binding primary aptamer (SEQ ID NO:202) showing stem and loop structures. (B) Dose/fluorescent response curve resulting from a primary aptamer comprising SEQ ID NO:202 and additional operative sequence complementary to a sensor oligonucleotide analogous to FIGURE 28A-C, showing binding to dopamine (top curve) relative to (curves in descending order) serotonin and then various others, including tyrosine.

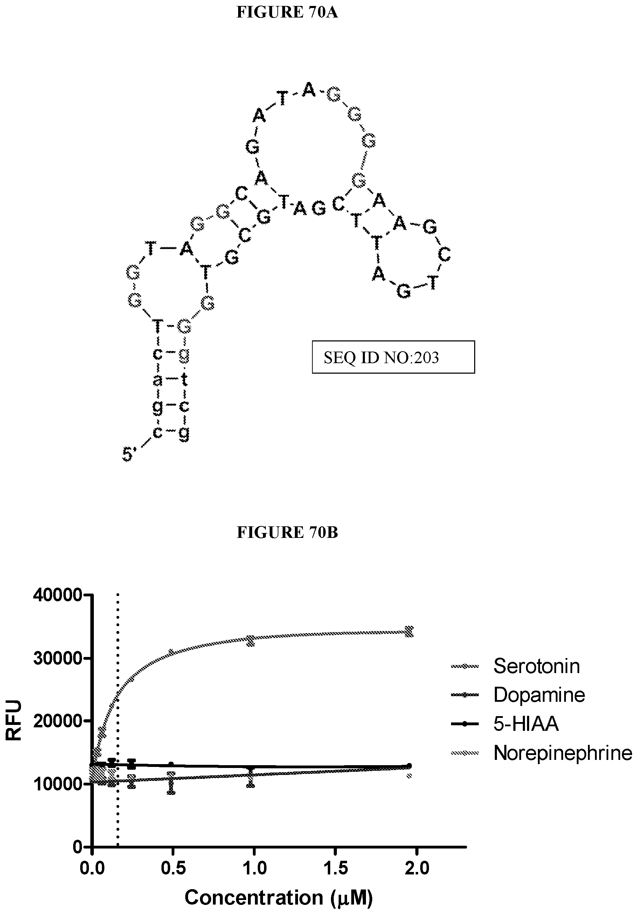

FIGURE 70A-B. (A) Serotonin-binding primary aptamer (SEQ ID NO:203) showing stem and loop structures. (B) Dose/fluorescent response curve resulting from a primary aptamer comprising SEQ ID NO:203 and additional operative sequence complementary to a sensor oligonucleotide analogous to FIGURE 34A-C, showing binding to serotonin (top curve) relative to (curves in descending order) H-IAA, norepinephrine and dopamine.

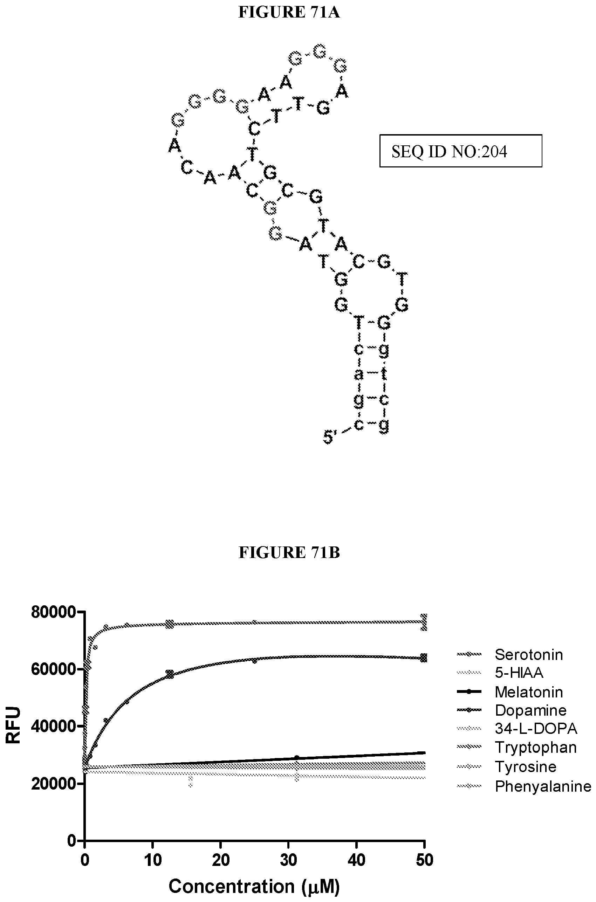

FIGURE 71A-B. (A) Serotonin-binding primary aptamer (SEQ ID NO:204) showing stem and loop structures. (B) Dose/fluorescent response curve resulting from a primary aptamer comprising SEQ ID NO:204 and additional operative sequence complementary to a sensor oligonucleotide analogous to FIGURE 37A-C, showing binding to serotonin (top curve) relative to (curves in descending order) dopamine and various other compounds.

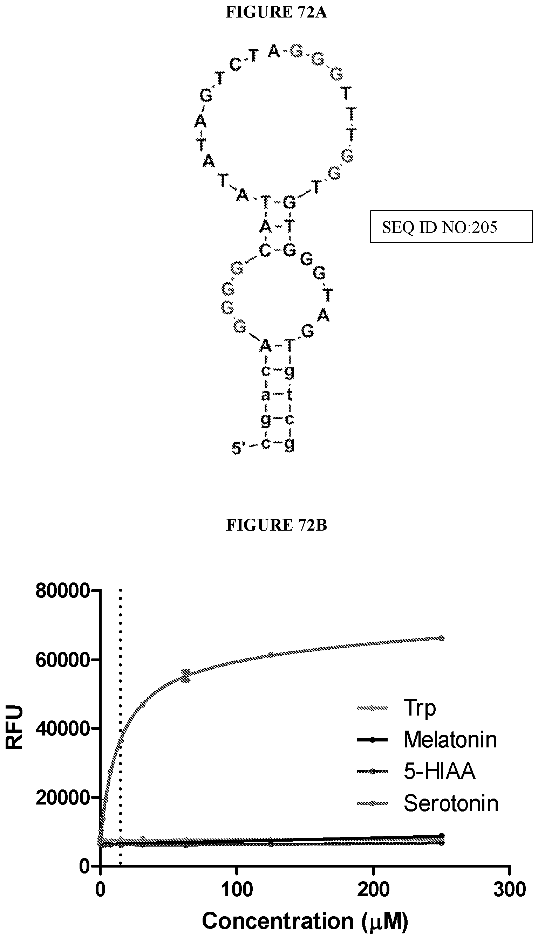

FIGURE 72A-B. (A) Serotonin-binding primary aptamer (SEQ ID NO:205) showing stem and loop structures. (B) Dose/fluorescent response curve resulting from a primary aptamer comprising SEQ ID NO:205 and additional operative sequence complementary to a sensor oligonucleotide analogous to FIGURE 33A-C, showing binding to serotonin (top curve) relative to e.g., melatonin, 5-HIAA and tryptophan.

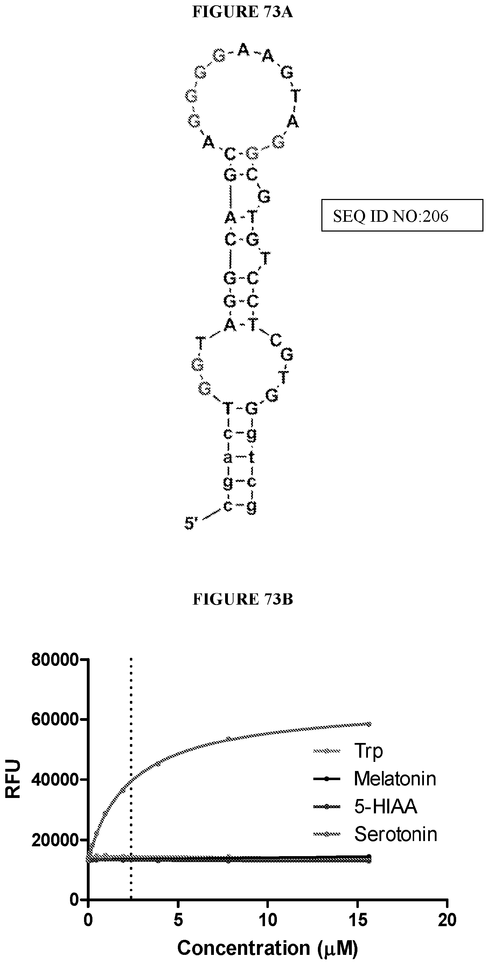

FIGURE 73A-B. (A) Serotonin-binding primary aptamer (SEQ ID NO:206) showing stem and loop structures. (B) Dose/fluorescent response curve resulting from a primary aptamer comprising SEQ ID NO:206 and additional operative sequence complementary to a sensor oligonucleotide analogous to FIGURE 35A-C, showing binding to serotonin (top curve) relative to e.g., melatonin, 5-HIAA and tryptophan.

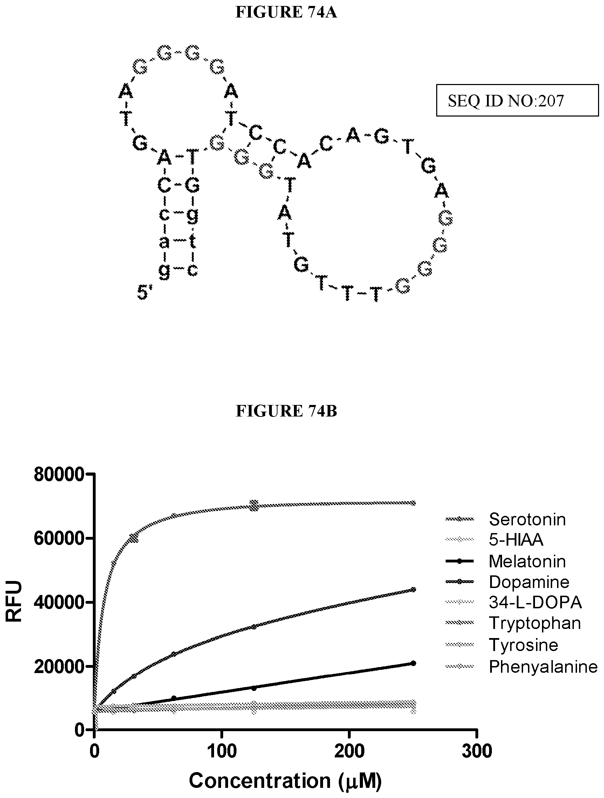

FIGURE 74A-B. (A) Serotonin-binding primary aptamer (SEQ ID NO:207) showing stem and loop structures. (B) Dose/fluorescent response curve resulting from a primary aptamer comprising SEQ ID NO:207 and additional operative sequence complementary to a sensor oligonucleotide analogous to FIGURE 36A-C, showing binding to serotonin (top curve) relative to other compounds.

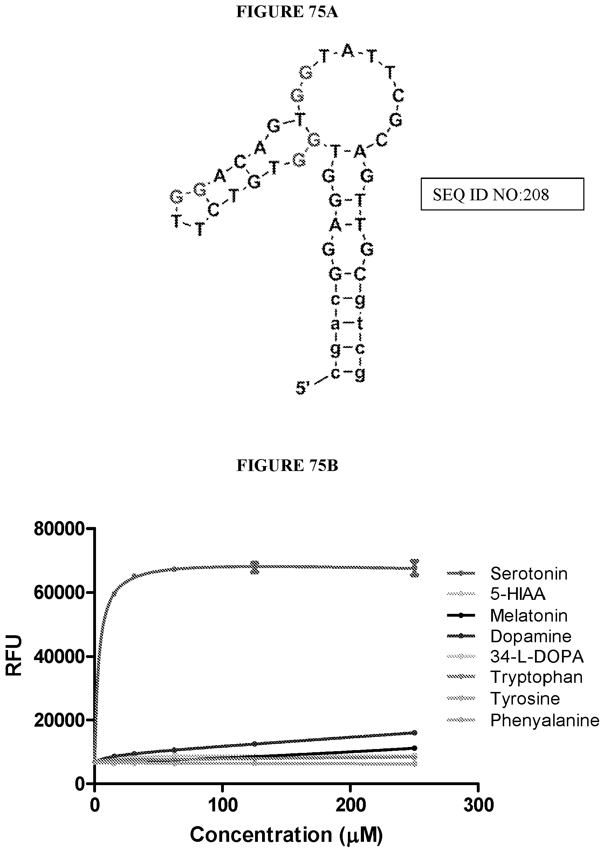

FIGURE 75A-B. (A) Serotonin-binding primary aptamer (SEQ ID NO:208) showing stem and loop structures. (B) Dose/fluorescent response curve resulting from a primary aptamer comprising SEQ ID NO:208 and additional operative sequence complementary to a sensor oligonucleotide analogous to FIGURE 38A-C, showing binding to serotonin (top curve) relative to other compounds.

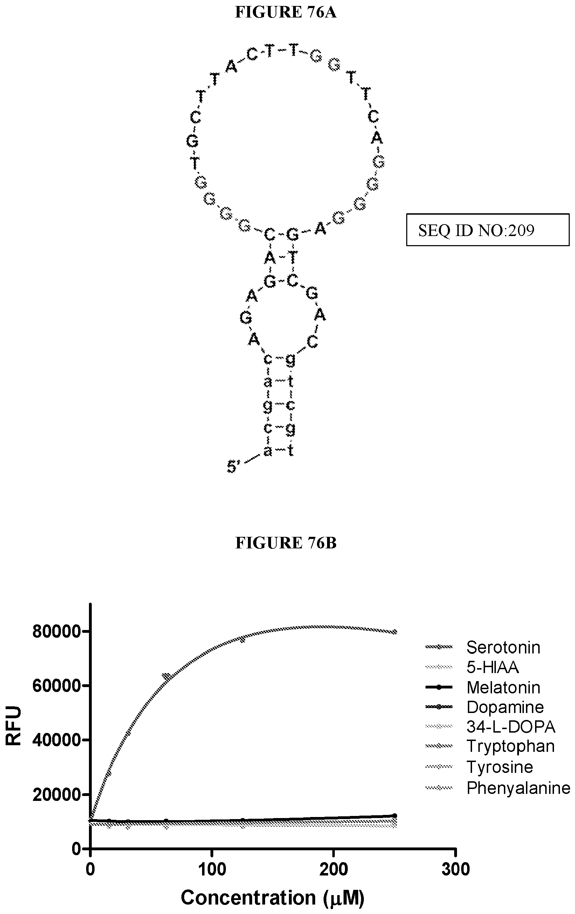

FIGURE 76A-B. (A) Serotonin-binding primary aptamer (SEQ ID NO:209) showing stem and loop structures. (B) Dose/fluorescent response curve resulting from a primary aptamer comprising SEQ ID NO:209 and additional operative sequence complementary to a sensor oligonucleotide analogous to FIGURE 39A-C, showing binding to serotonin (top curve) relative to other compounds.

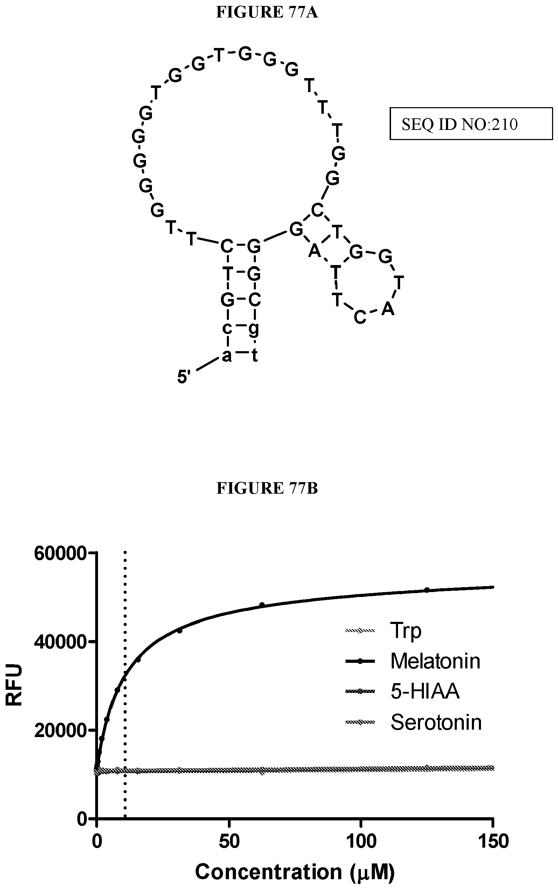

FIGURE 77A-B. (A) Melatonin-binding primary aptamer (SEQ ID NO:210) showing stem and loop structures. (B) Dose/fluorescent response curve resulting from a primary aptamer comprising SEQ ID NO:210 and additional operative sequence complementary to a sensor oligonucleotide analogous to FIGURE 42A-C, showing binding to melatonin (top curve) relative to other compounds.

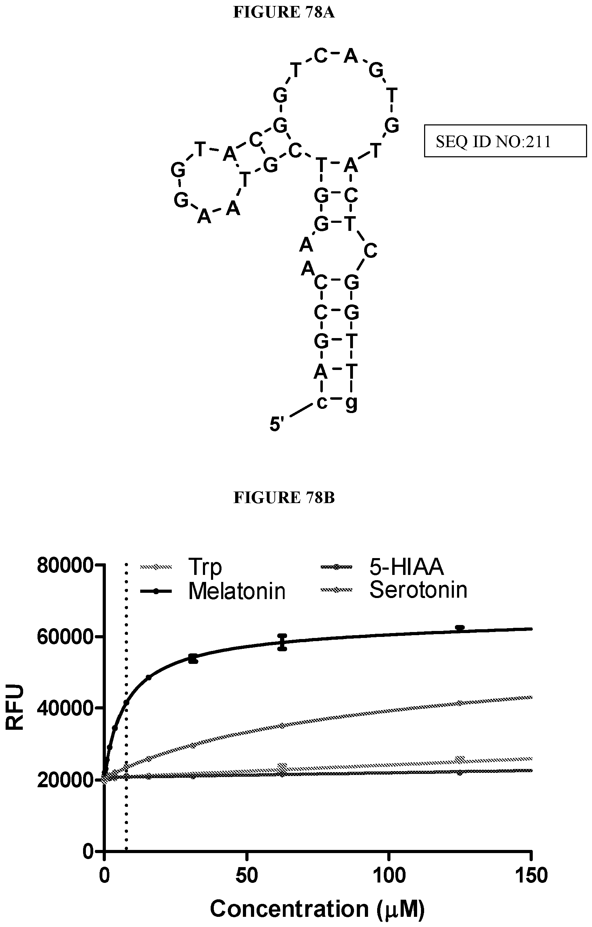

FIGURE 78A-B. (A) Melatonin-binding primary aptamer (SEQ ID NO:211) showing stem and loop structures. (B) Dose/fluorescent response curve resulting from a primary aptamer comprising SEQ ID NO:211 and additional operative sequence complementary to a sensor oligonucleotide analogous to FIGURE 40A-C, showing binding to melatonin (top curve) relative to other compounds.

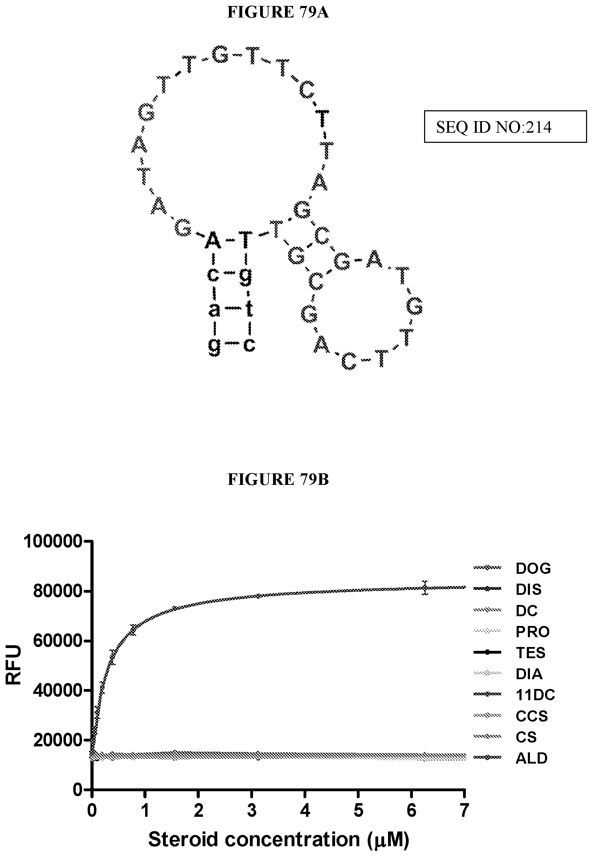

FIGURE 79A-B. Aldosterone- binding primary aptamer (SEQ ID NO:214) showing stem and loop structures. (B) Dose/fluorescent response curve resulting from a primary aptamer comprising SEQ ID NO:214 and additional operative sequence complementary to a sensor oligonucleotide, showing binding to aldosterone (top curve) relative to other steroid compounds.

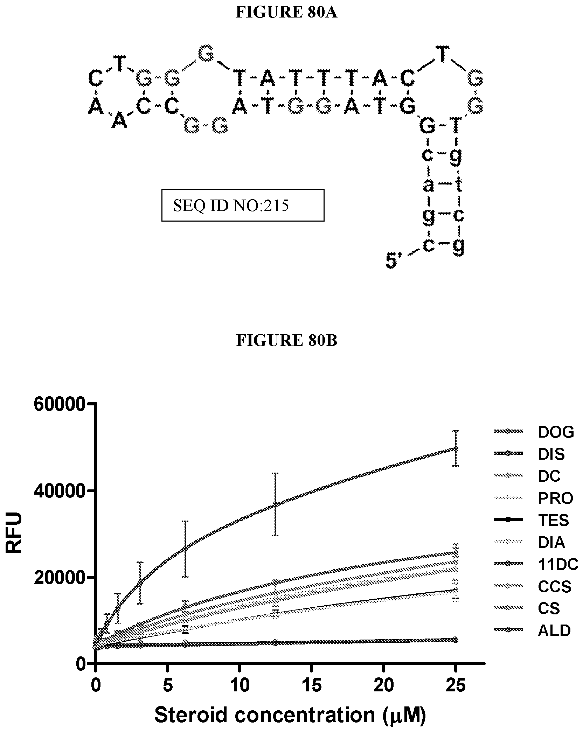

FIGURE 80A-B. Aldosterone- binding primary aptamer (SEQ ID NO:215) showing stem and loop structures. (B) Dose/fluorescent response curve resulting from a primary aptamer comprising SEQ ID NO:215 and additional operative sequence complementary to a sensor oligonucleotide, showing binding to aldosterone (top curve) relative to other steroid compounds.

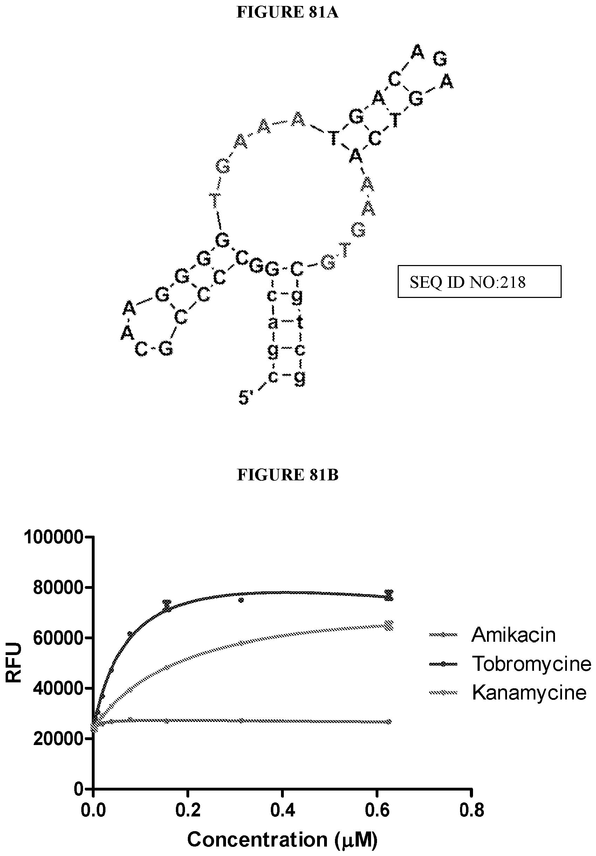

FIGURE 81A-B. Tobramycin- binding primary aptamer (SEQ ID NO:218) showing stem and loop structures. (B) Dose/fluorescent response curve resulting from a primary aptamer comprising SEQ ID NO:218 and additional operative sequence complementary to a sensor oligonucleotide, showing binding to tobramycin (top curve) relative to amikacin and kanamycin.

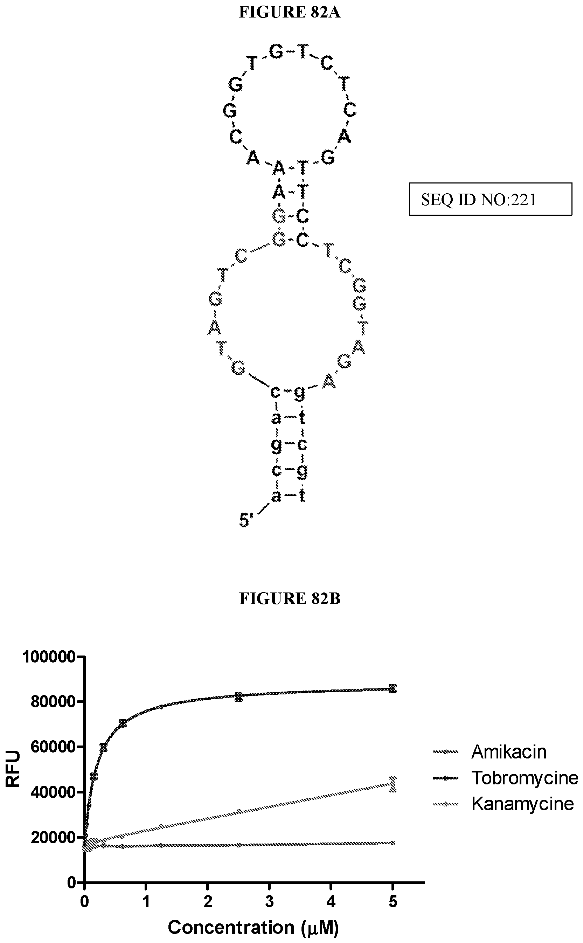

FIGURE 82A-B. Tobramycin- binding primary aptamer (SEQ ID NO:221) showing stem and loop structures. (B) Dose/fluorescent response curve resulting from a primary aptamer comprising SEQ ID NO:221 and additional operative sequence complementary to a sensor oligonucleotide, showing binding to tobramycin (top curve) relative to amikacin and kanamycin.

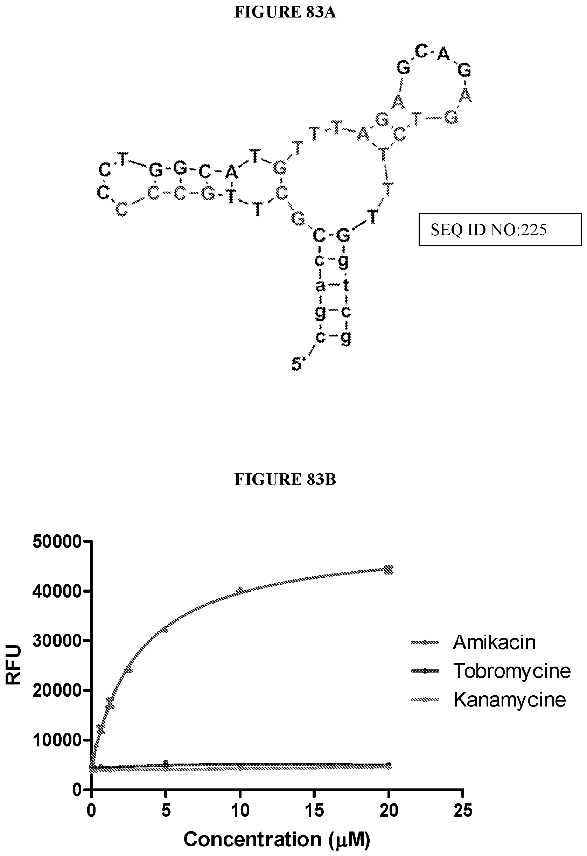

FIGURE 83A-B. Amikacin- binding primary aptamer (SEQ ID NO:225) showing stem and loop structures. (B) Dose/fluorescent response curve resulting from a primary aptamer comprising SEQ ID NO:225 and additional operative sequence complementary to a sensor oligonucleotide, showing binding to amikacin (top curve) relative to tobramycin and kanamycin.

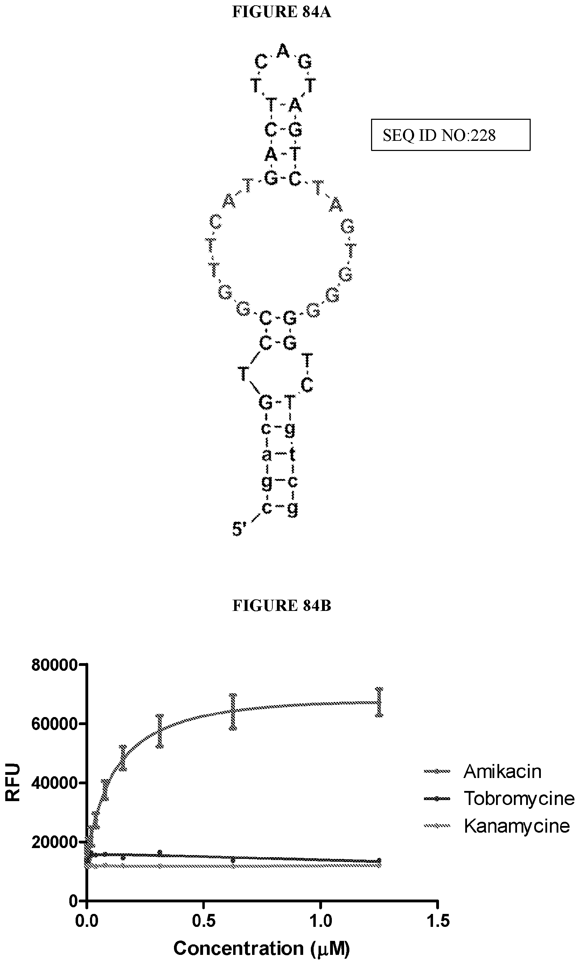

FIGURE 84A-B. Amikacin- binding primary aptamer (SEQ ID NO:228) showing stem and loop structures. (B) Dose/fluorescent response curve resulting from a primary aptamer comprising SEQ ID NO:228 and additional operative sequence complementary to a sensor oligonucleotide, showing binding to amikacin (top curve) relative to tobramycin and kanamycin.

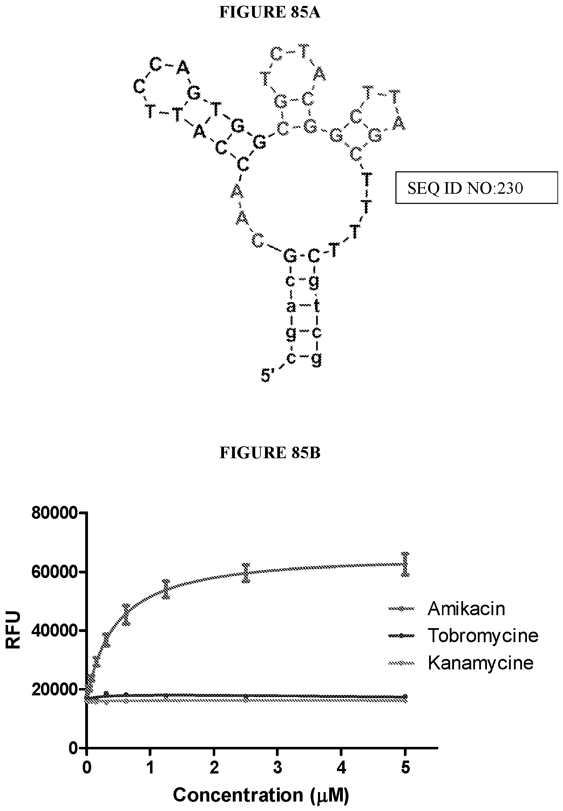

FIGURE 85A-B. Amikacin- binding primary aptamer (SEQ ID NO:230) showing stem and loop structures. (B) Dose/fluorescent response curve resulting from a primary aptamer comprising SEQ ID NO:230 and additional operative sequence complementary to a sensor oligonucleotide, showing binding to amikacin (top curve) relative to tobramycin and kanamycin.

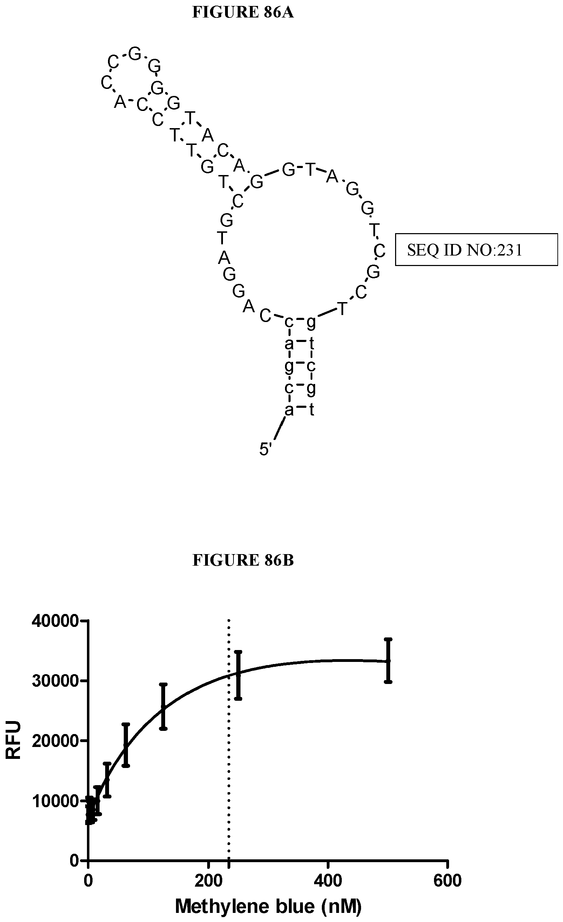

FIGURE 86A-B. Methylene blue- binding primary aptamer (SEQ ID NO:231) showing stem and loop structures. (B) Dose/fluorescent response curve resulting from a primary aptamer comprising SEQ ID NO: 231 and additional operative sequence complementary to a sensor oligonucleotide, showing binding to methylene blue.

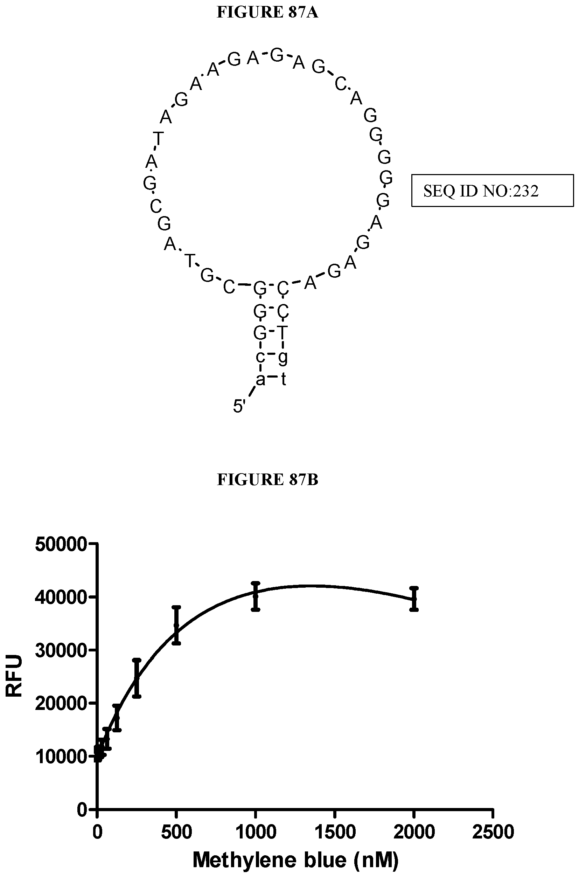

FIGURE 87A-B. Methylene blue- binding primary aptamer (SEQ ID NO:232) showing stem and loop structures. (B) Dose/fluorescent response curve resulting from a primary aptamer comprising SEQ ID NO: 232 and additional operative sequence complementary to a sensor oligonucleotide, showing binding to methylene blue.

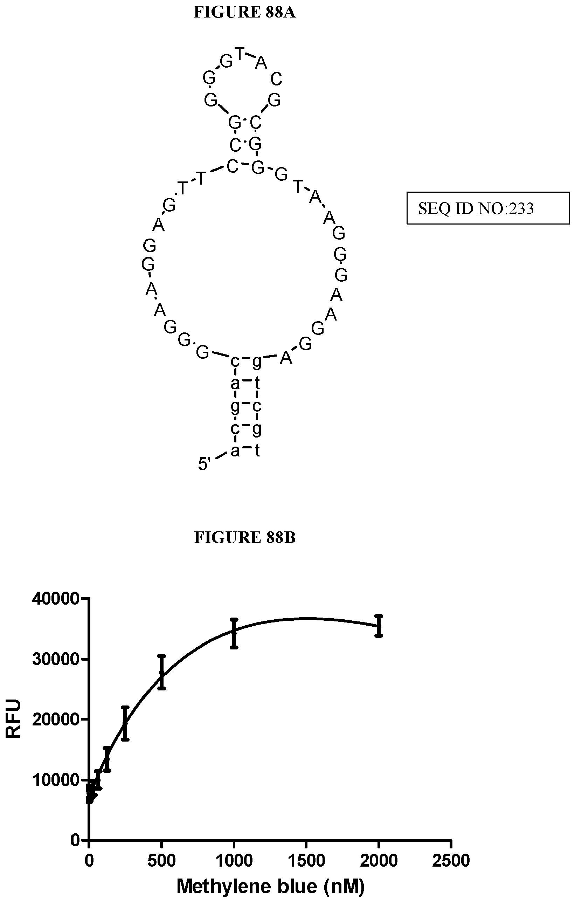

FIGURE 88A-B. Methylene blue- binding primary aptamer (SEQ ID NO:233) showing stem and loop structures. (B) Dose/fluorescent response curve resulting from a primary aptamer comprising SEQ ID NO:233 and additional operative sequence complementary to a sensor oligonucleotide, showing binding to methylene blue.

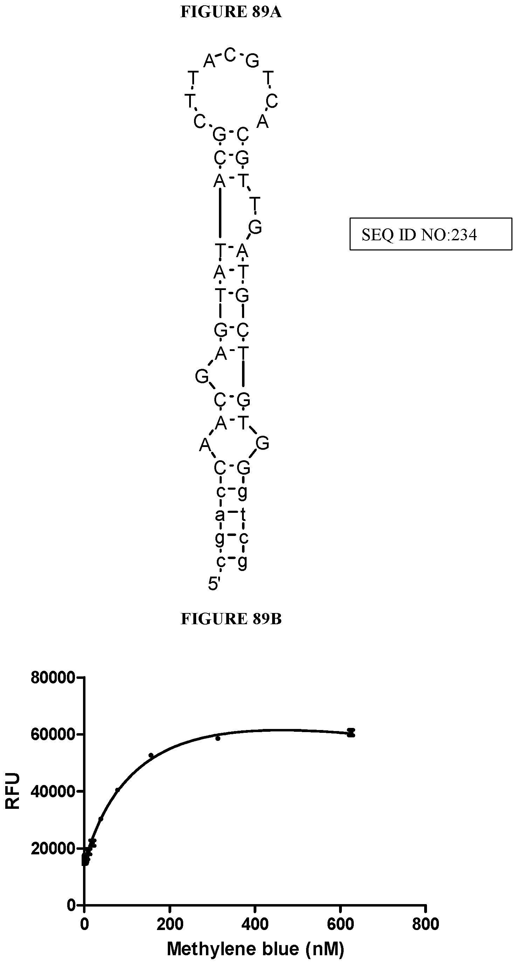

FIGURE 89A-B. Methylene blue- binding primary aptamer (SEQ ID NO:234) showing stem and loop structures. (B) Dose/fluorescent response curve resulting from a primary aptamer comprising SEQ ID NO:234 and additional operative sequence complementary to a sensor oligonucleotide, showing binding to methylene blue.

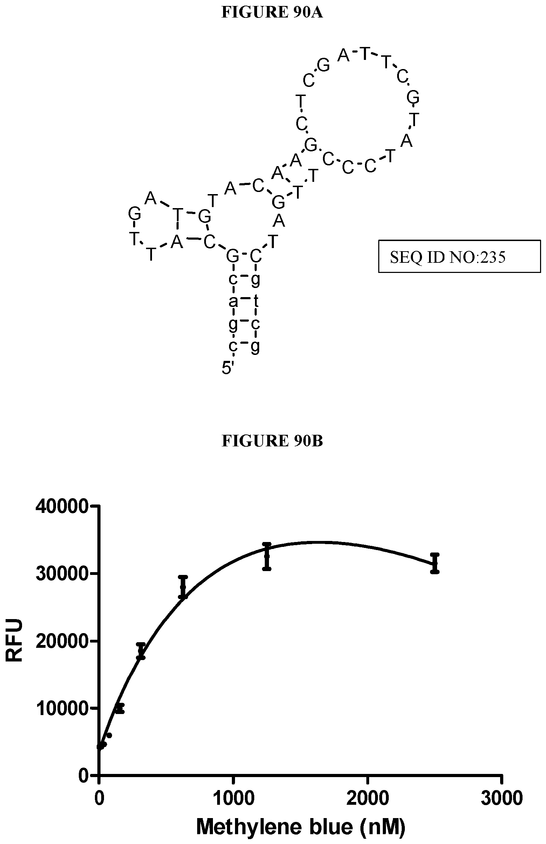

FIGURE 90A-B. Methylene blue- binding primary aptamer (SEQ ID NO:235) showing stem and loop structures. (B) Dose/fluorescent response curve resulting from a primary aptamer comprising SEQ ID NO:235 and additional operative sequence complementary to a sensor oligonucleotide, showing binding to methylene blue.

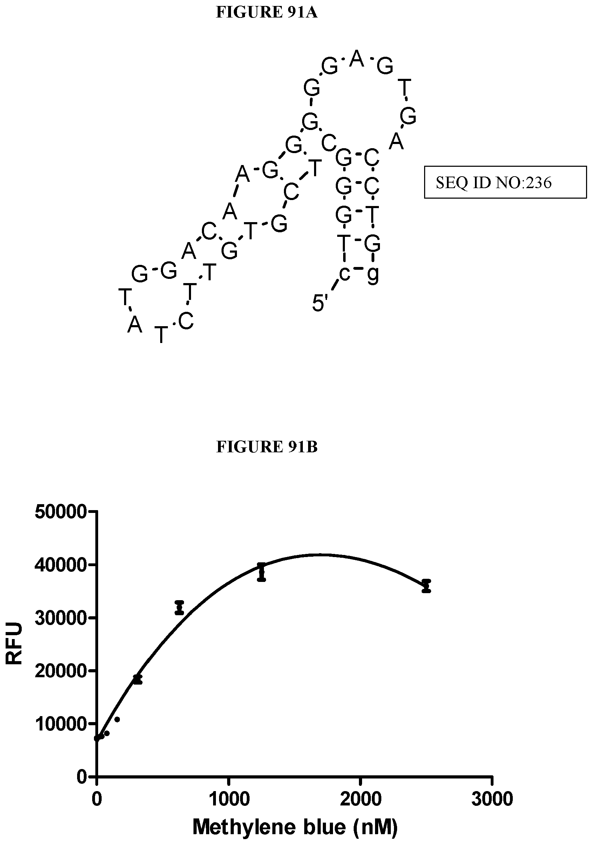

FIGURE 91A-B. Methylene blue- binding primary aptamer (SEQ ID NO:236) showing stem and loop structures. (B) Dose/fluorescent response curve resulting from a primary aptamer comprising SEQ ID NO:236 and additional operative sequence complementary to a sensor oligonucleotide, showing binding to methylene blue.

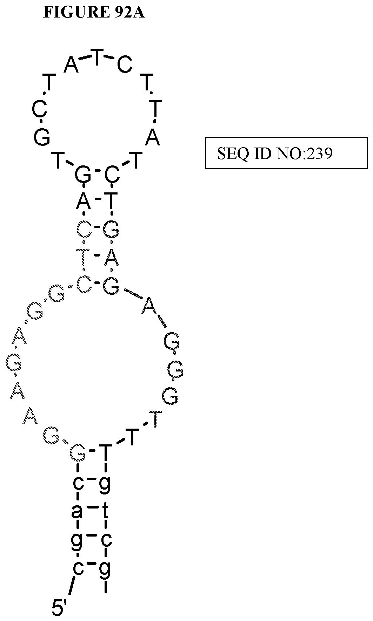

FIGURE 92A-B. Ammonium- binding primary aptamer (SEQ ID NO:239) showing stem and loop structures. (B) Dose/fluorescent response curve resulting from a primary aptamer comprising SEQ ID NO:239 and additional operative sequence complementary to a sensor oligonucleotide, showing selective binding to ammonium versus glycine or ethanolamine or potassium ion.

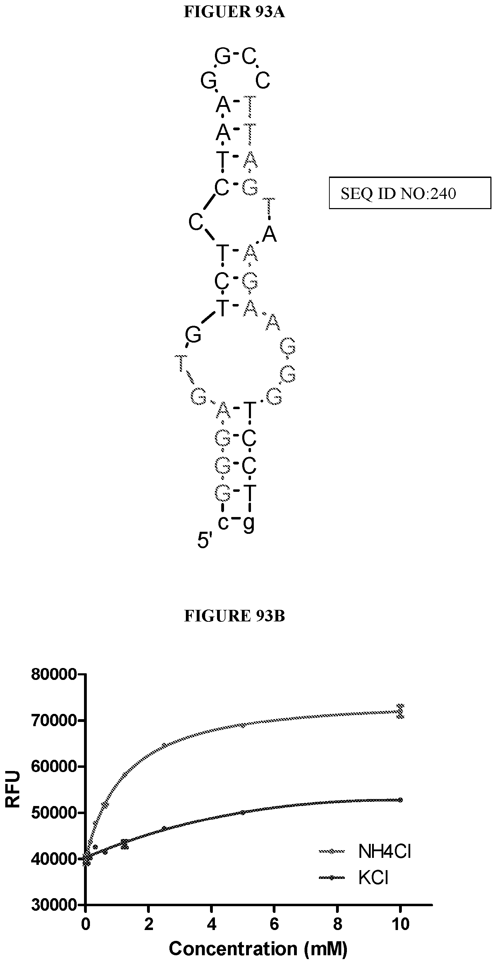

FIGURE 93A-B. Ammonium- binding primary aptamer (SEQ ID NO:240) showing stem and loop structures. (B) Dose/fluorescent response curve resulting from a primary aptamer comprising SEQ ID NO:240 and additional operative sequence complementary to a sensor oligonucleotide, showing selective binding to ammonium versus glycine or ethanolamine or potassium ion.

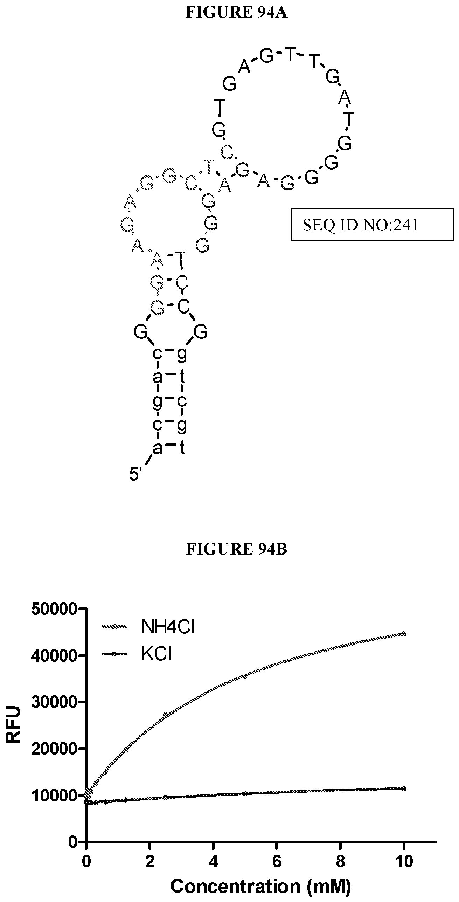

FIGURE 94A-B. Ammonium- binding primary aptamer (SEQ ID NO:241) showing stem and loop structures. (B) Dose/fluorescent response curve resulting from a primary aptamer comprising SEQ ID NO:241 and additional operative sequence complementary to a sensor oligonucleotide, showing selective binding to ammonium versus glycine or ethanolamine or potassium ion.

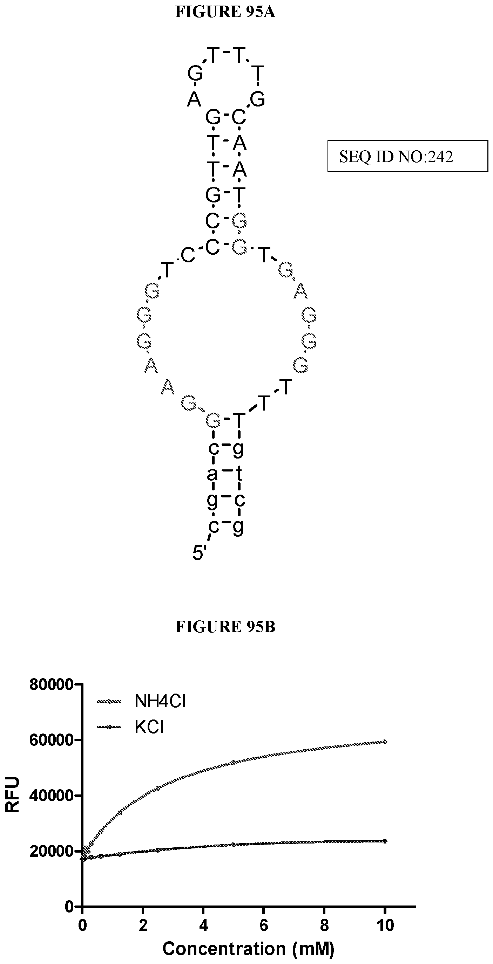

FIGURE 95A-B. Ammonium- binding primary aptamer (SEQ ID NO:242) showing stem and loop structures. (B) Dose/fluorescent response curve resulting from a primary aptamer comprising SEQ ID NO:242 and additional operative sequence complementary to a sensor oligonucleotide, showing selective binding to ammonium versus glycine or ethanolamine or potassium ion.

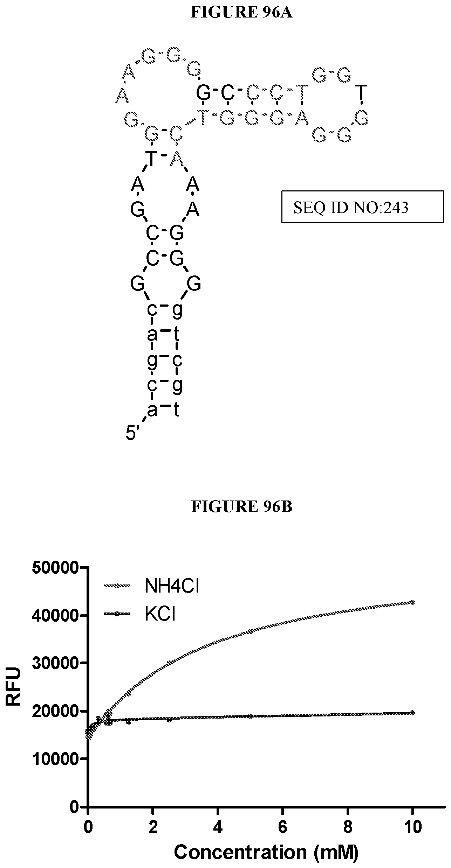

FIGURE 96A-B. Ammonium- binding primary aptamer (SEQ ID NO:243) showing stem and loop structures. (B) Dose/fluorescent response curve resulting from a primary aptamer comprising SEQ ID NO:243 and additional operative sequence complementary to a sensor oligonucleotide, showing selective binding to ammonium versus glycine or ethanolamine or potassium ion.

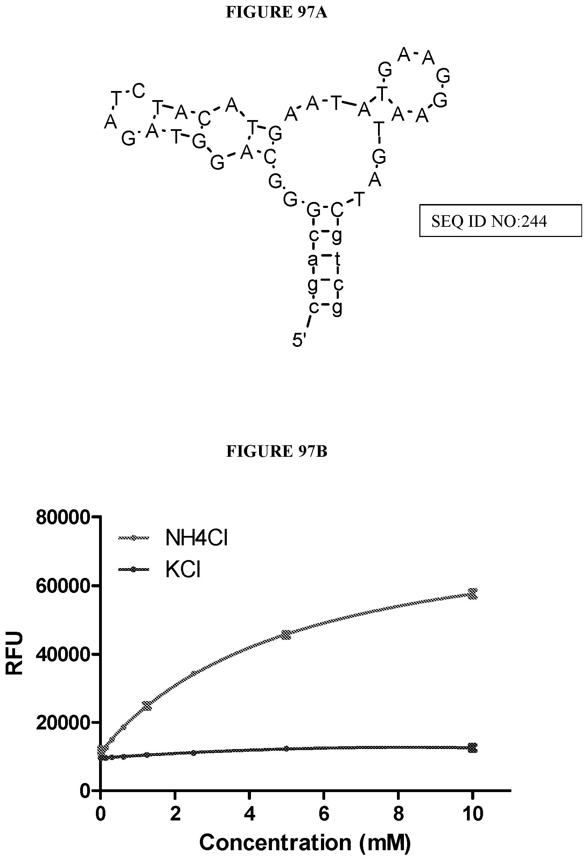

FIGURE 97A-B. Ammonium- binding primary aptamer (SEQ ID NO:244) showing stem and loop structures. (B) Dose/fluorescent response curve resulting from a primary aptamer comprising SEQ ID NO:244 and additional operative sequence complementary to a sensor oligonucleotide, showing selective binding to ammonium versus glycine or ethanolamine or potassium ion.

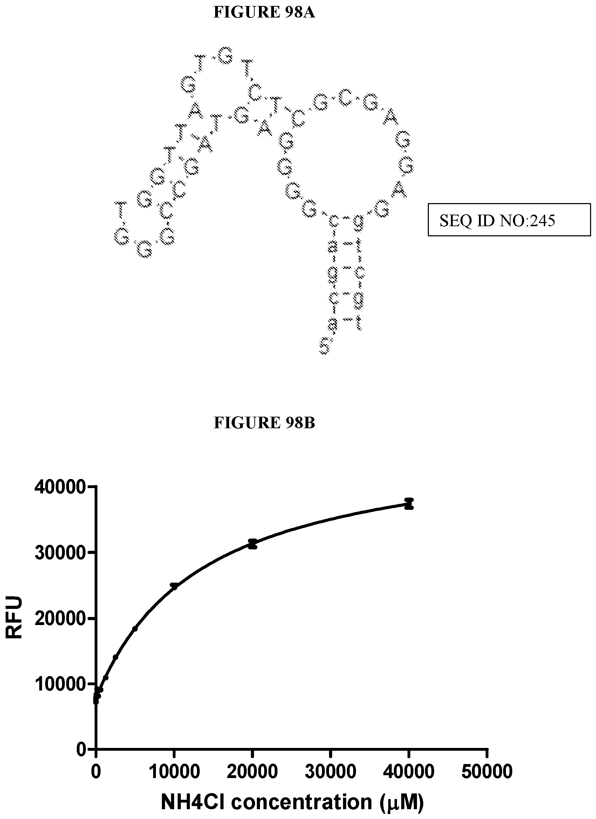

FIGURE 98A-B. Ammonium- binding primary aptamer (SEQ ID NO:245) showing stem and loop structures. (B) Dose/fluorescent response curve resulting from a primary aptamer comprising SEQ ID NO:245 and additional operative sequence complementary to a sensor oligonucleotide, showing selective binding to ammonium versus glycine or ethanolamine or potassium ion.

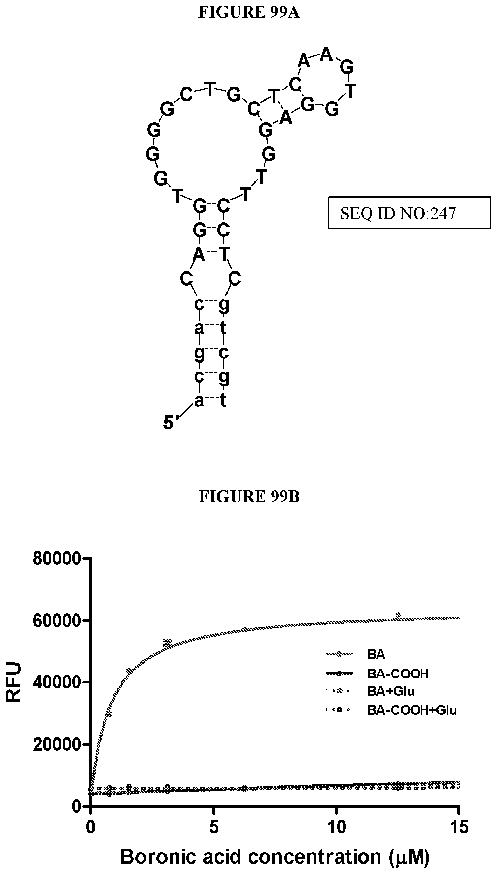

FIGURE 99A-B. Boronic acid- binding primary aptamer (SEQ ID NO:247) showing stem and loop structures. (B) Dose/fluorescent response curve resulting from a primary aptamer comprising SEQ ID NO:247 and additional operative sequence complementary to a sensor oligonucleotide, showing selective binding to boronic acid versus bisboronic acid, e.g., complexed to glucose.

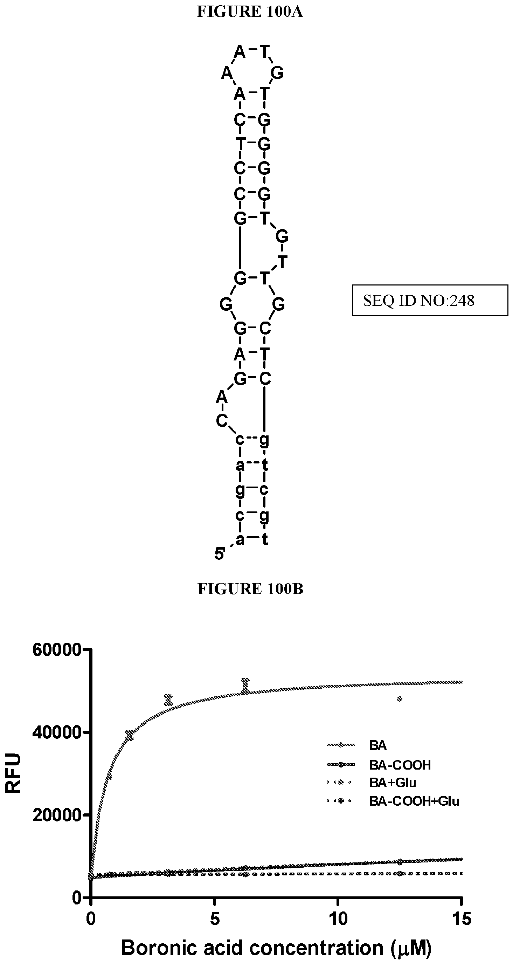

FIGURE 100A-B. Boronic acid- binding primary aptamer (SEQ ID NO:248) showing stem and loop structures. (B) Dose/fluorescent response curve resulting from a primary aptamer comprising SEQ ID NO:248 and additional operative sequence complementary to a sensor oligonucleotide, showing selective binding to boronic acid versus bisboronic acid, e.g., complexed to glucose.

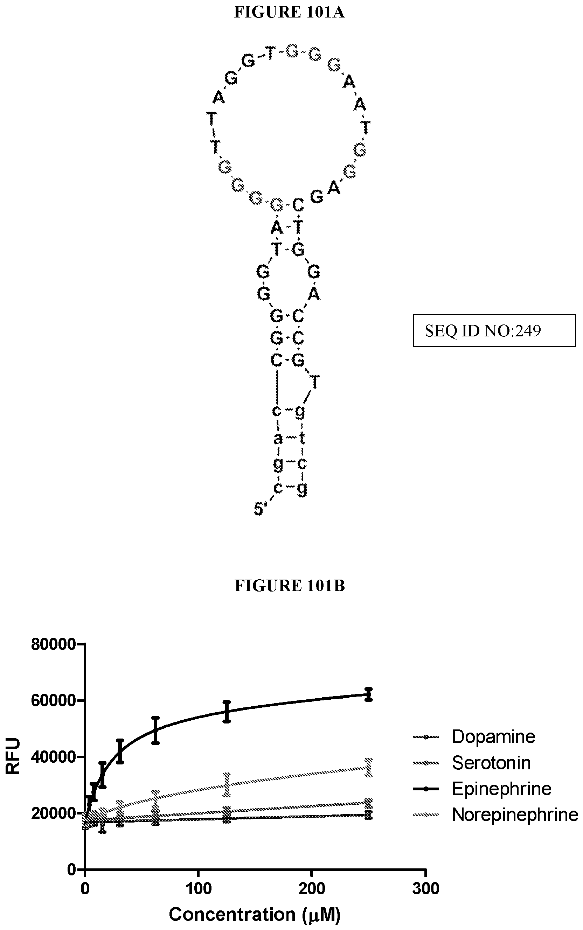

FIGURE 101A-B. Epinephrine - binding primary aptamer (SEQ ID NO:249) showing stem and loop structures. (B) Dose/fluorescent response curve resulting from a primary aptamer comprising SEQ ID NO:249 and additional operative sequence complementary to a sensor oligonucleotide, showing selective binding to epinephrine and, in descending order, serotonin, norepinephrine and dopamine.

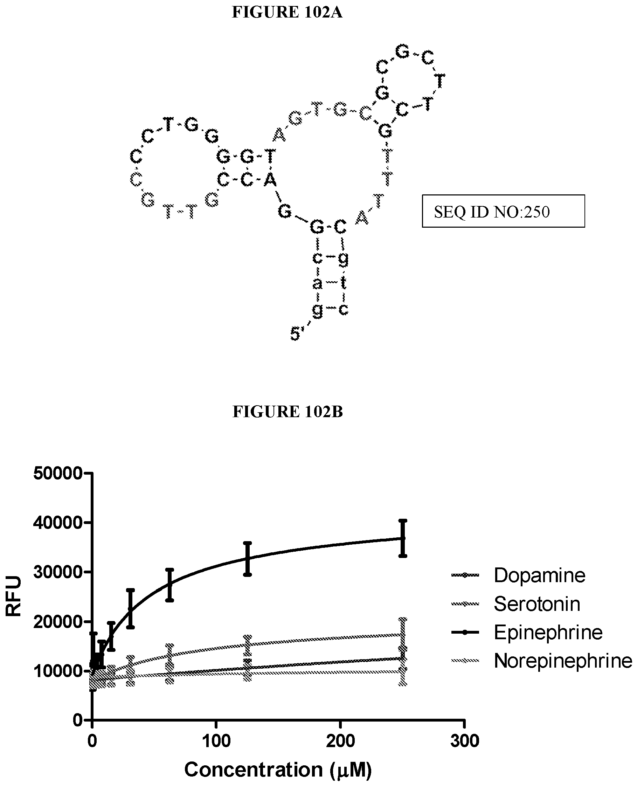

FIGURE 102A-B. Epinephrine - binding primary aptamer (SEQ ID NO:250) showing stem and loop structures. (B) Dose/fluorescent response curve resulting from a primary aptamer comprising SEQ ID NO:250 and additional operative sequence complementary to a sensor oligonucleotide, showing selective binding to epinephrine and, in descending order, serotonin, dopamine and norepinephrine.

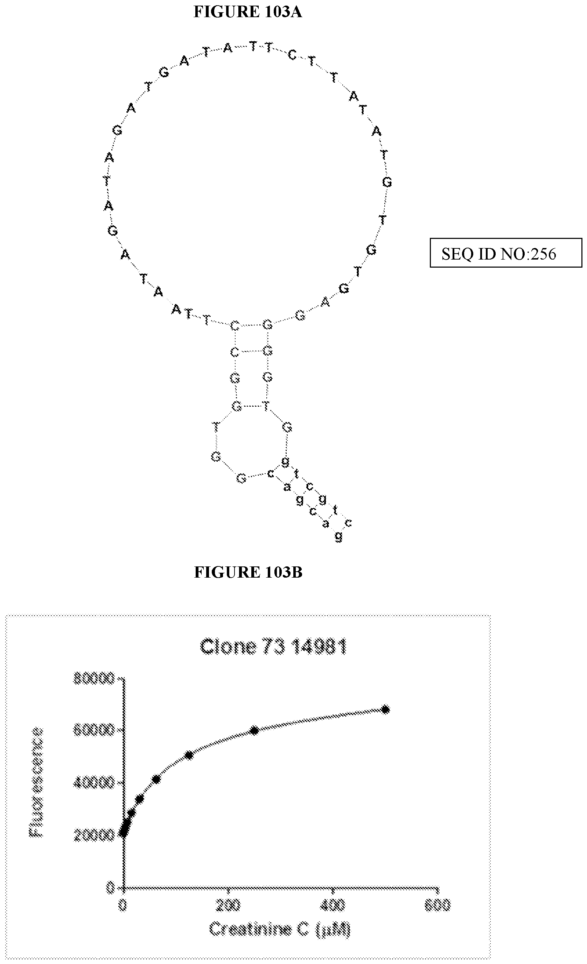

FIGURE 103A-B. Creatinine- binding primary aptamer (SEQ ID NO:256) showing stem and loop structures. (B) Dose/fluorescent response curve resulting from a primary aptamer comprising SEQ ID NO:256 showing binding to creatinine.

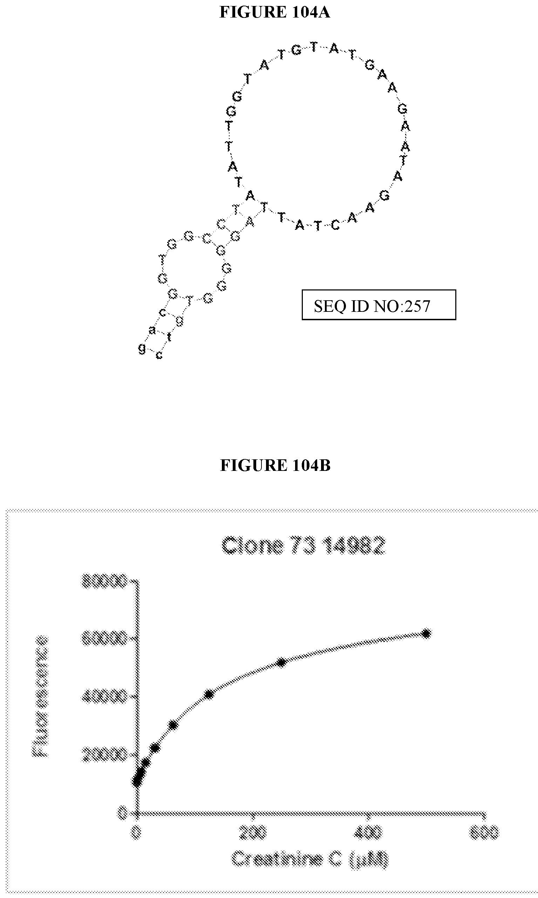

FIGURE 104A-B. Creatinine- binding primary aptamer (SEQ ID NO:257) showing stem and loop structures. (B) Dose/fluorescent response curve resulting from a primary aptamer comprising SEQ ID NO:257 showing binding to creatinine.

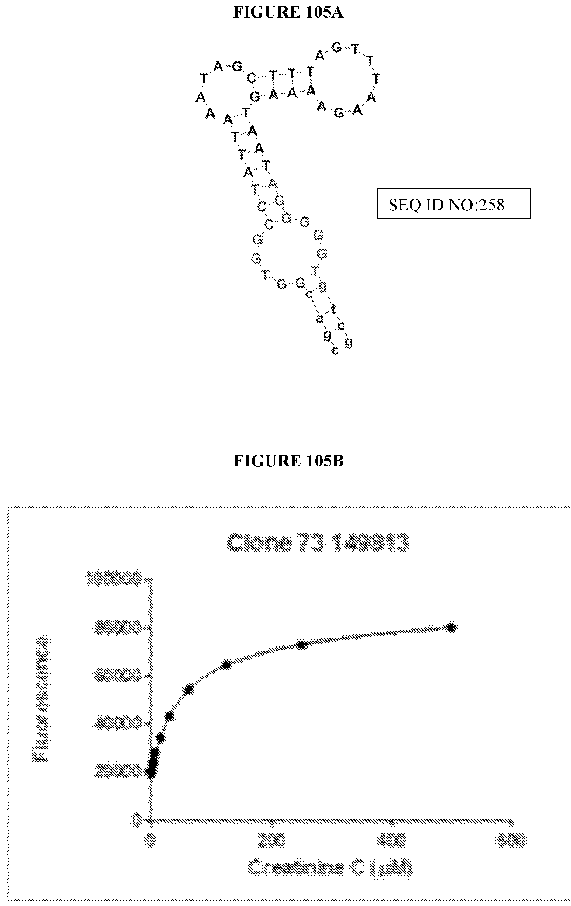

FIGURE 105A-B. Creatinine- binding primary aptamer (SEQ ID NO:258) showing stem and loop structures. (B) Dose/fluorescent response curve resulting from a primary aptamer comprising SEQ ID NO:258 showing binding to creatinine.

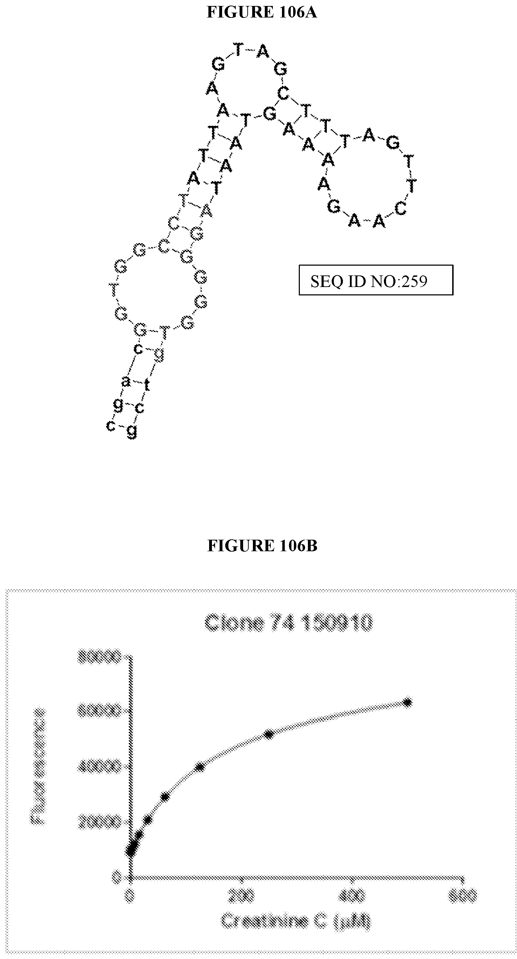

FIGURE 106A-B. Creatinine- binding primary aptamer (SEQ ID NO:259) showing stem and loop structures. (B) Dose/fluorescent response curve resulting from a primary aptamer comprising SEQ ID NO:259 showing binding to creatinine.

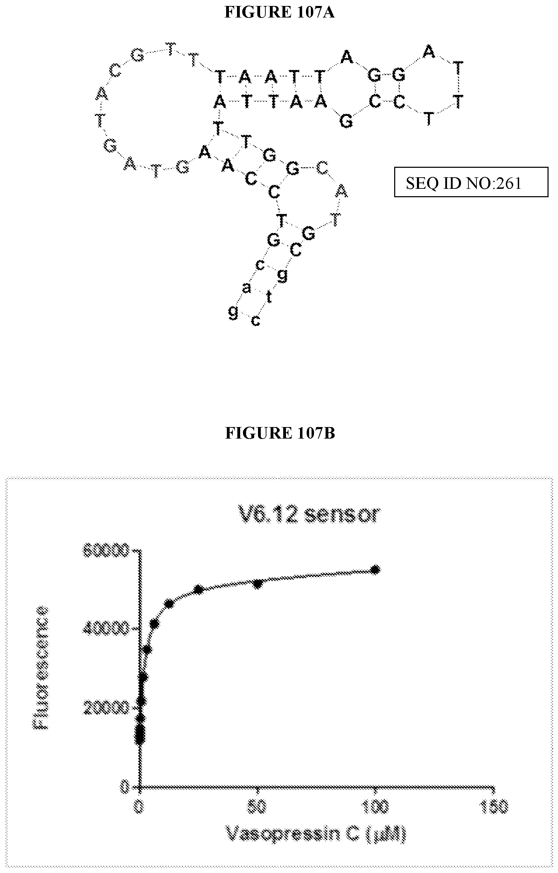

FIGURE 107A-B. Vasopressin- binding primary aptamer (SEQ ID NO:261) showing stem and loop structures. (B) Dose/fluorescent response curve resulting from a primary aptamer comprising SEQ ID NO:261 showing binding to vasopressin.

### 5. DETAILED DESCRIPTION OF THE INVENTION

The present disclosure relates to aptamer-based assays to capture and detect analytes. In addition to the primary aptamers, associated oligonucleotides and assay methods described herein, the methodology may be applied to design other aptamers, associated oligonucleotides, and analogous assays with aptamers or aptamer pairs, with adjustments related to the types of molecules, available reagents, and quantitative goals, for example established by the concentration in actual clinical samples and/or affinities of isolated reagents. Further, the assays, which utilize various mixtures of nucleic acids, can be combined with other nucleic acid elements such as those that form strand-displacement cascades.

For clarity of disclosure and not by way of limitation, this description is divided into the following sections:
5.1. Primary aptamers;
5.2. Anti-Aptamer Assays
5.3. Pseudosandwich Assays; and
5.4. Sandwich Assays.

Where a sequence provided herein refers to nucleotide "N", that position in the sequence may be filled by any natural or unnatural nucleotide, unless specified to the contrary.

The term "epitope" is used herein as referring to the binding site for the primary aptamer on the target analyte or, in the case of a sandwich assay, can be a binding site on the target analyte and a second aptamer.

In certain embodiments, where the invention provides for a sequence having a terminal CTCTC (SEQ ID NO:237) 5' sequence, the invention also provides for an alternative version of that sequence lacking the initial CTCTC (SEQ ID NO:237) sequence.

In certain embodiments, where the invention provides for a sequence having a CTCTC GGG (SEQ ID NO:238) 5' terminal sequence, the invention also provides for an alternative version of that sequence lacking the initial CTCTCGGG (SEQ ID NO:238) sequence. In certain embodiments, where the invention provides for a sequence having a TCCC (SEQ ID NO:246) 3' terminal sequence, the invention also provides for an alternative version of that sequence lacking the final TCCC (SEQ ID NO:246) sequence. In certain embodiments, where the invention provides for a sequence having a CTCTC GGG (SEQ ID NO:238) 5' terminal sequence and a TCCC (SEQ ID NO:246) 3' terminal sequence, the invention also provides for an alternative version lacking both these sequences.

In the assays described herein, detectable labels are used. In certain embodiments, a fluorescent moiety is comprised in one partner of a binding pair, and a quencher moiety is comprised in the other member of the binding pair. Assays may be designed so that the detectable moiety - e.g. the fluorescent moiety - is on either member of the pair. Fluorescent/quencher compounds are known in the art, and see Mary Katherine Johansson, Methods in Molecular Biol. 335:Fluorescent Energy Transfer Nucleic Acid Probes: Designs and Protocols, 2006, Didenko, ed., Humana Press, Totowa, NJ, and Marras et al., 2002, Nucl. Acids Res. 30, e122. Further, moieties that result in an increase in detectable signal when in proximity of each other may be used as alternative labels in the assays described herein, for example, as a result of fluorescence resonance energy transfer ("FRET"); suitable pairs include but are not limited to fluoroscein and tetramethylrhodamine; rhodamine 6G and malachite green, and FITC and thiosemicarbazole, to name a few.

### 5.1. PRIMARY APTAMERS

A "primary aptamer" (A^{P}) binds an target analyte (ligand, L). A primary aptamer may be isolated (identified as binding to its target analyte) by solution-phase or solidphase selection.

Primary aptamers (A^{p}) and associated oligonucleotides (e.g., anti-aptamers, sensor oligonucleotides, comp oligonucleotides, and sandwich oligonucleotides (also referred to as "secondary aptamers" or A^{s})), can have any size consistent with their intended function. In certain non-limiting embodiments, a primary aptamer or associated oligonucleotide is between about 20-250 nucleotides in length. For example, but not by way of limitation, the length can be between about 20-200 nucleotides, or between about 20-150 nucleotides, or between about 30 and 200 nucleotides, or between about 40-200 nucleotides, or between about 50-200 nucleotides, or between about 60-200 nucleotides, or between about 70-200 nucleotides, or between about 80-200 nucleotides, or between about 100-200 nucleotides, or between about 150-200 nucleotides, or between about 30-150 nucleotides, or between about 30-100 nucleotides, or between about 30-80 nucleotides, or between about 30-50 nucleotides, or between about 40-100 nucleotides; or at least about 20 nucleotides, or at least about 30 nucleotides, or up to about 100 nucleotides, or up to about 200 nucleotides (where "about" means plus or minus 20 percent), or between 20-250 nucleotides, or between 25 and 100 nucleotides. In certain non-limiting embodiments, primary aptamers and/or associated oligonucleotides can be spiegelmers or contain unnatural enantiomers of nucleic acids.²²⁻²⁵

In particular embodiments, a primary aptamer is provided comprising a core sequence that acts as a binding pocket for the target analyte. In certain embodiments, a primary aptamer comprising a particular core sequence, or a consensus core sequence, is provided. A primary aptamer may further comprise an operative sequence which plays a functional role in a particular assay, as will be described below. A primary aptamer may also optionally comprise additional sequence, other than core sequence or operative sequence, which does not substantially impact its functionality. A primary aptamer comprising a core sequence that binds to a target analyte of interest may be utilized in diverse assays, including but not limited to those exemplified herein.

### 5.1.1. METHODS FOR ISOLATING PRIMARY APTAMERS

Primary aptamers can be identified that selectively bind to diverse target analytes, including, but not limited to amino acids, mono- and oligo- saccharides, steroids, catecholamines, serotonin, melatonin, lipids, hormones, and/or peptides. In certain non-limiting embodiments, the method can be used to produce primary aptamers that bind to spiegelmers for vasopressin, aminoglycosides and other antibiotics, immunosupressants, anti-tumor agents, pesticides, hormones, etc..

For example, and not by way of limitation, the isolation of primary aptamers can be performed using the SELEX process. In certain non-limiting embodiments, the primary aptamers are isolated by either solid- (traditional) or newer solution-phase selections ¹⁶⁻²¹. Further, in certain non-limiting embodiments, the solution-phase selection has inherent advantages for small molecules, such as higher affinity and ease of screening of aptamers.

In certain non-limiting embodiments, the method comprises attaching a biotinylated strand complementary (C_{B}) to one of the PCR primers to agarose-streptavidin (FIGURE 7) and attaching sequences from a library (e.g., but not limited to, N₈-N₁₀₀) through complementary interactions to C_{B} of a primer. Two primers on library, 5'- and 3'-, are also partially complementary; members of the library which interact with a target in a way that favors stem formation between complementary region of these primers are released from the agarose by displacing complement C_{B}, and used in PCR amplification that now creates an enriched pool of potential aptamers.

In the solution-phase selection, molecules are used without any attachment to a matrix, thus, no functional groups are "wasted". This maximizes interactions with aptamers, leading to high affinities. Concentrations of compounds that can be used go up to the limit of solubility, allowing isolation of weak-affinity aptamers (e.g., against metabolites and glucose).

In certain non-limiting embodiments, fluorescent sensors can be directly obtained from this selection, by substituting biotin with dabcyl and attaching fluorescein to the aptamer (FIGURE 6, C_{D}), confirming that aptamers bind, determining their K_{d} (this is a competitive assay, so half-response is shifted away from the K_{d}⁸⁰, and C_{D} is present in an excess), and establishing selectivity.

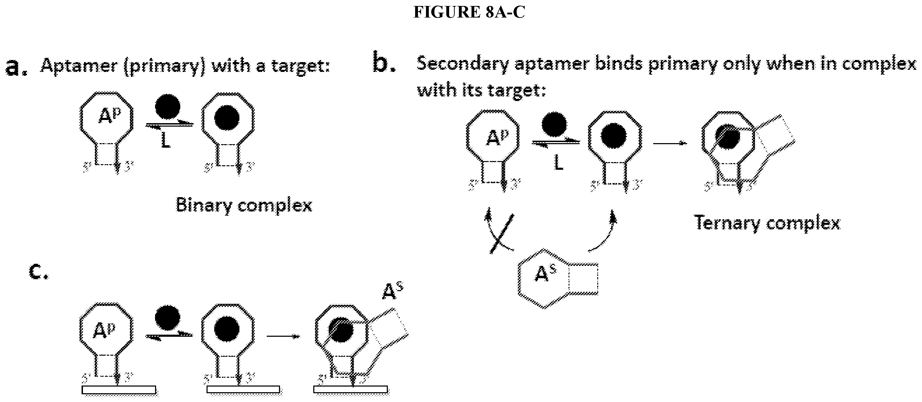

In certain non-limiting embodiments, the method comprises:
(1) Isolating primary aptamers (A^{p}s) by solution-phase (FIGURE 7) or solidphase selection, unless they are already available; use of enantiomeric aptamer (spiegelmers), if desired to minimize Watson-Crick base pairing (e.g., fusing aptamers or to minimize background interactions without analyte);
(2) Testing of the primary aptamer in its structure-switching form and modifying its structure switching form, which is then turned into pseudo-sandwich assay format (FIGURE 6C);
(3) Isolating secondary sandwich aptamers (A^{s}s) by either solution-phase or solid phase selections (FIGURE 9A-B), using primary aptamers or spiegelmers in their complexes with targets;
(4) Implementing sandwich assays for targets (FIGURE 8A-C) and/or
(5) Preparing a shortened form of the primary aptamer originally isolated;
(6) Modifying the optionally shortened primary aptamer by introducing an operative sequence; and/or
(7) Modifying the optionally shortened primary aptamer by substituting one or more nucleotides in its binding pocket to improve binding properties in the intended assay.

### 5.1.2 GLUCOSE-BINDING PRIMARY APTAMERS

In certain non-limiting embodiments, a primary aptamer binds to glucose in an aqueous solution at room temperature or 25°C with a dissociation constant of less than 10⁻¹ M (affinity 10) and binds selectively with glucose versus galactose.

In certain non-limiting instances, a glucose-binding primary aptamer comprises the sequences CCGTGTGT (SEQ ID NO: 157) and either AGTGTCCATTG (SEQ ID NO: 158) or AGTGTCCTTTG (SEQ ID NO: 159) or a variant of any of these sequences that differs in one or two bases by substitution, deletion, insertion or extension, where said primary aptamer binds to glucose in an aqueous solution at room temperature or 25°C with a dissociation constant of less than 10⁻¹ M (affinity 10), and binds selectivity to glucose versus galactose or fructose (see, for example, FIGURE 50B). In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, said glucose-binding primary aptamer comprising the sequences CCGTGTGT (SEQ ID NO: 157) and either AGTGTCCATTG (SEQ ID NO: 158) or AGTGTCCTTTG (SEQ ID NO: 159) or a variant thereof has a binding affinity for glucose that is at least about 50 percent or at least about 75 percent the binding affinity of the primary aptamer having SEQ ID NO:68.). In certain non-limiting instances, said glucose-binding primary aptamer comprising the sequences CCGTGTGT (SEQ ID NO: 157) and either AGTGTCCATTG (SEQ ID NO:158) or AGTGTCCTTTG (SEQ ID NO: 159) or a variant thereof competes with primary aptamer having SEQ ID NO:68 for glucose binding. In certain non-limiting instances, a glucose-binding primary aptamer comprises the sequences CCGTGTGT (SEQ ID NO: 157) and either AGTGTCCATTG (SEQ ID NO: 158) or AGTGTCCTTTG (SEQ ID NO: 159) or a variant thereof and further comprises at least one operative sequence. In certain non-limiting instances, a glucose-binding primary aptamer comprises the sequences CCGTGTGT (SEQ ID NO: 157) and either AGTGTCCATTG (SEQ ID NO: 158) or AGTGTCCTTTG (SEQ ID NO:159), or a variant thereof, and further comprises at least one operative sequence, said operative sequence complementary to a sequence comprised in a sensor oligonucleotide. In certain non-limiting instances, a glucose-binding primary aptamer comprises the sequences CCGTGTGT (SEQ ID NO: 157) and either AGTGTCCATTG (SEQ ID NO: 158) or AGTGTCCTTTG (SEQ ID NO: 159), or a variant thereof, and at least one operative sequence on either side (flanking) these two sequences. In certain non-limiting instances, a glucose-binding primary aptamer comprises the sequences CCGTGTGT (SEQ ID NO: 157) and either AGTGTCCATTG (SEQ ID NO: 158) or AGTGTCCTTTG (SEQ ID NO: 159), or a variant thereof, and at least one operative sequence on either side (flanking) these two sequences, where two of said operative sequences contain mutually complementary portions and can form a duplex.

In certain non-limiting instances, a glucose-binding primary aptamer has a predicted secondary structure that comprises two stems connected by sequences that bind to glucose (e.g., binding selectively to glucose versus galactose) and/or one or more of the following: a 4-O-R-glucose epitope, where R is hydrogen, an alkyl group, another carbohydrate or a protein; cellobiose; and/or maltose. See, for example, FIGURE 50A-B.

For example, but not by way of limitation, a glucose-binding primary aptamer may comprise a sequence selected from the group consisting of:

>S-Glu01: CTCTCGGGACGACCGTGTGTGTTGCTCTGTAAC---------
AGTGTCCATTGTCGTCCC (SEQ ID NO:160);

>S-Glu02:
CTCTCGGGACGACCGTGTGTGGTAGAGTCGTCGGGCTCTAACAGTGTCCTT
TGTCGTCCC (SEQ ID NO:161);

>S-Glu03:
CTCTCGGGACGACCGTGTGTGACGTGCGCCGTGGGGAACGTCAGTGTTCTT
TGTCGTCCC (SEQ ID NO:162);

>S-Glu04: CTCTCGGGACGACCGTGTGTCGACTTAGAGTCG---------
AGTGTCCTTTGTCGTCCC (SEQ ID NO:163); and

>S-Glu05: CTCTCGGGACGACCGTGTGTTGCAATTCTTGCA---------
AGTGTTCTTTGTCGTCCC (SEQ ID NO:164).

In certain non-limiting instances, a glucose-binding primary aptamer comprises a core sequence as set forth in SEQ ID NO:83 (FIGURE 10B). In certain non-limiting instances, a glucose-binding primary aptamer comprising a core sequence as set forth in SEQ ID NO:83 (FIGURE 10B) has a binding affinity for glucose that is at least about 50 percent or at least about 75 percent the binding affinity of the primary aptamer having SEQ ID NO:68). In certain non-limiting instances, a glucose-binding primary aptamer comprising a core sequence as set forth in SEQ ID NO:83 (FIGURE 10B) competes with primary aptamer having SEQ ID NO:68 for glucose binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a glucose-binding primary aptamer comprises a core sequence as set forth in SEQ ID NO:83 (FIGURE 10B) and further comprises at least one operative sequence. In certain non-limiting instances, a glucose-binding primary aptamer comprises a core sequence as set forth in SEQ ID NO:83 (FIGURE 10B) and further comprises at least one operative sequence, said operative sequence complementary to a sequence comprised in a sensor oligonucleotide. In certain non-limiting instances, a glucose-binding primary aptamer comprises a core sequence as set forth in SEQ ID NO:83 (FIGURE 10B) and at least one operative sequence on either side (flanking) the core sequence. In certain non-limiting instances, a glucose-binding primary aptamer comprises a core sequence as set forth in SEQ ID NO:83 (FIGURE 10B) and at least one operative sequence on either side (flanking) the core sequence, where two of said operative sequences contain mutually complementary portions and can form a duplex.

In certain non-limiting instances, isolated glucose- binding primary aptamers comprise the nucleotide sequence of SEQ ID NO: 1, SEQ ID NO:68, or SEQ ID NO:149 or variants of these sequences having at least about 80 percent, or at least about 85 percent, or at least about 90 percent, or at least about 95 percent, or at least about 98 percent homology to the original sequence, for example obtained by substitutions, deletions, and insertions of Watson-Crick base pairs or by mutations at non-conserved positions. Percent homology can be determined using standard software such as BLAST or FASTA. In certain non-limiting instances, isolated glucose-binding primary aptamers comprise the nucleotide sequence of SEQ ID NO:1. SEQ ID NO:68, or SEQ ID NO: 149. Said aptamers can bind to glucose and in their structure-switching formats (for example, in an anti-aptamer assay, a pseudosandwich assay, or a sandwich assay) they can respond by an increase in fluorescence.

### 5.1.3 PHENYLALANINE-BINDING PRIMARY APTAMERS

In certain non-limiting embodiments, a primary aptamer binds to phenylalanine in an aqueous solution at room temperature or 25°C with a dissociation constant of less than 10⁻⁴ M and binds selectively with phenylalanine versus tyrosine (or hydroxyl-phenylalanine) or tryptophan.

In certain non-limiting embodiments, a phenylalanine-binding primary aptamer comprises the sequences GCGT (SEQ ID NO: 165) and AGC and GGTT (SEQ ID NO: 166) or a variant of any of these sequences that differs in one or two bases by substitution, deletion, insertion or extension, where said primary aptamer binds to phenylalanine in an aqueous solution at room temperature or 25°C with a dissociation constant of less than 10⁻⁴ M and binds selectively to phenylalanine versus tyrosine (or hydroxyl-phenylalanine) or tryptophan (see, for example, FIGURE 51A-B). In certain non-limiting embodiments, said phenylalanine-binding primary aptamer comprising the sequences GCGT (SEQ ID NO: 165) and AGC and GGTT (SEQ ID NO: 166), or a variant thereof, has a binding affinity for phenylalanine that is at least about 50 percent or at least about 75 percent the binding affinity of a primary aptamer having SEQ ID NO:2, SEQ ID NO:3, SEQ ID NO:4, or SEQ ID NO:67. In certain non-limiting embodiments, said phenylalanine-binding primary aptamer comprising the sequences GCGT (SEQ ID NO: 165) and AGC and GGTT (SEQ ID NO: 166), or a variant thereof, competes with a phenylalanine-binding primary aptamer having SEQ ID NO: SEQ ID NO:2, SEQ ID NO:3, SEQ ID NO:4, or SEQ ID NO:67. In certain non-limiting embodiments, a phenylalanine-binding primary aptamer comprises the sequences GCGT (SEQ ID NO: 165) and AGC and GGTT (SEQ ID NO: 166) or a variant thereof and further comprises at least one operative sequence. In certain non-limiting embodiments, a phenylalanine-binding primary aptamer comprises the sequences GCGT (SEQ ID NO: 165) and AGC and GGTT (SEQ ID NO: 166), or a variant thereof, and further comprises at least one operative sequence, said operative sequence complementary to a sequence comprised in a sensor oligonucleotide. In certain non-limiting embodiments, a phenylalanine-binding primary aptamer comprises the sequences GCGT (SEQ ID NO: 165) and AGC and GGTT (SEQ ID NO: 166), or a variant thereof, and at least one operative sequence on either side (flanking) these three sequences. In certain non-limiting embodiments, a phenylalanine-binding primary aptamer comprises the sequences GCGT (SEQ ID NO: 165) and AGC and GGTT (SEQ ID NO: 166), or a variant thereof, and at least one operative sequence on either side (flanking) these three sequences, where two of said operative sequences contain mutually complementary portions and can form a duplex.

For example, but not by way of limitation, a phenylalanine-binding primary aptamer may comprise the sequence: CTC TCG GGA CGA CCG CGT TTC CCA AGA AAG CAA GTA TTG GTT GGT CGT CCC (SEQ ID NO:2)
or a portion thereof comprising the core, SEQ ID NO:85) set forth in FIGURE 11B, (see FIGURE 51A (SEQ ID NO: 167).

In certain non-limiting instances, a phenylalanine-binding primary aptamer comprises a core sequence as set forth in SEQ ID NO:85 (FIGURE 11B). In certain nonlimiting instances, a phenylalanine -binding primary aptamer comprising a core sequence as set forth in SEQ ID NO:85 (FIGURE 11B) has a binding affinity for phenylalanine that is at least about 50 percent or at least about 75 percent the binding affinity of the primary aptamer having SEQ ID NO: 167). In certain non-limiting instances, a phenylalanine - binding primary aptamer comprising a core sequence as set forth in SEQ ID NO:85 (FIGURE 11B) competes with primary aptamer having SEQ ID NO: 167 for phenylalanine binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a phenylalanine -binding primary aptamer comprises a core sequence as set forth in SEQ ID NO:85 (FIGURE 11B) and further comprises at least one operative sequence. In certain non-limiting instances, a phenylalanine -binding primary aptamer comprises a core sequence as set forth in SEQ ID NO:85 (FIGURE 11B) and further comprises at least one operative sequence, said operative sequence complementary to a sequence comprised in a sensor oligonucleotide. In certain non-limiting instances, a phenylalanine -binding primary aptamer comprises a core sequence as set forth in SEQ ID NO:85 (FIGURE 11B) and at least one operative sequence on either side (flanking) the core sequence. In certain non-limiting instances, a phenylalanine -binding primary aptamer comprises a core sequence as set forth in SEQ ID NO:85 (FIGURE 11B) and at least one operative sequence on either side (flanking) the core sequence, where two of said operative sequences contain mutually complementary portions and can form a duplex.

In certain non-limiting embodiments, a phenylalanine-binding primary aptamer comprises the sequences GG and GGGGG (SEQ ID NO: 168) and GGGG (SEQ ID NO: 169) or a variant of any of these sequences that differs in one or two bases by substitution, deletion, insertion or extension, where said primary aptamer binds to phenylalanine (see FIGURE 52A-B) in an aqueous solution at room temperature or 25°C with a dissociation constant of less than 10⁻⁴ M and binds to phenylalanine selectively versus tyrosine (or hydroxyl-phenylalanine) or tryptophan. In certain non-limiting embodiments, a phenylalanine-binding primary aptamer comprises the sequences GG and GGGGG (SEQ ID NO: 168) and GGGG (SEQ ID NO: 169) or a variant thereof and further comprises at least one operative sequence. In certain non-limiting embodiments, a phenylalanine-binding primary aptamer comprises the sequences GG and GGGGG (SEQ ID NO:168) and GGGG (SEQ ID NO:169), or a variant thereof, and further comprises at least one operative sequence, said operative sequence complementary to a sequence comprised in a sensor oligonucleotide. In certain non-limiting embodiments, a phenylalanine-binding primary aptamer comprises the sequences GG and GGGGG (SEQ ID NO: 168) and GGGG (SEQ ID NO: 169), or a variant thereof, and at least one operative sequence on either side (flanking) these three sequences. In certain non-limiting embodiments, a phenylalanine-binding primary aptamer comprises the sequences GG and GGGGG (SEQ ID NO: 168) and GGGG (SEQ ID NO: 169), or a variant thereof, and at least one operative sequence on either side (flanking) these three sequences, where two of said operative sequences contain mutually complementary portions and can form a duplex. For example, but not by way of limitation, a phenylalanine-bidning aptamer may comprise the sequence:

CTC TCG GGA CGA CCG GTG GGG GTT CTT TTT CAG GGG AGG TAC GGT
CGT CCC (SEQ ID NO:3).

In certain non-limiting instances, a phenylalanine-binding primary aptamer comprises a core sequence as set forth in SEQ ID NO: 87 (FIGURE 12B; FIGURE 52A). In certain non-limiting instances, a phenylalanine -binding primary aptamer comprising a core sequence as set forth in SEQ ID NO: 87 (FIGURE 12B) has a binding affinity for phenylalanine that is at least about 50 percent or at least about 75 percent the binding affinity of the primary aptamer having SEQ ID NO:67). In certain non-limiting instances, a phenylalanine -binding primary aptamer comprising a core sequence as set forth in SEQ ID NO:87 (FIGURE 12B) competes with primary aptamer having SEQ ID NO:67 for phenylalanine binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a phenylalanine -binding primary aptamer comprises a core sequence as set forth in SEQ ID NO:87 (FIGURE 12B) and further comprises at least one operative sequence. In certain non-limiting instances, a phenylalanine -binding primary aptamer comprises a core sequence as set forth in SEQ ID NO:87 (FIGURE 12B) and further comprises at least one operative sequence, said operative sequence complementary to a sequence comprised in a sensor oligonucleotide. In certain non-limiting instances, a phenylalanine -binding primary aptamer comprises a core sequence as set forth in SEQ ID NO:87 (FIGURE 12B) and at least one operative sequence on either side (flanking) the core sequence. In certain non-limiting instances, a phenylalanine -binding primary aptamer comprises a core sequence as set forth in SEQ ID NO:87 (FIGURE 12B) and at least one operative sequence on either side (flanking) the core sequence, where two of said operative sequences contain mutually complementary portions and can form a duplex.

In certain non-limiting embodiments, a phenylalanine-binding primary aptamer comprises the sequences GAGG (SEQ ID NO: 170) and CATT (SEQ ID NO: 171) or CCGG (SEQ ID NO: 172) and TGTT (SEQ ID NO: 173) or a variant of any of these sequences that differs in one or two bases by substitution, deletion, insertion or extension, where said primary aptamer binds to phenylalanine in an aqueous solution at room temperature or 25°C with a dissociation constant of less than 10⁻⁴ M and binds to phenylalanine selectively versus tyrosine (or hydroxyl-phenylalanine) or tryptophan. In certain non-limiting embodiments, a phenylalanine-binding primary aptamer comprises the sequences GAGG (SEQ ID NO: 170) and CATT (SEQ ID NO: 171) or CCGG (SEQ ID NO: 172) and TGTT (SEQ ID NO: 173)or a variant thereof and further comprises at least one operative sequence. In certain non-limiting embodiments, a phenylalanine-binding primary aptamer comprises the sequences GAGG (SEQ ID NO: 170) and CATT (SEQ ID NO: 171) or CCGG (SEQ ID NO: 172) and TGTT (SEQ ID NO: 173), or a variant thereof, and further comprises at least one operative sequence, said operative sequence complementary to a sequence comprised in a sensor oligonucleotide. In certain non-limiting embodiments, a phenylalanine-binding primary aptamer comprises the sequences GAGG (SEQ ID NO: 170) and CATT (SEQ ID NO: 171) or CCGG (SEQ ID NO: 172) and TGTT (SEQ ID NO: 173), or a variant thereof, and at least one operative sequence on either side (flanking) these four sequences. In certain non-limiting embodiments, a phenylalanine-binding primary aptamer comprises the sequences GAGG (SEQ ID NO: 170) and CATT (SEQ ID NO: 171) or CCGG (SEQ ID NO: 172) and TGTT (SEQ ID NO: 173), or a variant thereof, and at least one operative sequence on either side (flanking) these four sequences, where two of said operative sequences contain mutually complementary portions and can form a duplex. For example, but not by way of limitation, a phenylalanine-bidning aptamer may comprise the sequence: CTC TCG GGA CGA CGA GGC TGG ATG CAT TCG CCG GAT GTT CGA TGT CGT CCC (SEQ ID NO:4) or related sequence (SEQ ID NO: 174, FIGURE 53A).

In certain non-limiting instances, a phenylalanine-binding primary aptamer comprises a core sequence as set forth in SEQ ID NO: 89 (FIGURE 13B; FIGURE 53A). In certain non-limiting instances, a phenylalanine -binding primary aptamer comprising a core sequence as set forth in SEQ ID NO: 89 (FIGURE 13B) has a binding affinity for phenylalanine that is at least about 50 percent or at least about 75 percent the binding affinity of the primary aptamer having SEQ ID NO: 174). In certain non-limiting instances, a phenylalanine -binding primary aptamer comprising a core sequence as set forth in SEQ ID NO:89 (FIGURE 13B) competes with primary aptamer having SEQ ID NO: 174 for phenylalanine binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a phenylalanine -binding primary aptamer comprises a core sequence as set forth in SEQ ID NO: 89 (FIGURE 13B) and further comprises at least one operative sequence. In certain non-limiting instances, a phenylalanine -binding primary aptamer comprises a core sequence as set forth in SEQ ID NO:89 (FIGURE 13B) and further comprises at least one operative sequence, said operative sequence complementary to a sequence comprised in a sensor oligonucleotide. In certain non-limiting instances, a phenylalanine -binding primary aptamer comprises a core sequence as set forth in SEQ ID NO: 89 (FIGURE 13B) and at least one operative sequence on either side (flanking) the core sequence. In certain non-limiting instances, a phenylalanine -binding primary aptamer comprises a core sequence as set forth in SEQ ID NO:89 (FIGURE 13B) and at least one operative sequence on either side (flanking) the core sequence, where two of said operative sequences contain mutually complementary portions and can form a duplex.

Isolated phenylalanine- binding primary aptamers may comprise the nucleotide sequence of SEQ ID NO:2, SEQ ID NO:3, SEQ ID NO:4, SEQ ID NO: 67, SEQ ID NO: 167, SEQ ID NO: 174 or variants of these sequences having at least about 80 percent, or at least about 85 percent, or at least about 90 percent, or at least about 95 percent, or at least about 98 percent homology to the original sequence, for example obtained by substitutions, deletions, and insertions of Watson-Crick base pairs or by mutations at non-conserved positions. Percent homology can be determined using standard software such as BLAST or FASTA. Isolated phenylalanine-binding primary aptamers may comprise the nucleotide sequence of SEQ ID NO:2, SEQ ID NO:3, SEQ ID NO:4, SEQ ID NO: 67, SEQ ID NO: 167, or SEQ ID NO:174. Said aptamers can bind to phenylalanine and in their structure-switching formats (for example, in an anti-aptamer assay, a pseudosandwich assay, or a sandwich assay) they can respond by an increase in fluorescence.

### 5.1.4 HYDROCORTISONE-BINDING PRIMARY APTAMERS

In certain non-limiting instances, a primary aptamer binds to hydrocortisone in an aqueous solution at room temperature or 25°C with a dissociation constant of less than 10-4 M and binds selectively with corticosterone, 11-deoxycorticosterone and/or (17α,21-dihydroxyprogesterone, and/or that selectively binds to steroids with a C.17 carbon connected to one oxygen versus steroids that do not have an oxygen at C.17 position.

In certain non-limiting instances, a hydrocortisone-binding primary aptamer comprises the sequences CGCC (SEQ ID NO:175) and ATGTTC (SEQ ID NO:176) and GGATAGT(SEQ ID NO:177) or a variant of any of these sequences that differs in one or two bases by substitution, deletion, insertion or extension, where said primary aptamer binds to hydrocortisone in an aqueous solution at room temperature or 25°C with a dissociation constant of less than 10-4 M and binds to hydrocortisone selectively versus steroids that do not have an oxygen at C.17 position (see FIGURE 54B). In certain non-limiting instances, a hydrocortisone-binding primary aptamer comprises the sequences CGCC (SEQ ID NO:175) and ATGTTC (SEQ ID NO:176) and GGATAGT(SEQ ID NO: 177) or a variant thereof and further comprises at least one operative sequence. In certain non-limiting instances, a hydrocortisone-binding primary aptamer comprises the sequences CGCC (SEQ ID NO:175) and ATGTTC (SEQ ID NO:176) and GGATAGT(SEQ ID NO: 177), or a variant thereof , and further comprises at least one operative sequence, said operative sequence complementary to a sequence comprised in a sensor oligonucleotide. In certain non-limiting instances, a hydrocortisone-binding primary aptamer comprises the sequences CGCC (SEQ ID NO:175) and ATGTTC (SEQ ID NO:176) and GGATAGT(SEQ ID NO:177), or a variant thereof, and at least one operative sequence on either side (flanking) these three sequences. In certain non-limiting instances, a hydrocortisone-binding primary aptamer comprises the sequences CGCC (SEQ ID NO:175) and ATGTTC (SEQ ID NO:176) and GGATAGT(SEQ ID NO:177) or a variant thereof, and at least one operative sequence on either side (flanking) these four sequences, where two of said operative sequences contain mutually complementary portions and can form a duplex. For example, but not by way of limitation, a hydrocortisone-bidning aptamer may comprise the sequence: CTC TCG GGA CGA CGC CCG CAT GTT CCA TGG ATA GTC TTG ACT AGT CGT CCC (SEQ ID NO:5, FIGURE 14A) or a short version thereof (FIGURE 54A, SEQ ID NO: 178). In certain non-limiting instances, a hydrocortisone-binding primary aptamer comprises a core sequence as set forth in SEQ ID NO:91 (FIGURE 14B; FIGURE 54A). In certain non-limiting instances, a hydrocortisone -binding primary aptamer comprising a core sequence as set forth in SEQ ID NO:91 (FIGURE 14B) has a binding affinity for hydrocortisone that is at least about 50 percent or at least about 75 percent the binding affinity of the primary aptamer having SEQ ID NO: 178). In certain non-limiting instances, a hydrocortisone -binding primary aptamer comprising a core sequence as set forth in SEQ ID NO:91 (FIGURE 14B) competes with primary aptamer having SEQ ID NO: 178 for hydrocortisone binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a hydrocortisone -binding primary aptamer comprises a core sequence as set forth in SEQ ID NO:91 (FIGURE 14B) and further comprises at least one operative sequence. In certain non-limiting instances, a hydrocortisone -binding primary aptamer comprises a core sequence as set forth in SEQ ID NO:91 (FIGURE 14B) and further comprises at least one operative sequence, said operative sequence complementary to a sequence comprised in a sensor oligonucleotide. In certain non-limiting instances, a hydrocortisone -binding primary aptamer comprises a core sequence as set forth in SEQ ID NO:91 (FIGURE 14B) and at least one operative sequence on either side (flanking) the core sequence. In certain non-limiting instances, a hydrocortisone -binding primary aptamer comprises a core sequence as set forth in SEQ ID NO:91 (FIGURE 14B) and at least one operative sequence on either side (flanking) the core sequence, where two of said operative sequences contain mutually complementary portions and can form a duplex.

In certain non-limiting instances, a hydrocortisone-binding primary aptamer comprises the sequences CGCC (SEQ ID NO: 175) and TACGA(SEQ ID NO: 179) and GGATA (SEQ ID NO: 180) or a variant of any of these sequences that differs in one or two bases by substitution, deletion, insertion or extension, where said primary aptamer binds to hydrocortisone in an aqueous solution at room temperature or 25°C with a dissociation constant of less than 10-4 M and binds to hydrocortisone selectively versus steroids that do not have an oxygen at C. 17 position (see FIGURE 55B) In certain non-limiting instances, a hydrocortisone-binding primary aptamer comprises the sequences CGCC (SEQ ID NO: 175) and TACGA(SEQ ID NO: 179) and GGATA (SEQ ID NO: 180) or a variant thereof and further comprises at least one operative sequence. In certain non-limiting instances, a hydrocortisone-binding primary aptamer comprises the sequences CGCC (SEQ ID NO: 175) and TACGA(SEQ ID NO: 179) and GGATA (SEQ ID NO: 180), or a variant thereof, and further comprises at least one operative sequence, said operative sequence complementary to a sequence comprised in a sensor oligonucleotide. In certain non-limiting instances, a hydrocortisone-binding primary aptamer comprises the sequences CGCC (SEQ ID NO: 175) and TACGA(SEQ ID NO: 179) and GGATA (SEQ ID NO: 180), or a variant thereof, and at least one operative sequence on either side (flanking) these three sequences. In certain non-limiting instances, a hydrocortisone-binding primary aptamer comprises the sequences CGCC (SEQ ID NO: 175) and TACGA(SEQ ID NO: 179) and GGATA (SEQ ID NO: 180) or a variant thereof, and at least one operative sequence on either side (flanking) these four sequences, where two of said operative sequences contain mutually complementary portions and can form a duplex. For example, but not by way of limitation, a hydrocortisone-bidning aptamer may comprise the sequence: CTC TCG GGA CGA CTA GCG TAT GCG CCA GAA GTA TAC GAG GAT AGT CGT CCC (SEQ ID NO:6, FIGURE 15A) or a short version thereof (FIGURE 55A, SEQ ID NO:181).

In certain non-limiting instances, a hydrocortisone-binding primary aptamer comprises a core sequence as set forth in SEQ ID NO:93 (FIGURE 15B; FIGURE 55A). In certain non-limiting instances, a hydrocortisone -binding primary aptamer comprising a core sequence as set forth in SEQ ID NO:93 (FIGURE 15B) has a binding affinity for hydrocortisone that is at least about 50 percent or at least about 75 percent the binding affinity of the primary aptamer having SEQ ID NO: 181). In certain non-limiting instances, a hydrocortisone -binding primary aptamer comprising a core sequence as set forth in SEQ ID NO:93 (FIGURE 15B) competes with primary aptamer having SEQ ID NO: 181 for hydrocortisone binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a hydrocortisone -binding primary aptamer comprises a core sequence as set forth in SEQ ID NO:93 (FIGURE 15B) and further comprises at least one operative sequence. In certain non-limiting instances, a hydrocortisone -binding primary aptamer comprises a core sequence as set forth in SEQ ID NO:93 (FIGURE 15B) and further comprises at least one operative sequence, said operative sequence complementary to a sequence comprised in a sensor oligonucleotide. In certain non-limiting instances, a hydrocortisone -binding primary aptamer comprises a core sequence as set forth in SEQ ID NO:93 (FIGURE 15B) and at least one operative sequence on either side (flanking) the core sequence. In certain non-limiting instances, a hydrocortisone -binding primary aptamer comprises a core sequence as set forth in SEQ ID NO:93 (FIGURE 15B) and at least one operative sequence on either side (flanking) the core sequence, where two of said operative sequences contain mutually complementary portions and can form a duplex.

For example, but not by way of limitation, a hydrocortisone-bidning aptamer may comprise the sequence: CTC TCG GGA CGA CGC CAG AAG TTT ACG AGG ATA TGG TAA CAT AGT CGT CCC (SEQ ID NO:7, FIGURE 16A) or a short version thereof (FIGURE 56A, SEQ ID NO: 182).

In certain non-limiting instances, a hydrocortisone-binding primary aptamer comprises a core sequence as set forth in SEQ ID NO:95 (FIGURE 16B; FIGURE 56A-B). In certain non-limiting instances, a hydrocortisone -binding primary aptamer comprising a core sequence as set forth in SEQ ID NO:95 (FIGURE 16B) has a binding affinity for hydrocortisone that is at least about 50 percent or at least about 75 percent the binding affinity of the primary aptamer having SEQ ID NO: 182). In certain non-limiting instances, a hydrocortisone -binding primary aptamer comprising a core sequence as set forth in SEQ ID NO:95 (FIGURE 16B) competes with primary aptamer having SEQ ID NO: 182 for hydrocortisone binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a hydrocortisone -binding primary aptamer comprises a core sequence as set forth in SEQ ID NO:95 (FIGURE 16B) and further comprises at least one operative sequence. In certain non-limiting instances, a hydrocortisone -binding primary aptamer comprises a core sequence as set forth in SEQ ID NO:95 (FIGURE 16B) and further comprises at least one operative sequence, said operative sequence complementary to a sequence comprised in a sensor oligonucleotide. In certain non-limiting instances, a hydrocortisone -binding primary aptamer comprises a core sequence as set forth in SEQ ID NO:95 (FIGURE 16B) and at least one operative sequence on either side (flanking) the core sequence. In certain non-limiting instances, a hydrocortisone -binding primary aptamer comprises a core sequence as set forth in SEQ ID NO:95 (FIGURE 16B) and at least one operative sequence on either side (flanking) the core sequence, where two of said operative sequences contain mutually complementary portions and can form a duplex.

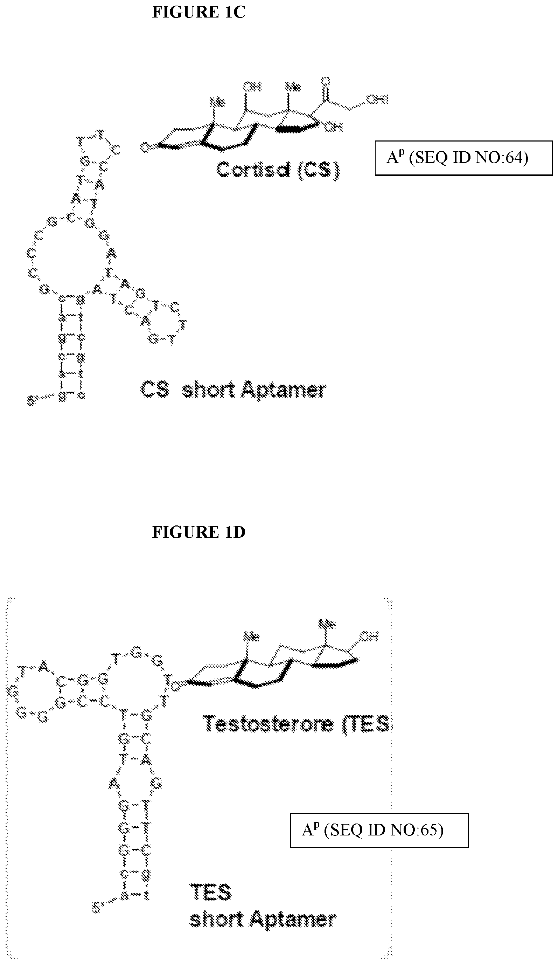

In certain non-limiting instances, isolated hydrocortisone- binding primary aptamers comprise the nucleotide sequence of SEQ ID NO:5, SEQ ID NO:6, SEQ ID NO:7, SEQ ID NO:64 (FIGURE 1C), SEQ ID NO: 181, SEQ ID NO: 148. or SEQ ID NO: 182, or variants of these sequences having at least about 80 percent, or at least about 85 percent, or at least about 90 percent, or at least about 95 percent, or at least about 98 percent homology to the original sequence, for example obtained by substitutions, deletions, and insertions of Watson-Crick base pairs or by mutations at non-conserved positions. Percent homology can be determined using standard software such as BLAST or FASTA. In certain non-limiting instances, isolated hydrocortisone-binding primary aptamers comprise the nucleotide sequence of SEQ ID NO:5, SEQ ID NO:6, SEQ ID NO:7, SEQ ID NO:64 (FIGURE 1C), SEQ ID NO: 181, SEQ ID NO: 148. or SEQ ID NO: 182. Said aptamers can bind to hydrocortisone and in their structure-switching formats (for example, in an anti-aptamer assay, a pseudosandwich assay, or a sandwich assay) they can respond by an increase in fluorescence.

### 5.1.5. DEHYDROISOANDROSTERONE-BINDING PRIMARY APTAMERS

In certain non-limiting instances, a primary aptamer binds to dehydroisoandrosterone in an aqueous solution at room temperature or 25°C with a dissociation constant of less than 10⁻⁴ M and binds selectively with dehydroisoandrosterone versus deoxycorticosterone.

In certain non-limiting instances, a dehydroisoandrosterone-binding primary aptamer comprises the sequences GGG, GGGG (SEQ ID NO:169) and GG or a variant of any of these sequences that differs in one or two bases by substitution, deletion, insertion or extension, where said primary aptamer binds to dehydroisoandrosterone in an aqueous solution at room temperature or 25°C with a dissociation constant of less than 10⁻⁴ M and binds to dehydroisoandrosterone selectively versus deoxycorticosterone and/or other steroids (see FIGURES 57B, 58B and 59B). In certain non-limiting instances, a dehydroisoandrosterone-binding primary aptamer comprises the sequences GGG, GGGG (SEQ ID NO: 169) and GG or a variant thereof and further comprises at least one operative sequence. In certain non-limiting instances, a dehydroisoandrosterone-binding primary aptamer comprises the sequences GGG, GGGG (SEQ ID NO: 169) and GG, or a variant thereof, and further comprises at least one operative sequence, said operative sequence complementary to a sequence comprised in a sensor oligonucleotide. In certain non-limiting instances, a dehydroisoandrosterone-binding primary aptamer comprises the sequences GGG, GGGG (SEQ ID NO: 169) and GG, or a variant thereof, and at least one operative sequence on either side (flanking) these three sequences. In certain non-limiting instances, a dehydroisoandrosterone-binding primary aptamer comprises the sequences GGG, GGGG (SEQ ID NO: 169) and GG, or a variant thereof, and at least one operative sequence on either side (flanking) these three sequences, where two of said operative sequences contain mutually complementary portions and can form a duplex.

For example, but not by way of limitation, a dehydroisoandrosterone-bidning aptamer may comprise the sequence: CTC TCG GGA CGA CGG GGG TGG CAT AGG GTA GGC TAG GGT CAC TGT CGT CCC (SEQ ID NO:9) or related sequence (SEQ ID NO: 183, FIGURE 57A). In certain non-limiting instances, a dehydroisoandrosterone-binding primary aptamer comprises a core sequence as set forth in SEQ ID NO:99 (FIGURE 18B; FIGURE 57A). In certain non-limiting instances, a dehydroisoandrosterone -binding primary aptamer comprising a core sequence as set forth in SEQ ID NO:99 (FIGURE 18B) has a binding affinity for dehydroisoandrosterone that is at least about 50 percent or at least about 75 percent the binding affinity of the primary aptamer having SEQ ID NO: 183). In certain non-limiting instances, a dehydroisoandrosterone -binding primary aptamer comprising a core sequence as set forth in SEQ ID NO:99 (FIGURE 18B) competes with primary aptamer having SEQ ID NO: 183 for dehydroisoandrosterone binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a dehydroisoandrosterone -binding primary aptamer comprises a core sequence as set forth in SEQ ID NO:99 (FIGURE 18B) and further comprises at least one operative sequence. In certain non-limiting instances, a dehydroisoandrosterone -binding primary aptamer comprises a core sequence as set forth in SEQ ID NO:99 (FIGURE 18B) and further comprises at least one operative sequence, said operative sequence complementary to a sequence comprised in a sensor oligonucleotide. In certain non-limiting instances, a dehydroisoandrosterone -binding primary aptamer comprises a core sequence as set forth in SEQ ID NO:99 (FIGURE 18B) and at least one operative sequence on either side (flanking) the core sequence. In certain non-limiting instances, a dehydroisoandrosterone -binding primary aptamer comprises a core sequence as set forth in SEQ ID NO:99 (FIGURE 18B) and at least one operative sequence on either side (flanking) the core sequence, where two of said operative sequences contain mutually complementary portions and can form a duplex.

For example, but not by way of limitation, a dehydroisoandrosterone-bidning aptamer may comprise the sequence: CTC TCG GGA CGA CGT GGC TAG GTA GGT TGC ATG CGG CAT AGG GGT CGT CCC (SEQ ID NO: 10) or related sequence (SEQ ID NO: 184, FIGURE 58A). In certain non-limiting instances, a dehydroisoandrosterone-binding primary aptamer comprises a core sequence as set forth in SEQ ID NO: 101 (FIGURE 19B; FIGURE 58A). In certain non-limiting instances, a dehydroisoandrosterone -binding primary aptamer comprising a core sequence as set forth in SEQ ID NO: 101 (FIGURE 19B) has a binding affinity for dehydroisoandrosterone that is at least about 50 percent or at least about 75 percent the binding affinity of the primary aptamer having SEQ ID NO: 184). In certain non-limiting instances, a dehydroisoandrosterone -binding primary aptamer comprising a core sequence as set forth in SEQ ID NO: 101 (FIGURE 19B) competes with primary aptamer having SEQ ID NO: 184 for dehydroisoandrosterone binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a dehydroisoandrosterone -binding primary aptamer comprises a core sequence as set forth in SEQ ID NO: 101 (FIGURE 19B) and further comprises at least one operative sequence. In certain non-limiting instances, a dehydroisoandrosterone -binding primary aptamer comprises a core sequence as set forth in SEQ ID NO: 101 (FIGURE 19B) and further comprises at least one operative sequence, said operative sequence complementary to a sequence comprised in a sensor oligonucleotide. In certain non-limiting instances, a dehydroisoandrosterone -binding primary aptamer comprises a core sequence as set forth in SEQ ID NO: 101 (FIGURE 19B) and at least one operative sequence on either side (flanking) the core sequence. In certain non-limiting instances, a dehydroisoandrosterone -binding primary aptamer comprises a core sequence as set forth in SEQ ID NO: 101 (FIGURE 19B) and at least one operative sequence on either side (flanking) the core sequence, where two of said operative sequences contain mutually complementary portions and can form a duplex.

For example, but not by way of limitation, a dehydroisoandrosterone-bidning aptamer may comprise the sequence: CTC TCG GGA CGA CGT GAC GGT GTG TAG TTG GGT TGT GGC AGG AGT CGT CCC (SEQ ID NO:11) or related sequence (SEQ ID NO:185, FIGURE 59A). In certain non-limiting instances, a dehydroisoandrosterone-binding primary aptamer comprises a core sequence as set forth in SEQ ID NO: 103 (FIGURE 20B; FIGURE 59A). In certain non-limiting instances, a dehydroisoandrosterone -binding primary aptamer comprising a core sequence as set forth in SEQ ID NO:103 (FIGURE 20B) has a binding affinity for dehydroisoandrosterone that is at least about 50 percent or at least about 75 percent the binding affinity of the primary aptamer having SEQ ID NO:185). In certain non-limiting instances, a dehydroisoandrosterone -binding primary aptamer comprising a core sequence as set forth in SEQ ID NO:103 (FIGURE 20B) competes with primary aptamer having SEQ ID NO: 185 for dehydroisoandrosterone binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a dehydroisoandrosterone -binding primary aptamer comprises a core sequence as set forth in SEQ ID NO:103 (FIGURE 20B) and further comprises at least one operative sequence. In certain non-limiting instances, a dehydroisoandrosterone -binding primary aptamer comprises a core sequence as set forth in SEQ ID NO:103 (FIGURE 20B) and further comprises at least one operative sequence, said operative sequence complementary to a sequence comprised in a sensor oligonucleotide. In certain non-limiting instances, a dehydroisoandrosterone -binding primary aptamer comprises a core sequence as set forth in SEQ ID NO:103 (FIGURE 20B) and at least one operative sequence on either side (flanking) the core sequence. In certain non-limiting instances, a dehydroisoandrosterone -binding primary aptamer comprises a core sequence as set forth in SEQ ID NO: 103 (FIGURE 20B) and at least one operative sequence on either side (flanking) the core sequence, where two of said operative sequences contain mutually complementary portions and can form a duplex.

In certain non-limiting instances, isolated dehydroisoandrosterone- binding primary aptamers comprise the nucleotide sequence of SEQ ID NO:9, SEQ ID NO:10, SEQ ID NO:11, SEQ ID NO: 183, SEQ ID NO: 184, SEQ ID NO:185 or variants of these sequences having at least about 80 percent, or at least about 85 percent, or at least about 90 percent, or at least about 95 percent, or at least about 98 percent homology to the original sequence, for example obtained by substitutions, deletions, and insertions of Watson-Crick base pairs or by mutations at non-conserved positions. Percent homology can be determined using standard software such as BLAST or FASTA. In certain non-limiting instances, isolated dehydroisoandrosterone-binding primary aptamers comprise the nucleotide sequence of SEQ ID NO:9, SEQ ID NO:10, SEQ ID NO:11, SEQ ID NO: 183, SEQ ID NO: 184, or SEQ ID NO:185. Said aptamers can bind to dehydroisoandrosterone and in their structure-switching formats (for example, in an anti-aptamer assay, a pseudosandwich assay, or a sandwich assay) they can respond by an increase in fluorescence.

### 5.1.6 DEOXYCORTICOSTERONE-BINDING PRIMARY APTAMERS

In certain non-limiting embodiments, a primary aptamer binds to deoxycorticosterone in an aqueous solution at room temperature or 25°C with a dissociation constant of less than 10⁻⁴ M and binds selectively with deoxycorticosterone versus dehydroisoandrosterone.

In certain non-limiting embodiments, a deoxycorticosterone-binding primary aptamer comprises the sequences AGCT (SEQ ID NO:186) and GCGG (SEQ ID NO:187) or a variant of any of these sequences that differs in one or two bases by substitution, deletion, insertion or extension, where said primary aptamer binds to deoxycorticosterone in an aqueous solution at room temperature or 25°C with a dissociation constant of less than 10⁻⁴ M and binds to deoxycorticosterone selectively versus dehydroisoandrosterone and/or other steroids (see FIGURES 60B, 61B). In certain non-limiting embodiments, a deoxycorticosterone-binding primary aptamer comprises the sequences AGCT (SEQ ID NO:186) and GCGG (SEQ ID NO:187) or a variant thereof and further comprises at least one operative sequence. In certain non-limiting embodiments, a deoxycorticosterone-binding primary aptamer comprises the sequences AGCT (SEQ ID NO:186) and GCGG (SEQ ID NO:187), or a variant thereof , and further comprises at least one operative sequence, said operative sequence complementary to a sequence comprised in a sensor oligonucleotide. In certain non-limiting embodiments, a deoxycorticosterone-binding primary aptamer comprises the sequences AGCT (SEQ ID NO: 186) and GCGG (SEQ ID NO: 187), or a variant thereof, and at least one operative sequence on either side (flanking) these two sequences. In certain non-limiting embodiments, a deoxycorticosterone-binding primary aptamer comprises the sequences AGCT (SEQ ID NO: 186) and GCGG (SEQ ID NO: 187), or a variant thereof, and at least one operative sequence on either side (flanking) these two sequences, where two of said operative sequences contain mutually complementary portions and can form a duplex.

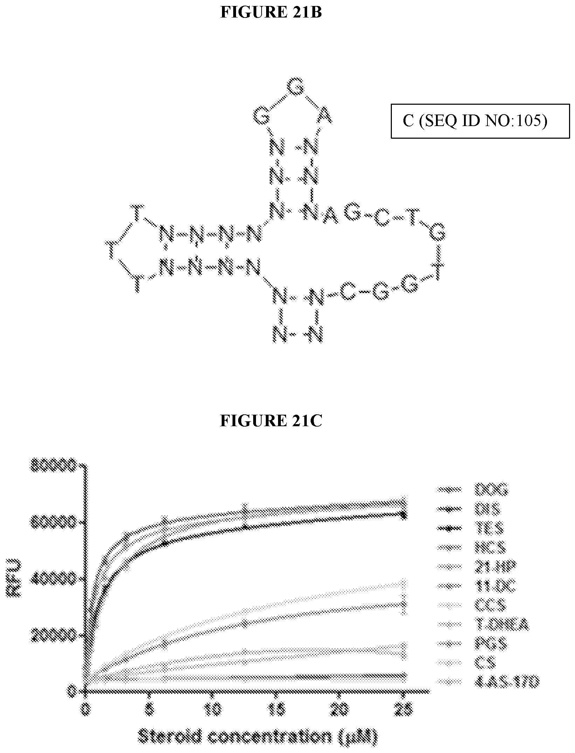

For example, but not by way of limitation, a deoxycorticosterone-bidning aptamer may comprise the sequence: CTC TCG GGA CGA CCC GGA TTT TCC GAG TGG AAC TAG CTG TGG CGG TCG TCC C (SEQ ID NO: 12) or related sequence (e.g., SEQ ID NO: 188, FIGURE 60A; SEQ ID NO:62, FIGURE 1A). In certain non-limiting instances, a deoxycorticosterone-binding primary aptamer comprises a core sequence as set forth in SEQ ID NO: 105 (FIGURE 21B; FIGURE 60A). In certain non-limiting instances, a deoxycorticosterone -binding primary aptamer comprising a core sequence as set forth in SEQ ID NO: 105 (FIGURE 21B) has a binding affinity for deoxycorticosterone that is at least about 50 percent or at least about 75 percent the binding affinity of the primary aptamer having SEQ ID NO: 188. In certain non-limiting instances, a deoxycorticosterone -binding primary aptamer comprising a core sequence as set forth in SEQ ID NO: 105 (FIGURE 21B) competes with primary aptamer having SEQ ID NO: 188 for deoxycorticosterone binding. In non-limiting embodiments, said primary aptamer has a length of between about 30 and about 100 nucleotides, or between about 30 and 80 nucleotides, or between about 30 and 70 nucleotides, or between about 30 and 60 nucleotides. In certain non-limiting instances, a deoxycorticosterone -binding primary aptamer comprises a core sequence as set forth in SEQ ID NO: 105 (FIGURE 21B) and further comprises at least one operative sequence. In certain non-limiting instances, a deoxycorticosterone -binding primary aptamer comprises a core sequence as set forth in SEQ ID NO: 105 (FIGURE 21B) and further comprises at least one operative sequence, said operative sequence complementary to a sequence comprised in a sensor oligonucleotide. In certain non-limiting instances, a deoxycorticosterone -binding primary aptamer comprises a core sequence as set forth in SEQ ID NO: 105 (FIGURE 21B) and at least one operative sequence on either side (flanking) the core sequence. In certain non-limiting instances, a deoxycorticosterone -binding primary aptamer comprises a core sequence as set forth in SEQ ID NO:105 (FIGURE 21B) and at least one operative sequence on either side (flanking) the core sequence, where two of said operative sequences contain mutually complementary portions and can form a duplex.

For example, but not by way of limitation, a deoxycorticosterone-bidning aptamer may comprise the sequence: CTC TCG GGA CGA CGG GGA TTT TCC AGT GCA ACT AGC TGA AAG CGG TCG TCC C (SEQ ID NO: 262).

For example, but not by way of limitation, a deoxycorticosterone-bidning aptamer may comprise the sequence: CTC TCG GGA CGA CCA GGA TTT TCC AGT GTA ACT AGC TAC AGC GGG TCG TCC C (SEQ ID NO: 263).

In certain non-limiting embodiments, isolated deoxycorticosterone- binding primary aptamers comprise the nucleotide sequence of SEQ ID NO:12, SEQ ID NO:62, SEQ ID NO:188, SEQ ID NO: 189, SEQ ID NO: 190, or variants of these sequences having at least about 80 percent, or at least about 85 percent, or at least about 90 percent, or at least about 95 percent, or at least about 98 percent homology to the original sequence, for example obtained by substitutions, deletions, and insertions of Watson-Crick base pairs or by mutations at non-conserved positions. Percent homology can be determined using standard software such as BLAST or FASTA. Isolated deoxycorticosterone-binding primary aptamers may comprise the nucleotide sequence of SEQ ID NO:12, SEQ ID NO:62, SEQ ID NO:188, SEQ ID NO: 189, or SEQ ID NO: 190. Said aptamers can bind to deoxycorticosterone and in their structure-switching formats (for example, in an anti-aptamer assay, a pseudosandwich assay, or a sandwich assay) they can respond by an increase in fluorescence.

### 5.1.7 TESTOSTERONE-BINDING PRIMARY APTAMERS

In certain non-limiting embodiments, a primary aptamer binds to testosterone in an aqueous solution at room temperature or 25°C with a dissociation constant of less than 10⁻⁴ M and binds selectively with testosterone versus 11-deoxycorticosterone.

In certain non-limiting embodiments, a testosterone-binding primary aptamer comprises the sequences GGG and GGGG (SEQ ID NO:169) and GG, or a variant of any of these sequences that differs in one or two bases by substitution, deletion, insertion or extension, including addition or deletion of a G residue, where said primary aptamer binds to testosterone in an aqueous solution at room temperature or 25°C with a dissociation constant of less than 10⁻⁴ M and binds to testosterone (see FIGURES 62B and 63B) selectively versus 11-deoxycorticosterone. In certain non-limiting embodiments, a testosterone-binding primary aptamer comprises the sequences GGG and GGGG (SEQ ID NO: 169) and GG or a variant thereof and further comprises at least one operative sequence. In certain non-limiting embodiments, a testosterone-binding primary aptamer comprises the sequences GGG and GGGG (SEQ ID NO: 169) and GG, or a variant thereof, and further comprises at least one operative sequence, said operative sequence complementary to a sequence comprised in a sensor oligonucleotide. In certain non-limiting embodiments, a testosterone-binding primary aptamer comprises the sequences GGG and GGGG (SEQ ID NO: 169) and GG, or a variant thereof, and at least one operative sequence on either side (flanking) these three sequences. In certain non-limiting embodiments, a testosterone-binding primary aptamer comprises the sequences GGG and GGGG (SEQ ID NO: 169) and GG, or a variant thereof, and at least one operative sequence on either side (flanking) these three sequences, where two of said operative sequences contain mutually complementary portions and can form a duplex.

For example, but not by way of limitation, a testosterone-binding aptamer may comprise the sequence: CTC TCG GGA CGA CGG GAT GTC CGG GGT ACG GTG GTT GCA GTT CGT CGT CCC (SEQ ID NO: 13) or a related sequence (e.g., SEQ ID NO:65, FIGURE 1D; SEQ ID NO: 191, FIGURE 62A). In certain non-limiting instances, a testosterone-binding primary aptamer comprises a core sequence as set forth in SEQ ID NO: 107 (FIGURE 22B; FIGURE 62A). In certain non-limiting instances, a testosterone - binding primary aptamer comprising a core sequence as set forth in SEQ ID NO: 107 (FIGURE 22B) has a binding affinity for testosterone that is at least about 50 percent or at least about 75 percent the binding affinity of the primary aptamer having SEQ ID NO: 191). In certain non-limiting instances, a testosterone -binding primary aptamer comprising a core sequence as set forth in SEQ ID NO: 107 (FIGURE 22B) competes with primary aptamer having SEQ ID NO: 191 for testosterone binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a testosterone -binding primary aptamer comprises a core sequence as set forth in SEQ ID NO: 107 (FIGURE 22B) and further comprises at least one operative sequence. In certain non-limiting instances, a testosterone -binding primary aptamer comprises a core sequence as set forth in SEQ ID NO:107 (FIGURE 22B) and further comprises at least one operative sequence, said operative sequence complementary to a sequence comprised in a sensor oligonucleotide. In certain non-limiting instances, a testosterone -binding primary aptamer comprises a core sequence as set forth in SEQ ID NO:107 (FIGURE 22B) and at least one operative sequence on either side (flanking) the core sequence. In certain non-limiting instances, a testosterone -binding primary aptamer comprises a core sequence as set forth in SEQ ID NO:107 (FIGURE 22B) and at least one operative sequence on either side (flanking) the core sequence, where two of said operative sequences contain mutually complementary portions and can form a duplex.

For example, but not by way of limitation, a testosterone-binding aptamer may comprise the sequence: CTC TCG GGA CGA CGG GTG GTC ATT GAG TGG TCT TAG GCA GGT AGT CGT CCC (SEQ ID NO:17) or related sequence (SEQ ID NO:192, FIGURE 63A). In certain non-limiting instances, a testosterone-binding primary aptamer comprises a core sequence as set forth in SEQ ID NO:115 (FIGURE 26B; FIGURE 63A). In certain non-limiting instances, a testosterone -binding primary aptamer comprising a core sequence as set forth in SEQ ID NO:115 (FIGURE 26B) has a binding affinity for testosterone that is at least about 50 percent or at least about 75 percent the binding affinity of the primary aptamer having SEQ ID NO:192). In certain non-limiting instances, a testosterone -binding primary aptamer comprising a core sequence as set forth in SEQ ID NO:115 (FIGURE 26B) competes with primary aptamer having SEQ ID NO:192 for testosterone binding. In non-limiting embodiments, said primary aptamer has a length of between about 30 and about 100 nucleotides, or between about 30 and 80 nucleotides, or between about 30 and 70 nucleotides, or between about 30 and 60 nucleotides. In certain non-limiting instances, a testosterone -binding primary aptamer comprises a core sequence as set forth in SEQ ID NO:115 (FIGURE 26B) and further comprises at least one operative sequence. In certain non-limiting instances, a testosterone -binding primary aptamer comprises a core sequence as set forth in SEQ ID NO:115 (FIGURE 26B) and further comprises at least one operative sequence, said operative sequence complementary to a sequence comprised in a sensor oligonucleotide. In certain non-limiting instances, a testosterone -binding primary aptamer comprises a core sequence as set forth in SEQ ID NO:115 (FIGURE 26B) and at least one operative sequence on either side (flanking) the core sequence. In certain non-limiting instances, a testosterone -binding primary aptamer comprises a core sequence as set forth in SEQ ID NO: 115 (FIGURE 26B) and at least one operative sequence on either side (flanking) the core sequence, where two of said operative sequences contain mutually complementary portions and can form a duplex.

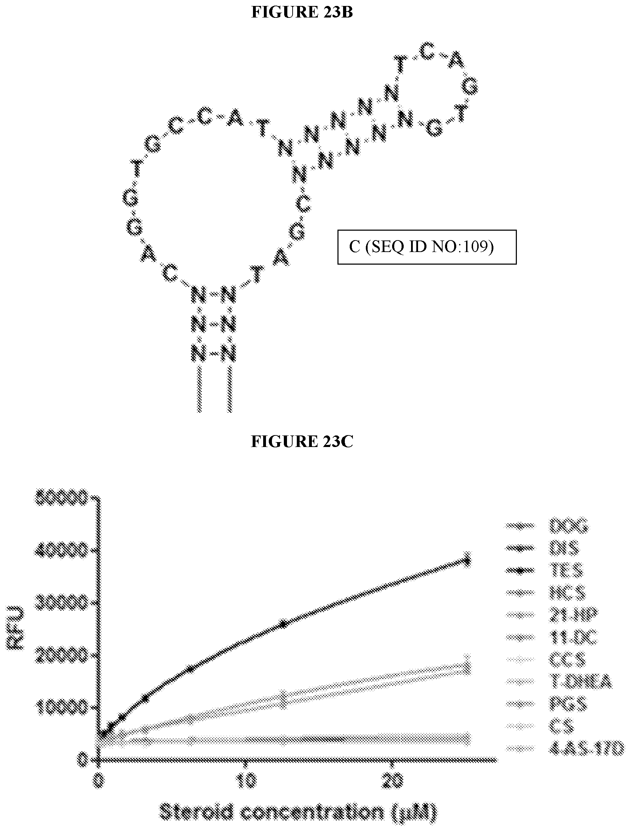

In certain non-limiting instances, a testosterone-binding primary aptamer comprises a core sequence as set forth in SEQ ID NO: 109 (FIGURE 23B). In certain non-limiting instances, a testosterone -binding primary aptamer comprising a core sequence as set forth in SEQ ID NO:109 (FIGURE 23B) has a binding affinity for testosterone that is at least about 50 percent or at least about 75 percent the binding affinity of the primary aptamer having SEQ ID NO: 14). In certain non-limiting instances, a testosterone -binding primary aptamer comprising a core sequence as set forth in SEQ ID NO: 109 (FIGURE 23B) competes with primary aptamer having SEQ ID NO: 14 for testosterone binding. In non-limiting embodiments, said primary aptamer has a length of between about 30 and about 100 nucleotides, or between about 30 and 80 nucleotides, or between about 30 and 70 nucleotides, or between about 30 and 60 nucleotides. In certain non-limiting instances, a testosterone -binding primary aptamer comprises a core sequence as set forth in SEQ ID NO:109 (FIGURE 23B) and further comprises at least one operative sequence. In certain non-limiting instances, a testosterone -binding primary aptamer comprises a core sequence as set forth in SEQ ID NO: 109 (FIGURE 23B) and further comprises at least one operative sequence, said operative sequence complementary to a sequence comprised in a sensor oligonucleotide.

In certain non-limiting instances, a testosterone -binding primary aptamer comprises a core sequence as set forth in SEQ ID NO: 109 (FIGURE 23B) and at least one operative sequence on either side (flanking) the core sequence. In certain non-limiting instances, a testosterone -binding primary aptamer comprises a core sequence as set forth in SEQ ID NO:109 (FIGURE 23B) and at least one operative sequence on either side (flanking) the core sequence, where two of said operative sequences contain mutually complementary portions and can form a duplex.

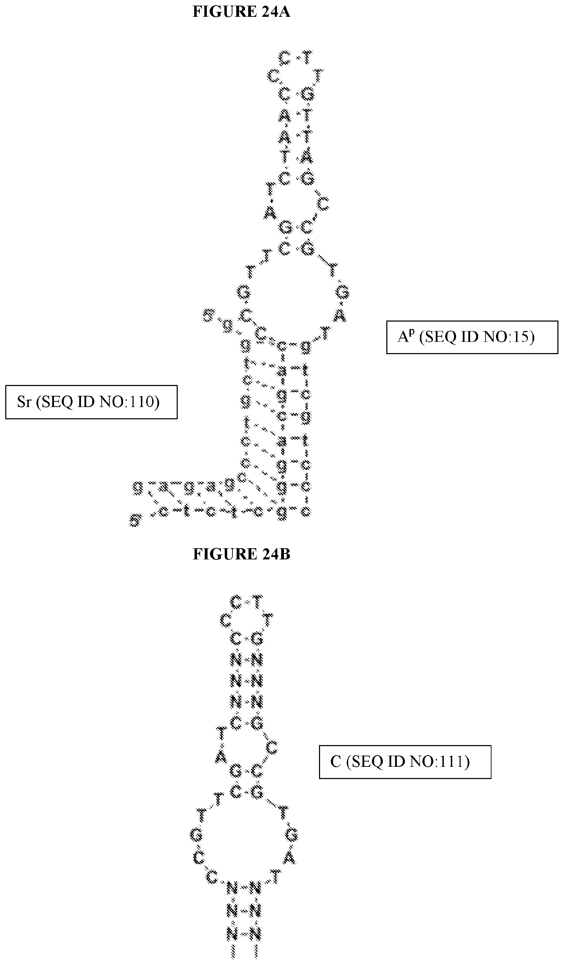

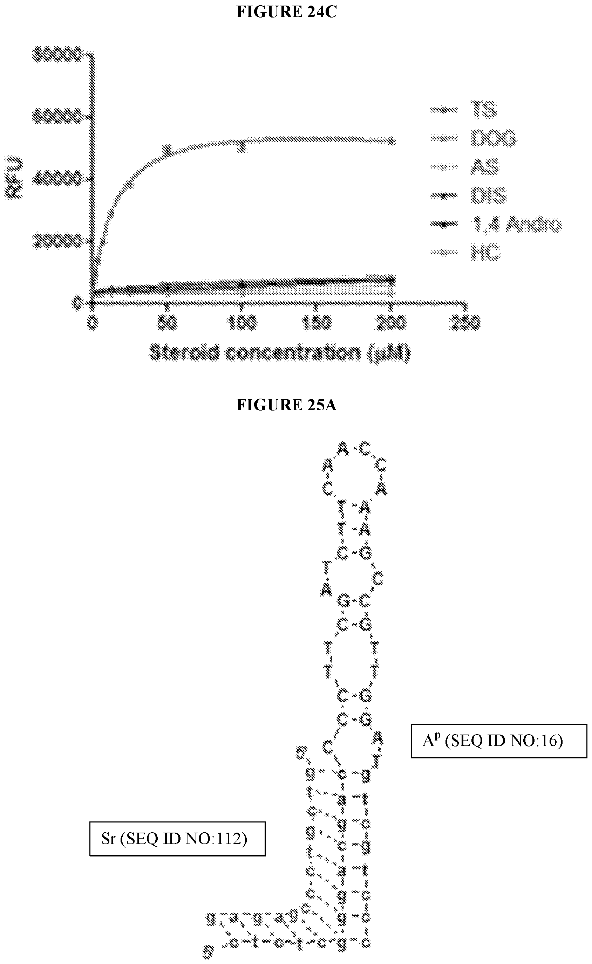

In certain non-limiting instances, a testosterone-binding primary aptamer comprises a core sequence as set forth in SEQ ID NO: 111 (FIGURE 24B). In certain non-limiting instances, a testosterone -binding primary aptamer comprising a core sequence as set forth in SEQ ID NO:111 (FIGURE 24B) has a binding affinity for testosterone that is at least about 50 percent or at least about 75 percent the binding affinity of the primary aptamer having SEQ ID NO:15). In certain non-limiting instances, a testosterone -binding primary aptamer comprising a core sequence as set forth in SEQ ID NO:111 (FIGURE 24B) competes with primary aptamer having SEQ ID NO:15 for testosterone binding. In non-limiting embodiments, said primary aptamer has a length of between about 30 and about 100 nucleotides, or between about 30 and 80 nucleotides, or between about 30 and 70 nucleotides, or between about 30 and 60 nucleotides. In certain non-limiting instances, a testosterone -binding primary aptamer comprises a core sequence as set forth in SEQ ID NO:111 (FIGURE 24B) and further comprises at least one operative sequence. In certain non-limiting instances, a testosterone -binding primary aptamer comprises a core sequence as set forth in SEQ ID NO:111 (FIGURE 24B) and further comprises at least one operative sequence, said operative sequence complementary to a sequence comprised in a sensor oligonucleotide. In certain non-limiting instances, a testosterone -binding primary aptamer comprises a core sequence as set forth in SEQ ID NO:111 (FIGURE 24B) and at least one operative sequence on either side (flanking) the core sequence. In certain non-limiting instances, a testosterone - binding primary aptamer comprises a core sequence as set forth in SEQ ID NO: 111 (FIGURE 24B) and at least one operative sequence on either side (flanking) the core sequence, where two of said operative sequences contain mutually complementary portions and can form a duplex.

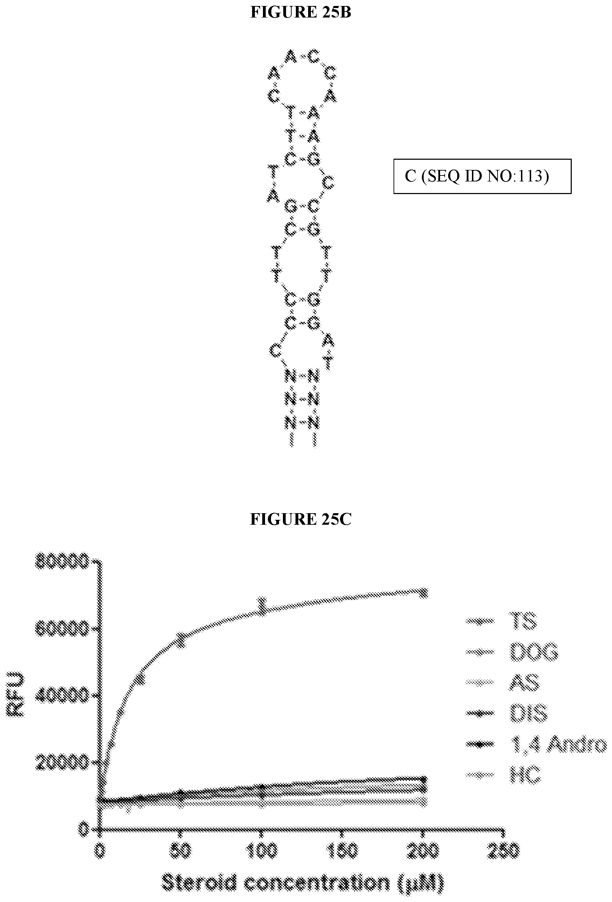

In certain non-limiting instances, a testosterone-binding primary aptamer comprises a core sequence as set forth in SEQ ID NO: 113 (FIGURE 25B). In certain non-limiting instances, a testosterone -binding primary aptamer comprising a core sequence as set forth in SEQ ID NO:111 (FIGURE 25B) has a binding affinity for testosterone that is at least about 50 percent or at least about 75 percent the binding affinity of the primary aptamer having SEQ ID NO:16). In certain non-limiting instances, a testosterone -binding primary aptamer comprising a core sequence as set forth in SEQ ID NO:113 (FIGURE 25B) competes with primary aptamer having SEQ ID NO:16 for testosterone binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a testosterone -binding primary aptamer comprises a core sequence as set forth in SEQ ID NO:113 (FIGURE 25B) and further comprises at least one operative sequence. In certain non-limiting instances, a testosterone -binding primary aptamer comprises a core sequence as set forth in SEQ ID NO:113 (FIGURE 25B) and further comprises at least one operative sequence, said operative sequence complementary to a sequence comprised in a sensor oligonucleotide. In certain non-limiting instances, a testosterone -binding primary aptamer comprises a core sequence as set forth in SEQ ID NO:113 (FIGURE 25B) and at least one operative sequence on either side (flanking) the core sequence. In certain non-limiting instances, a testosterone - binding primary aptamer comprises a core sequence as set forth in SEQ ID NO: 113 (FIGURE 25B) and at least one operative sequence on either side (flanking) the core sequence, where two of said operative sequences contain mutually complementary portions and can form a duplex.

Isolated testosterone- binding primary aptamers may comprise the nucleotide sequence of SEQ ID NO:13, SEQ ID NO:14, SEQ ID NO:15, SEQ ID NO: 16, SEQ ID NO: 17, SEQ ID NO: 65, SEQ ID NO:191, or SEQ ID NO:192, or variants of these sequences having at least about 80 percent, or at least about 85 percent, or at least about 90 percent, or at least about 95 percent, or at least about 98 percent homology to the original sequence, for example obtained by substitutions, deletions, and insertions of Watson-Crick base pairs or by mutations at non-conserved positions. Percent homology can be determined using standard software such as BLAST or FASTA. Isolated testosterone-binding primary aptamers may comprise the nucleotide sequence of SEQ ID NO: 13, SEQ ID NO:14, SEQ ID NO:15, SEQ ID NO: 16, SEQ ID NO: 17, SEQ ID NO:65, SEQ ID NO:191, or SEQ ID NO:192. Said aptamers can bind to testosterone and in their structure-switching formats (for example, in an anti-aptamer assay, a pseudosandwich assay, or a sandwich assay) they can respond by an increase in fluorescence.

### 5.1.8 SPHINGOSINE-1-PHOSPHATE-BINDING PRIMARY APTAMERS

In certain non-limiting instances, a primary aptamer binds to sphingosine-1-phosphate in an aqueous solution at room temperature or 25°C with a dissociation constant of less than 10⁻⁴ M (see FIGURE 64B).

In certain non-limiting instances, a sphingosine-1-phosphate-binding primary aptamer comprises the sequences GG and GGGG(SEQ ID NO:169) and GGGGG (SEQ ID NO:168) or a variant of any of these sequences that differs in one or two bases by substitution, deletion, insertion or extension, where said primary aptamer binds to sphingosine-1-phosphate in an aqueous solution at room temperature or 25°C with a dissociation constant of less than 10⁻⁴ M and binds selectively to sphingosine-1-phosphate. In certain non-limiting instances, a sphingosine-1-phosphate-binding primary aptamer comprises the sequences GG and GGGG(SEQ ID NO:169) and GGGGG (SEQ ID NO:168) or a variant thereof and further comprises at least one operative sequence. In certain non-limiting instances, a sphingosine-1-phosphate-binding primary aptamer comprises the sequences GG and GGGG(SEQ ID NO:169) and GGGGG (SEQ ID NO:168), or a variant thereof, and further comprises at least one operative sequence, said operative sequence complementary to a sequence comprised in a sensor oligonucleotide. In certain non-limiting instances, a sphingosine-1-phosphate-binding primary aptamer comprises the sequences GG and GGGG(SEQ ID NO:169) and GGGGG (SEQ ID NO:168), or a variant thereof, and at least one operative sequence on either side (flanking) these three sequences. In certain non-limiting instances, a sphingosine-1-phosphate-binding primary aptamer comprises the sequences GG and GGGG(SEQ ID NO:169) and GGGGG (SEQ ID NO:168) or a variant thereof, and at least one operative sequence on either side (flanking) these three sequences, where two of said operative sequences contain mutually complementary portions and can form a duplex.

For example, but not by way of limitation, a sphingosine-1-phosphate-binding aptamer may comprise the sequence: CTC TCG GGA CGA CGT GGT GTG GGA GAA AGA ATT TTC ATT GGG GTA GGG GGT CGT CCC (SEQ ID NO:18) or related sequence (SEQ ID NO:193, FIGURE 64A).

In certain non-limiting instances, a sphingosine-1-phosphate-binding primary aptamer comprises a core sequence as set forth in SEQ ID NO:117 (FIGURE 27B; FIGURE 64A). In certain non-limiting instances, a sphingosine-1-phosphate -binding primary aptamer comprising a core sequence as set forth in SEQ ID NO:117 (FIGURE 27B) has a binding affinity for sphingosine-1-phosphate that is at least about 50 percent or at least about 75 percent the binding affinity of the primary aptamer having SEQ ID NO:193). In certain non-limiting instances, a sphingosine-1-phosphate -binding primary aptamer comprising a core sequence as set forth in SEQ ID NO:117 (FIGURE 27B) competes with primary aptamer having SEQ ID NO:193 for sphingosine-1-phosphate binding. In non-limiting embodiments, said primary aptamer has a length of between about 30 and about 100 nucleotides, or between about 30 and 80 nucleotides, or between about 30 and 70 nucleotides, or between about 30 and 60 nucleotides. In certain non-limiting instances, a sphingosine-1-phosphate -binding primary aptamer comprises a core sequence as set forth in SEQ ID NO:117 (FIGURE 27B) and further comprises at least one operative sequence. In certain non-limiting instances, a sphingosine-1-phosphate - binding primary aptamer comprises a core sequence as set forth in SEQ ID NO: 117 (FIGURE 27B) and further comprises at least one operative sequence, said operative sequence complementary to a sequence comprised in a sensor oligonucleotide. In certain non-limiting instances, a sphingosine-1-phosphate -binding primary aptamer comprises a core sequence as set forth in SEQ ID NO: 117 (FIGURE 27B) and at least one operative sequence on either side (flanking) the core sequence. In certain non-limiting instances, a sphingosine-1-phosphate -binding primary aptamer comprises a core sequence as set forth in SEQ ID NO:117 (FIGURE 27B) and at least one operative sequence on either side (flanking) the core sequence, where two of said operative sequences contain mutually complementary portions and can form a duplex.

In certain non-limiting instances, isolated sphingosine-1-phosphate- binding primary aptamers comprise the nucleotide sequence of SEQ ID NO:18, SEQ ID NO:193, or variants of these sequences having at least about 80 percent, or at least about 85 percent, or at least about 90 percent, or at least about 95 percent, or at least about 98 percent homology to the original sequence, for example obtained by substitutions, deletions, and insertions of Watson-Crick base pairs or by mutations at non-conserved positions. Percent homology can be determined using standard software such as BLAST or FASTA. In certain non-limiting instances, isolated sphingosine-1-phosphate-binding primary aptamers comprise the nucleotide sequence of SEQ ID NO:18 or SEQ ID NO: 193. Said aptamers can bind to sphingosine-1-phosphate and in their structure-switching formats (for example, in an anti-aptamer assay, a pseudosandwich assay, or a sandwich assay) they can respond by an increase in fluorescence.

### 5.1.9 DOPAMINE-BINDING PRIMARY APTAMERS

In certain non-limiting instances, a primary aptamer binds to dopamine in an aqueous solution at room temperature or 25°C with a dissociation constant of less than 10⁻⁴ M and binds selectively with dopamine.

In certain non-limiting instances, a dopamine-binding primary aptamer comprises the sequences CCGAT (SEQ ID NO: 194) and GGTGT (SEQ ID NO:195) or a variant of any of these sequences that differs in one or two bases by substitution, deletion, insertion or extension, where said primary aptamer binds to dopamine in an aqueous solution at room temperature or 25°C with a dissociation constant of less than 10⁻⁴ M and binds to dopamine selectively versus serotonin or norepinephrine (FIGURE 65B). In certain non-limiting instances, a dopamine-binding primary aptamer comprises the sequences CCGAT (SEQ ID NO: 194) and GGTGT (SEQ ID NO: 195) or a variant thereof and further comprises at least one operative sequence. In certain non-limiting instances, a dopamine-binding primary aptamer comprises the sequences CCGAT (SEQ ID NO: 194) and GGTGT (SEQ ID NO: 195), or a variant thereof, and further comprises at least one operative sequence, said operative sequence complementary to a sequence comprised in a sensor oligonucleotide. In certain non-limiting instances, a dopamine-binding primary aptamer comprises the sequences CCGAT (SEQ ID NO: 194) and GGTGT (SEQ ID NO: 195), or a variant thereof, and at least one operative sequence on either side (flanking) these two sequences. In certain non-limiting instances, a dopamine-binding primary aptamer comprises the sequences CCGAT (SEQ ID NO: 194) and GGTGT (SEQ ID NO: 195) or a variant thereof, and at least one operative sequence on either side (flanking) these two sequences, where two of said operative sequences contain mutually complementary portions and can form a duplex. For example, but not by way of limitation, a dopamine-binding aptamer may comprise the sequence: CTC TCG GGA CGA CGC CAG TTT GAA GGT TCG TTC GCA GGT GTG GAG TGA CGT CGT CCC (SEQ ID NO:21) or related sequence (SEQ ID NO: 196, FIGURE 65A). In certain non-limiting instances, a dopamine-binding primary aptamer comprises a core sequence as set forth in SEQ ID NO: 123 (FIGURE 30B; FIGURE 65A). In certain non-limiting instances, a dopamine -binding primary aptamer comprising a core sequence as set forth in SEQ ID NO: 123 (FIGURE 30B) has a binding affinity for dopamine that is at least about 50 percent or at least about 75 percent the binding affinity of the primary aptamer having SEQ ID NO: 196). In certain non-limiting instances, a dopamine -binding primary aptamer comprising a core sequence as set forth in SEQ ID NO: 123 (FIGURE 30B) competes with primary aptamer having SEQ ID NO: 196 for dopamine binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a dopamine -binding primary aptamer comprises a core sequence as set forth in SEQ ID NO: 123 (FIGURE 30B) and further comprises at least one operative sequence. In certain non-limiting instances, a dopamine -binding primary aptamer comprises a core sequence as set forth in SEQ ID NO: 123 (FIGURE 30B) and further comprises at least one operative sequence, said operative sequence complementary to a sequence comprised in a sensor oligonucleotide. In certain non-limiting instances, a dopamine -binding primary aptamer comprises a core sequence as set forth in SEQ ID NO: 123 (FIGURE 30B) and at least one operative sequence on either side (flanking) the core sequence. In certain non-limiting instances, a dopamine -binding primary aptamer comprises a core sequence as set forth in SEQ ID NO: 123 (FIGURE 30B) and at least one operative sequence on either side (flanking) the core sequence, where two of said operative sequences contain mutually complementary portions and can form a duplex.

In certain non-limiting instances, a dopamine-binding primary aptamer comprises the sequences GGG and GGGG(SEQ ID NO: 169) or a variant of any of these sequences that differs in one or two bases by substitution, deletion, insertion or extension, where said primary aptamer binds to dopamine in an aqueous solution at room temperature or 25°C with a dissociation constant of less than 10⁻⁴ M and binds to dopamine selectively versus serotonin or tyrosine (FIGURE 66B). In certain non-limiting instances, a dopamine-binding primary aptamer comprises the sequences GGG and GGGG(SEQ ID NO: 169) or a variant thereof and further comprises at least one operative sequence. In certain non-limiting instances, a dopamine-binding primary aptamer comprises the sequences GGG and GGGG(SEQ ID NO: 169), or a variant thereof, and further comprises at least one operative sequence, said operative sequence complementary to a sequence comprised in a sensor oligonucleotide. In certain non-limiting instances, a dopamine-binding primary aptamer comprises the sequences GGG and GGGG(SEQ ID NO: 169), or a variant thereof, and at least one operative sequence on either side (flanking) these two sequences. In certain non-limiting instances, a dopamine-binding primary aptamer comprises the sequences GGG and GGGG(SEQ ID NO: 169) or a variant thereof, and at least one operative sequence on either side (flanking) these two sequences, where two of said operative sequences contain mutually complementary portions and can form a duplex. For example, but not by way of limitation, a dopamine-binding aptamer may comprise the sequence: CTC TCG GGA CGA CTG CAG CCT GGG GTT GTG GGG GGT AGG GGA GGT CTG AGT CGT CCC (SEQ ID NO:22; FIGURE 31A) or related sequence (SEQ ID NO: 197, FIGURE 66A). In certain non-limiting instances, a dopamine-binding primary aptamer comprises a core sequence as set forth in SEQ ID NO: 125 (FIGURE 31B; FIGURE 66A). In certain non-limiting instances, a dopamine - binding primary aptamer comprising a core sequence as set forth in SEQ ID NO: 125 (FIGURE 31B) has a binding affinity for dopamine that is at least about 50 percent or at least about 75 percent the binding affinity of the primary aptamer having SEQ ID NO: 197). In certain non-limiting instances, a dopamine -binding primary aptamer comprising a core sequence as set forth in SEQ ID NO: 125 (FIGURE 31B) competes with primary aptamer having SEQ ID NO: 197 for dopamine binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a dopamine -binding primary aptamer comprises a core sequence as set forth in SEQ ID NO: 125 (FIGURE 31B) and further comprises at least one operative sequence. In certain non-limiting instances, a dopamine -binding primary aptamer comprises a core sequence as set forth in SEQ ID NO: 125 (FIGURE 31B) and further comprises at least one operative sequence, said operative sequence complementary to a sequence comprised in a sensor oligonucleotide. In certain non-limiting instances, a dopamine -binding primary aptamer comprises a core sequence as set forth in SEQ ID NO: 125 (FIGURE 31B) and at least one operative sequence on either side (flanking) the core sequence. In certain non-limiting instances, a dopamine -binding primary aptamer comprises a core sequence as set forth in SEQ ID NO: 125 (FIGURE 31B) and at least one operative sequence on either side (flanking) the core sequence, where two of said operative sequences contain mutually complementary portions and can form a duplex.

In certain non-limiting instances, a dopamine-binding primary aptamer comprises the sequences CACAG (SEQ ID NO: 198) and CACAA (SEQ ID NO: 199) or a variant of any of these sequences that differs in one or two bases by substitution, deletion, insertion or extension, where said primary aptamer binds to dopamine in an aqueous solution at room temperature or 25°C with a dissociation constant of less than 10⁻⁴ M and binds to dopamine selectively versus serotonin, melatonin or tyrosine (FIGURE 67B). In certain non-limiting instances, a dopamine-binding primary aptamer comprises the sequences CACAG (SEQ ID NO: 198) and CACAA (SEQ ID NO: 199) or a variant thereof and further comprises at least one operative sequence. In certain non-limiting instances, a dopamine-binding primary aptamer comprises the sequences CACAG (SEQ ID NO: 198) and CACAA (SEQ ID NO: 199) or a variant thereof, and further comprises at least one operative sequence, said operative sequence complementary to a sequence comprised in a sensor oligonucleotide. In certain non-limiting instances, a dopamine-binding primary aptamer comprises the sequences CACAG (SEQ ID NO: 198) and CACAA (SEQ ID NO: 199), or a variant thereof, and at least one operative sequence on either side (flanking) these two sequences. In certain non-limiting instances, a dopamine-binding primary aptamer comprises the sequences CACAG (SEQ ID NO: 198) and CACAA (SEQ ID NO: 199) or a variant thereof, and at least one operative sequence on either side (flanking) these two sequences, where two of said operative sequences contain mutually complementary portions and can form a duplex. For example, but not by way of limitation, a dopamine-binding aptamer may comprise the sequence: CTC TCG GGA CGA CCA CAC AGA GGC ACA ACT CGC AGG AGC AAA GCG GCA GGT CGT CCC (SEQ ID NO:23; FIGURE 32A) or related sequence (SEQ ID NO:200, FIGURE 67A). In certain non-limiting instances, a dopamine-binding primary aptamer comprises a core sequence as set forth in SEQ ID NO: 127 (FIGURE 32B; FIGURE 67A). In certain non-limiting instances, a dopamine -binding primary aptamer comprising a core sequence as set forth in SEQ ID NO: 127 (FIGURE 32B) has a binding affinity for dopamine that is at least about 50 percent or at least about 75 percent the binding affinity of the primary aptamer having SEQ ID NO:200). In certain non-limiting instances, a dopamine -binding primary aptamer comprising a core sequence as set forth in SEQ ID NO: 125 (FIGURE 31B) competes with primary aptamer having SEQ ID NO:200 for dopamine binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a dopamine -binding primary aptamer comprises a core sequence as set forth in SEQ ID NO: 127 (FIGURE 32B) and further comprises at least one operative sequence. In certain non-limiting instances, a dopamine -binding primary aptamer comprises a core sequence as set forth in SEQ ID NO: 127 (FIGURE 32B) and further comprises at least one operative sequence, said operative sequence complementary to a sequence comprised in a sensor oligonucleotide. In certain non-limiting instances, a dopamine -binding primary aptamer comprises a core sequence as set forth in SEQ ID NO: 127 (FIGURE 32B) and at least one operative sequence on either side (flanking) the core sequence. In certain non-limiting instances, a dopamine -binding primary aptamer comprises a core sequence as set forth in SEQ ID NO: 127 (FIGURE 32B) and at least one operative sequence on either side (flanking) the core sequence, where two of said operative sequences contain mutually complementary portions and can form a duplex.

In certain non-limiting instances, a dopamine-binding primary aptamer comprises the sequences GGGG(SEQ ID NO: 169) and GG or a variant of any of these sequences that differs in one or two bases by substitution, deletion, insertion or extension, where said primary aptamer binds to dopamine in an aqueous solution at room temperature or 25°C with a dissociation constant of less than 10⁻⁴ M and binds to dopamine selectively versus serotonin, melatonin or tyrosine (FIGURE 68B). In certain non-limiting instances, a dopamine-binding primary aptamer comprises the sequences GGGG(SEQ ID NO: 169) and GG or a variant thereof and further comprises at least one operative sequence. In certain non-limiting instances, a dopamine-binding primary aptamer comprises the sequences GGGG(SEQ ID NO: 169) and GG or a variant thereof, and further comprises at least one operative sequence, said operative sequence complementary to a sequence comprised in a sensor oligonucleotide. In certain non-limiting instances, a dopamine-binding primary aptamer comprises the sequences GGGG(SEQ ID NO: 169) and GG, or a variant thereof, and at least one operative sequence on either side (flanking) these two sequences. In certain non-limiting instances, a dopamine-binding primary aptamer comprises the sequences GGGG(SEQ ID NO: 169) and GG or a variant thereof, and at least one operative sequence on either side (flanking) these two sequences, where two of said operative sequences contain mutually complementary portions and can form a duplex. For example, but not by way of limitation, a dopamine-binding aptamer may comprise the sequence: CTC TCG GGA CGA CGG GGA GGA GTT AGC ATG ACG GCA ACT TTA GTA CTT CGT CGT CCC (SEQ ID NO:20; FIGURE 29A) or related sequence (SEQ ID NO:201, FIGURE 68A). In certain non-limiting instances, a dopamine-binding primary aptamer comprises a core sequence as set forth in SEQ ID NO: 121 (FIGURE 29B; FIGURE 68A). In certain non-limiting instances, a dopamine - binding primary aptamer comprising a core sequence as set forth in SEQ ID NO: 121 (FIGURE 29B) has a binding affinity for dopamine that is at least about 50 percent or at least about 75 percent the binding affinity of the primary aptamer having SEQ ID NO:201). In certain non-limiting instances, a dopamine -binding primary aptamer comprising a core sequence as set forth in SEQ ID NO: 121 (FIGURE 29B)competes with primary aptamer having SEQ ID NO:201 for dopamine binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a dopamine -binding primary aptamer comprises a core sequence as set forth in SEQ ID NO: 121 (FIGURE 29B) and further comprises at least one operative sequence. In certain non-limiting instances, a dopamine -binding primary aptamer comprises a core sequence as set forth in SEQ ID NO: 121 (FIGURE 29B)and further comprises at least one operative sequence, said operative sequence complementary to a sequence comprised in a sensor oligonucleotide. In certain non-limiting instances, a dopamine -binding primary aptamer comprises a core sequence as set forth in SEQ ID NO: 121 (FIGURE 29B)and at least one operative sequence on either side (flanking) the core sequence. In certain non-limiting instances, a dopamine -binding primary aptamer comprises a core sequence as set forth in SEQ ID NO: 121 (FIGURE 29B)and at least one operative sequence on either side (flanking) the core sequence, where two of said operative sequences contain mutually complementary portions and can form a duplex.

In certain non-limiting instances, a dopamine-binding primary aptamer comprises the sequences GGGG(SEQ ID NO: 169) and GG or a variant of any of these sequences that differs in one or two bases by substitution, deletion, insertion or extension, where said primary aptamer binds to dopamine in an aqueous solution at room temperature or 25°C with a dissociation constant of less than 10⁻⁴ M and binds to dopamine selectively versus serotonin, melatonin or tyrosine (FIGURE 69B). In certain non-limiting instances, a dopamine-binding primary aptamer comprises the sequences GGGG(SEQ ID NO: 169) and GG or a variant thereof and further comprises at least one operative sequence. In certain non-limiting instances, a dopamine-binding primary aptamer comprises the sequences GGGG(SEQ ID NO: 169) and GG or a variant thereof, and further comprises at least one operative sequence, said operative sequence complementary to a sequence comprised in a sensor oligonucleotide. In certain non-limiting instances, a dopamine-binding primary aptamer comprises the sequences GGGG(SEQ ID NO: 169) and GG, or a variant thereof, and at least one operative sequence on either side (flanking) these two sequences. In certain non-limiting instances, a dopamine-binding primary aptamer comprises the sequences GGGG(SEQ ID NO: 169) and GG or a variant thereof, and at least one operative sequence on either side (flanking) these two sequences, where two of said operative sequences contain mutually complementary portions and can form a duplex. For example, but not by way of limitation, a dopamine-binding aptamer may comprise the sequence: CTC TCG GGA CGA CCA CTT CAG ACG CTC AAC GTT TGG GGA GGC ACG GCA GGT CGT CCC (SEQ ID NO: 19; FIGURE 28A) or related sequence (SEQ ID NO:202, FIGURE 69A). In certain non-limiting instances, a dopamine-binding primary aptamer comprises a core sequence as set forth in SEQ ID NO:119 (FIGURE 28B; FIGURE 69A). In certain non-limiting instances, a dopamine - binding primary aptamer comprising a core sequence as set forth in SEQ ID NO:119 (FIGURE 28B) has a binding affinity for dopamine that is at least about 50 percent or at least about 75 percent the binding affinity of the primary aptamer having SEQ ID NO:202). In certain non-limiting instances, a dopamine -binding primary aptamer comprising a core sequence as set forth in SEQ ID NO:119 (FIGURE 28B) competes with primary aptamer having SEQ ID NO:202 for dopamine binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a dopamine -binding primary aptamer comprises a core sequence as set forth in SEQ ID NO:119 (FIGURE 28B) and further comprises at least one operative sequence. In certain non-limiting instances, a dopamine -binding primary aptamer comprises a core sequence as set forth in SEQ ID NO:119 (FIGURE 28B) and further comprises at least one operative sequence, said operative sequence complementary to a sequence comprised in a sensor oligonucleotide. In certain non-limiting instances, a dopamine -binding primary aptamer comprises a core sequence as set forth in SEQ ID NO:119 (FIGURE 28B) and at least one operative sequence on either side (flanking) the core sequence. In certain non-limiting instances, a dopamine -binding primary aptamer comprises a core sequence as set forth in SEQ ID NO:119 (FIGURE 28B) and at least one operative sequence on either side (flanking) the core sequence, where two of said operative sequences contain mutually complementary portions and can form a duplex.

In certain non-limiting instances, isolated dopamine- binding primary aptamers comprise the nucleotide sequence of SEQ ID NO:21, SEQ ID NO:196, SEQ ID NO:22, SEQ ID NO: 197, SEQ ID NO:23, SEQ ID NO:200, SEQ ID NO:20, SEQ ID NO:201, SEQ ID NO:19, SEQ ID NO:202 or variants of these sequences having at least about 80 percent, or at least about 85 percent, or at least about 90 percent, or at least about 95 percent, or at least about 98 percent homology to the original sequence, for example obtained by substitutions, deletions, and insertions of Watson-Crick base pairs or by mutations at non-conserved positions. Percent homology can be determined using standard software such as BLAST or FASTA. In certain non-limiting instances, isolated dopamine-binding primary aptamers comprise the nucleotide sequence of SEQ ID NO:21, SEQ ID NO: 196, SEQ ID NO:22, SEQ ID NO: 197, SEQ ID NO:23, SEQ ID NO:200, SEQ ID NO:20, SEQ ID NO:201, SEQ ID NO: 19, or SEQ ID NO:202. Said aptamers can bind to dopamine and in their structure-switching formats (for example, in an anti-aptamer assay, a pseudosandwich assay, or a sandwich assay) they can respond by an increase in fluorescence.

### 5.1.10 SEROTONIN-BINDING PRIMARY APTAMERS

In certain non-limiting instances, a primary aptamer binds to serotonin in an aqueous solution at room temperature or 25°C with a dissociation constant of less than 10⁻⁴ M and binds selectively with serotonin versus dopamine, melatonin, and 5-hydroxytryptophan.

In certain non-limiting instances, a serotonin-binding primary aptamer comprises the sequences GG and GGGG(SEQ ID NO: 169) and GGG or a variant of any of these sequences that differs in one or two bases by substitution, deletion, insertion or extension, where said primary aptamer binds to serotonin in an aqueous solution at room temperature or 25°C with a dissociation constant of less than 10⁻⁴ M and binds to serotonin selectively versus dopamine, melatonin, or 5-hydroxytryptophan (FIGURES 70B, 71B, 72B, 73B, 74B, 75B, 76B). In certain non-limiting instances, a serotonin-binding primary aptamer comprises the sequences GG and GGGG(SEQ ID NO: 169) and GGG or a variant thereof and further comprises at least one operative sequence. In certain non-limiting instances, a serotonin-binding primary aptamer comprises the sequences GG and GGGG(SEQ ID NO: 169) and GGG or a variant thereof, and further comprises at least one operative sequence, said operative sequence complementary to a sequence comprised in a sensor oligonucleotide. In certain non-limiting instances, a serotonin-binding primary aptamer comprises the sequences GG and GGGG(SEQ ID NO: 169) and GGG, or a variant thereof, and at least one operative sequence on either side (flanking) these three sequences. In certain non-limiting instances, a serotonin-binding primary aptamer comprises the sequences GG and GGGG(SEQ ID NO: 169) and GGG or a variant thereof, and at least one operative sequence on either side (flanking) these three sequences, where two of said operative sequences contain mutually complementary portions and can form a duplex.

For example, but not by way of limitation, a serotonin-binding aptamer may comprise the sequence: CTC TCG GGA CGA CTG GTA GGC AGA TAG GGG AAG CTG ATT CGA TGC GTG GGT CGT CCC (SEQ ID NO:25; FIGURE 34A) or related sequence (SEQ ID NO:203, FIGURE 70A). In certain non-limiting instances, a serotonin-binding primary aptamer comprises a core sequence as set forth in SEQ ID NO: 131 (FIGURE 34B; FIGURE 70A). In certain non-limiting instances, a serotonin -binding primary aptamer comprising a core sequence as set forth in SEQ ID NO: 131 (FIGURE 34B) has a binding affinity for serotonin that is at least about 50 percent or at least about 75 percent the binding affinity of the primary aptamer having SEQ ID NO:203). In certain non-limiting instances, a serotonin -binding primary aptamer comprising a core sequence as set forth in SEQ ID NO: 131 (FIGURE 34B) competes with primary aptamer having SEQ ID NO:203 for serotonin binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a serotonin -binding primary aptamer comprises a core sequence as set forth in SEQ ID NO: 131 (FIGURE 34B) and further comprises at least one operative sequence. In certain non-limiting instances, a serotonin - binding primary aptamer comprises a core sequence as set forth in SEQ ID NO: 131 (FIGURE 34B) and further comprises at least one operative sequence, said operative sequence complementary to a sequence comprised in a sensor oligonucleotide. In certain non-limiting instances, a serotonin -binding primary aptamer comprises a core sequence as set forth in SEQ ID NO: 131 (FIGURE 34B) and at least one operative sequence on either side (flanking) the core sequence. In certain non-limiting instances, a serotonin - binding primary aptamer comprises a core sequence as set forth in SEQ ID NO: 131 (FIGURE 34B) and at least one operative sequence on either side (flanking) the core sequence, where two of said operative sequences contain mutually complementary portions and can form a duplex.

For example, but not by way of limitation, a serotonin-binding aptamer may comprise the sequence: CTC TCG GGA CGA CTG GTA GGC AAC AGG GGA AGG GAG TTC TGC GTA CGT GGG TCG TCC C (SEQ ID NO:28; FIGURE 37A) or related sequence (SEQ ID NO:204, FIGURE 71A). In certain non-limiting instances, a serotonin-binding primary aptamer comprises a core sequence as set forth in SEQ ID NO: 137 (FIGURE 37B; FIGURE 71A). In certain non-limiting instances, a serotonin - binding primary aptamer comprising a core sequence as set forth in SEQ ID NO: 137 (FIGURE 37B) has a binding affinity for serotonin that is at least about 50 percent or at least about 75 percent the binding affinity of the primary aptamer having SEQ ID NO:204). In certain non-limiting instances, a serotonin -binding primary aptamer comprising a core sequence as set forth in SEQ ID NO: 137 (FIGURE 37B) competes with primary aptamer having SEQ ID NO:204 for serotonin binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a serotonin -binding primary aptamer comprises a core sequence as set forth in SEQ ID NO: 137 (FIGURE 37B) and further comprises at least one operative sequence. In certain non-limiting instances, a serotonin -binding primary aptamer comprises a core sequence as set forth in SEQ ID NO: 137 (FIGURE 37B) and further comprises at least one operative sequence, said operative sequence complementary to a sequence comprised in a sensor oligonucleotide. In certain non-limiting instances, a serotonin -binding primary aptamer comprises a core sequence as set forth in SEQ ID NO: 137 (FIGURE 37 B) and at least one operative sequence on either side (flanking) the core sequence. In certain non-limiting instances, a serotonin -binding primary aptamer comprises a core sequence as set forth in SEQ ID NO: 137 (FIGURE 37B) and at least one operative sequence on either side (flanking) the core sequence, where two of said operative sequences contain mutually complementary portions and can form a duplex.

For example, but not by way of limitation, a serotonin-binding aptamer may comprise the sequence: CTC TCG GGA CGA CAG GGG CAT ATA TAG TCT AGG GTT TGG TGT GGG TAG TGT CGT CCC (SEQ ID NO:24; FIGURE 33A) or related sequence (SEQ ID NO:205, FIGURE 72A). In certain non-limiting instances, a serotonin-binding primary aptamer comprises a core sequence as set forth in SEQ ID NO: 129 (FIGURE 33B; FIGURE 72A). In certain non-limiting instances, a serotonin -binding primary aptamer comprising a core sequence as set forth in SEQ ID NO: 129 (FIGURE 33B) has a binding affinity for serotonin that is at least about 50 percent or at least about 75 percent the binding affinity of the primary aptamer having SEQ ID NO:205). In certain non-limiting instances, a serotonin -binding primary aptamer comprising a core sequence as set forth in SEQ ID NO: 129 (FIGURE 33B) competes with primary aptamer having SEQ ID NO:205 for serotonin binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a serotonin -binding primary aptamer comprises a core sequence as set forth in SEQ ID NO: 129 (FIGURE 33B) and further comprises at least one operative sequence. In certain non-limiting instances, a serotonin - binding primary aptamer comprises a core sequence as set forth in SEQ ID NO: 129 (FIGURE 33B and further comprises at least one operative sequence, said operative sequence complementary to a sequence comprised in a sensor oligonucleotide. In certain non-limiting instances, a serotonin -binding primary aptamer comprises a core sequence as set forth in SEQ ID NO: 129 (FIGURE 33B) and at least one operative sequence on either side (flanking) the core sequence. In certain non-limiting instances, a serotonin - binding primary aptamer comprises a core sequence as set forth in SEQ ID NO: 129 (FIGURE 33B) and at least one operative sequence on either side (flanking) the core sequence, where two of said operative sequences contain mutually complementary portions and can form a duplex.

For example, but not by way of limitation, a serotonin-binding aptamer may comprise the sequence: CTC TCG GGA CGA CTG GTA GGC AGC AGG GGA AGT AGG CGT GTC CTC GTG GGT CGT CCC (SEQ ID NO:26; FIGURE 35A) or related sequence (SEQ ID NO:206, FIGURE 73A). In certain non-limiting instances, a serotonin-binding primary aptamer comprises a core sequence as set forth in SEQ ID NO: 133 (FIGURE 35B; FIGURE 73A). In certain non-limiting instances, a serotonin -binding primary aptamer comprising a core sequence as set forth in SEQ ID NO: 133 (FIGURE 35B) has a binding affinity for serotonin that is at least about 50 percent or at least about 75 percent the binding affinity of the primary aptamer having SEQ ID NO:206). In certain non-limiting instances, a serotonin -binding primary aptamer comprising a core sequence as set forth in SEQ ID NO: 133 (FIGURE 35B) competes with primary aptamer having SEQ ID NO:206 for serotonin binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a serotonin -binding primary aptamer comprises a core sequence as set forth in SEQ ID NO: 133 (FIGURE 35B) and further comprises at least one operative sequence. In certain non-limiting instances, a serotonin - binding primary aptamer comprises a core sequence as set forth in SEQ ID NO: 133 (FIGURE 35B and further comprises at least one operative sequence, said operative sequence complementary to a sequence comprised in a sensor oligonucleotide. In certain non-limiting instances, a serotonin -binding primary aptamer comprises a core sequence as set forth in SEQ ID NO: 133 (FIGURE 35B) and at least one operative sequence on either side (flanking) the core sequence. In certain non-limiting instances, a serotonin - binding primary aptamer comprises a core sequence as set forth in SEQ ID NO: 133 (FIGURE 35 B) and at least one operative sequence on either side (flanking) the core sequence, where two of said operative sequences contain mutually complementary portions and can form a duplex.

For example, but not by way of limitation, a serotonin-binding aptamer may comprise the sequence: CTC TCG GGA CGA CCA GTA GGG GAT CCA CAG TGA GGG GTT TGT ATG GGT GGT CGT CCC (SEQ ID NO:27; FIGURE 36A) or related sequence (SEQ ID NO:207, FIGURE 74A). In certain non-limiting instances, a serotonin-binding primary aptamer comprises a core sequence as set forth in SEQ ID NO: 135 (FIGURE 36B; FIGURE 74A). In certain non-limiting instances, a serotonin -binding primary aptamer comprising a core sequence as set forth in SEQ ID NO: 135 (FIGURE 36B) has a binding affinity for serotonin that is at least about 50 percent or at least about 75 percent the binding affinity of the primary aptamer having SEQ ID NO:207). In certain non-limiting instances, a serotonin -binding primary aptamer comprising a core sequence as set forth in SEQ ID NO: 135 (FIGURE 36B) competes with primary aptamer having SEQ ID NO:207 for serotonin binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a serotonin -binding primary aptamer comprises a core sequence as set forth in SEQ ID NO: 135 (FIGURE 36B) and further comprises at least one operative sequence. In certain non-limiting instances, a serotonin - binding primary aptamer comprises a core sequence as set forth in SEQ ID NO: 135 (FIGURE 36B and further comprises at least one operative sequence, said operative sequence complementary to a sequence comprised in a sensor oligonucleotide. In certain non-limiting instances, a serotonin -binding primary aptamer comprises a core sequence as set forth in SEQ ID NO: 135 (FIGURE 36B) and at least one operative sequence on either side (flanking) the core sequence. In certain non-limiting instances, a serotonin - binding primary aptamer comprises a core sequence as set forth in SEQ ID NO: 135 (FIGURE 36 B) and at least one operative sequence on either side (flanking) the core sequence, where two of said operative sequences contain mutually complementary portions and can form a duplex.

For example, but not by way of limitation, a serotonin-binding aptamer may comprise the sequence: CTC TCG GGA CGA CGG AGG TGG TGT CTT GGA CAG TGG TAT TCG CAG TTG CGT CGT CCC (SEQ ID NO:29; FIGURE 38A) or related sequence (SEQ ID NO:208, FIGURE 75A). In certain non-limiting instances, a serotonin-binding primary aptamer comprises a core sequence as set forth in SEQ ID NO: 139 (FIGURE 38B; FIGURE 75A). In certain non-limiting instances, a serotonin -binding primary aptamer comprising a core sequence as set forth in SEQ ID NO: 139 (FIGURE 38B) has a binding affinity for serotonin that is at least about 50 percent or at least about 75 percent the binding affinity of the primary aptamer having SEQ ID NO:208). In certain non-limiting instances, a serotonin -binding primary aptamer comprising a core sequence as set forth in SEQ ID NO: 139 (FIGURE 38B) competes with primary aptamer having SEQ ID NO:208 for serotonin binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a serotonin -binding primary aptamer comprises a core sequence as set forth in SEQ ID NO: 139 (FIGURE 38B) and further comprises at least one operative sequence. In certain non-limiting instances, a serotonin - binding primary aptamer comprises a core sequence as set forth in SEQ ID NO: 139 (FIGURE 38B and further comprises at least one operative sequence, said operative sequence complementary to a sequence comprised in a sensor oligonucleotide. In certain non-limiting instances, a serotonin -binding primary aptamer comprises a core sequence as set forth in SEQ ID NO: 139 (FIGURE 38B) and at least one operative sequence on either side (flanking) the core sequence. In certain non-limiting instances, a serotonin - binding primary aptamer comprises a core sequence as set forth in SEQ ID NO: 139 (FIGURE 38 B) and at least one operative sequence on either side (flanking) the core sequence, where two of said operative sequences contain mutually complementary portions and can form a duplex.

For example, but not by way of limitation, a serotonin-binding aptamer may comprise the sequence: CTC TCG GGA CGA CAG AGA CGG GGT GCT TAC TTG GTT CAG GGG AGT CGA CGT CGT CCC (SEQ ID NO:30; FIGURE 39A) or related sequence (SEQ ID NO:209, FIGURE 76A). In certain non-limiting instances, a serotonin-binding primary aptamer comprises a core sequence as set forth in SEQ ID NO: 141 (FIGURE 39B; FIGURE 76A). In certain non-limiting instances, a serotonin -binding primary aptamer comprising a core sequence as set forth in SEQ ID NO: 141 (FIGURE 39B) has a binding affinity for serotonin that is at least about 50 percent or at least about 75 percent the binding affinity of the primary aptamer having SEQ ID NO:209). In certain non-limiting instances, a serotonin -binding primary aptamer comprising a core sequence as set forth in SEQ ID NO: 141 (FIGURE 39B) competes with primary aptamer having SEQ ID NO:209 for serotonin binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a serotonin -binding primary aptamer comprises a core sequence as set forth in SEQ ID NO: 141 (FIGURE 39B) and further comprises at least one operative sequence. In certain non-limiting instances, a serotonin - binding primary aptamer comprises a core sequence as set forth in SEQ ID NO: 141 (FIGURE 39B and further comprises at least one operative sequence, said operative sequence complementary to a sequence comprised in a sensor oligonucleotide. In certain non-limiting instances, a serotonin -binding primary aptamer comprises a core sequence as set forth in SEQ ID NO: 141 (FIGURE 39B) and at least one operative sequence on either side (flanking) the core sequence. In certain non-limiting instances, a serotonin - binding primary aptamer comprises a core sequence as set forth in SEQ ID NO: 139 (FIGURE 38 B) and at least one operative sequence on either side (flanking) the core sequence, where two of said operative sequences contain mutually complementary portions and can form a duplex.

In certain non-limiting instances, isolated serotonin- binding primary aptamers comprise the nucleotide sequence of SEQ ID NO:25, SEQ ID NO:203, SEQ ID NO:28, SEQ ID NO:204, SEQ ID NO:24, SEQ ID NO:205, SEQ ID NO:26, SEQ ID NO:206, SEQ ID NO:27, SEQ ID NO:207, SEQ ID NO:29, SEQ ID NO: 208, SEQ ID NO:30, SEQ ID NO:209, or variants of these sequences having at least about 80 percent, or at least about 85 percent, or at least about 90 percent, or at least about 95 percent, or at least about 98 percent homology to the original sequence, for example obtained by substitutions, deletions, and insertions of Watson-Crick base pairs or by mutations at non-conserved positions. Percent homology can be determined using standard software such as BLAST or FASTA. In certain non-limiting instances, isolated serotonin-binding primary aptamers comprise the nucleotide sequence of SEQ ID NO:25, SEQ ID NO:203, SEQ ID NO:28, SEQ ID NO:204, SEQ ID NO:24, SEQ ID NO:205, SEQ ID NO:26, SEQ ID NO:206, SEQ ID NO:27, SEQ ID NO:207, SEQ ID NO:29, SEQ ID NO: 208, SEQ ID NO:30, or SEQ ID NO:209. Said aptamers can bind to serotonin and in their structure-switching formats (for example, in an anti-aptamer assay, a pseudosandwich assay, or a sandwich assay) they can respond by an increase in fluorescence.

### 5.1.11 MELATONIN-BINDING PRIMARY APTAMERS

In certain non-limiting instances, a primary aptamer binds to melatonin in an aqueous solution at room temperature or 25°C with a dissociation constant of less than 10⁻⁴ M and binds selectively with melatonin versus serotonin or tryptophan (see FIGURES 77B and 78B).

For example, but not by way of limitation, a melatonin-binding aptamer may comprise the sequence: CTC TCG GGA CGA CGT CTT GGG GGT GGT GGG TTT GGC TGG TAC TTA GGG CGT CGT CCC (SEQ ID NO:32; FIGURE 41A) or related sequence (SEQ ID NO:210, FIGURE 77A). In certain non-limiting instances, a melatonin-binding primary aptamer comprises a core sequence as set forth in SEQ ID NO: 145 (FIGURE 41B; FIGURE 77A). In certain non-limiting instances, a melatonin - binding primary aptamer comprising a core sequence as set forth in SEQ ID NO: 145 (FIGURE 41B) has a binding affinity for melatonin that is at least about 50 percent or at least about 75 percent the binding affinity of the primary aptamer having SEQ ID NO:210). In certain non-limiting instances, a melatonin -binding primary aptamer comprising a core sequence as set forth in SEQ ID NO: 145 (FIGURE 41B) competes with primary aptamer having SEQ ID NO:210 for melatonin binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a melatonin -binding primary aptamer comprises a core sequence as set forth in SEQ ID NO: 145 (FIGURE 41B) and further comprises at least one operative sequence. In certain non-limiting instances, a melatonin -binding primary aptamer comprises a core sequence as set forth in SEQ ID NO: 145 (FIGURE 41B and further comprises at least one operative sequence, said operative sequence complementary to a sequence comprised in a sensor oligonucleotide. In certain non-limiting instances, a melatonin -binding primary aptamer comprises a core sequence as set forth in SEQ ID NO: 145 (FIGURE 41B) and at least one operative sequence on either side (flanking) the core sequence. In certain non-limiting instances, a melatonin -binding primary aptamer comprises a core sequence as set forth in SEQ ID NO: 145 (FIGURE 41 B) and at least one operative sequence on either side (flanking) the core sequence, where two of said operative sequences contain mutually complementary portions and can form a duplex.

For example, but not by way of limitation, a melatonin-binding aptamer may comprise the sequence: CTC TCG GGA CGA CAG CCA AGG TCG TAA GGT ACG GTC AGT GTA CTC GGT TGT CGT CCC (SEQ ID NO:31; FIGURE 40A) or related sequence (SEQ ID NO:211, FIGURE 78A). In certain non-limiting instances, a melatonin-binding primary aptamer comprises a core sequence as set forth in SEQ ID NO: 143 (FIGURE 40B; FIGURE 78A). In certain non-limiting instances, a melatonin - binding primary aptamer comprising a core sequence as set forth in SEQ ID NO: 143 (FIGURE 40B) has a binding affinity for melatonin that is at least about 50 percent or at least about 75 percent the binding affinity of the primary aptamer having SEQ ID NO:211). In certain non-limiting instances, a melatonin -binding primary aptamer comprising a core sequence as set forth in SEQ ID NO: 143 (FIGURE 40B) competes with primary aptamer having SEQ ID NO:211 for melatonin binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a melatonin -binding primary aptamer comprises a core sequence as set forth in SEQ ID NO: 143 (FIGURE 40B) and further comprises at least one operative sequence. In certain non-limiting instances, a melatonin -binding primary aptamer comprises a core sequence as set forth in SEQ ID NO: 143 (FIGURE 40B and further comprises at least one operative sequence, said operative sequence complementary to a sequence comprised in a sensor oligonucleotide. In certain non-limiting instances, a melatonin -binding primary aptamer comprises a core sequence as set forth in SEQ ID NO: 143 (FIGURE 40B) and at least one operative sequence on either side (flanking) the core sequence. In certain non-limiting instances, a melatonin -binding primary aptamer comprises a core sequence as set forth in SEQ ID NO: 143 (FIGURE 40 B) and at least one operative sequence on either side (flanking) the core sequence, where two of said operative sequences contain mutually complementary portions and can form a duplex.

In certain non-limiting instances, isolated melatonin- binding primary aptamers comprise the nucleotide sequence of SEQ ID NO:31, SEQ ID NO:211, SEQ ID NO:32, SEQ ID NO:210, or variants of these sequences having at least about 80 percent, or at least about 85 percent, or at least about 90 percent, or at least about 95 percent, or at least about 98 percent homology to the original sequence, for example obtained by substitutions, deletions, and insertions of Watson-Crick base pairs or by mutations at non-conserved positions. Percent homology can be determined using standard software such as BLAST or FASTA. In certain non-limiting instances, isolated melatonin-binding primary aptamers comprise the nucleotide sequence of SEQ ID NO:31, SEQ ID NO:21 1, SEQ ID NO:32, or SEQ ID NO:210. Said aptamers can bind to melatonin and in their structure-switching formats (for example, in an anti-aptamer assay, a pseudosandwich assay, or a sandwich assay) they can respond by an increase in fluorescence.

### 5.1.12 TYROSINE-BINDING PRIMARY APTAMERS

In certain non-limiting instances, a tyrosine-binding primary aptamer comprising a core sequence as set forth in SEQ ID NO: 147 (FIGURE 42B) has a binding affinity for tyrosine that is at least about 50 percent or at least about 75 percent the binding affinity of the primary aptamer having SEQ ID NO:33). In certain non-limiting instances, a tyrosine-binding primary aptamer comprising a core sequence as set forth in SEQ ID NO: 147 (FIGURE 42B) competes with primary aptamer having SEQ ID NO:33 for tyrosine binding. In non-limiting instances, said primary aptamer has a length of between about 30 and about 100 nucleotides, or between about 30 and 80 nucleotides, or between about 30 and 70 nucleotides, or between about 30 and 60 nucleotides. In certain non-limiting instances, a tyrosine -binding primary aptamer comprises a core sequence as set forth in SEQ ID NO: 147 (FIGURE 42B) and further comprises at least one operative sequence. In certain non-limiting instances, a tyrosine -binding primary aptamer comprises a core sequence as set forth in SEQ ID NO: 147 (FIGURE 42B) and further comprises at least one operative sequence, said operative sequence complementary to a sequence comprised in a sensor oligonucleotide. In certain non-limiting instances, a tyrosine -binding primary aptamer comprises a core sequence as set forth in SEQ ID NO: 147 (FIGURE 42B) and at least one operative sequence on either side (flanking) the core sequence. In certain non-limiting instances, a tyrosine -binding primary aptamer comprises a core sequence as set forth in SEQ ID NO: 147 (FIGURE 42 B) and at least one operative sequence on either side (flanking) the core sequence, where two of said operative sequences contain mutually complementary portions and can form a duplex.

In certain non-limiting instances, isolated tyrosine- binding primary aptamers comprise the nucleotide sequence of SEQ ID NO:33, SEQ ID NO: 147, or variants of these sequences having at least about 80 percent, or at least about 85 percent, or at least about 90 percent, or at least about 95 percent, or at least about 98 percent homology to the original sequence, for example obtained by substitutions, deletions, and insertions of Watson-Crick base pairs or by mutations at non-conserved positions. Percent homology can be determined using standard software such as BLAST or FASTA. In certain non-limiting instances, isolated tyrosine-binding primary aptamers comprise the nucleotide sequence of SEQ ID NO:33 or SEQ ID NO: 147. Said aptamers can bind to tyrosine and in their structure-switching formats (for example, in an anti-aptamer assay, a pseudosandwich assay, or a sandwich assay) they can respond by an increase in fluorescence.

### 5.1.13 ALDOSTERONE-BINDING PRIMARY APTAMERS

In certain non-limiting instances, a primary aptamer binds to aldosterone in an aqueous solution at room temperature or 25°C with a dissociation constant of less than 10⁻⁴ M and binds selectively with aldosterone (see FIGURES 79B and 80B) versus cortisone, cortisol or dexycortisone, or binds selectively to a steroid having C18 bound to one or two oxygen atoms, versus a steroid having a methyl linked to the C18 position.

In certain non-limiting instances, an aldosterone-binding primary aptamer comprises the sequences GATAGT (SEQ ID NO:212) and ATGTTC (SEQ ID NO:213) or a variant of any of these sequences that differs in one or two bases by substitution, deletion, insertion or extension, where said primary aptamer binds to aldosterone in an aqueous solution at room temperature or 25°C with a dissociation constant of less than 10⁻⁴ M and binds to aldosterone selectively versus cortisone, cortisol or deoxycortisone. In certain non-limiting instances, an aldosterone-binding primary aptamer comprises the sequences GATAGT (SEQ ID NO:212) and ATGTTC (SEQ ID NO:213) or a variant thereof and further comprises at least one operative sequence. In certain non-limiting instances, a aldosterone-binding primary aptamer comprises the sequences GATAGT (SEQ ID NO:212) and ATGTTC (SEQ ID NO:213) or a variant thereof , and further comprises at least one operative sequence, said operative sequence complementary to a sequence comprised in a sensor oligonucleotide. In certain non-limiting instances, a aldosterone-binding primary aptamer comprises the sequences GATAGT (SEQ ID NO:212) and ATGTTC (SEQ ID NO:213), or a variant thereof, and at least one operative sequence on either side (flanking) these two sequences. In certain non-limiting instances, a aldosterone-binding primary aptamer comprises the sequences GATAGT (SEQ ID NO:212) and ATGTTC (SEQ ID NO:213), or a variant thereof, and at least one operative sequence on either side (flanking) these two sequences, where two of said operative sequences contain mutually complementary portions and can form a duplex.

For example, but not by way of limitation, a aldosterone-binding aptamer may comprise the sequence: CTC TCG GGA CGA CAG ATA GTT GTT CTT AGC GAT GTT CAG CGT TGT CGT CCC (SEQ ID NO: 264) or related sequence (SEQ ID NO:63, FIGURE1B).

For example, but not by way of limitation, a aldosterone-binding aptamer may comprise the sequence: CTC TCG GGA CGA CGG TAG GTA GGC CAA CTG GGT ATT TAC TGG TGT CGT CCC (SEQ ID NO: 265).

Isolated aldosterone- binding primary aptamers may comprise the nucleotide sequence of SEQ ID NO:214, SEQ ID NO:215, SEQ ID NO:63, or variants of these sequences having at least about 80 percent, or at least about 85 percent, or at least about 90 percent, or at least about 95 percent, or at least about 98 percent homology to the original sequence, for example obtained by substitutions, deletions, and insertions of Watson-Crick base pairs or by mutations at non-conserved positions. Percent homology can be determined using standard software such as BLAST or FASTA. Isolated aldosterone-binding primary aptamers may comprise the nucleotide sequence of SEQ ID NO:214, SEQ ID NO:215, or SEQ ID NO:63. Said aptamers can bind to aldosterone and in their structure-switching formats (for example, in an anti-aptamer assay, a pseudosandwich assay, or a sandwich assay) they can respond by an increase in fluorescence.

### 5.1.14 TOBRAMYCIN-BINDING PRIMARY APTAMERS

In certain non-limiting instances, a primary aptamer binds to tobramycin in an aqueous solution at room temperature or 25°C with a dissociation constant of less than 10⁻³ M and binds selectively with tobramycin (see FIGURES 81B and 82B) versus amikacin or kanamycin.

In certain non-limiting instances, an tobramycin-binding primary aptamer comprises the sequence(s) TGAAA (SEQ ID NO:216) and/or AAGTG (SEQ ID NO:217) or a variant of any of these sequences that differs in one or two bases by substitution, deletion, insertion or extension, where said primary aptamer binds to tobramycin in an aqueous solution at room temperature or 25°C with a dissociation constant of less than 10⁻³ M and binds to tobramycin selectively versusamikacin or kanamycin. In certain non-limiting instances, an tobramycin-binding primary aptamer comprises the sequence(s) TGAAA (SEQ ID NO:216) and/or AAGTG (SEQ ID NO:217) or a variant thereof and further comprises at least one operative sequence. In certain non-limiting instances, a tobramycin-binding primary aptamer comprises the sequence(s) TGAAA (SEQ ID NO:216) and/or AAGTG (SEQ ID NO:217) or a variant thereof, and further comprises at least one operative sequence, said operative sequence complementary to a sequence comprised in a sensor oligonucleotide. In certain non-limiting instances, a tobramycin-binding primary aptamer comprises the sequence(s) TGAAA (SEQ ID NO:216) and/or AAGTG (SEQ ID NO:217), or a variant thereof, and at least one operative sequence on either side (flanking) this sequence or sequences. In certain non-limiting instances, a tobramycin-binding primary aptamer comprises the sequence(s) TGAAA (SEQ ID NO:216) and/or AAGTG (SEQ ID NO:217), or a variant thereof, and at least one operative sequence on either side (flanking) this sequence or sequences, where two of said operative sequences contain mutually complementary portions and can form a duplex.

For example, but not by way of limitation, a tobramycin-binding aptamer may comprise the sequence: CTC TCG GGA CGA CGG CCC CGC AAG GGG TGA AAT GAC AGA GTC AAA GTG CGT CGT CCC (SEQ ID NO: 266).

In certain non-limiting instances, an tobramycin-binding primary aptamer comprises the sequence(s) GTAGTC (SEQ ID NO:219) and/or TCGGTAG (SEQ ID NO:220) or a variant of any of these sequences that differs in one or two bases by substitution, deletion, insertion or extension, where said primary aptamer binds to tobramycin in an aqueous solution at room temperature or 25°C with a dissociation constant of less than 10⁻³ M and binds to tobramycin selectively versusamikacin or kanamycin. In certain non-limiting instances, an tobramycin-binding primary aptamer comprises the sequence(s) GTAGTC (SEQ ID NO:219) and/or TCGGTAG (SEQ ID NO:220) or a variant thereof and further comprises at least one operative sequence. In certain non-limiting instances, a tobramycin-binding primary aptamer comprises the sequence(s) GTAGTC (SEQ ID NO:219) and/or TCGGTAG (SEQ ID NO:220) or a variant thereof, and further comprises at least one operative sequence, said operative sequence complementary to a sequence comprised in a sensor oligonucleotide. In certain non-limiting instances, a tobramycin-binding primary aptamer comprises the sequence(s) GTAGTC (SEQ ID NO:219) and/or TCGGTAG (SEQ ID NO:220), or a variant thereof, and at least one operative sequence on either side (flanking) this sequence or sequences. In certain non-limiting instances, a tobramycin-binding primary aptamer comprises the sequence(s) GTAGTC (SEQ ID NO:219) and/or TCGGTAG (SEQ ID NO:220), or a variant thereof, and at least one operative sequence on either side (flanking) this sequence or sequences, where two of said operative sequences contain mutually complementary portions and can form a duplex.

For example, but not by way of limitation, a tobramycin-binding aptamer may comprise the sequence: CTC TCG GGA CGA CGT AGT CGG AAA CGG TGT CTC AGT TCC TCG GTA GAG TCG TCC C (SEQ ID NO: 267).

In certain non-limiting instances, isolated tobramycin- binding primary aptamers comprise the nucleotide sequence of SEQ ID NO:218, SEQ ID NO:221, or variants of these sequences having at least about 80 percent, or at least about 85 percent, or at least about 90 percent, or at least about 95 percent, or at least about 98 percent homology to the original sequence, for example obtained by substitutions, deletions, and insertions of Watson-Crick base pairs or by mutations at non-conserved positions. Percent homology can be determined using standard software such as BLAST or FASTA. In certain non-limiting instances, isolated tobramycin-binding primary aptamers comprise the nucleotide sequence of SEQ ID NO:218 or SEQ ID NO:221. Said aptamers can bind to tobramycin and in their structure-switching formats (for example, in an anti-aptamer assay, a pseudosandwich assay, or a sandwich assay) they can respond by an increase in fluorescence.

### 5.1.15 AMIKACIN-BINDING PRIMARY APTAMERS

In certain non-limiting instances, a primary aptamer binds to amikacin in an aqueous solution at room temperature or 25°C with a dissociation constant of less than 10⁻³ M and binds selectively with amikacin versus tobramycin or kanamycin (see FIGURES 83B, 84B abd 85B).

In certain non-limiting instances, an amikacin-binding primary aptamer comprises the sequences GC and GCCC(SEQ ID NO:222) and GTTTAGA (SEQ ID NO:223) and AGTCTT (SEQ ID NO:224) or a variant of any of these sequences that differs in one or two bases by substitution, deletion, insertion or extension, where said primary aptamer binds to amikacin in an aqueous solution at room temperature or 25°C with a dissociation constant of less than 10⁻³ M and binds to amikacin selectively versustobramycin and kanamycin. In certain non-limiting instances, a amikacin-binding primary aptamer comprises the sequences GC and GCCC(SEQ ID NO:222) and GTTTAGA (SEQ ID NO:223) and AGTCTT (SEQ ID NO:224) or a variant thereof and further comprises at least one operative sequence. In certain non-limiting instances, a amikacin-binding primary aptamer comprises the sequences GC and GCCC(SEQ ID NO:222) and GTTTAGA (SEQ ID NO:223) and AGTCTT (SEQ ID NO:224), or a variant thereof, and further comprises at least one operative sequence, said operative sequence complementary to a sequence comprised in a sensor oligonucleotide. In certain non-limiting instances, a amikacin-binding primary aptamer comprises the sequences GC and GCCC(SEQ ID NO:222) and GTTTAGA (SEQ ID NO:223) and AGTCTT (SEQ ID NO:224), or a variant thereof, and at least one operative sequence on either side (flanking) these four sequences. In certain non-limiting instances, a amikacin-binding primary aptamer comprises the sequences GC and GCCC(SEQ ID NO:222) and GTTTAGA (SEQ ID NO:223) and AGTCTT (SEQ ID NO:224), or a variant thereof, and at least one operative sequence on either side (flanking) these four sequences, where two of said operative sequences contain mutually complementary portions and can form a duplex. For example, but not by way of limitation, a amikacin-binding aptamer may comprise the sequence: CTC TCG GGA CGA CCG CTT GCC CCC TGG CAT GTT TAG AGC AGA GTC TTT GGT CGT CCC (SEQ ID NO: 268).

In certain non-limiting instances, an amikacin-binding primary aptamer comprises the sequences GGTTCAT (SEQ ID NO:226) and ATGTGGG (SEQ ID NO:227) or a variant of any of these sequences that differs in one or two bases by substitution, deletion, insertion or extension, where said primary aptamer binds to amikacin in an aqueous solution at room temperature or 25°C with a dissociation constant of less than 10⁻³ M and binds to amikacin selectively versustobramycin and kanamycin. In certain non-limiting instances, a amikacin-binding primary aptamer comprises the sequences GGTTCAT (SEQ ID NO:226) and ATGTGGG (SEQ ID NO:227)or a variant thereof and further comprises at least one operative sequence. In certain non-limiting instances, a amikacin-binding primary aptamer comprises the sequences GGTTCAT (SEQ ID NO:226) and ATGTGGG (SEQ ID NO:227), or a variant thereof, and further comprises at least one operative sequence, said operative sequence complementary to a sequence comprised in a sensor oligonucleotide. In certain non-limiting instances, a amikacin-binding primary aptamer comprises the sequences GGTTCAT (SEQ ID NO:226) and ATGTGGG (SEQ ID NO:227), or a variant thereof, and at least one operative sequence on either side (flanking) these two sequences. In certain non-limiting instances, a amikacin-binding primary aptamer comprises the sequences GGTTCAT (SEQ ID NO:226) and ATGTGGG (SEQ ID NO:227), or a variant thereof, and at least one operative sequence on either side (flanking) these two sequences, where two of said operative sequences contain mutually complementary portions and can form a duplex. For example, but not by way of limitation, a amikacin-binding aptamer may comprise the sequence: CTC TCG GGA CGA CGT CCG GTT CAT GAC TTC AGT AGT CTA GTG GGG GTC TGT CGT CCC (SEQ ID NO: 269).

In certain non-limiting instances, an amikacin-binding primary aptamer comprises the sequences CAA and CGTCTACGGCTTAGC (SEQ ID NO:229) or a variant of any of these sequences that differs in one or two bases by substitution, deletion, insertion or extension, where said primary aptamer binds to amikacin in an aqueous solution at room temperature or 25°C with a dissociation constant of less than 10⁻³ M and binds to amikacin selectively versustobramycin and kanamycin. In certain non-limiting instances, a amikacin-binding primary aptamer comprises the sequences CAA and CGTCTACGGCTTAGC (SEQ ID NO:229) or a variant thereof and further comprises at least one operative sequence. In certain non-limiting instances, a amikacin-binding primary aptamer comprises the sequences CAA and CGTCTACGGCTTAGC (SEQ ID NO:229), or a variant thereof, and further comprises at least one operative sequence, said operative sequence complementary to a sequence comprised in a sensor oligonucleotide. In certain non-limiting instances, a amikacin-binding primary aptamer comprises the sequences CAA and CGTCTACGGCTTAGC (SEQ ID NO:229), or a variant thereof, and at least one operative sequence on either side (flanking) these two sequences. In certain non-limiting instances, a amikacin-binding primary aptamer comprises the sequences CAA and CGTCTACGGCTTAGC (SEQ ID NO:229), or a variant thereof, and at least one operative sequence on either side (flanking) these two sequences, where two of said operative sequences contain mutually complementary portions and can form a duplex. For example, but not by way of limitation, a amikacin-binding aptamer may comprise the sequence: CTC TCG GGA CGA CGC AAC CAT TCC AGT GGC GTC TAC GGC TTA GCT TTT CGT CGT CCC (SEQ ID NO: 270).

In certain non-limiting instances, isolated amikacin- binding primary aptamers comprise the nucleotide sequence of SEQ ID NO:225, SEQ ID NO:228, SEQ ID NO:230, or variants of these sequences having at least about 80 percent, or at least about 85 percent, or at least about 90 percent, or at least about 95 percent, or at least about 98 percent homology to the original sequence, for example obtained by substitutions, deletions, and insertions of Watson-Crick base pairs or by mutations at non-conserved positions. Percent homology can be determined using standard software such as BLAST or FASTA. In certain non-limiting instances, isolated amikacin-binding primary aptamers comprise the nucleotide sequence of SEQ ID NO:225, SEQ ID NO:228, or SEQ ID NO:230. Said aptamers can bind to amikacin and in their structure-switching formats (for example, in an anti-aptamer assay, a pseudosandwich assay, or a sandwich assay) they can respond by an increase in fluorescence.

### 5.1.16 METHYLENE BLUE-BINDING PRIMARY APTAMERS

In certain non-limiting instances, a primary aptamer binds to methylene blue in an aqueous solution at room temperature or 25°C with a dissociation constant of less than 10⁻⁶ M and binds selectively with methylene blue (see FIGURES 86B, 87B, 88B, 89B, 90B and 91B).

For example, but not by way of limitation, a methylene blue -binding aptamer may comprise the sequence: CTC TCG GGA CGA CCA GGA TGC TGT TCC ACC GGG GTA CAG GTA GGT CGC TGT CGT CCC (SEQ ID NO: 271).

For example, but not by way of limitation, a methylene blue -binding aptamer may comprise the sequence: CTC TCG GGA CGA CGG GCG TAG CGA TAG AAG AGA GCA GGG GGA GAG ACC TGT CGT CCC (SEQ ID NO: 272).

For example, but not by way of limitation, a methylene blue -binding aptamer may comprise the sequence: CTC TCG GGA CGA CGG GAA GGA GTT CCG GGG TAC GCG GGT AAG GGA AGG AGT CGT CCC (SEQ ID NO: 273).

For example, but not by way of limitation, a methylene blue -binding aptamer may comprise the sequence: CTC TCG GGA CGA CCA ACG AGT ATA CGC TTA CGT CAC GTT GAT GCT GTG GGT CGT CCC (SEQ ID NO: 274).

For example, but not by way of limitation, a methylene blue -binding aptamer may comprise the sequence: CTC TCG GGA CGA CGC ATT GAT GTA CAA GCT CGA TTC GTA TCC CTT GAT CGT CGT CCC (SEQ ID NO: 275).

For example, but not by way of limitation, a methylene blue -binding aptamer may comprise the sequence: CTC TCG GGA CGA CTG GGC TCG TGT TCT ATG GAC AAG GGG GAG TGA CCT GGT CGT CCC (SEQ ID NO: 276).

In certain non-limiting instances, isolated methlene blue- binding primary aptamers comprise the nucleotide sequence of SEQ ID NO:231, SEQ ID NO:232, SEQ ID NO:233, SEQ ID NO:234, SEQ ID NO:235, SEQ ID NO:236 or variants of these sequences having at least about 80 percent, or at least about 85 percent, or at least about 90 percent, or at least about 95 percent, or at least about 98 percent homology to the original sequence, for example obtained by substitutions, deletions, and insertions of Watson-Crick base pairs or by mutations at non-conserved positions. Percent homology can be determined using standard software such as BLAST or FASTA. In certain non-limiting instances, isolated methylene blue -binding primary aptamers comprise the nucleotide sequence of SEQ ID NO:231, SEQ ID NO:232, SEQ ID NO:233, SEQ ID NO:234, SEQ ID NO:235, or SEQ ID NO:236. Said aptamers can bind to methylene blue and in their structure-switching formats (for example, in an anti-aptamer assay, a pseudosandwich assay, or a sandwich assay) they can respond by an increase in fluorescence.

### 5.1.17 AMMONIUM-BINDING PRIMARY APTAMERS

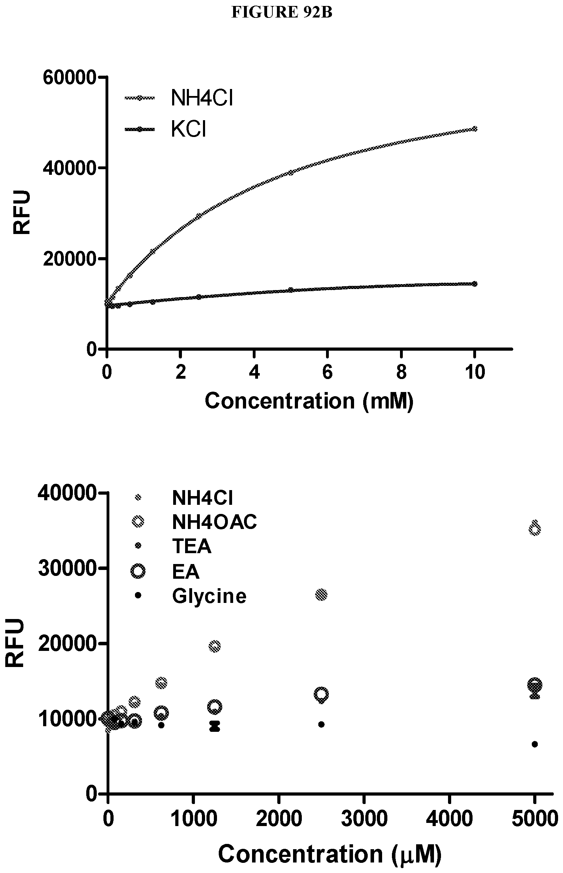

In certain non-limiting instances, a primary aptamer binds to ammonium ion in an aqueous solution at room temperature or 25°C with a dissociation constant of less than 10⁻² M and binds selectively with ammonium versus glycine or ethanolamine or potassium ion (see FIGURES 92B, 93B, 94B, 95B, 96B, 97B, and 98B).

For example, but not by way of limitation, an ammonium -binding aptamer may comprise the sequence: CTC TCG GGA CGA CGG AAG AGG CTC AGT GCT ATC TTA TCT GAG AGG GTT TGT CGT CCC (SEQ ID NO: 277).

For example, but not by way of limitation, an ammonium -binding aptamer may comprise the sequence: CTC TCG GGA CGA CGG GAG TGT CTC CTA AGG CCT TAG TAA GAA GGG TCC TGT CGT CCC (SEQ ID NO: 278).

For example, but not by way of limitation, an ammonium -binding aptamer may comprise the sequence: CTC TCG GGA CGA CGG GAA GAG GCT CGT GAG TTG ATG GGG AGA GGG TCC GGT CGT CCC (SEQ ID NO: 279).

For example, but not by way of limitation, an ammonium -binding aptamer may comprise the sequence: CTC TCG GGA CGA CGG AAG GGT CCC GTT GAG TTT GCA ATG GTG AGG GTT TGT CGT CCC (SEQ ID NO: 280).

For example, but not by way of limitation, an ammonium -binding aptamer may comprise the sequence: CTC TCG GGA CGA CGC CGA TGG AAG GGG CCC TGG TGG GAG GGT CAA AGG GGT CGT CCC (SEQ ID NO: 281).

For example, but not by way of limitation, an ammonium -binding aptamer may comprise the sequence: CTC TCG GGA CGA CGG GCA GGT AGA TCT ACA TGA ATA TGA AGG AAT GAT CGT CGT CCC (SEQ ID NO: 282).

For example, but not by way of limitation, an ammonium -binding aptamer may comprise the sequence: CTC TCG GGA CGA CGG GGA GTA GCC GGG TGG TTA GTG TCT CGC GAG GAA GTC GTC CC (SEQ ID NO: 283).

In certain non-limiting instances, isolated ammonium- binding primary aptamers comprise the nucleotide sequence of SEQ ID NO:239, SEQ ID NO:240, SEQ ID NO:241, SEQ ID NO:242, SEQ ID NO:243, SEQ ID NO:244, SEQ ID NO:245 or variants of these sequences having at least about 80 percent, or at least about 85 percent, or at least about 90 percent, or at least about 95 percent, or at least about 98 percent homology to the original sequence, for example obtained by substitutions, deletions, and insertions of Watson-Crick base pairs or by mutations at non-conserved positions. Percent homology can be determined using standard software such as BLAST or FASTA. In certain non-limiting instances, isolated ammonium-binding primary aptamers comprise the nucleotide sequence of, SEQ ID NO:239, SEQ ID NO:240, SEQ ID NO:241, SEQ ID NO:242, SEQ ID NO:243, SEQ ID NO:244, SEQ ID NO:245. Said aptamers can bind to ammonium and in their structure-switching formats (for example, in an anti-aptamer assay, a pseudosandwich assay, or a sandwich assay) they can respond by an increase in fluorescence.

### 5.1.18 BORONIC ACID-BINDING PRIMARY APTAMERS

In certain non-limiting instances, a primary aptamer binds to boronic acid in an aqueous solution at room temperature or 25°C with a dissociation constant of less than 10⁻² M and binds selectively with boronic acid versus bisboronic acid, e.g., complexed with glucose (FIGURE 99B, 100B).

For example, but not by way of limitation, a boronic acid -binding aptamer may comprise the sequence: CTC TCG GGA CGA CCA GGT GGG GCT GCT CAA GTG GAG GTT CCT CGT CGT CCC (SEQ ID NO: 284).

For example, but not by way of limitation, a boronic acid -binding aptamer may comprise the sequence: CTC TCG GGA CGA CCA GAG GGG CCT CAA ATG TGG GGT GTT GCT CGT CGT CCC (SEQ ID NO: 285).

In certain non-limiting instances, isolated boronic acid- binding primary aptamers comprise the nucleotide sequence of SEQ ID NO:247, SEQ ID NO:248, or variants of these sequences having at least about 80 percent, or at least about 85 percent, or at least about 90 percent, or at least about 95 percent, or at least about 98 percent homology to the original sequence, for example obtained by substitutions, deletions, and insertions of Watson-Crick base pairs or by mutations at non-conserved positions. Percent homology can be determined using standard software such as BLAST or FASTA. In certain non-limiting instances, isolated boronic acid-binding primary aptamers comprise the nucleotide sequence of, SEQ ID NO:247 or SEQ ID NO:248. Said aptamers can bind to boronic acid and in their structure-switching formats (for example, in an anti-aptamer assay, a pseudosandwich assay, or a sandwich assay) they can respond by an increase in fluorescence.

### 5.1.19 EPINEPHRINE-BINDING PRIMARY APTAMERS

In certain non-limiting instances, a primary aptamer binds to epinephrine in an aqueous solution at room temperature or 25°C with a dissociation constant of less than 10⁻³ M and binds selectively with epinephrine versus serotonin, norepinephrine or dpoamine (FIGURE 101B, 102B).

For example, but not by way of limitation, an epinephrine-binding aptamer may comprise the sequence: CTC TCG GGA CGA CCG GGG TAG GGG TTA GGT GGG AAT GGA GCT GGA CCG TGT CGT CCC (SEQ ID NO: 286).

For example, but not by way of limitation, an epinephrine-binding aptamer may comprise the sequence: CTC TCG GGA CGA CGG ACC GTT GCC CTG GGG TAG TGC GCG CTT CGT TTA CGT CGT CCC (SEQ ID NO: 287).

In certain non-limiting instances, isolated epinephrine - binding primary aptamers comprise the nucleotide sequence of SEQ ID NO:249, SEQ ID NO:250, or variants of these sequences having at least about 80 percent, or at least about 85 percent, or at least about 90 percent, or at least about 95 percent, or at least about 98 percent homology to the original sequence, for example obtained by substitutions, deletions, and insertions of Watson-Crick base pairs or by mutations at non-conserved positions. Percent homology can be determined using standard software such as BLAST or FASTA. In certain non-limiting instances, isolated epinephrine -binding primary aptamers comprise the nucleotide sequence of, SEQ ID NO:249 or SEQ ID NO:250. Said aptamers can bind to epinephrine and in their structure-switching formats (for example, in an anti-aptamer assay, a pseudosandwich assay, or a sandwich assay) they can respond by an increase in fluorescence.

### 5.1.20 CREATININE-BINDING PRIMARY APTAMERS

In certain non-limiting instances, a primary aptamer binds to creatinine in an aqueous solution at room temperature or 25°C with a dissociation constant of less than 10⁻² M and binds selectively with creatinine versus creatine or urea.

In certain non-limiting instances, a creatinine-binding primary aptamer comprises the sequences GGTGGCCT (SEQ ID NO:254) and AGGGGTG (SEQ ID NO:255) or a variant of any of these sequences that differs in one or two bases by substitution, deletion, insertion or extension, where said primary aptamer binds to creatinine (see FIGURES 103B, 104B. 105B, 106B) in an aqueous solution at room temperature or 25°C with a dissociation constant of less than 10⁻² M and binds to creatinine selectively versus creatine or urea. In certain non-limiting instances, a creatinine-binding primary aptamer comprises the sequences GGTGGCCT (SEQ ID NO:254) and AGGGGTG (SEQ ID NO:255) or a variant thereof and further comprises at least one operative sequence. In certain non-limiting instances, a creatinine-binding primary aptamer comprises the sequences GGTGGCCT (SEQ ID NO:254) and AGGGGTG (SEQ ID NO:255) or a variant thereof, and further comprises at least one operative sequence, said operative sequence complementary to a sequence comprised in a sensor oligonucleotide. In certain non-limiting instances, a creatinine-binding primary aptamer comprises the sequences GGTGGCCT (SEQ ID NO:254) and AGGGGTG (SEQ ID NO:255), or a variant thereof, and at least one operative sequence on either side (flanking) these two sequences. In certain non-limiting instances, a creatinine-binding primary aptamer comprises the sequences GGTGGCCT (SEQ ID NO:254) and AGGGGTG (SEQ ID NO:255) or a variant thereof, and at least one operative sequence on either side (flanking) these two sequences, where two of said operative sequences contain mutually complementary portions and can form a duplex.

For example, but not by way of limitation, a creatinine-binding aptamer may comprise the sequence: GA CGA CGGTGGCCTTAATAGATAGATGATATTCTTAT ATGTG TGAGGGGTG GT CGT C (SEQ ID NO:256; FIGURE 103A).

For example, but not by way of limitation, a creatinine-binding aptamer may comprise the sequence: GA CGA C GGTGGCCTATATTGGTATGTATGAA GAATAGAACTATTAGGGGGT GT C (SEQ ID NO: 288).

For example, but not by way of limitation, a creatinine-binding aptamer may comprise the sequence: CGA C GGTGGCCTATTAAATAGCTTTAGTT TAAGAAAAGTAATAGGGGGT GT CG (SEQ ID NO:258; FIGURE 105A).

For example, but not by way of limitation, a creatinine-binding aptamer may comprise the sequence: CTC TCG GGA CGA C GGTGGCCTATTAAGTAGCTTTA GTTCAAGAAAAGTAATAGGGGGT GT CGT CCC (SEQ ID NO: 289).

In certain non-limiting instances, isolated creatinine- binding primary aptamers comprise the nucleotide sequence of SEQ ID NO:256, SEQ ID NO:257, SEQ ID NO:258, SEQ ID NO:259 or variants of these sequences having at least about 80 percent, or at least about 85 percent, or at least about 90 percent, or at least about 95 percent, or at least about 98 percent homology to the original sequence, for example obtained by substitutions, deletions, and insertions of Watson-Crick base pairs or by mutations at non-conserved positions. Percent homology can be determined using standard software such as BLAST or FASTA. In certain non-limiting instances, isolated creatinine-binding primary aptamers comprise the nucleotide sequence of SEQ ID NO:256, SEQ ID NO:257, SEQ ID NO:258, or SEQ ID NO:259. Said aptamers can bind to creatinine and in their structure-switching formats (for example, in an anti-aptamer assay, a pseudosandwich assay, or a sandwich assay) they can respond by an increase in fluorescence.

### 5.1.21. VASOPRESSIN-BINDING PRIMARY APTAMERS

In certain non-limiting instances, a primary aptamer binds to vasopressin in an aqueous solution at room temperature or 25°C with a dissociation constant of less than 10⁻⁴ M and binds selectively with vasopressin versus oxytocin or pressinoic acid.

In certain non-limiting instances, a vasopressin-binding primary aptamer comprises the sequences GTAGTACGTT (SEQ ID NO:260) and CAT or a variant of any of these sequences that differs in one or two bases by substitution, deletion, insertion or extension, where said primary aptamer binds to vasopressin (see FIGURE 107B) in an aqueous solution at room temperature or 25°C with a dissociation constant of less than 10⁻⁴ M and binds to vasopressin selectively versusoxytocin or pressinoic acid. In certain non-limiting instances, a vasopressin-binding primary aptamer comprises the sequences GTAGTACGTT (SEQ ID NO:260) and CAT or a variant thereof and further comprises at least one operative sequence. In certain non-limiting instances, a vasopressin-binding primary aptamer comprises the sequences GTAGTACGTT (SEQ ID NO:260) and CATor a variant thereof, and further comprises at least one operative sequence, said operative sequence complementary to a sequence comprised in a sensor oligonucleotide. In certain non-limiting instances, a vasopressin-binding primary aptamer comprises the sequencesGTAGTACGTT (SEQ ID NO:260) and CAT, or a variant thereof, and at least one operative sequence on either side (flanking) these two sequences. In certain non-limiting instances, a vasopressin-binding primary aptamer comprises the sequences GTAGTACGTT (SEQ ID NO:260) and CATor a variant thereof, and at least one operative sequence on either side (flanking) these two sequences, where two of said operative sequences contain mutually complementary portions and can form a duplex.

For example, but not by way of limitation, a vasopressin-binding aptamer may comprise the sequence: GA C GTCCAAGTAGTACGTTTAATTAGG ATTTCCGAATTATTGGCATGC GT C (SEQ ID NO:261; FIGURE 107A)

In certain non-limiting instances, isolated vasopressin- binding primary aptamers comprise the nucleotide sequence of SEQ ID NO:261 or variants of these sequences having at least about 80 percent, or at least about 85 percent, or at least about 90 percent, or at least about 95 percent, or at least about 98 percent homology to the original sequence, for example obtained by substitutions, deletions, and insertions of Watson-Crick base pairs or by mutations at non-conserved positions. Percent homology can be determined using standard software such as BLAST or FASTA. In certain non-limiting instances, isolated vasopressin-binding primary aptamers comprise the nucleotide sequence of SEQ ID NO:261. Said aptamers can bind to vasopressin and in their structure-switching formats (for example, in an anti-aptamer assay, a pseudosandwich assay, or a sandwich assay) they can respond by an increase in fluorescence.

### 5.2 ANTI-APTAMER ASSAYS

An "anti-aptamer assay" is provided, in which a sample to be tested for the presence and/or amount of an analyte of interest is contacted with effective amounts of (1) a primary aptamer comprising a core sequence that binds to the analyte and (2) an "anti-aptamer" which is complementary to at least a portion of the primary aptamer, wherein the primary aptamer and/or anti-aptamer comprise a detectable moiety(ies) which detect whether the primary aptamer and anti-aptamer are bound to each other or unbound; and wherein a primary aptamer bound to the analyte does not bind to its anti-aptamer.

Certain instances provide for a method of detecting or measuring the presence or amount of analyte of interest in a sample, comprising (i) contacting at least a portion of a sample with effective amounts of a primary aptamer and an anti-aptamer that is complementary to at least a portion of the primary aptamer, said primary aptamer and/or anti-aptamer comprising a moiety which allows the amount of primary aptamer bound to analyte to be detected and/or measured, under conditions that would permit duplex formation between the primary aptamer and anti-aptamer if target analyte were not present; and (ii) detecting and optionally quantifying the amount of primary aptamer that is not bound to anti-aptamer.

A sample may be, for example and not limitation, a blood sample, a plasma sample, a serum sample, a urine sample, a tissue sample, a cerebrospinal fluid sample, a sputum sample, a fecal sample, a water sample, an industrial sample, etc.

As an illustrative example and not by way of limitation, the presence or amount of analyte of interest in a sample may be determined by (i) contacting at least a portion of the sample with effective amounts of (a) a primary aptamer comprising a fluorescent label and (b) an anti-aptamer, complementary to at least 80 percent or at least 90 percent or at least 95 percent or at least 98 percent of the primary aptamer, comprising a moiety that quenches fluorescence of said fluorescent label if primary aptamer and anti-aptamer are bound together in a duplex (for example, as a double-stranded molecule), under conditions that would permit duplex formation between primary aptamer and anti-aptamer to occur if analyte were not present; and (ii) detecting and optionally quantifying the amount of fluorescence. The amount of fluorescence may further be compared to the amount of fluorescence that results from a control mixture of primary aptamer and anti-aptamer in the absence of sample or in the presence of a known amount of analyte (e.g., a standard curve). This example may be modified as would be known to one skilled in the art. For example, different moieties may be used to detect duplex formation, such as a colorimetric label, an enzymatic label, a radiolabel, etc., and the detectable label may be carried on the anti-aptamer, depending on assay design.

In certain embodiments, a primary aptamer comprises one or more operative sequence which is complementary to anti-aptamer and facilitates duplex formation. For example, an operative sequence with a nucleotide composition which favors duplex formation may be used (a.k.a. a "toe hold" sequence).

The anti-aptamer assay and method of using it may utilize any primary aptamer that binds an analyte of interest, including but not limited to those set forth herein.

An anti-aptamer has a sequence which is complementary to at least 80 percent or at least 90 percent or at least 95 percent or at least 98 percent of its corresponding primary aptamer.

A primary aptamer or anti-aptamer for use in the anti-aptamer assay may be between about 30 and about 200 nucleotides, or between about 30 and 100, or between about 30 and 80, nucleotides in length.

In certain non-limiting embodiments, the primary aptamer and anti-aptamer may be present, in the assay, in a ratio of about 1: 1. Depending on the strength of binding between analyte and primary aptamer and/or between primary aptamer and anti-aptamer, it may be desirable to alter this ratio to produce a dose-response curve having the desired features (for example, being able to measure/detect analyte at a particular concentration). As an illustrative example, and not by way of limitation, if binding between primary aptamer and analyte is very strong relative to the affinity between primary aptamer and anti-aptamer, it may be desirable to increase the relative amount of anti-aptamer, so that the ratio is 1:greater than 1. Non-limiting examples are about 1: 1;

In certain non-limiting embodiments, an anti-aptamer assay may be performed in solution. Non-limiting examples of the use of anti-aptamer assay, in solution, for detecting various analytes are shown in FIGURE 3A-L, showing results for measuring deoxycortisoen (FIGURE 3A-B); aldosterone (FIGURE 3C-D); cortison (FIGURE 3E-F); testosterone (FIGURE 3G-H); Phen-CpRh (FIGUR 3I-J), and phenylalanine (FIGURE 3K-L). In a specific non-limiting example, provided as illustration, 400 nM FAMaptamer solution and four-times concentrated Iowa Black-anti-aptamer ("complement strand") solution are prepared separately in buffer (20 mM HEPES, 1 M NaCl, 10 mM MgCl2, 5 mM KCl, pH 7.5). These solutions are annealed separately by incubating in boiling water for 5 min, and cooled down at room temperature for ~ 30 min. Meanwhile, a two-times concentrate of each aptamer's target solutions are prepared. After the 30 min incubation period of the aptamer and anti-aptamer solutions, 18.75 ul of the aptamer solution is added first to the 384-well plate, then 37.5 ul of the target solution is added, and then immediately added is 18.75 ul of anti-aptamer solution. Without incubation, the fluorescent measurement is started and read every 5 min at room temperature for > 8 hours by using Fluorescence plate reader (Victor II microplate reader, PerkinElmer). The final concentration of aptamer is 100 nM, the concentration of complementary strand is 100 nM for all except CS aptamer (1000 nM) in 75 µL reaction volume.

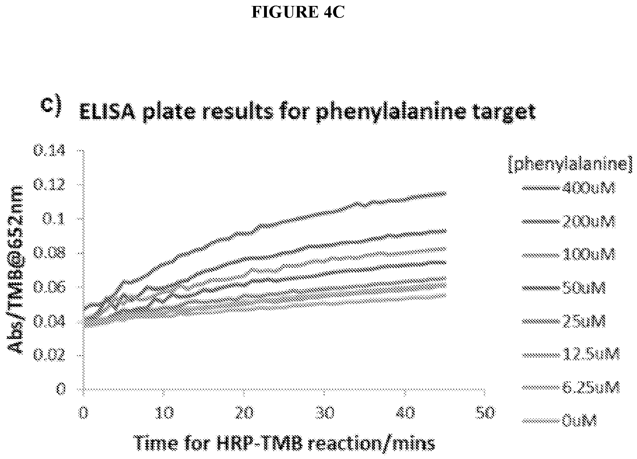

In certain non-limiting embodiments, an anti-aptamer assay may be performed inas a solid phase assay, with primary aptamer or anti-aptamer bound a solid phase (e.g., an ELISA-like format). Non-limiting examples of the use of anti-aptamer assayas a solidphase assay, for detecting various analytes are shown in FIGURE 4A-C for deoxycortisone (FIGURE 4A), glucose (FIGURE 4B) and phenylalanine (FIGURE 4C).

In certain non-limiting instances, a kit is provided for practicing an anti-aptamer assay as described herein. For example, said kit comprises a primary aptamer directed toward an analyte of interest and a corresponding anti-aptamer. Non-limiting examples of primary aptamers that may be comprised in such a kit include the primary aptamers set forth herein, for example as described in Section 5.1 and FIGURES 50A-B to 102AB, and a corresponding anti-aptamer as described herein. Said kit may optionally further comprise target analyte, for example a control solution comprising target analyte, and/or a standard curve.

### 5.3 PSEUDO SANDWICH ASSAY

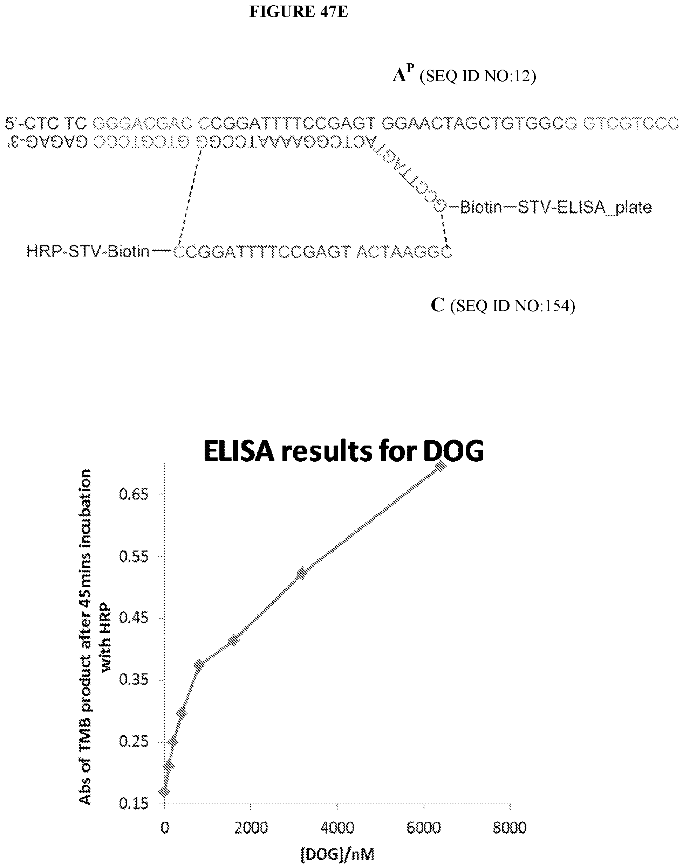

The present application discloses pseudosandwich assays in solution (FIGURE 47A-D) and on plates (FIGURE 47E-F). In certain non-limiting embodiments, the pseudosandwich assays employ a primary aptamer.

Certain instances provide for a "pseudosandwich assay", or method of detecting or measuring the presence or amount of an analyte of interest in a sample, comprising (i) contacting at least a portion of a sample with effective amounts of (a) a primary aptamer comprising a core sequence that binds to the analyte and a portion complementary to a sensor oligonucleotide; (b) a sensor oligonucleotide; and optionally (c) a comp oligonucleotide; one or more of which is bound to a detectable moity(ies) which can detect whether the primary aptamer and sensor oligonucleotide are bound to each other or whether primary aptamer is bound to analyte.

In certain non-limiting instances, the method comprises:
(1) Isolating primary aptamers (A^{p}s) by solution-phase (FIGURE 7) or solidphase selection, unless they are already available; use of enantiomeric aptamer (spiegelmers), if desired to minimize Watson-Crick base pairing (e.g., fusing aptamers or to minimize background interactions without analyte);
(2) Testing of the primary aptamer in its structure-switching form and modifying its structure switching form, which is then turned into pseudo-sandwich assay format (FIGURE 6C);
(3) Isolating secondary aptamers (A^{s}s) by either solution-phase or solid phase selections (FIGURE 9), using primary aptamers or spiegelmers in their complexes with targets; and
(4) Implementing sandwich assays for targets (FIGURE 8).

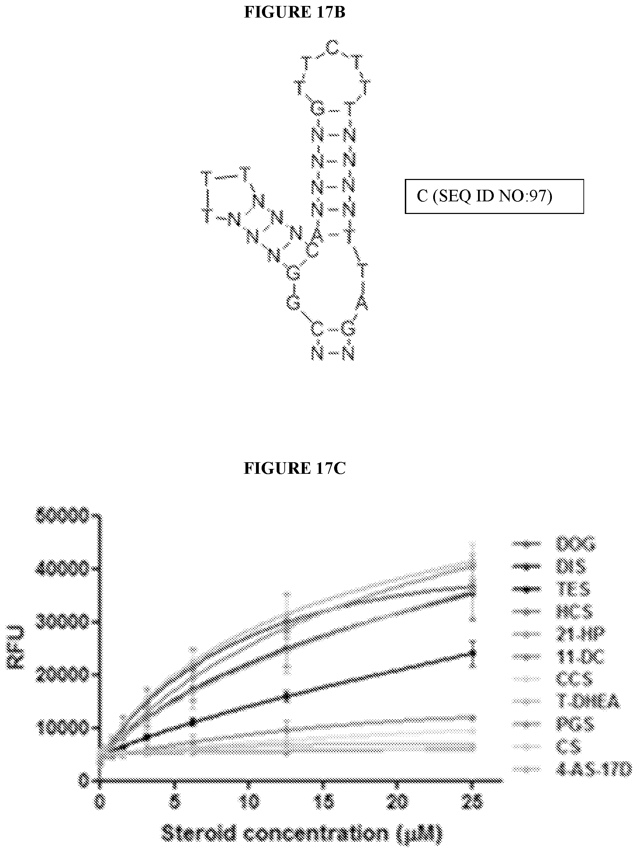

An exemplary list of the disclosed primary aptamers are provided in FIGURE 10-46 in their sensor (structure-switching) forms ¹⁶⁻²¹, together with their associated sensor oligonucleotides. The disclosed primary aptamers were isolated using solution-phase selection and can be used in aptamer-based assays, not limited to sandwich and pseudosandwich assays. In certain non-limiting embodiments, primary aptamers (FIGURE 10-46) can be spiegelmers or unnatural enantiomers of nucleic acids ²²⁻²⁵. In certain non-limiting embodiments, primary aptamers can be directly engineered to be used in "pseudosandwich" assays (see FIGURE 6, FIGURE 47 and FIGURE 48).

A "pseudo-sandwich" assay transforms reversible interactions of analyte-binding aptamers (primary aptamers or A^{P}) into a more stable partially double helical complex, which can be used in solution-phase assays (FIGURE 6 and FIGURE 47A-D), or in solution-state assays (plates, beads or components of lateral flow devices), in which case it may allow extensive washing (FIGURE 6 and FIGURE 47E-F).

The stable double helical product released once a primary aptamer is bound to its analyte can be also captured to a solid state surface (e.g., plate well or beads or lateral flow pad) and subjected to extensive washing, therefore eliminating many sources of high background in other aptamer-based assays using structure switching principles. The oligonucleotide in complex with capture oligonucleotide can be then used to generate amplified readout indicative of presence and quantity initially bound analyte, like in a typical sandwich ELISA.

For example, a complementary oligonucleotide (C_{D}) can be extended (C_{Dext}) into an aptamer. While C_{D} on its own is in equilibrium and 3-5 equivalents are used to achieve quenching of aptamers with fluorescein, C_{Dext} requires only one equivalent and it binds nearly irreversibly to the aptamers. This helps with the half-response point.

C_{Dext} can then be further extended by adding a "toehold" region that allows this kinetically protected stable complex to interact with an oligonucleotide complementary to C_{Dext} (C_{Dext}^{comp}). The presence of an excess of C_{Dext}^{comp} in solution allows small amounts of aptamer to form binding pockets and interact with the L. This triggers establishment of equilibrium leading to a certain amount of double helix formations which is substantially increase with the release of double helix into solution. This process can be monitored by an increase in fluorescence in solution.

### Pseudosandwich assays - in solution:

In certain non-limiting instances pseudosandwich assays in solution can be performed, wherein a double helix formation is triggered by deoxycorticosterone. In certain non-limiting instances, primary aptamers comprising the nucleotide sequence of SEQ ID NO: 12 or analogs can form a complex with C_{Dext} and in the presence of C_{Dext}^{comp} can produce an increase in fluorescence in the presence of deoxycorticosterone and analogs.

In certain non-limiting instances pseudosandwich assays in solution can be performed, wherein a double helix formation is triggered by tyrosine. In certain non-limiting instances, primary aptamers comprising the nucleotide sequence of SEQ ID NO: 33 or analogs can form a complex with C_{Dext} and in the presence of C_{Dext}^{comp} can produce an increase in fluorescence in the presence of tyrosine and analogs.

In certain non-limiting instances pseudosandwich assays in solution can be performed, wherein a double helix formation is triggered by glucose. In certain non-limiting instances, primary aptamers comprising the nucleotide sequence of SEQ ID NO: 1 or analogs can form a complex with C_{Dext} and in the presence of C_{Dext}^{comp} can produce an increase in fluorescence in the presence of glucose and analogs.

In certain non-limiting instances pseudosandwich assays in solution can be performed, wherein a double helix formation is triggered by Phenylalanine. In certain non-limiting instances, primary aptamers comprising the nucleotide sequence of SEQ ID NO: 2 or analogs can form a complex with C_{Dext} and in the presence of C_{Dext}^{comp} can produce an increase in fluorescence in the presence of Phenylalanine and analogs.

In certain non-limiting instances, if the C_{Dext} is deposited on plates, the double helix remains attached to the plate when the rest of solution is removed and it can now be extensively washed. This double helix can be used in numerous ways for the signal development.

### Pseudosandwich assays on plate:

Solution-evolved aptameric sensors can be adapted for use in a solid surfaceformat application, for example, ELISA-type assays. Specifically, the solution sensor - composed of an analyte and a specific aptamer comprising the nucleotide sequence of SEQ ID NO: 12, which is partially hybridized to C_{Dext} - is modified with attachment chemistry to enable its anchoring to a solid surface.

The attachment chemistry can comprise use of biotin or covalent bonding between maleimide and sulfhydryl functional groups. In addition, the complementary strand to the competitor oligonucleotide C_{Dext} (C_{Dext}^{comp}) (optimized to bind to the C_{Dext} in the presence of analyte) can be modified with a biotin-tag, which is used to capture a "read-out" producing molecule e.g. HRP conjugate. The "read-out" can be performed using 3,3',5,5'-Tetramethylbenzidine (TMB), the substrate for HRP, for visualization.

Optimization of response of the surface-bound sensor, relative to +/- analyte, can be carried out by adjustment of several parameters: Amount of sensor on surface; attachment chemistry, concentration of complementary competitor oligonucleotide; buffer in each step; washing steps; HRP concentration; HRP substrate.

A primary aptamer can comprise at least one operative sequence which is complementary to at least a portion of a sensor oligonucleotide.

Non-limiting illustrative examples showing pairs of primary aptamer and sensor oligonucleotide are shown in FIGURES 10A-42A. For example, but not by way of limitation, glucose-binding primary aptamer (SEQ ID NO:1) comprises an operative sequence complementary to sensor oligonucleotide (SEQ ID NO: 82); phenylalanine-binding primary aptamer SEQ ID NO:2 comprises an operative sequence complementary to sensor oligonucleotide (SEQ ID NO:84); phenylalanine-binding primary aptamer SEQ ID NO:3 comprises an operative sequence complementary to sensor oligonucleotide (SEQ ID NO:86; hydrocortisone-binding primary aptamer (SEQ ID NO5) comprises an operative sequence complementary to sensor oligonucleotide (SEQ ID NO:90), etc. In certain non-limiting embodiments, a pseudosandwich assay on plate can be performed to detect deoxycorticosterone.

A sample may be, for example and not limitation, a blood sample, a plasma sample, a serum sample, a urine sample, a tissue sample, a cerebrospinal fluid sample, a sputum sample, a fecal sample, a water sample, an industrial sample, etc.

In certain non-limiting instances, the foregoing assays can be used to detect and/or quantitate any analyte of interest, and can be particularly advantageous over existing methods in detecting and/or quantitating analytes that have a molecular weight less than about 1000 Daltons, or less than about 500 Daltons, or less than about 200 Daltons, including but not limited to steroid compounds, such as but not limited to cortisol, aldosterone, dehydroisoandrosterone, progesterone, testosterone; glucose; amino acids such as but not limited to phenylalanine, leucine, isoleucine, valine, citrulline, tyrosine, alanine; pharmaceutical compounds, vitamins, toxins, neurotransmitters (e.g., catecholamines, serotonin), peptides (vasopressin, oxytocin, angiotensin, natriuretic peptides, glucagon, insulin and others), antibiotics and antifungal compounds (e.g., aminoglucosides), macrocyclic immunosuppresants, or lipids or lipid complexes.

In certain non-limiting instances, the assays can also be applied to large molecules, for example, above 1000 D.

In certain non-limiting instances, a kit is provided for practicing a pseudosandwich assay as described herein. In certain instances, said kit comprises a primary aptamer directed toward an analyte of interest and a corresponding sensor oligonucleotide. Non-limiting examples of primary aptamers that may be comprised in such a kit include the primary aptamers set forth herein, for example as described in Section 6.1 and FIGURES 50A-B to 102AB, and a corresponding sensor oligonucleotide as described herein. Non-limiting examples of primary aptamer/sensor oligonucleotide pairs are set forth in FIGURES 10A-42A. Said kit may further comprise one or more comp oligonucleotide, as described herein. Said kit may optionally further comprise target analyte, for example a control solution comprising target analyte, and/or a standard curve.

### 5.4 SANDWICH ASSAY

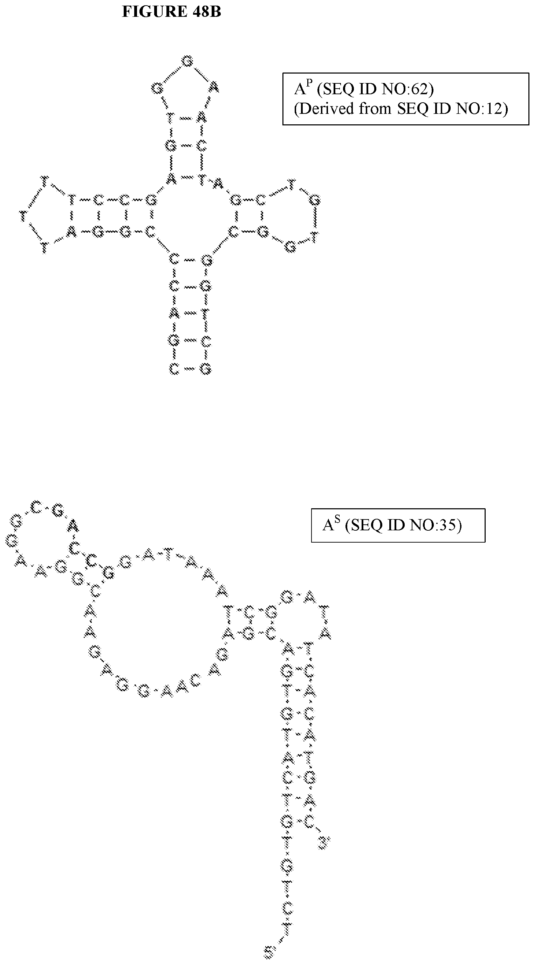

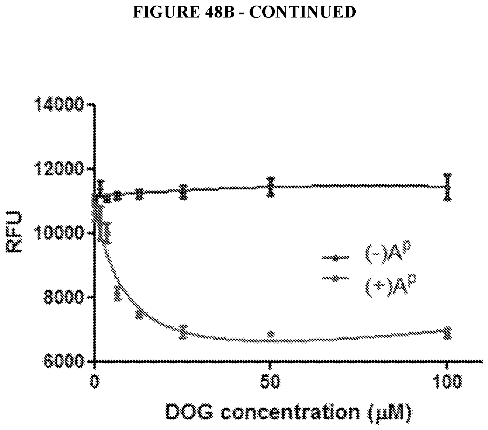

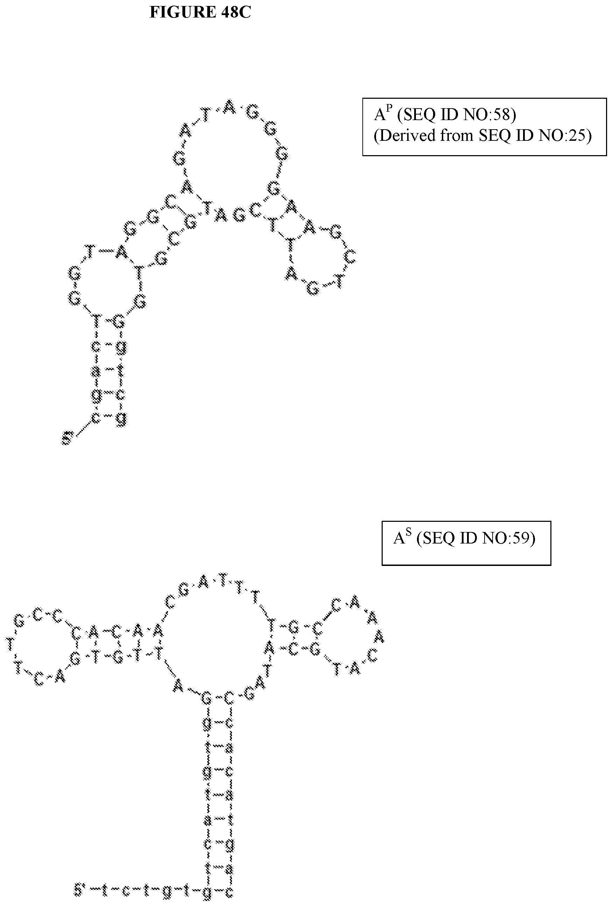

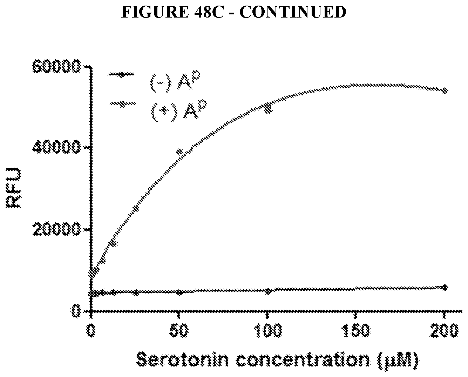

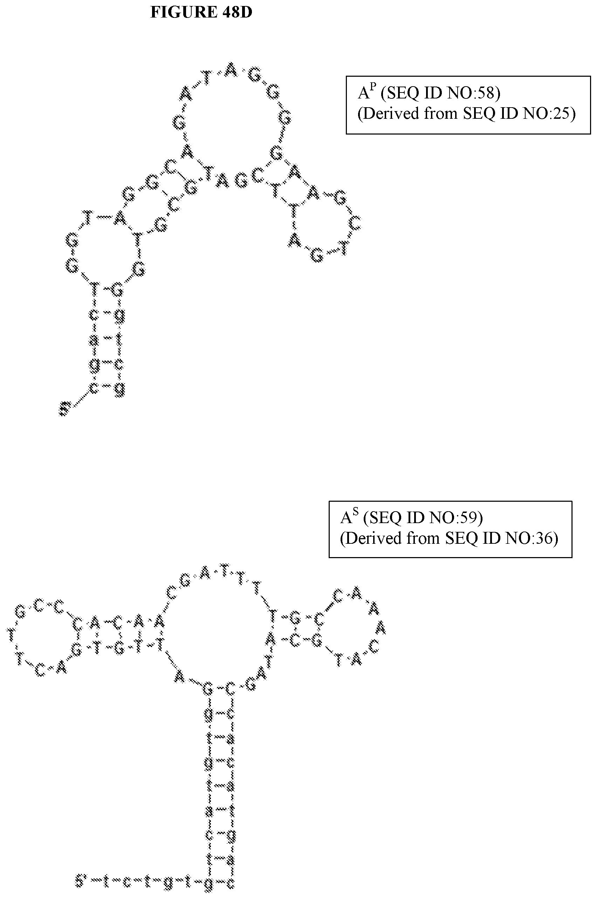

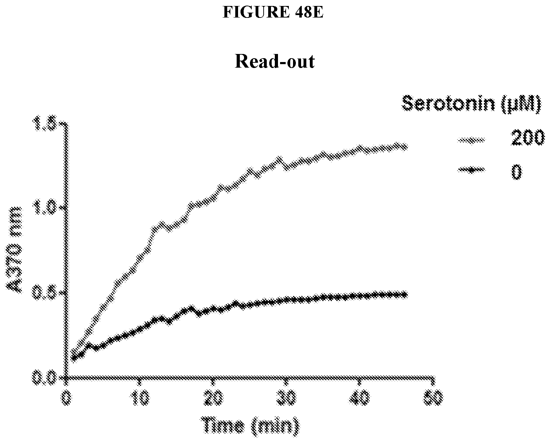

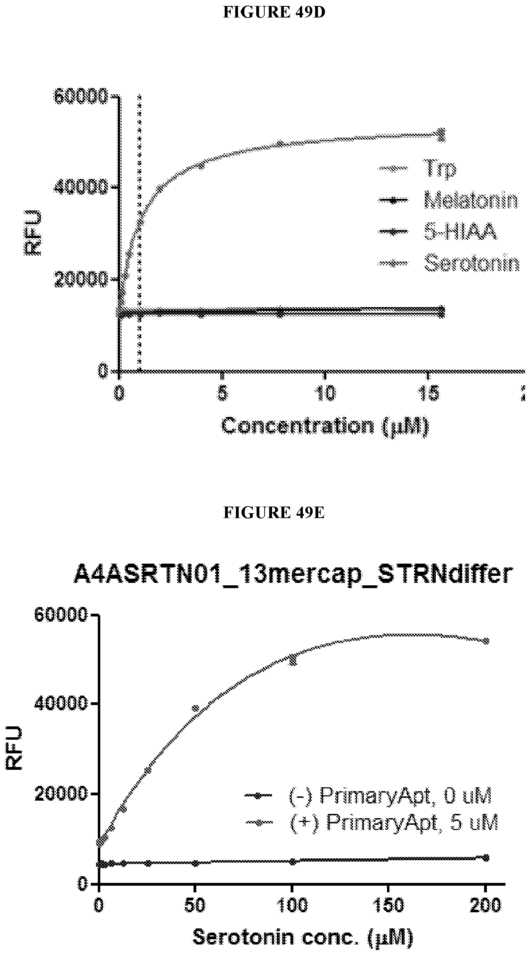

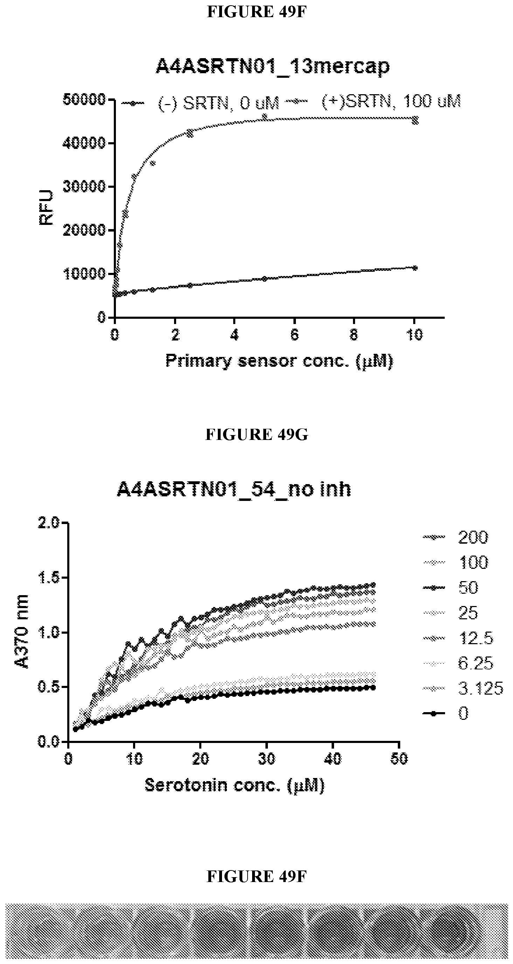

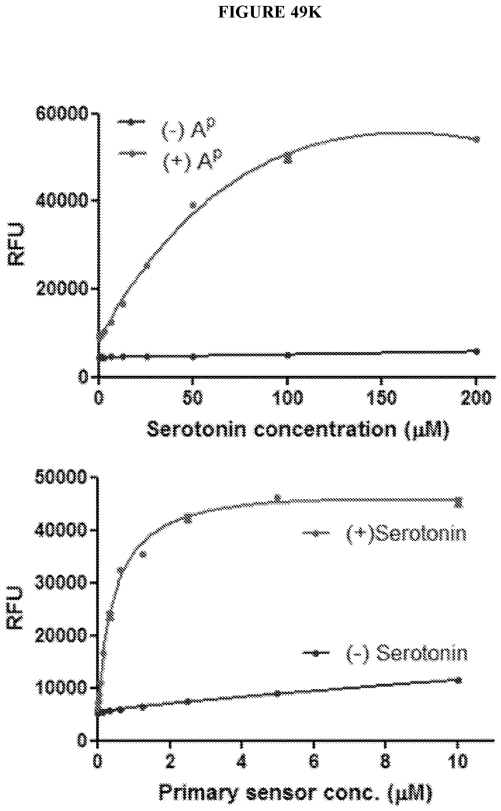

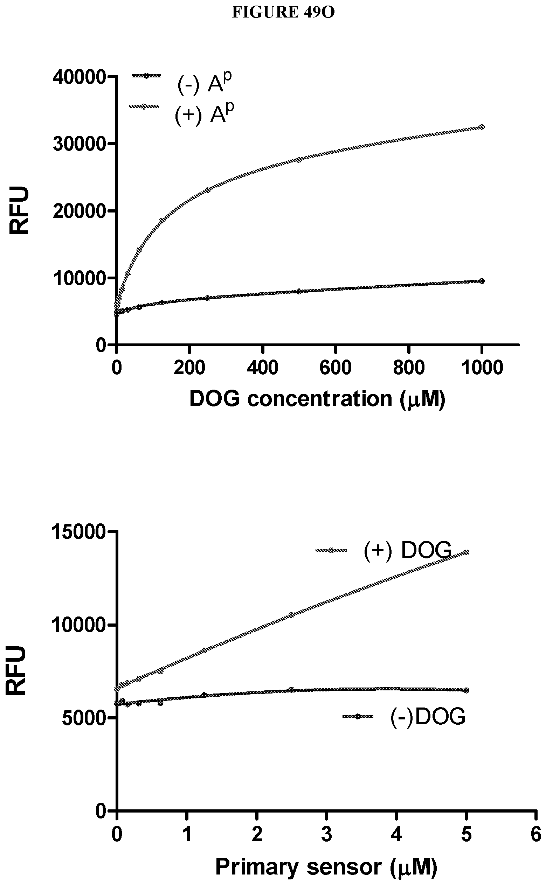

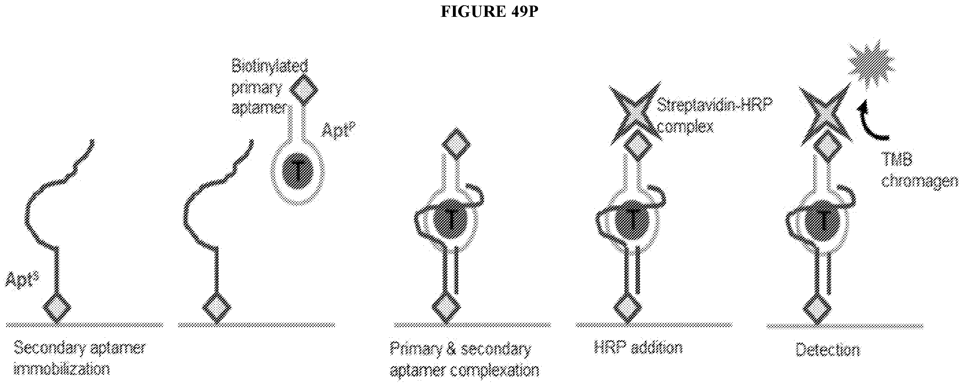

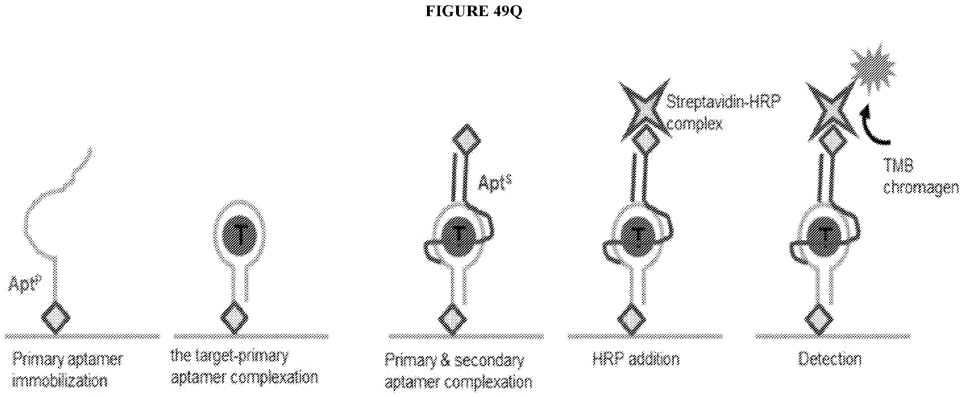

A "secondary aptamer" (A^{S}) binds a complex (A^{P∗}L) of a primary aptamer (A^{P}) to its target analyte (ligand, L) selectively over free primary aptamers, forming a ternary complex (A^{P∗}L^{∗}A^{S}). The secondary aptamers are isolated by solution-phase or solidphase selections. An exemplary non-limiting list of the disclosed secondary aptamers is provided in FIGURES 48 and 49. The disclosed secondary aptamers were isolated by either of the aforementioned selections.

In certain embodiments, a sandwich assay is provided, which comprises a method of detecting or measuring the presence or amount of an analyte of interest in a sample, comprising (i) contacting at least a portion of a sample with effective amounts of (a) a primary aptamer comprising a core sequence that binds to the analyte and a portion that, when primary aptamer is bound to analyte, binds to a secondary "sandwich" aptamer; (b) a secondary "sandwich"aptamer; one or more of which is bound to a detectable moity(ies) which can detect whether the primary aptamer and secondary "sandwich" aptamer are bound to each other or unbound.

In certain embodiments, a primary aptamer comprises at least one operative sequence which binds to a sandwich aptamer (a.k.a. secondary aptamer).

In certain, non-limiting embodiments, a secondary aptamer comprises an aptamer ('secondary') that binds to an aptamer (primary) with former binding to the latter preferentially when the latter is bound to its ligand (an analyte of interest).

In certain, non-limiting embodiments, a secondary aptamer comprises a sequence ('secondary') that binds to another sequence (primary) with former binding to the latter preferentially when the latter bound to a molecule.

In certain, non-limiting embodiments, the present invention discloses an assay for a molecule (fluorescence assay and ELISA-like) that uses two oligonucleotides with one oligonucleotide that preferentially binds to the other, when the latter is bound to that molecule.

In certain, non-limiting embodiments, one aptamer (primary) is sufficient to form a receptor.

A "sandwich" assay, without availability of the second binding site (epitope) in a molecule, is enabled by a secondary aptamer (A^{S}; a.k.a. sandwich aptamer or secondary sandwich aptamer) forming a ternary complex (A^{P∗}L^{∗}A^{S}) (FIGURE 8). In the disclosed sandwich assay, the primary and secondary aptamers are "sandwiching" components, with L being a target. In certain non-limiting embodiments, the sandwich assay can be implemented in solution (FIGURES 48 and 49) or on solid surface (FIGURE 49) in non-limiting examples, plates, beads, or any component of a lateral flow device).

In certain non-limiting instances, a kit is provided for practicing a sandwich assay as described herein. For example, said kit comprises a primary aptamer directed toward an analyte of interest and a corresponding sandwich aptamer (a.k.a., secondary aptamer . Non-limiting examples of primary aptamers that may be comprised in such a kit include the primary aptamers set forth herein, for example as described in Section 6.1 and FIGURES 50A-B to 102AB, and a corresponding sandwich/secondary aptamer as described herein. Said kit may optionally further comprise target analyte, for example a control solution comprising target analyte, and/or a standard curve.

A sample may be, for example and not limitation, a blood sample, a plasma sample, a serum sample, a urine sample, a tissue sample, a cerebrospinal fluid sample, a sputum sample, a fecal sample, a water sample, an industrial sample, etc.

### 5.4.1. ISOLATION OF SECONDARY APTAMERS

The present application discloses the isolation of secondary aptamers using the SELEX process.

In certain non-limiting instances, methods for selecting secondary aptamers (A^{s}) can be performed, which can also be combined in some cases (FIGURE 9) comprise a solid-state selection and a solution-phase selection.

### Solid-state election

During the solid-state selection a target aptamer (A^{p}) is attached to a matrix (e.g., beads), incubated with a library (e.g., but not limited to, pre-structured or unstructured N₂₀₋₁₀₀) in the presence of target analyte L to isolate aptamer candidates with affinity for A^{p∗}L complex (FIGURE 9A). These oligonucleotides are PCR-amplified, single-stranded species regenerated, and then used in the next selection cycle. The process is repeated until convergence is reached, pools cloned and sequenced, leading to A^{s} candidates. The counter-selection is performed by elimination of binders to A^{p} in the absence of L, ensuring binding to the complex.

### Solution-phase selection

The process in solution-phase uses pre-structured library to enable formation of stem between primers attached to matrix via complementary oligonucleotides, and A^{s} candidates are selected because their loop is closed through binding to A^{p} and they get released from the solid surface (FIGURE 9B). Counter-selection in this case is against A^{p} without ligand, and ligand itself.

In certain non-limiting embodiments, primary aptamers used in selection can be made from DNA, RNA, modified nucleotides, or spiegelmers. Spiegelmers are particularly suitable to minimize background, increase affinity, and improve properties of secondary aptamers, because these are mirror-image nucleic acids ²⁰⁻²³ with L-deoxyribose or ribose, that do not firm contiguous Watson Crick base pairing with natural DNA. In certain non-limiting embodiments, such spiegelmers can be obtained by inverting aptamers for planar molecules or molecules with planes of symmetry, such as serotonin or dopamine (aptamers depicted in FIGURE 28-39 can be inverted) or other neurotransmitters.

### 5.4.2. SECONDARY APTAMERS TO GLUCOSE-BINDING PRIMARY APTAMERS

More specifically, in certain non-limiting instances, secondary aptamers can be isolated according to the aforementioned method for glucose. In certain non-limiting instances, secondary aptamers or their analogs obtained by substitutions, deletions, and insertions of Watson-Crick base pairs or by mutations at non-conserved positions, bind to a primary aptamer, when the latter is in the complex with glucose. In certain non-limiting instances, secondary aptamers or their analogs obtained by substitutions, deletions, and insertions of Watson-Crick base pairs or by mutations at non-conserved positions, bind to a primary aptamer, when the latter is in the complex with a mono- or oligo - saccharide. In certain non-limiting instances, secondary aptamers or their analogs bind to a primary aptamer, when the latter is in the complex with glucose, wherein the analog comprises a nucleotide sequence at least 80% identical to the nucleotide sequence of the secondary aptamer; a nucleotide sequence at least 95% identical to the nucleotide sequence of the secondary aptamer; and a nucleotide sequence at least 99% identical to the nucleotide sequence of the secondary aptamer.

### 5.4.3. SECONDARY APTAMERS TO PHENYLALANINE-BINDING PRIMARY APTAMERS

In certain non-limiting embodiments, secondary aptamers can be isolated according to the aforementioned method for phenylalanine. In certain non-limiting embodiments, secondary aptamers or their analogs obtained by substitutions, deletions, and insertions of Watson-Crick base pairs or by mutations at non-conserved positions, bind to a primary aptamer, when the latter is in the complex with Phenylalanine. In certain non-limiting embodiments, secondary aptamers or their analogs obtained by substitutions, deletions, and insertions of Watson-Crick base pairs or by mutations at non-conserved positions, bind to a primary aptamer, when the latter is in the complex with an amino acid or a peptide. In certain non-limiting embodiments, secondary aptamers or their analogs bind to a primary aptamer, when the latter is in the complex with Phenylalanine, wherein the analog comprises a nucleotide sequence at least 80% identical to the nucleotide sequence of the secondary aptamer; a nucleotide sequence at least 95% identical to the nucleotide sequence of the secondary aptamer; and a nucleotide sequence at least 99% identical to the nucleotide sequence of the secondary aptamer.

### 5.4.4. SECONDARY APTAMERS TO HYDROCORTISONE-BINDING PRIMARY APTAMERS

In certain non-limiting instances, secondary aptamers can be isolated according to the aforementioned method for hydrocortisone. In certain non-limiting instances, secondary aptamers or their analogs obtained by substitutions, deletions, and insertions of Watson-Crick base pairs or by mutations at non-conserved positions, bind to a primary aptamer, when the latter is in the complex with hydrocortisone. In certain non-limiting instances, secondary aptamers or their analogs bind to a primary aptamer, when the latter is in the complex with hydrocortisone, wherein the analog comprises a nucleotide sequence at least 80% identical to the nucleotide sequence of the secondary aptamer; a nucleotide sequence at least 95% identical to the nucleotide sequence of the secondary aptamer; and a nucleotide sequence at least 99% identical to the nucleotide sequence of the secondary aptamer.

### 5.4.5. SECONDARY APTAMERS TO DEHYDROISOANDROSTERONE-BINDING PRIMARY APTAMERS

In certain non-limiting instances, secondary aptamers can be isolated according to the aforementioned method for dehydroisoandrosterone. In certain non-limiting instances, secondary aptamers or their analogs obtained by substitutions, deletions, and insertions of Watson-Crick base pairs or by mutations at non-conserved positions, bind to a primary aptamer, when the latter is in the complex with dehydroisoandrosterone. In certain non-limiting instances, secondary aptamers or their analogs bind to a primary aptamer, when the latter is in the complex with dehydroisoandrosterone, wherein the analog comprises a nucleotide sequence at least 80% identical to the nucleotide sequence of the secondary aptamer; a nucleotide sequence at least 95% identical to the nucleotide sequence of the secondary aptamer; and a nucleotide sequence at least 99% identical to the nucleotide sequence of the secondary aptamer.

### 5.4.6. SECONDARY APTAMERS TO DEOXYCORTICOSTERONE-BINDING PRIMARY APTAMERS

In certain non-limiting instances, secondary aptamers can be isolated using the aforementioned method for deoxycorticosterone. In certain non-limiting instances, secondary aptamers comprising the nucleotide sequence of any one of SEQ ID NO: 34-35 (FIGURE 48) and their analogs obtained by substitutions, deletions, and insertions of Watson-Crick base pairs or by mutations at non-conserved positions, bind to a primary aptamer comprising the nucleotide sequence of SEQ ID NO: 12 (FIGURE 21), when the latter is in the complex with deoxycorticosterone. In certain non-limiting instances, secondary aptamers or their analogs bind to a primary aptamer, when the latter is in the complex with deoxycorticosterone, wherein the analog comprises a nucleotide sequence at least 80% identical to the nucleotide sequence of the secondary aptamer; a nucleotide sequence at least 95% identical to the nucleotide sequence of the secondary aptamer; and a nucleotide sequence at least 99% identical to the nucleotide sequence of the secondary aptamer.

### 5.4.7. SECONDARY APTAMERS TO TESTOSTERONE-BINDING PRIMARY APTAMERS

In certain non-limiting instances, secondary aptamers can be isolated according to the aforementioned method for testosterone. In certain non-limiting instances, secondary aptamers or their analogs obtained by substitutions, deletions, and insertions of Watson-Crick base pairs or by mutations at non-conserved positions, bind to a primary aptamer, when the latter is in the complex with testosterone. In certain non-limiting instances, secondary aptamers or their analogs bind to a primary aptamer, when the latter is in the complex with testosterone, wherein the analog comprises a nucleotide sequence at least 80% identical to the nucleotide sequence of the secondary aptamer; a nucleotide sequence at least 95% identical to the nucleotide sequence of the secondary aptamer; and a nucleotide sequence at least 99% identical to the nucleotide sequence of the secondary aptamer.

### 5.4.8. SECONDARY APTAMERS TO SPHINGOSINE-1-PHOSPHATE-BINDING PRIMARY APTAMERS

In certain non-limiting instances, secondary aptamers can be isolated according to the aforementioned method for sphingosine-1-phosphate. In certain non-limiting instances, secondary aptamers or their analogs obtained by substitutions, deletions, and insertions of Watson-Crick base pairs or by mutations at non-conserved positions, bind to a primary aptamer, when the latter is in the complex with sphingosine-1-phosphate. In certain non-limiting instances, secondary aptamers or their analogs bind to a primary aptamer, when the latter is in the complex with sphingosine-1-phosphate, wherein the analog comprises a nucleotide sequence at least 80% identical to the nucleotide sequence of the secondary aptamer; a nucleotide sequence at least 95% identical to the nucleotide sequence of the secondary aptamer; and a nucleotide sequence at least 99% identical to the nucleotide sequence of the secondary aptamer.

### 5.4.9. SECONDARY APTAMERS TO DOPAMINE-BINDING PRIMARY APTAMERS

In certain non-limiting instances, secondary aptamers can be isolated according to the aforementioned method for dopamine. In certain non-limiting instances, secondary aptamers or their analogs obtained by substitutions, deletions, and insertions of Watson-Crick base pairs or by mutations at non-conserved positions, bind to a primary aptamer, when the latter is in the complex with dopamine. In certain non-limiting instances, secondary aptamers or their analogs bind to a primary aptamer, when the latter is in the complex with dopamine, wherein the analog comprises a nucleotide sequence at least 80% identical to the nucleotide sequence of the secondary aptamer; a nucleotide sequence at least 95% identical to the nucleotide sequence of the secondary aptamer; and a nucleotide sequence at least 99% identical to the nucleotide sequence of the secondary aptamer.

### 5.4.10. SECONDARY APTAMERS TO SEROTONIN-BINDING PRIMARY APTAMERS

In certain non-limiting instances, secondary aptamers were isolated using the aforementioned method for serotonin. In certain non-limiting instances, secondary aptamers comprising any one of the nucleotide sequences of SEQ ID NO: 36, SEQ ID NO: 59, and SEQ ID NO: 60 (FIGURE 48) and any one of their analogs obtained by substitutions, deletions, and insertions of Watson-Crick base pairs or by mutations at non-conserved positions, bind to a primary aptamer comprising any one of the nucleotide sequences of SEQ ID NO: 25 and SEQ ID NO: 58 (FIGURE 34 and FIGURE 48C and D), when the latter is in the complex with serotonin. In certain non-limiting instances, secondary aptamers or their analogs bind to a primary aptamer, when the latter is in the complex with serotonin, wherein the analog comprises a nucleotide sequence at least 80% identical to the nucleotide sequence of the secondary aptamer; a nucleotide sequence at least 95% identical to the nucleotide sequence of the secondary aptamer; and a nucleotide sequence at least 99% identical to the nucleotide sequence of the secondary aptamer.

### 5.4.11. SECONDARY APTAMERS TO TYROSINE-BINDING PRIMARY APTAMERS

In certain non-limiting instances, secondary aptamers can be isolated according to the aforementioned method for tyrosine. In certain non-limiting instances, secondary aptamers or their analogs obtained by substitutions, deletions, and insertions of Watson-Crick base pairs or by mutations at non-conserved positions, bind to a primary aptamer, when the latter is in the complex with tyrosine. In certain non-limiting instances, secondary aptamers or their analogs bind to a primary aptamer, when the latter is in the complex with tyrosine, wherein the analog comprises a nucleotide sequence at least 80% identical to the nucleotide sequence of the secondary aptamer; a nucleotide sequence at least 95% identical to the nucleotide sequence of the secondary aptamer; and a nucleotide sequence at least 99% identical to the nucleotide sequence of the secondary aptamer.

### 5.4.12. SECONDARY APTAMERS TO L-TYROSINE-BINDING PRIMARY APTAMERS

In certain non-limiting instances, secondary aptamers can be isolated according to the aforementioned method for L-tyrosine. In certain non-limiting instances, secondary aptamers or their analogs obtained by substitutions, deletions, and insertions of Watson-Crick base pairs or by mutations at non-conserved positions, bind to a primary aptamer, when the latter is in the complex with L-tyrosine. In certain non-limiting instances, secondary aptamers or their analogs bind to a primary aptamer, when the latter is in the complex with L-tyrosine, wherein the analog comprises a nucleotide sequence at least 80% identical to the nucleotide sequence of the secondary aptamer; a nucleotide sequence at least 95% identical to the nucleotide sequence of the secondary aptamer; and a nucleotide sequence at least 99% identical to the nucleotide sequence of the secondary aptamer.

In certain non-limiting instances, this protocol can be suitably modified and optimized to isolate secondary aptamers to all non-limiting examples of primary aptamers and their analogs (including spiegelmers) as depicted in FIGURE 49. It can also be applied to primary aptamers for any molecule that has molecular mass below 100000 Daltons or below 2000 Daltons or below 1000 Daltons or below 500 Daltons or below 200 Daltons, and it cannot have two epitopes that do not compete against each other.

### 5.4.13 ADDITIONAL SANDWICH ASSAY EMBODIMENTS

In certain non-limiting embodiments, the present application provides sandwich assays, with fluorescent detections and ELISA-like formats.

In the pseudosandwich assay described above, there were no true sandwich interactions (thus, 'pseudo-sandwich') and ligand*aptamer interactions were "translated" into a presence of a double helix allowing a format similar to a non-competitive sandwich ELISA. However, in certain embodiments a sandwich assay comprises two aptamers binding to one molecule at the same time without competing with each other.

In certain non-limiting embodiments of the present application aptamers against aptamer*ligand complexes or secondary aptamer bind primary aptamers when in complexes with ligands have been generated (FIGURE 8 and FIGURE 48). In certain embodiments a sandwich assay leads to high sensitivity. In certain non-limiting embodiments, aptamers against aptamer*ligand complexes can act as sophisticated switches with finely tuned complementarity; upon sensing ligand by one of them, they come together in a ternary (tertiary) complex (FIGURE 49).

### Sandwich assay in solution

In certain non-limiting embodiments, sandwiches are formed in solution for deoxycorticosterone and serotonin and fluorescence read-outs are measured (FIGURE 48A-C). In this assay, a primary aptamer is in solution and a secondary aptamer, which is also in solution, is turned in structure-switching fluorescent form, and is interacting with the primary aptamer when the primary aptamer is in complex with the targeted steroid through the release of a quencher-labeled competitor oligonucleotide.

### Sandwich assay on plate

In certain non-limiting instances, a sandwich assay on plate is performed for serotonin (FIGURE 48D-E). In this assay, a primary aptamer is deposited on plate, while a secondary aptamer binds to the primary aptamer when it is in its complex with ligand (in this case serotonin).

In certain non-limiting instances, the foregoing assays can be used to detect and/or quantitate any analyte of interest, and can be particularly advantageous over existing methods in detecting and/or quantitating analytes that have a molecular weight less than about 1000 Daltons or less than about 500 Daltons or less than about 200 Daltons, including but not limited to steroid compounds, such as but not limited to cortisol, aldosterone, dehydroepiandrosterone, progesterone, testosterone; glucose; amino acids such as but not limited to phenylalanine, leucine, isoleucine, valine, citrulline, tyrosine, alanine; pharmaceutical compounds, vitamins, toxins, neurotransmitters (e.g., catecholamines, serotonin), peptides (vasopressin, oxytocin, angiotensin, natriuretic peptides, glucagon, insulin and others), antibiotics and antifungal compounds (e.g., aminoglucosides), macrocyclic immunosuppresants, or lipids or lipid complexes.

In certain non-limiting embodiments, these assays can also be applied to larger molecules, with molecular weight above 1000 Daltons.

### 6. EXAMPLE 1 - ISOLATION OF PRIMARY APTAMERS

The present example provides methods for isolating primary aptamers (A^{P}) and examples of their structures.

The isolation of primary aptamers was performed using the SELEX process. Primary aptamers were isolated by solution-phase selection, as this method has inherent advantages for small molecules, such as higher affinity and ease of screening of aptamers (Table 1). Non-limiting examples are provided in FIGURE 10-46.

The method was based on attaching a biotinylated strand complementary (**C_{B}**) to one of the PCR primers to agarose-streptavidin (FIGURE 7) and attaching sequences from a library (e.g., but not limited to, N₈-N₁₀₀) through complementary interactions to **C_{B}** of a primer. Two primers on library, 5'- and 3'-, were also partially complementary; all members of the library which interacted with a target in a way that favors stem formation between complementary region of these primers were released from the agarose by displacing the complementary nucleotide (C_{B}), and were used in PCR amplification. This created an enriched pool of potential aptamers.

Fluorescent sensors were directly obtained from this selection, by substituting biotin with dabcyl and attaching fluorescein to the aptamer (FIGURE 6, C_{D}), confirming that aptamers bind, determining their K_{d} (this was a competitive assay, so half-response is shifted away from the K_{d}⁸⁰, and C_{D} was present in an excess), and establishing selectivity.

Primary aptamers were isolated according to the aforementioned method for:
1. D-Glucose (SEQ ID NO:1);
2. L-Phenylalanine (SEQ ID NOS: 2-4);
3. Hydrocortisone (SEQ ID NOS: 5-7);
4. Dehydroisoandrosterone and Deoxycorticosterone 21-glucoside (DOG) (SEQ ID NOS: 8-12);
5. Testosterone (SEQ ID NOS: 13-17);
6. Sphingosine-1-phosphate (SEQ ID NO: 18);
7. Dopamine (SEQ ID NOS: 19-23);
8. Serotonin (SEQ ID NOS: 24-30);
9. Melatonine (SEQ ID NOS: 31-32); and
10. L-Tyrosine (SEQ ID NO: 33).

The disclosed primary aptamers bound to their target analyte and in their structure-switching formats they responded to its presence by an increase in fluorescence (FIGURE 10-46 and FIGURE 47A-D).

**Table 1. Primary Aptamer Sequences.**

| **Target** | **Primary Aptamer Sequence** | **SEQ ID NO:** |
|---|---|---|
| Glucose | CTCTCGGGACGACCGTGTGTGTTGCTCTGTAACAGTGTCCATTGTCG TCCC | 1 |
| Phenylalanine | CTCTCGGGACGACCGCGTTTCCCAAGAAAGCAAGTATTGGTTGGTCG TCCC | 2 |
| | CTCTCGGGACGACCGGTGGGGGTTCTTTTCAGGGGAGGTACGGTCG TCCC | 3 |
| | CTCTCGGGACGACGAGGCTGGATGCATTCGCCGGATGTTCGATGTCG TCCC | 4 |
| Hydrocortisone | CTCTCGGGACGACGCCCGCATGTTCCATGGATAGTCTTGACTAGTCG TCCC | 5 |
| | CTCTCGGGACGACTAGCGTATGCGCCAGAAGTATACGAGGATAGTC GTCCC | 6 |
| | CTCTCGGGACGACGCCAGAAGTTTACGAGGATATGGTAACATAGTC GTCCC | 7 |
| Dehydroisoandroster one and Deoxycorticosterone | CTCTCGGGACGACGGGGATTTTCCCAATTGGTTCTTTCAATTTAGTCG TCCC | 8 |
| Dehydroisoandroster one | CTCTCGGGACGACGGGGGTGGCATAGGGTAGGCTAGGGTCACTGTC GTCCC | 9 |
| | CTCTCGGGACGACGTGGCTAGGTAGGTTGCATGCGGCATAGGGGTC GTCCC | 10 |
| | CTCTCGGGACGACGTGACGGTGTGTAGTTGGGTTGTGGCAGGAGTCG TCCC | 11 |
| Deoxycorticosterone | CTCTCGGGACGACCCGGATTTTCCGAGTGGAACTAGCTGTGGCGGTC GTCCC | 12 |
| Testosterone | CTCTCGGGACGACGGGATGTCCGGGGTACGGTGGTTGCAGTTCGTCG TCCC | 13 |
| | CTCTCGGGACGACCAGGTGCCATTAGCGTCAGTGTGCTACGATGTCG TCCC | 14 |
| | CTCTCGGGACGACCCGTTCGATCTAACCCTTGTTAGCCGTGATGTCG TCCC | 15 |
| | CTCTCGGGACGACCCCTTCGATCTTCAACCAAAGCCGTTGGATGTCG TCCC | 16 |
| | CTCTCGGGACGACGGGTGGTCATTGAGTGGTCTTAGGCAGGTAGTCG TCCC | 17 |
| Sphingosine-1-phosphate | CTCTCGGGACGACGTGGTGTGGGAGAAAGAATTTTCATTGGGGTAG GGGGTCGTCCC | 18 |
| Dopamine | CTCTCGGGACGACCACTTCAGACGCTCAACGTTTGGGGAGGCACGG CAGGTCGTCCC | 19 |
| | CTCTCGGGACGACGGGGAGGAGTTAGCATGACGGCAACTTTAGTAC TTCGTCGTCCC | 20 |
| | CTCTCGGGACGACGCCAGTTTGAAGGTTCGTTCGCAGGTGTGGAGTG ACGTCGTCCC | 21 |
| | CTCTCGGGACGACTGCAGCCTGGGGTTGTGGGGGGTAGGGGAGGTC TGAGTCGTCCC | 22 |
| | CTCTCGGGACGACCACACAGAGGCACAACTCGCAGGAGCAAAGCGG CAGGTCGTCCC | 23 |
| Serotonin | CTCTCGGGACGACAGGGGCATATATAGTCTAGGGTTTGGTGTGGGTA GTGTCGTCCC | 24 |
| | CTCTCGGGACGACTGGTAGGCAGATAGGGGAAGCTGATTCGATGCG TGGGTCGTCCC | 25 |
| | CTCTCGGGACGACTGGTAGGCAGCAGGGGAAGTAGGCGTGTCCTCG TGGGTCGTCCC | 26 |
| | CTCTCGGGACGACCAGTAGGGGATCCACAGTGAGGGGTTTGTATGG GTGGTCGTCCC | 27 |
| | CTCTCGGGACGACTGGTAGGCAACAGGGGAAGGGAGTTCTGCGTAC GTGGGTCGTCCC | 28 |
| | CTCTCGGGACGACGGAGGTGGTGTCTTGGACAGTGGTATTCGCAGTT GCGTCGTCCC | 29 |
| | CTCTCGGGACGACAGAGACGGGGTGCTTACTTGGTTCAGGGGAGTC GACGTCGTCCC | 30 |
| Melatonin | CTCTCGGGACGACAGCCAAGGTCGTAAGGTACGGTCAGTGTACTCG GTTGTCGTCCC | 31 |
| | CTCTCGGGACGACGTCTTGGGGGTGGTGGGTTTGGCTGGTACTTAGG GCGTCGTCCC | 32 |
| Tyrosine | CTCTCGGGACGACGGCCCGATCTCAGAGTAGTCGTCCC | 33 |

The sequences of the primary aptamers that were isolated for serotonin in their presumed secondary structure, as shown with the complementary oligonucleotide (C) that was used in selection (C_{B}) or fluoresence sensing (C_{D}), and the presumed core pocket (as shown in FIGURE 10-46) are included in Table 2.

**Table 2. Sequences of Primary Aptamers for serotonin in their presumed secondary structure, the complementary oligonucleotides used in their selection, and their presumed core pockets.**

| **For Primary Aptamers isolated for Serotonin comprising the nucleotide sequence of SEQ ID NO: 24** | |
|---|---|
| Primary Aptamer | CTCTCGGGACGACAGGGGCATATATAGTCTAGGGTTTGGTGTGGGTAGTGTCGT CCC (SEQ ID NO: 37) |
| Complementary oligonucleotide | TGTCGTCCCGAGAG (SEQ ID NO: 38) |
| Core Pocket | NAGGGGCATATATAGTCTAGGGTTTGGTGTGGGTAGTN, where N Can be any one of A, T, G, or (SEQ ID NO: 39) |

| **For Primary Aptamers isolated for Serotonin comprising the nucleotide sequence of SEQ ID NO: 25** | |
|---|---|
| Primary Aptamer | CTCTCGGGACGACTGGTAGGCAGATAGGGGAAGCTGATTCGATGCGTGGGTCGT CCC (SEQ ID NO: 40) |
| Complementary oligonucleotide | GTCGTCCCGAGAG (SEQ ID NO: 41) |
| Core Pocket | NTGGTAGNNNGATAGGGNNNNGCTGANNNGANNNGTGGN, where N can be any one of A, T, G, or C (SEQ ID NO: 42) |

| For Primary Aptamers isolated for Serotonin comprising the nucleotide sequence of SEQ ID NO: 26 | |
|---|---|
| Primary Aptamer | CTCTCGGGACGACTGGTAGGCAGCAGGGGAAGTAGGCGTGTCCTCGTGGGTCGT CCC (SEQ ID NO: 43) |
| Complementary oligonucleotide | GTCGTCCCGAGAG (SEQ ID NO: 44) |
| Core Pocket | NTGGTAGGCAGCAGGGGAAGTAGGCGTGTCCTCGTGGN, where N can be any one of A, T, G, or C (SEQ ID NO: 45), |

| **For Primary Aptamers isolated for Serotonin comprising the nucleotide sequence of SEQ ID NO: 27** | |
|---|---|
| Primary Aptamer | CTCTCGGGACGACCAGTAGGGGATCCACAGTGAGGGGTTTGTATGGGTGGTCGT CCC (SEQ ID NO: 46) |
| Complementary oligonucleotide | GGTCGTCCCGAGAG (SEQ ID NO: 47) |
| Core Pocket | NAGTAGGGGANNNCAGTGAGGGGTTTGTANNNNTN, where N can be any one of A, T, G, or C (SEQ ID NO: 48) |

| **For Primary Aptamers isolated for Serotonin comprising the nucleotide sequence of SEQ ID NO: 28** | |
|---|---|
| Primary Aptamer | CTCTCGGACGACTGGTAGGCAACAGGGAAGGAGTTCTGCGTACGTGGGTC GTCCC (SEQ ID NO: 49) |
| Complementary oligonucleotide | GTCGTCCCGAGAG (SEQ ID NO: 50) |
| Core Pocket | NTGGNAGGNAACAGGGGNGGGAGNNCTNCGTNCGTGGN, where N can be any one of A, T, G, or C (SEQ ID NO: 51) |

| **For Primary Aptamers isolated for Serotonin comprising the nucleotide sequence of SEQ ID NO: 29** | |
|---|---|
| Primary Aptamer | CTCTCGGGACGACGGAGGTGGTGTCTTGGACAGTGGTATTCGCAGTTGCGTCGT CCC (SEQ ID NO: 52) |
| Complementary oligonucleotide | CGTCGTCCCGAGAG (SEQ ID NO: 53) |
| Core Pocket | NGGAGGTGGNNNNNNNNNNNGTGGTATTCGCAGTTGCN, where N can be any one of A, T, G, or C (SEQ ID NO: 54) |

| **For Primary Aptamers isolated for Serotonin comprising the nucleotide sequence of SEQ ID NO: 30** | |
|---|---|
| Primary Aptamer | CTCTCGGGACGACAGAGACGGGGTGCTTACTTGGTTCAGGGGAGTCGACGTCGT CCC (SEQ ID NO 55) |
| Complementary oligonucleotide | TGTCGTCCCGAGAG (SEQ ID NO: 56) |
| Core Pocket | NNAGANNNGGGGTGCTTACTTGGTTCAGGGGANNNGACNN, where N can be any one of A, T, G, or C (SEQ ID NO: 57) |

### 7. EXAMPLE 2 - PSEUDOSANDWICH ASSAYS

In the present example pseudosandwich assays, with fluorescent detection in solution and on plates (ELISA-like format) are provided.

Solution-evolved aptameric sensors were adapted for use in a solid surface-format application, for example, ELISA-type assays. The solution sensor - composed of an analyte and a primary aptamer partially hybridized to a competitor oligonucleotide - was modified using attachment chemistry to enable its anchoring to a solid surface. The surface attachment chemistry used was biotin-streptavidin interaction. In addition, the complementary strand to the competitor oligonucleotide (optimized to bind to the competitor oligonucleotide in the presence of analyte) was modified with a biotin-tag, which was used to capture a "detection"
molecule e.g. streptavidin-HRP conjugate. The HRP substrate, TMB, was used for visualization.

Optimization of response of the surface-bound sensor, relative to +/- analyte, was carried out by adjusting parameters including: amount of sensor on surface, attachment chemistry, concentration of complementary competitor oligonucleotide, buffer in each step, washing steps, HRP concentration, and HRP substrate.

### Experimental procedure for Pseudosandwich assay - in solution

The solution buffer had the composition of the SELEX buffer or any other buffer that the sensor was shown to work in. Each sample well contained a final concentration of 50 nM aptamer-C_{Dext} duplex (with a stoichiometric amount of C_{Dext} in relation to aptamer, or in two- or three-fold excess). Selected concentrations of analyte were added, and complement to C_{Dext} was added to a final concentration of 2 µM. Final mixtures were incubated at room temperature for 20 minutes and then the fluorescence signal was measured.

Pseudosandwich assays in solution, where a double helix formation was triggered by an analyte, were performed for:
1. Deoxycorticosterone 21-glucoside (DOG);
2. L-Tyrosine;
3. D-Glucose; and
4. L-Phenylalanine

The primary aptamers formed a complex with C_{Dext} and in the presence of C_{Dext}^{comp} produced an increase in fluorescence in the presence of their target analytes (FIGURE 47A-D).

If the C_{Dext} was deposited on plates, the double helix remained attached to the plate when the rest of solution was removed and it could be extensively washed.

### Experimental procedure for Pseudosandwich assay - in solid state

Deoxycorticosterone 21-glucoside (DOG) sensor ELISA on streptavidin-coated plates was performed as following: 151.5 pmoles (6.6 µL x 30 µM) of DOG sensor in PBS pH 7.4 buffer solution, was added per well of the streptavidin-coated ELISA plate (Thermo, binding capacity 5pmoles) containing 100µL of PBS buffer, to give a final sensor concentration of 1.44µM. The mixture was incubated at room temperature for 5 minutes. The solution was then removed from the well and the well washed thoroughly eight times with PBS buffer. Next, 100 µL of TRIS pH 7.4 buffer (composed of 20 mM TRIS, 140 mM NaCl, 5 mM KCl, and 2 mM MgCh) was added per well, followed by 2 µL of 2 mM DOG dissolved in DMSO, to give a final Deoxycorticosterone 21-glucoside (DOG)concentration of 50 µM. The single-stranded complement containing the biotin tag was then add (2 µL of 100 µM stock) to give a final concentration of 1.79 µM. The mixture was incubated at room temperature of 20 minutes. The solution was then removed and the wells washed thoroughly 8x with PBS buffer. 100 µL of PBS containing 1% BSA and 8000-fold diluted HRP-STV conjugate was then added per well, and incubated for 5 minutes at room temperature. The solution was removed and wells were washed thoroughly 8x with PBS buffer. 100 µL of TMB/H₂O₂ substrate was then added to each well and the reaction progression was monitored on a plate reader at 652nm.

Results for Deoxycorticosterone 21-glucoside (DOG) concentration dependence on a streptavidin-ELISA 96-well plate are shown in FIGURE 47E. Eight wells were coated with aptamer sensor and exposed to various concentrations of Deoxycorticosterone 21-glucoside (DOG) analyte, followed by tagging with HRP enzyme, and quantified by adding TMB substrate, which is oxidized to a blue product by H₂O₂/HRP. A time course of the oxidation of TMB proportional to the amount of HRP enzyme bound in each well, as well as a concentration dependence were measured (FIGURE 47E).

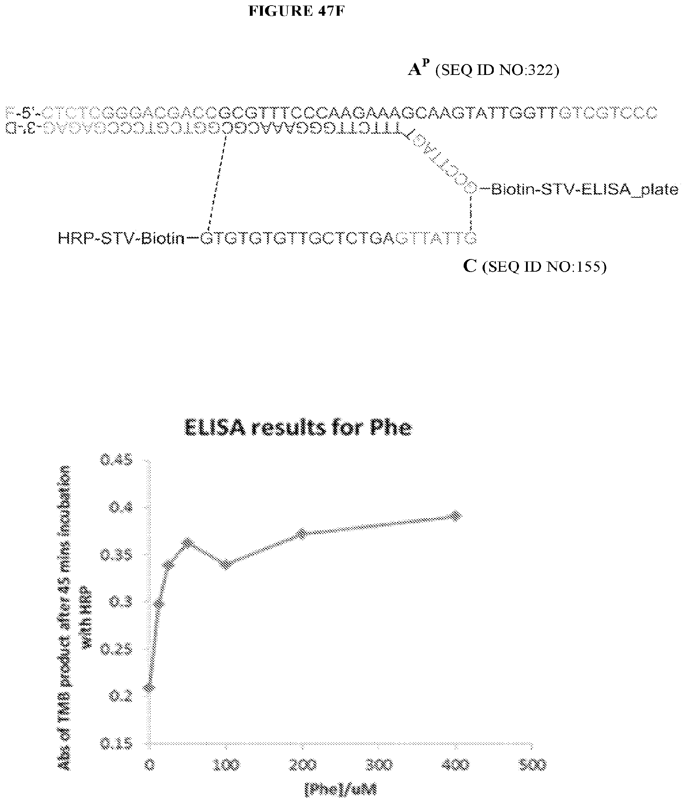

Phenylalanine (Phe) sensor ELISA on streptavidin-coated plates was performed as following: The aforementioned procedure was carried out for the Phe sensor, except the 20 minute incubation step was carried out in 20 mM HEPES pH 7.5, 1 M NaCl, 10 mM MgClz, 5 mM KCl buffer. Phe concentrations used were 0-200 µM from a 2 mM stock solution in water. Results for Phe concentration dependence on a streptavidin-ELISA 96-well plate are shown in FIGURE 47F.

### 8. EXAMPLE 3 - ISOLATION OF SECONDARY APTAMERS

The present example discloses methods for isolating secondary aptamers (A^{S}) and their structure.

The isolation of secondary aptamers was performed using the SELEX process. Two methods for selecting secondary aptamers (A^{s}) were performed, which can also be combined in some cases (FIGURE 9:

### (1) A solid-state selection.

A target aptamer (A^{p}) was attached to a matrix (e.g., beads), incubated with a library (e.g., but not limited to, prestructured or unstructured N₂₀₋₁₀₀) in the presence of target analyte L to isolate aptamer candidates with affinity for A^{p∗}L complex (FIGURE 9A). These oligonucleotides were PCR-amplified, single-stranded species regenerated, and then used in the next selection cycle. The process was repeated until convergence is reached, pools cloned and sequenced, leading to A^{s} candidates. The counter-selection was performed by elimination of binders to A^{P} in the absence of L, ensuring binding to the complex.

### (2) A solution-phase selection.

The process in solution-phase used pre-structured library to enable formation of stem between primers attached to matrix via complementary oligonucleotides, and A^{s} candidates were selected because their loop was closed through binding to A^{p} and they get released from the solid surface (FIGURE 9B). Counter-selection in this case was against A^{p} without ligand, and ligand itself.

Secondary aptamers were isolated using the aforementioned methods for:
1. Deoxycorticosterone (SEQ ID NOS: 34-35);
2. Serotonin (SEQ ID NOS: 36, 59, and 60);

The secondary aptamers bound to the primary aptamers, when the latter is in the complex with their target analytes (FIGURE 48).

The nucleotide sequences of the secondary aptamers are shown in Table 3.

**Table 3. Secondary Aptamer Sequences.**

| **Target** | **Secondary Aptamer Sequence** | **SEQ ID NO:** |
|---|---|---|
| Deoxycorticosterone | GTCATGTGGCGCCTCGACCCCAGCCTTGGTAGCTGTTGGCCACACAA GAGCACATGAC | 290 |
| | GTCATGTGACGAGACAAGGAGAACGGAAGGCGACCGGATAAATCG GATATCACATGAC | 291 |
| Serotonin | GTCATGTGGATTGTGACTTGCCCACAACGATTTTGCCAAACATGCAT AGCCACATGAC | 36 |
| | CATGTGGATTGTGACTTGCCCACAACGATTTTGCCAAACATGCATAG CCACATG | 60 |

Secondary aptamers for serotonin were isolated based on nucleotide sequences of primary aptamer for serotonin comprising:
CGACTGGTAGGCAGATAGGGGAAGCTGATTCGATGCGTGGGTCG (SEQ ID NO: 58), which was derived from the nucleotide sequence SEQ ID NO: 25 (FIGURE 48A-C). Secondary aptamers were isolated for serotonin comprising the following nucleotide sequence:
TCTGTGTCATGTGGATTGTGACTTGCCCACAACGATTTTGCCAAACATGCATA GCCACATGAC (SEQ ID NO: 59), in their presumed secondary structure (FIGURE 48A-C).

Isolated primary and secondary aptamers were used to perform sandwich assays as described in Example 4. Complementary oligonucleotides comprising the following nucleotide sequence: CACATGACACAGA (SEQ ID NO: 61) were used in fluorescence sensing.

### 9. EXAMPLE 4 - SANDWICH ASSAYS

In the present example, examples of sandwich assays, with fluorescent detections and ELISA-like formats are provided.

In the pseudosandwich assay described above, there were no true sandwich interactions (thus, 'pseudo-sandwich') and ligand*aptamer interactions were "translated" into a presence of a double helix allowing a format similar to non-competitive sandwich ELISAs. However, the true sandwich requires two aptamers (similar to two antibodies) binding to one molecule at the same time without competing with each other. This can be difficult for small molecules, simply because they lack two epitopes.

An alternate approach is depicted in FIGURE 8 and FIGURE 48, wherein aptamers against aptamer*ligand complexes or secondary aptamer binding primary aptamers when in complexes with ligands have been generated. The advantage of this method was that it is a true sandwich which could lead to better sensitivity. Aptamers against aptamer*ligand complexes acted as sophisticated switches with finely tuned complementarity; upon sensing ligand by one of them, they came together in a ternary (tertiary) complex.

### Experimental procedure for sandwich assay - in solution

The solution buffer had the composition of the SELEX buffer or any other buffer that the sensor was shown to work in. The buffer used for the measurement of serotonin was PBS buffer including 2 mM MgCh. For the measurement of deoxycorticosterone and testosterone, the buffer consisted of 20 mM HEPES, 1 M NaCl, 10 mM MgClz, and 5 mM KCl (pH 7.5).

Separate solutions were prepared in parallel: (1) Four times concentrated primary aptamer (A^{P}) was prepared in buffer and incubated for > 5 min in a water bath heated to boiling until use for unfolding secondary structure, then mixed with four times concentrated analyte (L) and incubated > 30 min. e.g. 20 µL of 4X concentrated A^{P} (e.g. 10 µM) and 20 µL of 4X concentrated serotonin solution (e.g. 800 µM). The final concentration of serotonin was 400 µM and of the complex A^{P}-serotonin was 5 µM. This is the 2X concentration of Ap-serotonin complex solution. (2) Two times concentrated secondary aptamer (A^{S}) solution was prepared. FAM fluorescent conjugated A^{S} (100 nM) and its dabcyl-quencher strand were mixed, the dabcyl-quencher strand was in three to five-fold excess for the sufficient quenching. This mixture was incubated for > 5 min in a water bath heated to boiling - used to promote unfolding of secondary structure and hybridization with the quencher strand. 40 µL of each of the separately prepared solutions (1) and (2) were mixed and incubated for 40 min. Then 75 µL of the mixed solution was taken for measurement. The fluorescent signal was measured. Each sample contained a final concentration of 2.5 - 5 µM of primary aptamer (A^{P}), 50 nM of secondary aptamer A^{S}, 150 nM - 250 nM of dabcyl quencher strand, and the indicated concentrations of analyte on the plots.

### Sandwich assay - in solid state

First, ligand (L) and primary aptamer (Ap) solution were prepared. 400 µL of 2X concentrated Bio-TEG conjugated primary aptamer (0.6 pmole/µL) was prepared in buffer, and incubated in the boiling water for > 5 min until use. Two times concentrated serotonin solution (e.g. 400 µM in buffer, 50 µL) was prepared, then mixed with 50 µL ligand and 50 µL of A^{P} solution and incubated > 30 min. The final concentration of serotonin was 200 µM and of the complex A^{P}-serotonin was 30 pmole per well. Second, a Bio-TEG modified secondary aptamer solution was prepared in bulk and 100 µL were applied (0.5 pmole/µl) per well. This solution was incubated for > 5 min in a water bath heated to boiling and was allowed to cool until use. After preparing these solutions, the ELISA assay was carried out. The ELISA plate wells were washed with PBS (+ 2 mM MgCh) buffer, 2 times, with soaking 5 min between washes. The A^{S} solution (100 µL) was added to the wells, then incubated for ~ 20 min at room temperature to allow biotin binding to the plate. The solution was then removed and the wells were washed thoroughly 8 times with the same buffer. Then, 100 µL of [L* A^{P} ] solution was added to the wells and incubated for > 30 min at room temperature. The solution was then removed and the wells were washed thoroughly 8 times with the same buffer. 100 µL of PBS containing 1% BSA and 10000-fold diluted HRP-STV conjugate was then added per well, and incubated for 5 minutes at room temperature. The solution was removed and wells washed thoroughly 10 times with the same buffer. Directly after this wash, 100 µL of TMB/H₂O₂ substrate was then added to each well and the reaction progression monitored on a plate reader at 370 or 652 nm.

Fluorescence read-outs when sandwiches are formed in solution (FIGURE 48A-C) were performed for the following analytes:
1. Deoxycorticosterone 21-glucoside (DOG); and
2. Serotonin.

In this assay, a primary aptamer is in solution and a secondary aptamer, which is also in solution, was turned in structure-switching fluorescent form, and was interacting with the primary aptamer when the primary aptamer was in complex with the targeted steroid through the release of a quencher-labeled competitor oligonucleotide (FIGURE 48A-C).

A sandwich assay on plate was performed for the following analyte:
1. Serotonin. In this assay, a primary aptamer was deposited on plate, while a secondary aptamer bound to the primary aptamer when it was in its complex with serotonin (FIGURE 48D-E).

### 10. EXAMPLE 5 - ANTI-APTAMER ASSAY

400 nM FAM-aptamer solution and four-times concentrated Iowa Black-complement strand solution are prepared separately in buffer (20 mM HEPES, 1 M NaCl, 10 mM MgCl2, 5 mM KCl, pH 7.5). These solutions were annealed separately by incubating in boiling water for 5 min, and cooled down at room temperature for ~ 30 min. Meanwhile, a two-times concentrate of each aptamer's target solutions were prepared; the final concentration is indicated in each plot. After the 30 min incubation period of the aptamer and complement strand solutions, 18.75 ul of the aptamer solution is added first to the 384-well plate, then 37.5 ul of the target solution is added, and then immediately added is 18.75 ul of complementary strand solution. Without incubation, the fluorescent measurement was started and read every 5 min at room temperature for > 8 hours by using Fluorescence plate reader (Victor II microplate reader, PerkinElmer). The final concentration of aptamer is 100 nM, the concentration of complementary strand is 100 nM for all except CS aptamer (1000 nM) in 75 µL reaction volume. The final concentration of target/ligand is indicated in each plot.

Each aptamer and its complementary strand is listed in below. The results are shown in FIGURE 3A-L.
(A) DOG aptamer/complementary (100 nM: 100 nM)
   - DOGS.2_sht/ DOGS.2_sht_anti: CGA CCC GGA TTT TCC GAG TGG AAC TAG CTG TGG CGG TCG /36-FAM/ (SEQ ID NO: 292); /5IABkFQ/CGA CCG CCA CAG CTA GTT CCA CTC GGA AAA TCC GGG TCG (SEQ ID NO: 293)
   - DOGS.2_Full/ DOGS.2_Full_anti: /56-FAM/CTC TCG GGA CGA CCC GGA TTT TCC GAG TGG AAC TAG CTG TGG CGG TCG TCC C (SEQ ID NO: 294); GGG ACG ACC GCC ACA GCT AGT TCC ACT CGG AAA ATC CGG GTC GTC CCG AGA G/3IABkFQ/ (SEQ ID NO: 295)
(B) ALD Aptamer/ complementary: (100 nM: 100 nM)
   - ALDS.1_sht/ ALDS.1_sht_anti: CGA CAG ATA GTT GTT CTT AGC GAT GTT CAG CGT TGT CG/36-FAM/ (SEQ ID NO: 296); /5IABkFQ/CGA CAA CGC TGA ACA TCG CTA AGA ACA ACT ATC TGT CG (SEQ ID NO: 297)
   - ALDS.1 Full/ ALDS.1_Full_anti: /56-FAM/CTC TCG GGA CGA CAG ATA GTT GTT CTT AGC GAT GTT CAG CGT TGT CGT CCC (SEQ ID NO: 298); GGG ACG ACA ACG CTG AAC ATC GCT AAG AAC AAC TAT CTG TCG TCC CGA GAG /3IABkFQ/ (SEQ ID NO: 299)
(C) CS Aptamer/ complementary: (100 nM: 1000 nM)
   CSS.1_sht/ CSS.1_sht_anti: GAC GAC GCC CGC ATG TTC CAT GGA TAG TCT TGA CTA GTC GTC /36-FAM/ (SEQ ID NO: 300); /5IABkFQ/GAC GAC TAG TCA AGA CTA TCC ATG GAA CAT GCG GGC GTC GTC (SEQ ID NO: 301)
(D) TES Aptamer/complementary: TES.1_sht/ TES.1_sht_anti: ACG GGA TGT CCG GGG TAC GGT GGT TGC AGT TCG T/36-FAM/ (SEQ ID NO: 302); /5IABkFQ/ACG AAC TGC AAC CAC CGT ACC CCG GAC ATC CCG T (SEQ ID NO: 303)
(E) Phe-CpRh Aptamer/complementary:
   Phe-CpRh_sht/ Phe-CpRh_sht_anti: /56-FAM/CGA CAC AGC GTG AGC CAA CTA ATT AGT GCG TAT TGT CG (SEQ ID NO: 304); CGA CAA TAC GCA CTA ATT AGT TGG CTC ACG CTG TGT CG/3IABkFQ/ (SEQ ID NO: 305)
(F) Phe Aptamer/complementary:
   Phe_sht/ Phe sht anti: / GAC CGG TGG GGG TTC TTT TTC AGG GGA GGT ACG GTC /36-FAM/ (SEQ ID NO: 306); /5IABkFQ/GAC CGT ACC TCC CCT GAA AAA GAA CCC CCA CCG GTC (SEQ ID NO: 307)

### 11. EXAMPLE 6 - SANDWICH ASSAY

FIGURE 49H shows a primary aptamer (A^{P}) that binds to serotonin (SRTNS.1) and a secondary aptamer (SRTN 2nd Apt1: GTG GTT AGT AAC TTG CAC GCC GCC CAA TTG CTA TTC ATG ACA AGC CAC (SEQ ID NO: 251) that binds to an aptamer (primary) with former binding to the latter preferentially when the latter is bound to its ligand. The colored letters with the dot lines indicate the hypothetical partial hybridization region between two aptamers. Those two aptamers are applied to ELISA-like assay through immobilizing the secondary aptamer on the streptavidin-coated plate and the primary in the solution. The spectrophotometric signal increases in proportion to the serotonin analyte. The signal is produced through the biotin conjugated on the SRTNS.1 binds the streptavidin-HRP conjugate, and catalyze the substrate TMB (FIGURE 49I). Increase in signal confirms that there is more binding when serotonin is present in increasing concentrations.

Figure 49 J shows a primary aptamer that binds to serotonin (gold ball) and a secondary aptamer (A^{S}) (SRTN 2^{nd} Apt.2: TCT GTG TCA TGT GGA TTG TGA CTT GCC CAC AAC GAT TTT GCC AAA CAT GCA TAG CCA CAT GAC (SEQ ID NO: 252) that binds to an aptamer (primary) with former binding to the latter preferentially when the latter is bound to its ligand. The colored letters with the dot lines indicate the partial hybridization region between two aptamers. SRTN 2^{nd} Apt.2 (F conjugated strand, 'F' indicates fluorescein) is shown in a format that was based on a competition between ligand and complementary oligonucleotide carrying a quencher (D, dabcyl). The quencher strand (grey C_{D}) first hybridizes with the 5' end of the aptamer, through the secondary conformational change upon target (SRTNS.1 + serotonin) binding. This quencher is released from the aptamer strand and results to the fluorescent signal increase. Red line higher than blue line indicates that complementary oligonucleotide with a quencher (C_{D}) is being displaced when (LOWER) either primary aptamer (A^{P}) is present (i.e., that binding occurs when primary aptamer to the ligand already present was added) or when (UPPER) serotonin to solution when primary aptamer is already present was added (FIGURE 49K).

Figure 49L shows an ELISA assay with the trimmed secondary aptamer (SRTN 2^{nd} Apt1) on the plate and primary aptamer (SRTNS.1 ) in solution with amplification through HRP-conjugated to streptavidin, streptavidin-HRP conjugate, and catalyze the substrate TMB. Red line shows that there is more binding when serotonin is present (FIGURE 49M). In certain non-limiting embodiments, reverse format in which primary and secondary aptamers are present are both possible.

Figure 49N shows a primary aptamer (A^{P}) that binds to the DOG (DOGS.2), a secondary aptamer (A^{S}) that binds to an aptamer (primary) with former binding to the latter preferentially when the latter is bound to its ligand (DOG 2nd Apt. 1: TCT GTG TCA TGT GGC GCC TCG ACC CCA GCC TTG GTA GCT GTT GGC CAC ACA AGA GCA CAT GAC (SEQ ID NO: 253). The colored letters with the dot lines indicate the partial hybridization region between two aptamers. DOG 2nd Apt.2 (F conjugated strand, 'F' indicates fluorescein) is shown in a format that was based on a competition between ligand and complementary oligonucleotide carrying a quencher (D, dabcyl). The quencher strand (grey) first hybridizes with the 5' end of the aptamer, through the secondary conformational change upon target (DOGS.2 + DOG) binding, this quencher is released from the aptamer strand. It results the fluorescent signal increase. (TOP) shows that secondary aptamer binds to primary aptamer when we add DOG (steroid concentration on X-axis) and (BOTTOM) shows that secondary aptamer binds to DOG only in the presence of primary aptamer (FIGURE 49O).

### 12. EXAMPLE 7 - PROTOCOLS FOR SECONDARY APTAMER ELISA (SANDWICH ASSAY)

Secondary aptamer on the plate and primary aptamer in solution method (FIGURE 49P). A biotinylated secondary aptamer is immobilized on a streptavidin-coated plate in appropriate buffer for about 30 minutes at room temperature, and then washed with buffer to remove excess unbound aptamers. A primary aptamer and its target are pre-incubated for 40 min at room temperature and then added to the immobilized secondary aptamer, incubating on the plate for 40 minutes. Streptavidin-HRP, 10000-fold diluted in 100 µL of PBS (w. 1% BSA), is added next, and incubated for 20 mins, then washed thoroughly with PBS. TMB substrate mix is then added and change in absorbance at 370 nm recorded for 45 mins.

Primary aptamer on the plate and secondary aptamer in solution" method (FIGURE 49Q). A biotinylated primary aptamer is immobilized on a streptavidin-coated plate in appropriate buffer for about 30 minutes at room temperature, and then washed with buffer to remove excess unbound aptamers. A target solution is then added to the immobilized primary aptamer and incubated for about 40 min. The secondary aptamer is added to the plate on top of the target solution and incubated about 40 min, then washed with buffer to remove the unbound secondary aptamers. Streptavidin-HRP, 10000-fold diluted in 100 µL of PBS (w. 1% BSA), is added next, and incubated for 20 mins, then washed thoroughly with PBS. TMB substrate mix is then added and change in absorbance at 370 nm recorded for 45 mins.

### 13. REFERENCES

1. Cox, K. L.; Devanarayan, V.; Kriauciunas, A.; Manetta, J.' Montrose, C.; Sittampalam, S. "Immunoassay Methods" in Assay Guidance Manual (Internet), NCBI Resources Bookshelf; last updated: December 24, 2014..
2. Gan, S.D.; Patel, K. R. "Enzyme Immunoassay and Enzyme-Linked Immunosorbent Assay" J Investig Derm 133, e12 (2013)
3. Lequin, R. "Enzyme Immunoassay (EIA)/Enzyme-Linked Immunosorbent Assay (ELISA)" Clinical Chemistry 51(12): 2415-2418 (2005).
4. Fan, M.; He, J. "Recent Progress in Noncompetitive Hapten Immunoassays: A review" Chapter 5 in Trends in Immunolabelled and Related Techniques, www.intechopen.com, accessed at: 06/01/2015.
5. Wilson, R. "Sensitivity and Specificity: Twin Goals of Proteomics Assays" Expert Rev Proteomics 10(2): 135-149 (2013).
6. Dong, J.-X.; Xu, C.; Wang, H.' Xiao, Z.-L.; Gee, S. J.; Li, Z.-F.; Wang, F.; Wu, W.-J.; Shen, Y.-D.; Yang, J.-Y.; Sun, Y.-M.; Hammock, B. D. "Enhanced Sensitive Immunoassay" Noncompetitive Phage AntiImmune Complex Assay for the Determination of Malachite Green and Leucomalachite Green" J Agric Food Chem 62(34): 8752-8758 (2014).
7. Ihara, M.; Suzuki, T.; Kobayashi, N.; Goto, J.; Ueda, H. "Open-Sandwich Enzyme Immunoassay for One-Step Noncompetitive Detection of Corticosteroid 11-Deoxycortisol" Anal Chem 81(20): 8298-8304 (2009).
8. Shen, J.; :Li, Y.; Gu, H.; Xia, F.; Zuo, X. "Recent Development of Sandwich Assay based on Nanobiotechnologies for Proteins, Nucleic Acids, Small Molecules, and Ions" Chem Rev 114(15): 7631-7677 (page 7662) (2014).
9. Ozer, A.; Pagano, J. M.; Lis, J. T. "New Technologies Provide Quantum Changes in the Scale, Speed, and Success of SELEX Methods and Aptamer Characterization" Molecular Therapy Nucleic Acids 3: e 183 (2014).
10. McKeague, M.; Derosa, M. C. "Challenges and opportunities for small molecule aptamer development." J Nucleic Acids, Article ID 748913 (2012).
11. Rohloff, J. C.; Gelinas, A. D.; Jarvis, T. C.; Ochsner, U. A.; Schenider, D. J.; Gold, L.; Janjic, N. "Nucleic Acid Ligands with Protein-like Side Chains: Modified Aptamers and Their Use as Diagnostic and Therapeutic Agents" Molecular Therapy Nucleic Acids 3: e:201 (2014).
12. Ellington, A. D. & Szostak, J. W. "In vitro selection of RNA molecules that bind specific ligands" Nature (London) 346: 818-822 (1990).
13. Tuerk, C. & Gold, L. "Systematic evolution of ligands by exponential enrichment: RNA ligands to bacteriophage T4 DNA polymerase" Science 249(4968):505-510 (1990).
14. Cho, E. J.; Lee, J.-W.; Ellington, A. D. "Applications of Aptamers as Sensors" Annu. Rev. Anal Chem 2:241-264 (2009).
15. Keefe, A. D.; Pai, S.; Ellington, A. "Aptamers as therapeutics" Nat Rev Drug Discovery, 9:537-550 (2010).
16. Yang, K.-A.; Pei, R.; Stefanovic, D.; Stojanovic, M. N. "Optimizing Cros-reactivity with Evolutionary Search for Sensors" J Am Chem Soc 134:1642-1647 (2012).
17. Stoltenburg, R.; Nikolaus, N.; Strehlitz, B. "Capture-SELEX: Selection of DNA Aptamers for Aminoglycoside Antibiotics" J Anal Meth Chem 15697 (2012).
18. Rajendran M,; Ellington, A. D. "Selection of fluorescent aptamer beacons that light up in the presence of zinc" Anal Bioanal Chem 390:1067-75 (2008).
19. Nutiu, R.; Li, Y. "In vitro selection of structure-switching signaling aptamers" Angew Chem Int Ed 44: 1061-1065 (2005).
20. Hu, J.; Easley, C. J.; "A simple and rapid approach for measurement of dissociation constants of DNA aptamers against proteins and small molecules via automated microchip electrophoresis" Analyst 136:3461-3468 (2011).
21. Nutiu, R., and Li, Y. "Structure-switching signaling aptamers" J Am Chem Soc 125:4771-4778 (2003).
22. Vater, A.; Klussmann, S. "Toward third-generation aptamers: Spiegelmers and their therapeutics prospects." Curr Opin Drug Discov Devel 6: 253-261 (2003).
23. Vater, A.; Klussmann, S. "Turning mirror-image oligonucleotides into drugs: the evolution of Spiegelmer therapeutics" Drug Discovery Today 20(1): 147-155 (2015).
24. Williams, K. P.; Liu, X. N.; Schumacher, T. N.; Lin, H. Y.; Ausiello, D. A.; Kim, P. S.; Bartel, D. P. "Bioactive and Nuclease-resistant L-DNA Ligand of Vasopressin." Proc Natl Acad Sci. USA, 94(21):11285-11290 (1997).
25. Purschke, W. G.; Eulberg, D.; Buchner, K.; Vonhoff, S.; Klussmann, S. "An L-RNA-based aquaretic agent that inhibits vasopressin in vivo." Proc Natl Acad Sci USA 103:5173-5178 (2003).
26. Yang, K.A; Barbu, M.; Halim, M.; Pallavi, P.; Kim, B.; Kokpashchikov, D.; Pecic, S.; Taylor, S.; Worgall, T. S.; Stojanovic, M. N. "Recognition and Sensing of Low-Epitope Targets via Ternary Complexes with Oligonucleotides and Synthetic Receptors" Nat Chem. 6:1003-1008 (2014).
27. Ozer, A.; Pagano, J. M.; Lis, J. T. "New Technologies Provide Quantum Changes in the Scale, Speed, and Success of SELEX Methods and Aptamer Characterization" Molecular Therapy Nucleic Acids 3: e 183 (2014).
28. McKeague, M.; Derosa, M. C. "Challenges and opportunities for small molecule aptamer development." J Nucleic Acids, Article ID 748913 (2012).
29. Rohloff, J. C.; Gelinas, A. D.; Jarvis, T. C.; Ochsner, U. A.; Schenider, D. J.; Gold, L.; Janjic, N. "Nucleic Acid Ligands with Protein-like Side Chains: Modified Aptamers and Their Use as Diagnostic and Therapeutic Agents" Molecular Therapy Nucleic Acids 3: e:201 (2014).
30. Ellington, A. D. & Szostak, J. W. "In vitro selection of RNA molecules that bind specific ligands" Nature (London) 346: 818-822 (1990).
31. Tuerk, C. & Gold, L. "Systematic evolution of ligands by exponential enrichment: RNA ligands to bacteriophage T4 DNA polymerase" Science 249(4968):505-510 (1990).
32. Robertson, D. L.; Joyce, G. F. "Selection in vitro of an RNA enzyme that specifically cleaves singlestranded DNA" Nature 344:467-468 (1990).
33. Cho, E. J.; Lee, J.-W.; Ellington, A. D. "Applications of Aptamers as Sensors" Annu. Rev. Anal Chem 2:241-264 (2009).
34. Keefe, A. D.; Pai, S.; Ellington, A. "Aptamers as therapeutics" Nat Rev Drug Discovery, 9:537-550 (2010).
35. Anslyn, E. "Supramolecular Analytical Chemistry" J Org Chem 72(3):687-699 (2007).
36. Gennady V.; Oshovsky, D.; Reinhoudt, D. V.;Verboom, W. "Supramolecular Chemistry in Water" Angew. Chem. Int. Ed. 46(14): 2366-2393 (2007).
37. Jonathan W. Steed (Editor-in-Chief), Philip A. Gale (Editor-in-Chief) "Supramolecular Chemistry: From Molecules to Nanomaterials" Wiley, 8 Volume Set (2012).
38. Steed, J. W.; Atwood, J. L.; Supramolecular Chemistry, 2nd Edition, Willey, (2009).
39. Pedersen, C. J. "Cyclic polyethers and their complexes with metal salts" J Am Chem Soc 89(26):7017-7036 (1967).
40. Kyba, E. P.; Siegel, M. G.; Sousa, L. R.; Sogah, G. D. Y.; Cram, D. J. "Chiral, hinged, and functionalized multiheteromacrocycles" J Am Chem Soc 95:2691-2692 (1973).
41. Dietrich, B.; Lehn, J.-M.; Sauvage, J.-P. "Diaza-polyoxa- macrocycles et macrobicycles" Tet Lett 2885-2888 (1969).
42. Costa J.B.; Andreiev A.I.; Dieckmann T. "Thermodynamics and kinetics of adaptive binding in the malachite green RNA aptamer" Biochemistry 24;52(38):6575-83 (2013).
43. Hermann, T.; Patel, D. J. "Adaptive Recognition by Nucleic Acids" Science 287:820-825 (2000).
44. Xia, T.; Yuan, J.; Fang, X.; Conformational dynamics of an ATP-binding DNA aptamer: a single molecule study. J Phys Chem B 117(48): 14994-50003 (2013).
45. Vater, A.; Klussmann, S. "Toward third-generation aptamers: Spiegelmers and their therapeutics prospects." Curr Opin Drug Discov Devel 6: 253-261 (2003).
46. Vater, A.; Klussmann, S. "Turning mirror-image oligonucleotides into drugs: the evolution of Spiegelmer therapeutics" Drug Discovery Today 20(1): 147-155 (2015).
47. Williams, K. P.; Liu, X. N.; Schumacher, T. N.; Lin, H. Y.; Ausiello, D. A.; Kim, P. S.; Bartel, D. P. "Bioactive and Nuclease-resistant L-DNA Ligand of Vasopressin." Proc Natl Acad Sci. USA, 94(21):11285-11290 (1997).
48. Purschke, W. G.; Eulberg, D.; Buchner, K.; Vonhoff, S.; Klussmann, S. "An L-RNA-based aquaretic agent that inhibits vasopressin in vivo." Proc Natl Acad Sci USA 103:5173-5178 (2003).
49. Kato, T.; Yano, K.; Ikebukuro, K.;and Karube, I. "Interaction of three-way DNA junctions with steroids" Nucleic Acids Res 28: 1963-1968 (2000).
50. Stojanovic, M. N.; Green, E. G.; Semova, S.; Nikic, D.B.; Landry, D.W. "Cross-reactive arrays based on three-way junctions" J Am Chem Soc 125:6085-6089 (2003).
51. Yang, K.-A.; Pei, R.; Stefanovic, D.; Stojanovic, M. N. "Optimizing Cros-reactivity with Evolutionary Search for Sensors" J Am Chem Soc 134:1642-1647 (2012).
52. Iskander, K. N.; Osuchowski, M. F.; Steams-Kurosava, D. J.; Stepien, D.; Valentine, C.; Remick, D. G. "Sepsis: multiple abnormalities, heterogenous responses, and evolving understanding" Phys. Rev. 93(3): 1247-1288 (2013).
53. Benedict, C. R.; Rose, J. A. "Arterial norepinephrine changes in patients with septic shock" Circ Shock 38(3): 165-172 (1992).
54. Wilson, M. F.; Brackett, D. J.; Tompkins, P, Benjamin, B.; Archer, L. T.; Hinshaw, L. B. "Elevated plasma vasopressin concentrations during endotoxin and E. coli shock" Adv Shock Res 6:15-26 (1981).
55. Landry D. W., Levin H. R., Gallant E. M., Ashton R. C., Seo S., D'Alessandro D., Oz M. C., Oliver J. A. "Vasopressin deficiency contributes to the vasodilation of septic shock" Circulation 95: 1122-1125 (1997).
56. Lin, I. Y.; Ma, H. P.; Lin, A. C.; Chong, C. F.; Lin, C. M.; Wang, T. "Low plasma vasopressin/norepinephrine ratio predicts septic shock" Am J Emrg Med 23(6): 718-724 (2005).
57. Sam, S.; Corbridge, T. C.; Mokhlesi, B.; Comellas, A. P.; Molitch, M. E. "Cortisol levels and mortality in severe sepsis" Clin Endorinol (Oxf) 60(1): 29-35 (2004).
58. Bendel, S.; Karlsson, S.; Pettila, V.; Loisa, P.; Varpula, M.; Ruokonen, E.; Finnsepsis Study Group "Freel cortisol in sepsis and septic shock" Anesth Analq 106(6): 1813-1819 (2008).
59. Annane, D.; Maximi, V.; Ibrahim, F.; Alvarez, J. C.; Abe, E.; Boudou, P. "Diagnosis of Adrenal Insufficiency in Severe Sepsis and Septic Shock" Am J Respir Crit Care Med 174:1319-1326, 2006.
60. Moares, R. B.; Freidman, G.; Viana, M. V.; Tonietto, T.; Saltz, H.; Czepielewski, M. A. "Aldosterone secretion in patients with septic shock: a prospective study." Arq Bras Endorcinol Metabol 57(8): 636-641 (2013).
61. Salgado, D. R.; Rocco, J. R.; Silva, E.; Vincent, J. L. "Modulation of the renin-angiotensin-aldosterone system in sepsis: a new therapeutic approach?" Expert Opin Ther Targets 14(1): 11-20 (2010).
62. Zhang, W.; Chen, X.; Huang, L.; Lu, N.; Zhou, L.; Wu, G.; Chen, Y. "Severe sepsis: Low expression of renin-angiotensin system is associated with poor prognosis" Exper Therap Med 7(5): 1342-1348 (2014).
63. Sato, Y.; Fujiwara, H.; Takatsu, Y. "Cardiac troponin and heart failure in the era of high-sensitivity assays" J. Cardiology 60(3): 160-167 (2012).
64. Sherwood, M. W.; Newby, K. "High-Sensitivity Troponin Assays: Evidence, Indications, and Reasonable Use" J Am Heath Assoc.3: e000403 (2014).
65. Cox, K. L.;D evanarayan, V.; Kriauciunas, A.; Manetta, J.' Montrose, C.; Sittampalam, S. "Immunoassay Methods" in Assay Guidance Manual (Internet), NCBI Resources Bookshelf; last updated: December 24, 2014.
66. Gan, S.D.; Patel, K. R. "Enzyme Immunoassay and Enzyme-Linked Immunosorbent Assay" J Investig Derm 133, e12 (2013).
67. Lequin, R. "Enzyme Immunoassay (EIA)/Enzyme-Linked Immunosorbent Assay (ELISA)" Clinical Chemistry 51(12): 2415-2418 (2005).
68. Fan, M.; He, J. "Recent Progress in Noncompetitive Hapten Immunoassays: A review" Chapter 5 in Trends in Immunolabelled and Related Techniques, www.intechopen.com, accessed at: 06/01/2015.
69. Wilson, R. "Sensitivity and Specificity: Twin Goals of Proteomics Assays" Expert Rev Proteomics 10(2): 135-149 (2013).
70. De Lemos, J. A.; Morrow, D. A.; "Brain Natriuretic Peptide Measurement in Acute Coronary Syndromes" Circulation 106: 2686-2870 (2002).
71. Osborne, S. E.; Ellington, E. D. "Nucleic acid selection and the challenge of combinatorial chemistry" Chemical Reviews 97 (2): 349-370 (1997).
72. Vant-Hull B., Gold L, Zichi DA. "Theoretical principles of in vitro selection using combinatorial nucleic acid libraries" Curr Protoc Nucleic Acid Chem. Chapter 9: Unit 9.1 (2000).
73. Dong, J.-X.; Xu, C.; Wang, H.' Xiao, Z.-L.; Gee, S. J.; Li, Z.-F.; Wang, F.; Wu, W.-J.; Shen, Y.-D.; Yang, J.-Y.; Sun, Y.-M.; Hammock, B. D. "Enhanced Sensitive Immunoassay" Noncompetitive Phage AntiImmune Complex Assay for the Determination of Malachite Green and Leucomalachite Green" J Agric Food Chem 62(34): 8752-8758 (2014).
74. Ihara, M.; Suzuki, T.; Kobayashi, N.; Goto, J.; Ueda, H. "Open-Sandwich Enzyme Immunoassay for One-Step Noncompetitive Detection of Corticosteroid 11-Deoxycortisol" Anal Chem 81(20): 8298-8304 (2009).
75. Shen, J.; :Li, Y.; Gu, H.; Xia, F.; Zuo, X. "Recent Development of Sandwich Assay based on Nanobiotechnologies for Proteins, Nucleic Acids, Small Molecules, and Ions" Chem Rev 114(15): 7631-7677 (page 7662) (2014).
76. Stojanovic, M. N., de Prada, P., Landry, D. W. "Self-assembling aptameric sensors" J Am Chem Soc, 122:11547-11548 (2000).
77. Chandra, M.; Silverman, S. K. "DNA and RNA Can Be Equally Efficient Catalysts for Carbon-Carbon Bond Formation", J Am Chem Soc 130:2936-2937 (2008).
78. Pinheiro, V. B.; Holliger, P. "Towards XNA nanotechnology: new materials from synthetic genetic polymers" Trends Biotechnology 32(6): 321-328 (2014).
79. Santoro, S. W.; Joyce, G. F.; Sakthivel, K.; Gramatikova, S.; Barbas, C. F. "RNA cleavage by a DNA enzyme with extended chemical functionality" J Am Chem Soc 122:2433-2439 (2000).
80. Perrin D.M.; Garestier, T.; Helene, C. "Bridging the gap between proteins and nucleic acids: a metal-independent RNAseA mimic with two protein-like functionalities" J Am Chem Soc 123:1556-1563 (2001).
81. Kimoto, M.; Yamashige, R.; Matsunaga, K-I.; Yokoyama, S.; Hirao, I. "Generation of high-affinity DNA aptamers using an expanded genetic alphabet" Nature Biotechnol 31,453-457 (2013).
82. Imaizumi, Y.; Kasahare, Y.; Fujita, H.; Kitadume, S.; Ozaki, H.; Endoh, T.; Kuwhara, M.; Sugimoto, N. "Efficacy of base-modification on target binding of small molecule DNA aptamers" J Am Chem Soc 135, 9412-9419 (2013).
83. Brukner, I.; El-Ramahi, R.; Gorska-Flipot, I.; Krajinovic, M.; Labuda, D. "An in vitro selection scheme for oligonucleotide probes to discriminate between closely related DNA sequences." Nucleic Acids Res. 35(9): e66, (2007), and literature cited therein.
84. Brukner, I.; Krajinovic, M.; Dascal, A.; Labuda, D. "A protocol for the in vitro selection of specific oligonucleotide probes for high-resolution DNA typing." Nat Protoc 2(11):2807-14 (2007).
85. Ayel, E.; Escudé, C. "In vitro selection of oligonucleotides that bind doublestranded DNA in the presence of triplex-stabilizing agents." Nucleic Acids Res 38(5):e31 (2010), and literature cited therein.
86. Darfeuille, F.; S. Reigadas; J. Hansen; H. Orum; C. Di Primo; J. Toulme (2006). "Aptamers targeted to an RNA hairpin show improved specificity compared to that of complementary oligonucleotides." Biochemistry 45 (39):12076-12082.
87. Durand, G.; Lisi, S.; Ravelet, C.; Dausse, E.; Peyrin, E.; Toulme, J.-J. "Riboswitches Based on Kissing Complexes for the Detection of Small Ligands" Angew Chem Int Ed 53(27): 6942-6945 (2014).
88. Luense, C. E.; Michlewski, G.; Hopp, C. S.; Rentmeister, A.; Caceres, J. F.; Famulok, M.; Mayer, G. "An aptamer targeting the apical-loop domain modulates pri-miRNA processing" Angew Chem Int Ed 49(27):4674-4677, (2010).
89. Butcher, S. E.; Pyle, A. M. "The Molecular Interactions that Stabilize RNA Tertiary Structure: RNA Motifs, Patterns, and Networks" Acc. Chem. Res. 44(12): 1302-1311 (2011).
90. Abraham, M.; Dror, O.; Nussinov, R.; Wolfson, H. J. "Analysis and classification of RNA tertiary structures" RNA 14(11):2274-2289 (2008).
91. Butcher, S. E.; Heckman, J. E.; Burke, J. M. "Reconstitution of hairpin ribozyme activity following separation of functional domains". Journal Biol Chem 270 (50): 29648-29651 (1995).
92. Doherty, EA; Doudna JA "Ribozyme structures and mechanisms". Annu. Rev. Biophys. Biomol. Struct. 30: 457-475 (2001).
93. Rinker, S.; Ke, Y.; Liu, Y.; Chhabra, R.; Yan, H. "Self-assembled DNA nanostructures for distance dependent multivalent ligand-protein binding" Nature Nanotech 3:418-422 (2008).
94. Corbett, P. T.; Leclaire, J.; Vial, L.; West, K. R.; Wietor, J.-L.; Sanders, J. K. M.; Otto, S. "Dynamic combinatorial chemistry". Chem Rev 106 (9):3652-3711 (2006).
95. Wang Y.; Rando R.R. "Specific binding of aminoglycoside antibiotics to RNA" Chemistry & Biology 2:281-290 (1995).
96. Stojanovic, M.N.; de Prada, P.; Landry, D.W. "Fluorescent Sensors based on aptamer self-assembly" J Am Chem Soc 122: 9678-9679 (2000).
97. Stojanovic, M.N.; de Prada, P.; Landry, D.W. "Aptamer-based folding fluorescent sensor for cocaine", J Am Chem Soc 123: 4928-4929, (2001).
98. Stojanovic, M.N.; Landry, D.W. "Aptamer-based colorimetric sensor for cocaine" J Am Chem Soc 124: 9678-9679 (2002).
99. Stojanovic, M.N. Kolpashchikov, D.M. "Modular allosteric sensors" J Am Chem Soc 126: 9266-9270, (2004).
100. Pei, R.; Shen, A; Olah, M.; Stefanovic, D.; Worgall, T.; Stojanović, M.N. " High-resolution crossreactive array for alkaloids" Chem Commun. (Camb);(22):3193-5 (2009).
101. Green, E.G.; Olah, M.J.; Abramova, T.; Williams, L.; Stefanović, D.; Worgall, T.; Stojanović, M.N. "Rational Approach to Minimal Cross-reactive Arrays" J Am Chem Soc 128: 15278-15282 (2006).
102. Stoltenburg, R.; Nikolaus, N.; Strehlitz, B. "Capture-SELEX: Selection of DNA Aptamers for Aminoglycoside Antibiotics" J Anal Meth Chem 15697 (2012).
103. Rajendran M,; Ellington, A. D. "Selection of fluorescent aptamer beacons that light up in the presence of zinc" Anal Bioanal Chem 390:1067-75 (2008).
104. Nutiu, R.; Li, Y. "In vitro selection of structure-switching signaling aptamers" Angew Chem Int Ed 44: 1061-1065 (2005).
105. Hu, J.; Easley, C. J.; "A simple and rapid approach for measurement of dissociation constants of DNA aptamers against proteins and small molecules via automated microchip electrophoresis" Analyst 136:3461-3468 (2011).
106. Nutiu, R., and Li, Y. "Structure-switching signaling aptamers" J Am Chem Soc 125:4771-4778 (2003).
107. Nguyen, T. H.; Pei, R.; Landry, D.; Stojanovic, M.; Lin. Q "Label-free microfluidic characterization of temperature dependent biomolecular interactions," Biomicrofluid 5:34118-341187 (2011).
108. Nguyen, T. H.; Pei, R.; Landry, D.; Stojanovic, M.; Lin. Q "Microfluidic Aptameric Affinity Sensing of Vasopressin for Clinical Diagnostic and Therapeutic Applications," Sensors and Actuators B: Chemical 154: 59-66 (2011).
109. Nguyen, T. H.; Pei, R.; Landry, D.; Stojanovic, M.; Lin. Q. "An Aptameric Microfluidic System for Specific Purification, Enrichment and Mass Spectrometric Detection of Biomolecules," J of Microelectromechanical Syst 18(6): 1198-1207 (2009).
110. Hilton, J. P.; Olsen, T.; Kim, J.; Zhu, J.; Nguyen, T. H.; Barbu, M.; Pei, R.; Stojanovic, M.; Lin, Q. "Isolation of thermally sensitive protein-binding oligonucleotides on a microchip" Microfluidics and Nanofluidics, 19(4):795-804 (2015).
111. Linnet, K. "Necessary sample size for method comparison studies based on regression analysis" Clin Chem 45:882-894 (1999).
112. Magari, R. T. "Evaluating Agreement Between Two Analytical Methods in Clinical Chemistry" Clin Chem Lab Med 38: 1021-1025 (2000).
113. Linnet, K. "Performance of Deming regression analysis in case of misspecified analytic error ratio in method comparison studies" Clin Chem 44:1024-1031 (1998).
114. Dewitte, K.; Fierens, C.; Stockl, D.; Thienport, L. M. "Application of the Bland-Altman plot for interpretation of method-comparison studies: a critical investigation of its practice" Clin Chem 48:799-801 (2002).
115. Yang, L.; Flint Beal, M. "Determination of Neurotransmitter Levels in Models of Parkinson' s Disease by HPLC-ECD" in Giovanni Manfredi and Hibiki Kawamata (eds.), Neurodegeneration: Methods and Protocols, Methods in Molecular Biology,. vol. 793: 401-415, 2011.
116. Kennedy, B.; Ziegler, M. G. "A more sensitive and specific radioenzymatic assay for catecholamines" Life Sci 47(23):2143-53 (1990).
117. "http://www.abnova.com/products/products_detail.asp?catalog_id=KA1887" and other vendors, accessed on 3/2/2016.
118. Clark, J. J.; Sandberg, S. G.; Wanat, M. J.; Gan, J. O.; Home, E. A.; Hart, A. S.; Akers, C. A. et al. "Chronic microsensors for longitudinal subsecond dopamine detection in behaving animals" Nat. Methods 7(2):126-129 (2010).
119. Mannironi, C.; Di Nardo, A.; Fruscoloni, P.; Tocchini-Valentini, G. P. "In vitro selection of dopamine RNA ligands" Biochem 36(32): 9726-9734 (1997).
120. Simpson, N.; Maffei, A.; Freeby, M.; Burroughs, S.; Freyberg, Z.; Javitch, J.; Leibel, R. L.; Harris, P. E. "Dopamine-mediated autocrine inhibitory circuit regulating human insulin secretion in vitro" Mol Endocrinol (10):1757-72 (2012).
121. Hauser, N. C.; Martinez, R.; Jacob, A.; Rupp, S.; Hoheisel, J. D.; Matysiak, S. "Utilising the left helical conformation of L-DNA for analyzing different marker types on a single universal microarrays platform" Nucleic Acids Res 34(18): 501-5111 (2006).
122. Hampshire, A. J.; Rusling, D. A.; Broughton-Head, V. J.; Fox, K. R. "Footprinting: A method for determining the sequence selectivity, affinity, and kinetics of DNA_binding ligands" Methods 42(2): 128-140 (2007).
123. Regulski, E. E.; Breaker, R. R. "In-Line Probing Analysis of Riboswitches" Methods in Molecular Biology 419: 53-75, Totowa, NJ (2008).
124. Franksson, G.; Anggard, E. "The Plasma Protein Binding of Amphetamine, Catecholamines and Related Compounds" Acta Pharm Tox 28(3):209-214 (2009).
125. Appendix. Normal Hormone Reference Ranges. Greenspan's Basic & Clinical Endocrinology. Ninth Edition, the McGraw-Hill Companies (2011).
126. Elias, A. N.; Nosratola, D.; Vaziri, M. D.; Maksy, M. "Plasma Norepinephrine, Epinephrine, and Dopamine Levels in End-Stage Renal Disease" Arch Intern Med 145:101301015 (1985).
127. Plunker, J. J.; Reeves, J. D.; Ngo, L.; Bellows, W., Shafer, S. L.; Roache, G.; Howse, J.; Herskowitz, A.; Mangano, D. T. et al "Urine and Plasma Catecholamine and Cortisol Concentrations after Myocardial Revascularization" Anest 86:785-796 (1997).
128. Zuker, M. "Mfold web server for nucleic acid folding and hybridization prediction" Nucleic Acids Res 31 (13), 3406-15, (2003).
129. Zadeh, J.N., Steenberg, C. D.; Bois, J. S.; Wolfe, B. R.; Khan, A. R.; Pierce, M. B.; Dirks, R. M.; Pierce, N. A. "NUPACK: analysis and design of nucleic acid systems" J Comp Chem 32:170-173 (2011).
130. (http://www.enzolifesciences.com/ADI-900-017A/arg8-vasopressin-elisa-kit/) and other similar kits, accessed on 03/03/2015.
131. (https://www.buhlmannlabs.ch/products-solutions/special-products/vasopressin-adh/) and similar kits, accessed on 03/03/2015.
132. Zhang, D.; Rios, D. R.; Tam, V. H.; Chow, D. S. "Development and validation of a highly sensitive LC-MS/MS assay for the quantification of arginine vasopressin in human plasma and urine: Application in preterm neonates and child" J Pharm Biomed Anal 96:67-73 (2014).
133. Bassi, E.; Park, M.; Azavedo, L. C. P. "Therapeutic Strategies for High-Dose Vasopressor-Dependent Shock" volume 2013: ID 654708, 10 pages (2013).
134. Raff, H.; Findling, J. W. "Aldosterone control in critically ill patients: ACTH, metoclopramide, and atrial natriuretric peptide" Crit Care Med 18(9):915-920 (1997).
135. Dick, M.; Dasta, J. F.; Choban, P. S.; Sinha, R.; Flancbaum, L. "Serum Aldosterone Concentrations and Urine Output in Oliguric Intensive Care Unit Patients Receiving Low-Dose Dopamine" Ann Pharmacotherapy 28:837-840 (1994).
136. Lichtarowicz-Krynska, E. J.; Cole, T. J.; Camacho-Hubner, C.; Britto, J.; Levin, M.; Klein, N.; Aynsley-Green, A. "Circulating Aldosterone Levels are Unexpectedly Low in Children with Actute Meningococcal Disease" J Clin Endocrin Met 89(3):1410-1414 (2004).
137. Meurant, G. "The Adrenal Cortex" chapter in Molecular and Cell Endocrinology in Principles of Medical Biology, v. 10A, p. 241 (1997).
138. Folan, M. M.; Stone, R. A.; Pittenger, A. L.; Pittenger, A. L.; Stofferl, J. A.; Hess, M. M.; Kroboth, P. D. "Dehydroepiisoandreosterone, dehydroepiisoandrosterone-sulfate, and cortisol concentrations in intensive care unit patients" Crit Care Med 20(5):965-970 (2001).
139. Vd Berghe, G.; d Zegher, F.; Wouters, P.; Schetz, M.; Verwaest, C.; Ferdinande, P.; Lauwers, P. "Dehydroepiandrosterone sulphate in critical illness: effect of dopamine" Clin Endocrin 43:457-463 (1995).

## Claims

1. An assay for testing a sample for the presence and/or amount of an analyte of interest comprising contacting at least a portion of the sample with effective amounts of (1) a primary aptamer comprising a core sequence that binds to the analyte and (2) an anti-aptamer which is complementary to at least a portion of the primary aptamer, wherein the primary aptamer and/or anti-aptamer comprise a detectable moiety(ies) which detect whether the primary aptamer and anti-aptamer are bound to each other or unbound; and wherein a primary aptamer bound to the analyte does not bind to its anti-aptamer, and wherein the primary aptamer comprises a fluorescent label and the anti-aptamer comprises a quencher moiety; wherein the analyte, primary aptamer and anti-aptamer are selected from the group consisting of:
(a) the analyte is deoxycorticosterone 21-glucoside (DOG) and either
i. the primary aptamer comprises SEQ ID NO: 62 and the anti-aptamer comprises SEQ ID NO:69, or
ii. the primary aptamer comprises SEQ ID NO: 76 and the anti-aptamer comprises SEQ ID NO:77;
(b) the analyte is aldosterone (ALDS) and either
i. the primary aptamer comprises SEQ ID NO: 63 and the anti-aptamer comprises the complement of SEQ ID NO: 63, or
ii. the primary aptamer comprises SEQ ID NO: 79 and the anti-aptamer comprises SEQ ID NO:80;
(c) the analyte is cortisol (CS) and the primary aptamer comprises SEQ ID NO: 64 and the anti-aptamer comprises the complement of SEQ ID NO: 64;
(d) the analyte is testosterone (TES) and the primary aptamer comprises SEQ ID NO: 65 and the anti-aptamer comprises the complement of SEQ ID NO: 65;
(e) the analyte is Phe-CpRh and the primary aptamer comprises SEQ ID NO: 66 and the anti-aptamer comprises the complement of SEQ ID NO: 66; and
(f) the analyte is phenylalanine (Phe) and the primary aptamer comprises SEQ ID NO: 67 and the anti-aptamer comprises the complement of SEQ ID NO: 67.

2. A method of detecting or measuring the presence or amount of an analyte of interest in a sample, comprising (i) contacting at least a portion of a sample with effective amounts of a primary aptamer and an anti-aptamer that is complementary to at least a portion of the primary aptamer, said primary aptamer and/or anti-aptamer comprising a moiety which allows the amount of primary aptamer bound to analyte to be detected and/or measured, under conditions that would permit duplex formation between the primary aptamer and anti-aptamer if target analyte were not present; and (ii) detecting the amount of primary aptamer that is not bound to anti-aptamer, and wherein the primary aptamer comprises a fluorescent label and the anti-aptamer comprises a quencher moiety ; wherein the analyte, primary aptamer and anti-aptamer are selected from the group consisting of:
(a) the analyte is deoxycorticosterone 21-glucoside (DOG) and either
i. the primary aptamer comprises SEQ ID NO: 62 and the anti-aptamer comprises SEQ ID NO:69, or
ii. the primary aptamer comprises SEQ ID NO: 76 and the anti-aptamer comprises SEQ ID NO:77;
(b) the analyte is aldosterone (ALDS) and either
iii. the primary aptamer comprises SEQ ID NO: 63 and the anti-aptamer comprises the complement of SEQ ID NO: 63, or
iv. the primary aptamer comprises SEQ ID NO: 79 and the anti-aptamer comprises SEQ ID NO:80;
(c) the analyte is cortisol (CS) and the primary aptamer comprises SEQ ID NO: 64 and the anti-aptamer comprises the complement of SEQ ID NO: 64;
(d) the analyte is testosterone (TES) and the primary aptamer comprises SEQ ID NO: 65 and the anti-aptamer comprises the complement of SEQ ID NO: 65;
(e) the analyte is Phe-CpRh and the primary aptamer comprises SEQ ID NO: 66 and the anti-aptamer comprises the complement of SEQ ID NO: 66; and
(f) the analyte is phenylalanine (Phe) and the primary aptamer comprises SEQ ID NO: 67 and the anti-aptamer comprises the complement of SEQ ID NO: 67.

3. A method of detecting or measuring the presence or amount of an analyte of interest in a sample according to claim 2, comprising (i) contacting at least a portion of the sample with effective amounts of (a) a primary aptamer comprising a fluorescent label and (b) an anti-aptamer, comprising a moiety that quenches fluorescence of said fluorescent label if primary aptamer and anti-aptamer are bound together in a duplex, under conditions that would permit duplex formation between primary aptamer and anti-aptamer to occur if analyte were not present; and (ii) detecting the amount of fluorescence.

4. The method of claim 2 wherein the primary aptamer comprises a core sequence that binds to the analyte and a portion complementary to a sensor oligonucleotide, and the method further comprises contacting at least a portion of the sample with an effective amounts of a sensor oligonucleotide one or more of which is bound to a detectable moity(ies) which can detect whether the primary aptamer and sensor oligonucleotide are bound to each other or whether primary aptamer is bound to analyte.

5. The method of claim 2 wherein the primary aptamer comprises a core sequence that binds to the analyte and a portion that, when primary aptamer is bound to analyte, binds to a secondary "sandwich" aptamer, and the method further comprises contacting at least a portion of the sample with an effective amounts of a secondary "sandwich" aptamer; one or more of which is bound to a detectable moity(ies) which can detect whether the primary aptamer and secondary "sandwich" aptamer are bound to each other or unbound.

## Patentansprüche

1. Testverfahren zum Testen einer Probe auf das Vorliegen und/oder die Menge eines Analyten von Interesse, umfassend In-Kontakt-Bringen wenigstens eines Teils der Probe mit wirksamen Mengen (1) eines primären Aptamers, das eine Kernsequenz umfasst, die an den Analyten bindet, und (2) eines Antiaptamers, das zu wenigstens einem Teil des primären Aptamers komplementär ist, wobei das primäre Aptamer und/oder Antiaptamer eine nachweisbare Gruppierung bzw. nachweisbare Gruppierungen umfassen/umfasst, mit denen nachgewiesen wird, ob das primäre Aptamer u Antiaptamer aneinander gebunden oder ungebunden sind; und wobei ein an den Analyten gebundenes primäres Aptamer nicht an sein Antiaptamer bindet und wobei das primäre Aptamer eine Fluoreszenzmarkierung und das Antiaptamer eine Quencher-Gruppierung umfasst; wobei Analyt, primäres Aptamer und Antiaptamer aus der aus Folgendem bestehenden Gruppe ausgewählt sind:
(a) bei dem Analyten handelt es sich um Desoxycorticosteron-21-glucosid (DOG), und entweder
i. das primäre Aptamer umfasst SEQ ID NO: 62 und das Antiaptamer umfasst SEQ ID NO: 69 oder
ii. das primäre Aptamer umfasst SEQ ID NO: 76 und das Antiaptamer umfasst SEQ ID NO: 77;
(b) bei dem Analyten handelt es sich um Aldosteron (ALDS), und entweder
i. das primäre Aptamer umfasst SEQ ID NO: 63 und das Antiaptamer umfasst das Komplement von SEQ ID NO: 63 oder
ii. das primäre Aptamer umfasst SEQ ID NO: 79 und das Antiaptamer umfasst SEQ ID NO: 80;
(c) bei dem Analyten handelt es sich um Cortisol (CS), und das primäre Aptamer umfasst SEQ ID NO: 64 und das Antiaptamer umfasst das Komplement von SEQ ID NO: 64;
(d) bei dem Analyten handelt es sich um Testosteron (TES), und das primäre Aptamer umfasst SEQ ID NO: 65 und das Antiaptamer umfasst das Komplement von SEQ ID NO: 65;
(e) bei dem Analyten handelt es sich um Phe-CpRh, und das primäre Aptamer umfasst SEQ ID NO: 66 und das Antiaptamer umfasst das Komplement von SEQ ID NO: 66; und
(f) bei dem Analyten handelt es sich um Phenylalanin (Phe), und das primäre Aptamer umfasst SEQ ID NO: 67 und das Antiaptamer umfasst das Komplement von SEQ ID NO: 67.

2. Verfahren zum Nachweisen oder Messen des Vorliegens bzw. der Menge eines Analyten von Interesse in einer Probe, umfassend (i) In-Kontakt-Bringen wenigstens eines Teils einer Probe mit wirksamen Mengen eines primären Aptamers und eines Antiaptamers, das zu wenigstens einem Teil des primären Aptamers komplementär ist, wobei das primäre Aptamer und/oder Antiaptamer eine Gruppierung umfassen/umfasst, die das Nachweisen und/oder Messen der Menge von an Analyt gebundenem primärem Aptamer unter Bedingungen erlaubt, die eine Duplexbildung zwischen dem primären Aptamer und Antiaptamer gestatten würden, falls kein Zielanalyt vorläge; und (ii) Nachweisen der Menge von primärem Aptamer, das nicht an Antiaptamer gebunden ist, und wobei das primäre Aptamer eine Fluoreszenzmarkierung und das Antiaptamer eine Quencher-Gruppierung umfasst; wobei Analyt, primäres Aptamer und Antiaptamer aus der aus Folgendem bestehenden Gruppe ausgewählt sind:
(a) bei dem Analyten handelt es sich um Desoxycorticosteron-21-glucosid (DOG), und entweder
i. das primäre Aptamer umfasst SEQ ID NO: 62 und das Antiaptamer umfasst SEQ ID NO: 69 oder
ii. das primäre Aptamer umfasst SEQ ID NO: 76 und das Antiaptamer umfasst SEQ ID NO: 77;
(b) bei dem Analyten handelt es sich um Aldosteron (ALDS), und entweder
iii. das primäre Aptamer umfasst SEQ ID NO: 63 und das Antiaptamer umfasst das Komplement von SEQ ID NO: 63 oder
iv. das primäre Aptamer umfasst SEQ ID NO: 79 und das Antiaptamer umfasst SEQ ID NO: 80;
(c) bei dem Analyten handelt es sich um Cortisol (CS), und das primäre Aptamer umfasst SEQ ID NO: 64 und das Antiaptamer umfasst das Komplement von SEQ ID NO: 64;
(d) bei dem Analyten handelt es sich um Testosteron (TES), und das primäre Aptamer umfasst SEQ ID NO: 65 und das Antiaptamer umfasst das Komplement von SEQ ID NO: 65;
(e) bei dem Analyten handelt es sich um Phe-CpRh, und das primäre Aptamer umfasst SEQ ID NO: 66 und das Antiaptamer umfasst das Komplement von SEQ ID NO: 66; und
(f) bei dem Analyten handelt es sich um Phenylalanin (Phe), und das primäre Aptamer umfasst SEQ ID NO: 67 und das Antiaptamer umfasst das Komplement von SEQ ID NO: 67.

3. Verfahren zum Nachweisen oder Messen des Vorliegens bzw. der Menge eines Analyten von Interesse in einer Probe gemäß Anspruch 2, umfassend (i) In-Kontakt-Bringen wenigstens eines Teils der Probe mit wirksamen Mengen (a) eines primären Aptamers, das eine Fluoreszenzmarkierung umfasst, und (b) eines Antiaptamers, das eine Gruppierung umfasst, die Quenchen von Fluoreszenz der Fluoreszenzmarkierung bewirkt, falls primäres Aptamer und Anti-aptamer zusammen in einem Duplex gebunden sind, unter Bedingungen, die das Auftreten einer Duplexbildung zwischen primärem Aptamer und Antiaptamer gestatten würden, falls kein Analyt vorläge; und (ii) Nachweisen der Menge an Fluoreszenz.

4. Verfahren nach Anspruch 2, wobei das primäre Aptamer eine Kernsequenz, die an den Analyten bindet, und einen Teil, der zu einem Sensor-Oligonukleotid komplementär ist, umfasst und das Verfahren ferner In-Kontakt-Bringen wenigstens eines Teils der Probe mit einer wirksamen Menge eines Sensor-Oligonukleotids umfasst, von dem ein oder mehrere an eine nachweisbare Gruppierung bzw. nachweisbare Gruppierungen gebunden ist/sind, mit der/denen sich nachweisen lässt, ob das primäre Aptamer und Sensor-Oligonukleotid aneinander gebunden sind oder ob primäres Aptamer an Analyt gebunden ist.

5. Verfahren nach Anspruch 2, wobei das primäre Aptamer eine Kernsequenz, die an den Analyten bindet, und einen Teil, der, wenn primäres Aptamer an Analyt gebunden ist, an ein sekundäres "Sandwich"-Aptamer bindet, umfasst und das Verfahren ferner In-Kontakt-Bringen wenigstens eines Teils der Probe mit einer wirksamen Menge eines sekundären "Sandwich"-Aptamers umfasst, von dem ein oder mehrere an eine nachweisbare Gruppierung bzw. nachweisbare Gruppierungen gebunden ist/sind, mit der/denen sich nachweisen lässt, ob das primäre Aptamer und sekundäres "Sandwich"-Aptamer aneinander gebunden oder ungebunden vorliegen.

## Revendications

1. Dosage pour le test d'un échantillon en ce qui concerne la présence et/ou la quantité d'un analyte d'intérêt comprenant la mise en contact d'au moins une partie de l'échantillon avec des quantités efficaces de (1) un aptamère primaire comprenant une séquence centrale qui se lie à l'analyte et (2) un anti-aptamère qui est complémentaire à au moins une partie de l'aptamère primaire, l'aptamère primaire et/ou l'anti-aptamère comprenant un ou plusieurs groupements détectables qui détectent si l'aptamère primaire et l'anti-aptamère sont liés l'un à l'autre ou non liés ; et un aptamère primaire lié à l'analyte ne se liant pas à son anti-aptamère, et l'aptamère primaire comprenant un marqueur fluorescent et l'anti-aptamère comprenant un groupement d'extinction ; l'analyte, l'aptamère primaire et l'anti-aptamère étant choisis dans le groupe constitué par :
(a) l'analyte étant le désoxycorticostérone 21-glucoside (DOG) et soit
i. l'aptamère primaire comprenant la SEQ ID NO: 62 et l'anti-aptamère comprenant la SEQ ID NO: 69, soit
ii. l'aptamère primaire comprenant la SEQ ID NO: 76 et l'anti-aptamère comprenant la SEQ ID NO: 77 ;
(b) l'analyte étant l'aldostérone (ALDS) et soit
i. l'aptamère primaire comprenant la SEQ ID NO: 63 et l'anti-aptamère comprenant le complément de la SEQ ID NO: 63, soit
ii. l'aptamère primaire comprenant la SEQ ID NO: 79 et l'anti-aptamère comprenant la SEQ ID NO: 80 ;
(c) l'analyte étant le cortisol (CS) et l'aptamère primaire comprenant la SEQ ID NO: 64 et l'anti-aptamère comprenant le complément de la SEQ ID NO: 64 ;
(d) l'analyte étant la testostérone (TES) et l'aptamère primaire comprenant la SEQ ID NO: 65 et l'anti-aptamère comprenant le complément de la SEQ ID NO: 65 ;
(e) l'analyte étant Phe-CpRh et l'aptamère primaire comprenant la SEQ ID NO: 66 et l'anti-aptamère comprenant le complément de la SEQ ID NO: 66 ; et
(f) l'analyte étant la phénylalanine (Phe) et l'aptamère primaire comprenant la SEQ ID NO: 67 et l'anti-aptamère comprenant le complément de la SEQ ID NO: 67.

2. Procédé de détection ou de mesure de la présence ou de la quantité d'un analyte d'intérêt dans un échantillon, comprenant (i) la mise en contact d'au moins une partie d'un échantillon avec des quantités efficaces d'un aptamère primaire et d'un anti-aptamère qui est complémentaire à au moins une partie de l'aptamère primaire, ledit aptamère primaire et/ou anti-aptamère comprenant un groupement qui permet à la quantité d'aptamère primaire lié à l'analyte d'être détectée et/ou mesurée, dans des conditions qui permettraient la formation d'un duplex entre l'aptamère primaire et l'anti-aptamère si l'analyte cible n'était pas présent ; et (ii) la détection de la quantité d'aptamère primaire qui n'est pas lié à l'anti-aptamère, et l'aptamère primaire comprenant un marqueur fluorescent et l'anti-aptamère comprenant un groupement d'extinction ; l'analyte, l'aptamère primaire et l'anti-aptamère étant choisis dans le groupe constitué par :
(a) l'analyte étant le désoxycorticostérone 21-glucoside (DOG) et soit
i. l'aptamère primaire comprenant la SEQ ID NO: 62 et l'anti-aptamère comprenant la SEQ ID NO: 69, soit
ii. l'aptamère primaire comprenant la SEQ ID NO: 76 et l'anti-aptamère comprenant la SEQ ID NO: 77 ;
(b) l'analyte étant l'aldostérone (ALDS) et soit
iii. l'aptamère primaire comprenant la SEQ ID NO: 63 et l'anti-aptamère comprenant le complément de la SEQ ID NO: 63, soit
iv. l'aptamère primaire comprenant la SEQ ID NO: 79 et l'anti-aptamère comprenant la SEQ ID NO: 80 ;
(c) l'analyte étant le cortisol (CS) et l'aptamère primaire comprenant la SEQ ID NO: 64 et l'anti-aptamère comprenant le complément de la SEQ ID NO: 64 ;
(d) l'analyte étant la testostérone (TES) et l'aptamère primaire comprenant la SEQ ID NO: 65 et l'anti-aptamère comprenant le complément de la SEQ ID NO: 65 ;
(e) l'analyte étant Phe-CpRh et l'aptamère primaire comprenant la SEQ ID NO: 66 et l'anti-aptamère comprenant le complément de la SEQ ID NO: 66 ; et
(f) l'analyte étant la phénylalanine (Phe) et l'aptamère primaire comprenant la SEQ ID NO: 67 et l'anti-aptamère comprenant le complément de la SEQ ID NO: 67.

3. Procédé de détection ou de mesure de la présence ou de la quantité d'un analyte d'intérêt dans un échantillon selon la revendication 2, comprenant (i) la mise en contact d'au moins une partie de l'échantillon avec des quantités efficaces de (a) un aptamère primaire comprenant un marqueur fluorescent et (b) un anti-aptamère, comprenant un groupement qui éteint la fluorescence dudit marqueur fluorescent si l'aptamère primaire et l'anti-aptamère sont liés ensemble dans un duplex, dans des conditions qui permettraient la formation d'un duplex entre l'aptamère primaire et l'anti-aptamère si l'analyte cible n'était pas présent ; et (ii) la détection de la quantité de fluorescence.

4. Procédé selon la revendication 2, l'aptamère primaire comprenant une séquence centrale qui se lie à l'analyte et une partie complémentaire à un oligonucléotide capteur, et le procédé comprenant en outre la mise en contact d'au moins une partie de l'échantillon avec des quantités efficaces d'un oligonucléotide capteur, l'un ou plusieurs desquels étant liés à un ou plusieurs groupements détectables qui peuvent détecter si l'aptamère primaire et l'oligonucléotide capteur sont liés l'un à l'autre ou si l'aptamère primaire est lié à l'analyte.

5. Procédé selon la revendication 2, l'aptamère primaire comprenant une séquence centrale qui se lie à l'analyte et une partie qui, lorsque l'aptamère primaire est lié à l'analyte, se lie à un aptamère « sandwich » secondaire, et le procédé comprenant en outre la mise en contact d'au moins une partie de l'échantillon avec des quantités efficaces d'un aptamère « sandwich » secondaire ; l'un ou plusieurs desquels étant liés à un ou plusieurs groupements détectables qui peuvent détecter si l'aptamère primaire et l'aptamère « sandwich » secondaire sont liés l'un à l'autre ou non liés.
